(19) Europäisches Patentamt
European Patent Office
Office européen des brevets

(11) EP 4 567 112 A1

(12) **EUROPEAN PATENT APPLICATION**
published in accordance with Art. 153(4) EPC

(43) Date of publication:
**11.06.2025 Bulletin 2025/24**

(21) Application number: **23849513.9**

(22) Date of filing: **04.08.2023**

(51) International Patent Classification (IPC):
***C12N 15/113*** *(2010.01)* ***A61K 31/713*** *(2006.01)*
***A61P 1/16*** *(2006.01)* ***A61P 29/00*** *(2006.01)*
***A61P 3/06*** *(2006.01)* ***A61P 9/10*** *(2006.01)*
***A61P 7/02*** *(2006.01)* ***A61P 9/00*** *(2006.01)*

(52) Cooperative Patent Classification (CPC):
**A61K 31/713; A61P 1/16; A61P 3/06; A61P 7/02; A61P 9/00; A61P 9/10; A61P 29/00; C12N 15/113**

(86) International application number:
**PCT/CN2023/111121**

(87) International publication number:
**WO 2024/027809 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.08.2022 CN 202210936491**
**17.03.2023 CN 202310260531**
**05.06.2023 CN 202310655563**

(71) Applicant: **Kylonova (Xiamen) Biopharma Co., Ltd.**
**Xiamen, Fujian 361022 (CN)**

(72) Inventors:
- **CUI, Kunyuan**
**Xiamen, Fujian 361022 (CN)**
- **LU, Xueqin**
**Xiamen, Fujian 361022 (CN)**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

# (54) RNA INHIBITOR FOR INHIBITING APOC3 GENE EXPRESSION AND USE THEREOF

(57) The present invention pertains to the field of biopharmaceutics, and particularly relates to an RNA inhibitor for inhibiting APOC3 gene expression or a pharmaceutically acceptable salt thereof. The RNA inhibitor comprises a sense strand and an antisense strand. The sense strand and the antisense strand are each independently 15-30 nucleotides, preferably 19-23 nucleotides in length. At least 80% of the bases are complementary between the sense strand and the antisense strand. The RNA inhibitor further comprises the carrier structures 5'MVIP and 3'MVIP. The present application further relates to a pharmaceutical composition comprising the RNA inhibitor for inhibiting APOC3 gene expression and use thereof in the treatment and prevention of cardiovascular and cerebrovascular diseases.

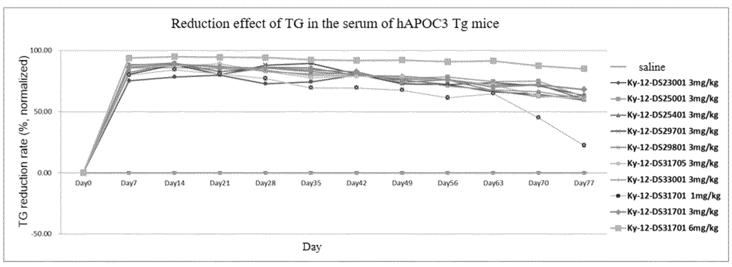


**Figure 9**



Processed by Luminess, 75001 PARIS (FR)

## Description

### Cross-reference of the relevant application

The present invention claims the priority of the Chinese patent applications 202210936491.5, 202310260531.3 and 202310655563.3 with the names of "An RNAinhibitor for inhibiting APOC3 gene expression and application thereof", "APOC3 inhibitors and application thereof" and "An RNA inhibitor for inhibiting APOC3 gene expression and application thereof" filed on August 5, 2022, March 17, 2023 and June 5, 2023, the entire content of which are incorporated in the present invention by reference.

### Technical field

The present invention relates to the biomedical field, specifically involving an RNA inhibitor that suppresses APOC3 gene expression and its application.

### Background Art

### RNA inhibitor

RNA inhibitor (RNA interference) was discovered by Andrew Z. Fire et al. during antisense RNA inhibition experiments in Caenorhabditis elegans in 1998, and this process was called RNA inhibitor. This discovery was rated as one of the top ten scientific advances in 2001 by Science magazine, and ranked first among the top ten scientific advances in 2002. Since then, siRNA with RNAi as its mechanism of action has received widespread attention as a potential gene therapy drug. In 2006, Andrew Z. Fire and Craig C. Mello won the Nobel Prize in physiology or medicine for their contributions in the study of RNAi mechanism. RNAi is triggered by double stranded RNA (dsRNA) in many organisms, including animals, plants and fungi. In the process of RNAi, an endonuclease called "Dicer" cleaves or "dices" long strand of dsRNA into small segments of 21-25 nucleotides long. These small segments are known as small interfering RNAs (siRNAs), whose antisense strands are loaded onto Argonaute protein (AGO2). AGO2 loading occurs in the RISC-loading complex, a ternary complex consisting of Argonaute protein, Dicer, and dsRNA binding protein (TRBP for short). During loading, the sense strand is cleaved by AGO2 and discharged. Then, AGO2 uses the antisense strand to bind to mRNA containing fully complementary sequences, and then catalyzes the cleavage of these mRNA, resulting in mRNA cleavage to loss the role as a translation template, thereby preventing the synthesis of related proteins. After cleavage, the cleaved mRNA is released, and the RISC-loading complex loaded with the antisense strand is recycled for another round of cleavage.

According to statistics, among the disease-related proteins in human body, about more than 80% of them cannot be targeted by current conventional small molecule drugs and biological macromolecule preparations, and are considered as undruggable proteins. Gene therapy, which aims to treat diseases through gene expression, silencing and other functions, is considered by the industry to be the third generation of therapeutic drugs after chemical small molecule drugs and biological macromolecular drugs. This therapy treats diseases at the genetic level and is not restricted by undruggable proteins. As the most mainstream type of gene therapy, RNAi technology treats diseases at the mRNA level and has higher efficiency as compared with chemical small molecule drugs and biological macromolecular drugs at the protein level. RNAi technology can be used to design the sense strand and antisense strand sequences of siRNAs with high specificity and good inhibitory effect according to the specific gene sequence. These single strand sequences are synthesized through solid-phase synthesis, and then the sense strand and antisense strand are paired into siRNA in a specific annealing buffer according to the principle of base pairing. Finally, it is delivered to the corresponding target site in the body through the vehicle system, degrading the target mRNA, and destroying the function of the target mRNA as a translation template, thus preventing the synthesis of related proteins.

### Delivery system of siRNA

siRNA is unstable in blood and tissues and easily degraded by nucleases. In order to improve the stability of siRNA, the sense strand and/or antisense strand of siRNA can be modified, but these chemical modifications only provide limited protection from nuclease degradation and may ultimately affect the activity of siRNA. Therefore, a corresponding delivery system is needed to ensure that siRNA can safely and efficiently pass through cell membrane. Due to its large molecular weight, large amount of negative charge, and high water solubility, siRNA itself cannot smoothly pass through cell membrane to enter the cell.

Liposome is basically composed of a hydrophilic core and a phospholipid bilayer. It has a phospholipid bilayer similar to a biological membrane and has high biocompatibility, therefore liposome once became the most popular and

widely used siRNA vehicle. Liposome-mediated siRNA delivery mainly encapsulates siRNA into liposomes to protect siRNA from degradation by nucleases, improves the efficiency of siRNA passing through cell membrane barriers, and thereby promotes cellular absorption. Examples of liposomes include, for example, anionic liposomes, pH sensitive liposomes, immunoliposomes, fusogenic liposomes, and cationic lipids etc. Despite some progress, liposomes themselves are prone to triggering inflammatory reactions, a variety of antihistamines and hormones such as cetirizine and dexamethasone must be used before administration to reduce possible acute inflammatory reactions. Therefore, they are not suitable for all treatment fields in actual clinical application, especially some chronic disease treatment fields. Possible accumulated toxicity resulting from long-term use is a potential safety hazard. Therefore, a safer and more effective vehicle system is needed to deliver siRNA.

Asialoglycoprotein receptor (ASGPR) in the liver is a receptor specifically expressed on hepatocytes and is a highly efficient endocytic receptor. Due to the fact that under physiological conditions in the body, after enzymatic or acid hydrolysis of sialic acids, the exposed secondary ends of various glycoproteins are galactose residues, the sugar specifically bound to ASGPR is galactosyl, so ASGPR is also called galactose-specific receptor. Monosaccharide and polysaccharide molecules such as galactose, galactosamine, and N-acetylgalactosamine all have high affinity for ASGPR. ASGPR mainly has the physiological function of mediating the clearance of asialoglycoprotein, lipoprotein and other substances in the blood, and it is closely related to the occurrence and development of liver diseases such as viral hepatitis, liver cirrhosis, liver cancer and so on. The discovery of this characteristic of ASGPR plays an important role in the diagnosis and treatment of hepatogenic diseases (Ashwell G and Harford J, Carbohydrate specific Receptors of the Liver, Ann Rev Biochem 1982 51:531-554). The therapeutic drug for hepatogenic diseases containing galactose or galactosamine and their derivatives in the structure can have specific affinity with ASGPR, so it actively targets liver and does not need other vehicle systems to deliver.

## APOC3 and cardiovascular and cerebrovascular diseases

APOC3(apolipoprotein C-III) is the gene encoding apolipoprotein C3, with a full length of about 3.1 kb. It is an 8.8 kD glycoprotein composed of 79 amino acid residues and mainly synthesized in the liver. APOC3 has the function of inhibiting the activities of lipoprotein lipase and hepatic lipase, it interferes with the ApoE-mediated binding of triglyceride (TG)-rich lipoproteins to hepatic lipase, affects the balance of lipid metabolism, and easily leads to hypertriglyceridemia. A large number of related studies have shown that the polymorphism at the Sst I restriction site of the APOC3 gene is associated with hypertriglyceridemia, and therefore may also be associated with other cardiovascular diseases caused by hypertriglyceridemia.

APOC3 plays a key role in the metabolism of triglyceride-rich lipoproteins. It is mainly combined with high-density lipoprotein cholesterol (HDL-c) (about 70%) in normal fasting plasma, while the plasma APOC3 levels in patients with hypertriglyceridemia is significantly increased, which are primarily (about 86%) bound to very low-density lipoprotein (VLDL).

Domestic and foreign studies have shown that elevated plasma APOC3 level can cause hypertriglyceridemia, and plasma APOC3 level is positively correlated with plasma triglyceride and triglyceride level in very low-density lipoprotein. Currently, a large number of literature have reported that APOC3 gene functions are related to cardiovascular and cerebrovascular diseases such as coronary atherosclerosis, type 2 diabetes, and cerebral infarction.

Familial hyperchylomicronemia (FCS), also known as lipoprotein lipase deficiency, is a rare autosomal recessive disorder, also known as primary hyperlipoproteinemia type I, which requires long-term restriction of the fat amount in the diet. Before November 2012, there was no specific drug to treat FCS. In addition to elevated triglycerides, the clinical features of FCS also include recurrent pancreatitis. In November 2012, the European Commission (EC) approved Glybera, the first gene therapy drug in the Western world, but Glybera is very expensive. The cost per patient is expected to be up to 1.25 million Euros (approximately $1.6 million). There is an urgent need for a specific drug to treat FCS. APOC3 is an important regulator of lipid metabolism. Loss-of-function mutations in APOC3 will inhibit hepatocytes from clearing triglyceride-rich lipoprotein particles, leading to increased TG and VLDL levels and decreased HDL-c level in the blood. By silencing the expression of APOC3, harmful lipoprotein indicators (i.e. TG and VLDL levels) are reduced and the level of "good" cholesterol HDL-c is increased. APOC3 small interfering inhibitors are being developed for the treatment of hypertriglyceridemia (HTG), severe hypertriglyceridemia (sHTG), and familial chylomicronemia syndrome (FCS). This gives new hope for the treatment of chylomicronemia.

Existing technology can already use RNA interference technology to inhibit APOC3 gene expression, but the inhibition efficiency and persistence still need to be improved. The market needs an RNA inhibitor with higher inhibition efficiency and longer lasting effect.

## Invention content

In order to solve the above problems, the present invention provides an RNA inhibitor for inhibiting the expression

of APOC3 gene or a pharmaceutically acceptable salt thereof, which specifically interferes with the mRNA of APOC3 gene, destroys its function as a translation template, and prevents the expression of APOC3 protein, thereby preventing and/or treating APOC3 gene-mediated related diseases. The RNA inhibitor of the present invention has excellent APOC3 protein expression inhibition effect, long-lasting action time, and therefore has high medical application value.

In one aspect, the present invention provides an RNA inhibitor for inhibiting the expression of APOC3 gene or a pharmaceutically acceptable salt thereof, wherein the RNA inhibitor is formed by base pairing of a sense strand and an antisense strand with chain length of 15-30 nucleotides each independently, the chain length is preferably 19-23 nucleotides each independently, and at least 80% of the bases between the sense strand and the antisense strand are complementary.

In some embodiments, the antisense strand is preferably selected from a) the following sequences, b) sequences having at least 15 consecutive nucleotides identical to those in a), and c) sequences having no more than 3 nucleotides different from those in a) and b): 5'uugguggcgugcuucauguaatt 3' (SEQ ID NO. 212), 5'uaacccugcaugaagcugagatt 3'(SEQ ID NO. 216), 5'uuaacggugcuccaguagucutt 3' (SEQ ID NO. 224) , 5'cagagaacuuguccuuaacggtt 3'(SEQ ID NO. 225), 5' uauugaggucucaggcagccatt 3' (SEQ ID NO. 241) , 5'ugaaguuggucugaccucaggtt 3' (SEQ ID NO. 255), 5' gcacugagaauacugucccuuuu 3' (SEQ ID NO. 256), 5'gcacugagaauacugucccuu3'(SEQ ID NO. 459), 5' acacugagaauacugucccua 3' (SEQ ID NO.461), 5' ugaauacugucccuuuuaagc 3' (SEQ ID NO.465) , 5' cugagaauacugucccuuuua 3' (SEQ ID NO.471), 5' acugagaauacugucccuuua 3' (SEQ ID NO.472) , 5' uaauacugucccuuuuaagcaa 3' (SEQ ID NO.438), 5'ugaggucucaggcagccacgg3' (SEQ ID NO.600), 5'uggauaggcagguggacuugg3' (SEQ ID NO. 620), 5'caggauggauaggcaggugga3' (SEQ ID NO.624), 5'gagcacugagaauacugucccc3'(SEQ ID NO. 668), 5'acacugagaauacugucgcuc3'(SEQ ID NO. 687), 5'acacugagaauacugucgcuu3'(SEQ ID NO. 700) , 5' ucacugagaauacugucccuu 3'(SEQ ID NO. 519);
where, g= guanylate, a= adenylate, u= uridylate, c= cytidylate, t= thymidylate

It should be noted that, at least 15 consecutive nucleotides each independently represent: 15, 16, 17, 18, 19, 20, 21 ... etc. consecutive nucleotides, and the difference of no more than 3 nucleotides each independently indicates: 1, 2 or 3 different nucleotides. The sense strands and antisense strands exemplified below are not limited to the combinations shown in the examples of the present invention to form RNA inhibitors. As long as they can be complementary paired to form double strands, the sense strand and the antisense strand can be combined at will and are not limited by the examples.

In some embodiments, more preferably, the sense strand and the antisense strand are selected from: a) the following sequences, b) sequences having at least 15 consecutive nucleotides identical to those in a), and c) sequences having no more than 3 nucleotides different from those in a) and b). The sequence combination of the sense strand and the antisense strand is as follows:

| SEQ ID NO. | single strand code | sense strand 5'→3' | SEQ ID NO. | single strand code | antisense strand 5'→3' |
|---|---|---|---|---|---|
| 161 | S58 | uuacaugaagcacgccaccaatt | 212 | AS58 | uugguggcgugcuucauguaatt |
| 165 | S62 | ucucagcuucaugcaggguuat t | 216 | AS62 | uaacccugcaugaagcugagatt |
| 173 | S70 | agacuacuggagcaccguuaat t | 224 | AS70 | uuaacggugcuccaguagucutt |
| 174 | S71 | ccguuaaggacaaguucucugt t | 225 | AS71 | cagagaacuuguccuuaacggtt |
| 190 | S87 | uggcugccugagaccucaauat t | 241 | AS87 | uauugaggucucaggcagccatt |
| 204 | S101 | ccugaggucagaccaacuucat t | 255 | AS10 1 | ugaaguuggucugaccucaggtt |
| 205 | S102 | aagggacaguauucucagugct t | 256 | AS10 2 | gcacugagaauacugucccuuuu |
| 440 | S268 | gggacaguauucucagugcua | 459 | AS27 0 | gcacugagaauacugucccuu |
| 442 | S270 | uagggacaguauuctcagugu | 461 | AS27 2 | acacugagaauacugucccua |

(continued)

| SEQ ID NO. | single strand code | sense strand 5'→3' | SEQ ID NO. | single strand code | antisense strand 5'→3' |
|---|---|---|---|---|---|
| 446 | S274 | gcuuaaaagggacaguauuca | 465 | AS27 6 | ugaauacugucccuuuuaagc |
| 452 | S280 | aaagggacaguauucucagug | 471 | AS28 2 | cugagaauacugucccuuuua |
| 453 | S281 | aagggacaguauucucagugc | 472 | AS28 3 | acugagaauacugucccuuua |
| 702 | S194 | gcuuaaaagggacaguauuca | 438 | AS19 4 | uaauacugucccuuuuaagcaa |
| 864 | S356 | guggcugccugagaccucaau | 600 | AS35 6 | ugaggucucaggcagccacgg |
| 884 | S376 | aaguccaccugccuauccauc | 620 | AS37 6 | uggauaggcagguggacuugg |
| 888 | S380 | caccugccuauccauccugaa | 624 | AS38 0 | caggauggauaggcaggugga |
| 931 | S423 | gggacaguauucucagugcuu | 667 | AS42 3 | gcacugagaauacugucccuu |
| 932 | S424 | gacaguauucucagugcucuu | 668 | AS42 4 | gagcacugagaauacuguccc |
| 951 | S443 | gagcgacaguauucucagugu | 687 | AS44 3 | acacugagaauacugucgcuc |
| 964 | S456 | aagcgacaguauucucagugu | 700 | AS45 6 | acacugagaauacugucgcuu |
| 781 | S273 | aagggacaguauucucaguga | 519 | AS27 5 | ucacugagaauacugucccuu |

where, g=guanylate, a=adenylate, u=uridylate, c=cytidylate, t=thymidylate。

In the RNA inhibitor of the present invention, one or more nucleotides on the sense strand and/or the antisense strand can be modified to form modified nucleotides.

Preferably, in the RNA inhibitor of the present invention, the sense strand and/or the antisense strand contains at least one 2'-modified nucleotide, and the 2'-modified nucleotide includes: 2'-O-methyl nucleotide, 2'-deoxy-2'-fluoronucleotide, 2'-deoxynucleotide, 2'-methoxyethyl nucleotide, 2'-amino nucleotide or 2'-alkyl nucleotide.

More preferably, in the RNAinhibitor of the present invention, the sense strand and/or the antisense strand contains at least one 2'-O-methyl nucleotide or 2'-deoxy-2'-fluoronucleotide.

Preferably, in the RNA inhibitor of the present invention, the phosphate bonds among three adjacent nucleotides at at least one of the ends of the sense strand and/or antisense strand can be thiolated.

In the above technical solutions, preferably, the sense strand and the antisense strand are selected from: a) the following sequences, b) sequences having at least 15 consecutive nucleotides identical to those in a), and c) sequences having no more than 3 nucleotides different from those in a) and b).

| SE Q ID NO. | singl e stran d code | sense strand 5'→3' | SE Q ID NO. | single strand code | antisense strand 5'→3' |
|---|---|---|---|---|---|
| 411 | S16 7 | UsUsACAUGAfAfGfCACGCCACCsAsA | 381 | AS16 7 | UsfUsGfGUfGGfCGUGCUfUCfAUGUsAsA |
| 412 | S16 8 | UsCsUCAGCUfUfCfAUGCAGGGUsUsA | 382 | AS16 8 | UsfAsAfCCfCUfGCAUGAfAGfCUGAsGsA |
| 417 | S17 3 | AsGsACUACUfGfGfAGCACCGUUsAsA | 387 | AS17 3 | UsfUsAfACfGGfUGCUCCfAGfUAGUsCsU |
| 418 | S17 4 | CsCsGUUAAGfGfAfCAAGUUCUCsUsG | 388 | AS17 4 | CsfAsGfAGfAAfCUUGUCfCUfUAACsGsG |
| 428 | S18 4 | UsGsGCUGCCfUfGfAGACCUCAAsUsA | 398 | AS18 4 | UsfAsUfUGfAGfGUCUCAfGGfCAGCsCsA |
| 435 | S19 1 | CsCsUGAGGUfCfAfGACCAACUUsCsA | 405 | AS19 1 | UsfGsAfAGfUUfGGUCUGfACfCUCAsGsG |
| 436 | S19 2 | AsAsGGGACAfGfUfAUUCUCAGUsGsC | 406 | AS19 2 | GsfCsAfCUfGAfGAAUACfUGfUCCCUUsUsU |
| 478 | S28 7 | GsGsGAfCAfGfUfAUUCUCAGUGCsUsA | 507 | AS28 9 | GsCsACUGfAGAAUfACfUGUCCCsUsU |
| 481 | S29 0 | UsAsGGfGAfCfAfGUAUUCTCAGUsGsU | 510 | AS29 2 | AsCsACUGfAGAAUfACfUGUCCCsUsA |
| 486 | S29 5 | GsCsUUAAAAfGfGfGACAGUAUUsCsA | 515 | AS29 7 | UsfGsAfAUfACfUGfUCfCCfUUfUUfAAsGsC |
| 497 | S30 6 | AsAsAGfGGfAfCfAGUAUUCUCAGsUsG | 526 | AS30 8 | CsfUsGfAGfAAfUACUGUfCCfCUUUsUsA |

(continued)

| SE Q ID NO. | singl e stran d code | sense strand 5'→3' | SE Q ID NO. | single strand code | antisense strand 5'→3' |
|---|---|---|---|---|---|
| 499 | S30 8 | AsAsGGfGAfCfAfGUAUUCU CAGUsGsC | 527 | AS30 9 | AsCsUGAGfAAUACfUGfUCCCU UsUsA |
| 486 | S29 5 | GsCsUUAAAAfGfGfGACAG UAUUsCsA | 532 | AS31 4 | UsAsfAUfACfUGfUCfCCfUUfUU fAAGCsAsA |
| 102 8 | S45 7 | GsUsGGCUGCfCfUfGAGACC UCAsAsU | 965 | AS45 7 | UsfGsAfGGfUCfUCAGGCfAGfC CfACsfGsG |
| 102 9 | S45 8 | AsAsGUCCACfCfUfGCCUAU CCAsUsC | 966 | AS45 8 | UsfGsGfAUfAGfGCAGGUfGGfA CfUUsfGsG |
| 103 0 | S45 9 | CsAsCCUGCCfUfAfUCCAUC CUGsAsA | 967 | AS45 9 | CsfAsGfGAfUGfGAUAGGfCAfG GfUGsfGsA |
| 103 1 | S46 0 | GsGsGACAGUfAfUfUCUCA GUGCsUsU | 968 | AS46 0 | GsfCsAfCUfGAfGAAUACfUGfU CfCCsfUsU |
| 103 2 | S46 1 | GsAsCAGUAUfUfCfUCAGU GCUCsUsU | 969 | AS46 1 | GsfAsGfCAfCUfGAGAAUfACfU GfUCsfCsC |
| 103 3 | S46 2 | GsAsGCGACAfGfUfAUUCU CAGUsGsU | 970 | AS46 2 | AsfCsAfCUfGAfGAAUACfUGfU CfGCsfUsC |
| 103 9 | S46 8 | GsAsGCGAfCAfGfUfAUUCU CAGUsGsU | 976 | AS46 8 | AsfCsAfCUfGAfGAfAUACfUGfU CfGCsUsC |
| 111 0 | S53 9 | AsAsGCGACAfGfUfAUUCU CAGUsGsU | 102 4 | AS51 6 | AsfCsAfCUfGAfGAAUACfUGfU CfGCsfUsU |

(continued)

| SE Q ID NO. | singl e stran d code | sense strand 5'→3' | SE Q ID NO. | single strand code | antisense strand 5'→3' |
|---|---|---|---|---|---|
| 112 1 | S55 0 | AsAsGGfGAfCfAfGUAUUCU CAGUsGsA | 115 1 | AS52 9 | UsCsACUGfAGAAUfACfUGUCC CsUsU |

where, G=2'-O-methylguanylate, A=2'-O-methyladenylate, U=2'-O-methyluridylate, C=2'-O-methylcytidylate; fG=2'-fluoroguanylate, fA=2'-fluoroadenylate, fU=2'-fluorouridylate, fC=2'-fluorocytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate, fGs=2'-fluoro-3'-thioguanylate, fAs=2'-fluoro-3'-thioadenylate, fUs=2'-fluoro-3'-thiouridylate, fCs=2'-fluoro-3'-thiocytidylate, T=thymidylate.

In some technical solutions, preferably, the RNA inhibitor or pharmaceutically acceptable salt thereof further contains a carrier structure, and the RNA inhibitor is as shown in Formule Ia, Ib or Ic:

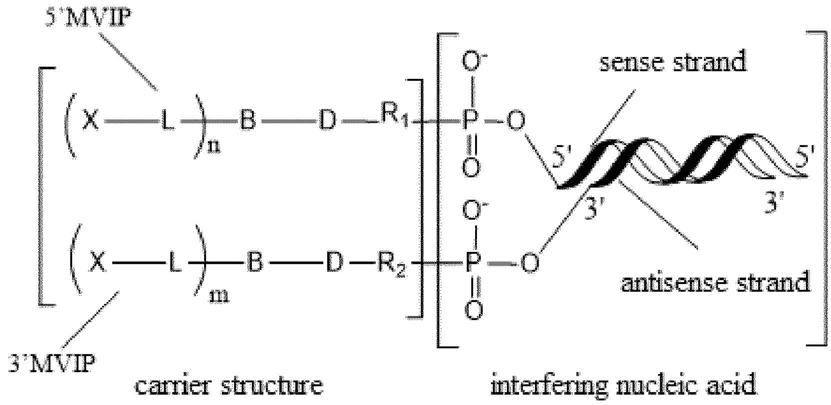


Ia

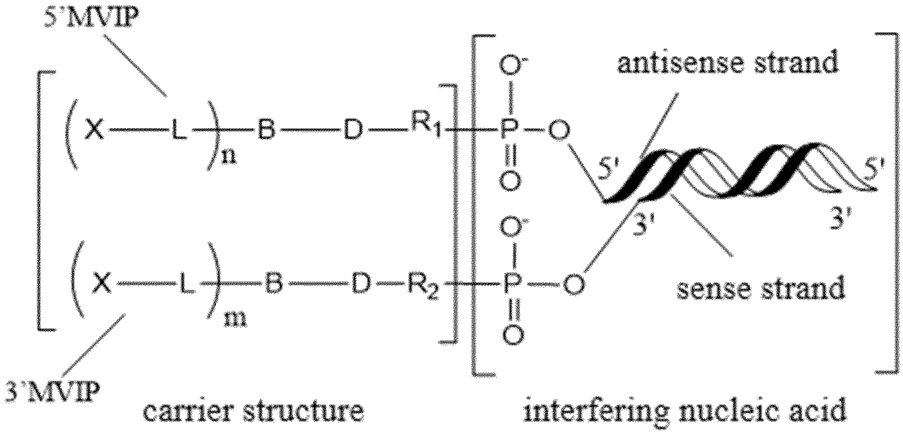


Ib

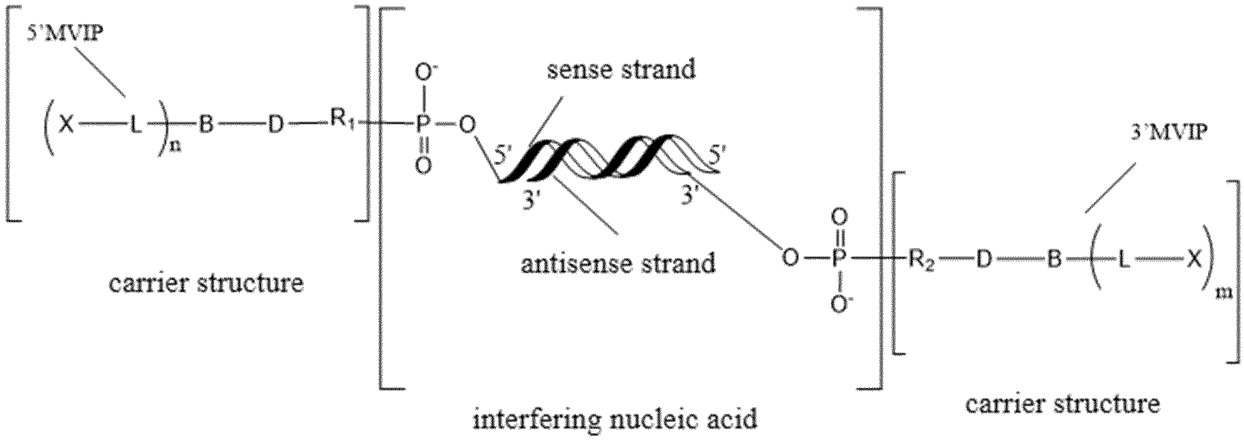


Ic

wherein,

the carrier structure includes a 5'MVIP and a 3 'MVIP;

The 5'MVIP is composed of a transition point $R_1$, a linking chain D, a linker B, a branched chain L and a liver targeting specific ligand X and is connected with the 5' end of sense strand or the 5' end of antisense strand through the transition point $R_1$. The structure is shown in the formula I:

$$(X\text{-}L)_n\text{-}B\text{-}D\text{-}R_1 \qquad I$$

The 3'MVIP is composed of a transition point $R_2$, a linking chain D, a linker B, a branched chain L and a liver targeting specific ligand X and is connected with the 3' end of sense strand or the 3' end of antisense strand through the transition point $R_2$. The structure is shown in the formula II:

$$(X\text{-}L)_m\text{-}B\text{-}D\text{-}R_2 \qquad II$$

wherein,

n and m are each independently any integer of 0-4, each independently preferably an integer of 1-3, and n+m = an integer of 2-6, preferably n+m=2, 3 or 4, more preferably 4.

As an example, the transition point $R_1$ is a heterocyclic or carbocyclic structure containing N, S or O as shown below,

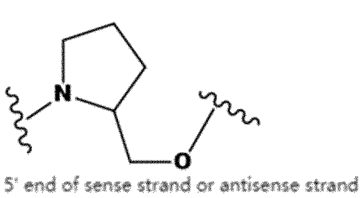


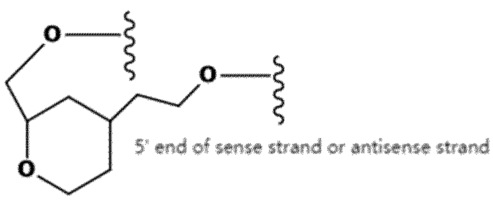


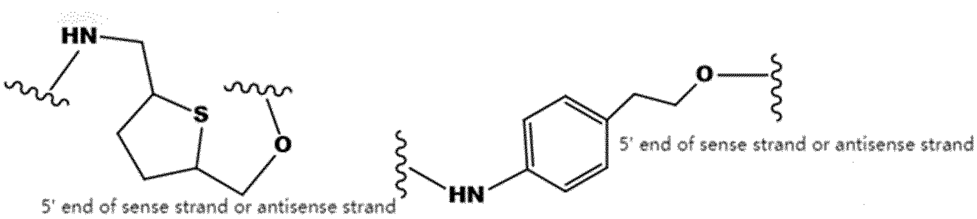


Alternatively, $R_1$ is $-NH(CH_2)_xCH_2O-$, wherein x is any integer of 3-12, preferably any integer of 4-6;

As an example, the transition point $R_2$ is a heterocyclic or carbocyclic structure containing N, S or O as shown below,

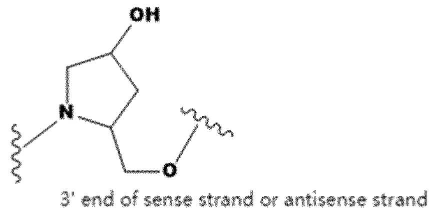
3' end of sense strand or antisense strand

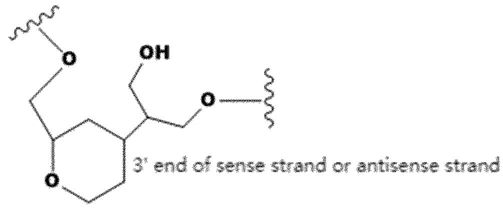
3' end of sense strand or antisense strand

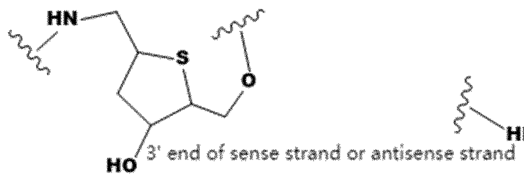
3' end of sense strand or antisense strand

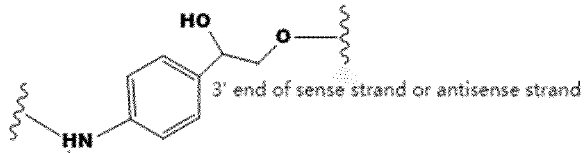
3' end of sense strand or antisense strand

Alternatively, the transition point $R_2$ is $-NH(CH_2)_{x1}CH(OH)(CH_2)_{x2}CH_2O-$, wherein x1 is any integer of 1-4, and x2 is any integer of 0-4;
The liver targeting specific ligand X may be the same or different within each of 5'MVIP and 3'MVIP or between 5'MVIP and 3'MVIP, and is selected from monosaccharides and their derivatives, preferably selected from N-acetylgalactosamine and their derivatives, and more preferably selected from the following structures:

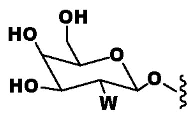 ; 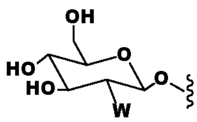 ; 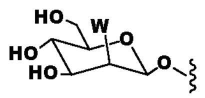 ;

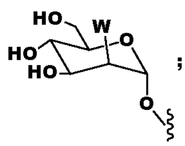 ; 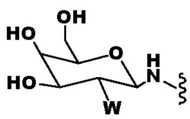 ; 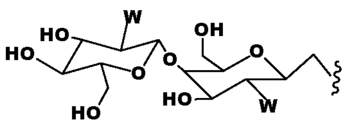 ;

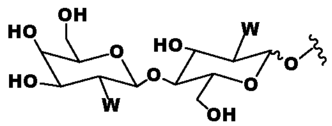 ; 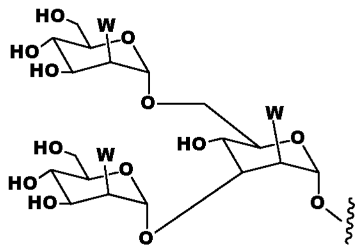 ;

Wherein, as an example, W is selected from one or two of -OH, -NHCOOH and $-NHCO(CH_2)q\ CH_3$, wherein q is an integer of 0-4.;

As an example, the branch chain L is the same or different within each of 5'MVIP and 3'MVIP or between 5'MVIP and 3'MVIP, which is selected from one or more of the following structures:

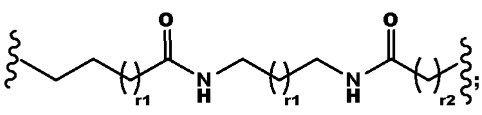 ; 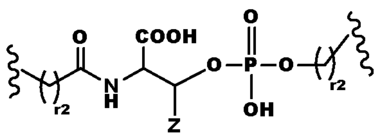 ;

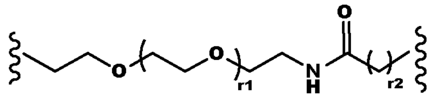 ; 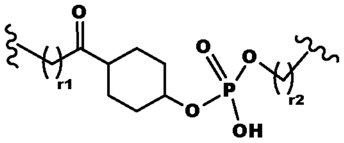 ;

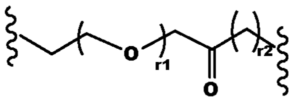 ; 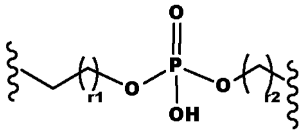 ; 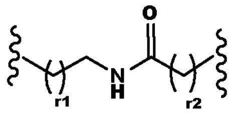 .

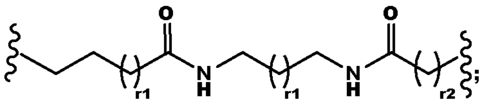 ; 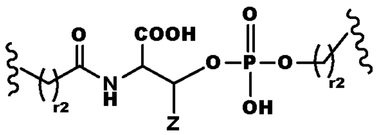 ;

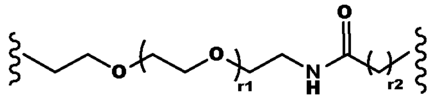 ; 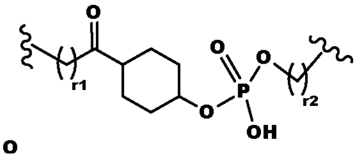 ;

o

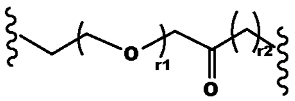 ; 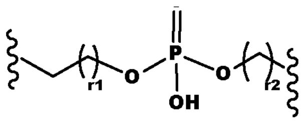 ; 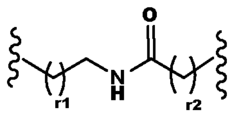 .

wherein, r1 is any integer of 1-12, r2 is any integer of 0-20, and Z is H, alkyl or amido. Alkyl is, for example, $C_1$-$C_5$ alkyl; As an example, the linker B is the same or different within each of 5'MVIP and 3'MVIP or between 5'MVIP and 3'MVIP, which is selected from the following structures:

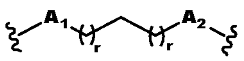 ; 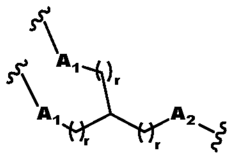 ; 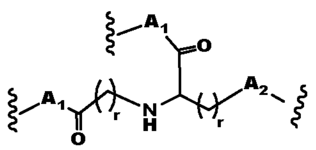 ;

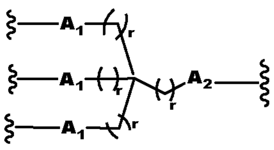 ; 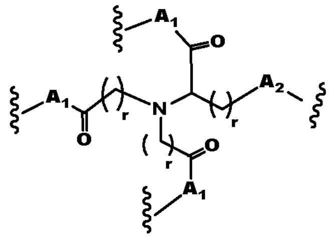 ; 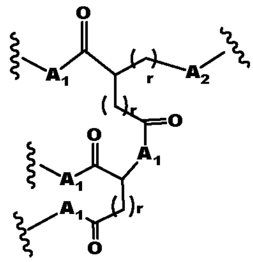 ;

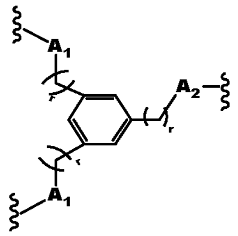 ; 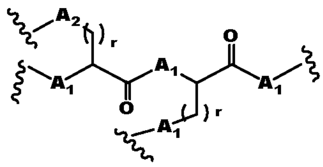 ;

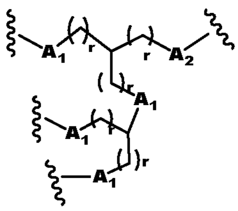 ; 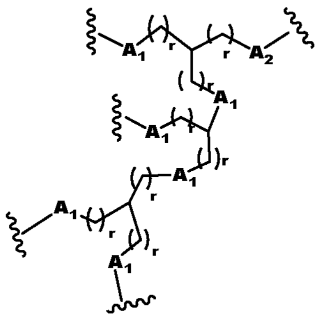 ; 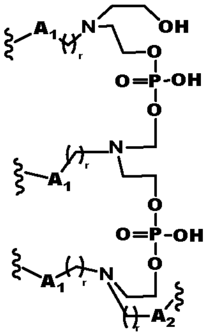 .

wherein, $A_1$ and $A_2$ are each independently C, O, S, -NH-, carbonyl, amido, phosphoryl or thiophosphoryl, and r is any integer of 0-4;

As an example, the linking chain D is the same or different within each of 5'MVIP and 3'MVIP or between 5'MVIP and 3'MVIP, which is selected from the following structures:

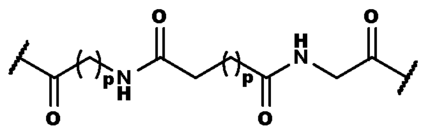 ; 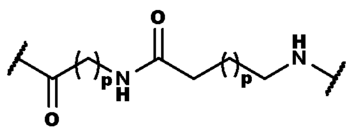 ; 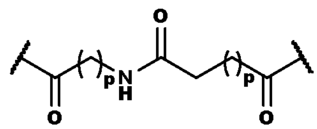 ;

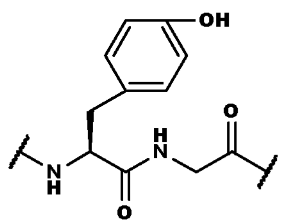 ; 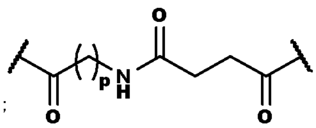 ; 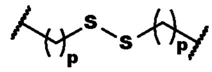 ; 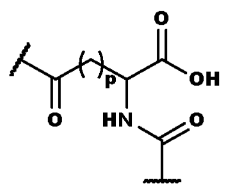 ;

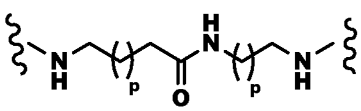 ; 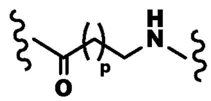 ; 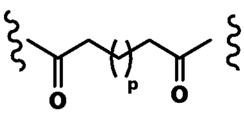 ;

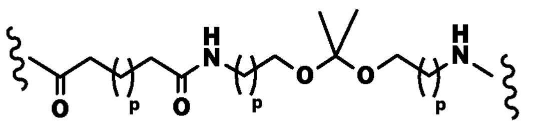 ; 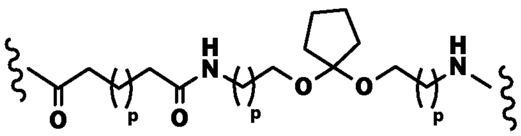 ;

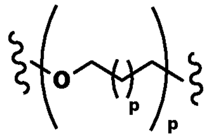 ; 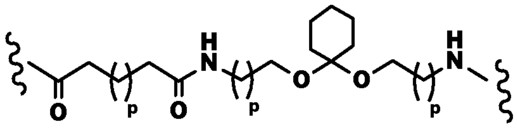 ; 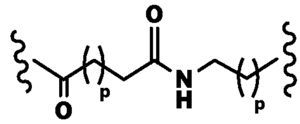 ;

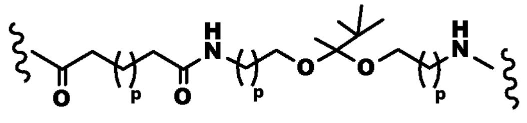 ; 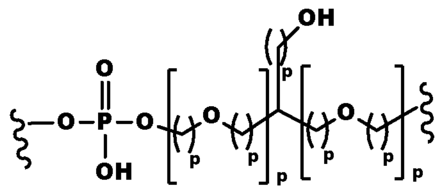 ;

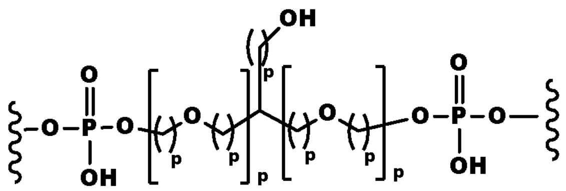 ; 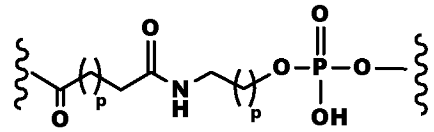 ;

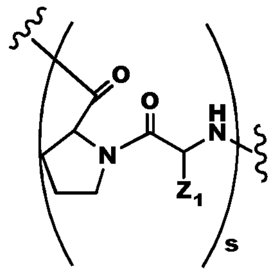 ; 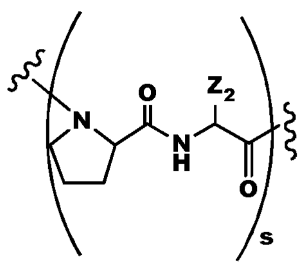 ; 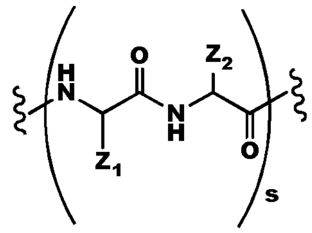 ;

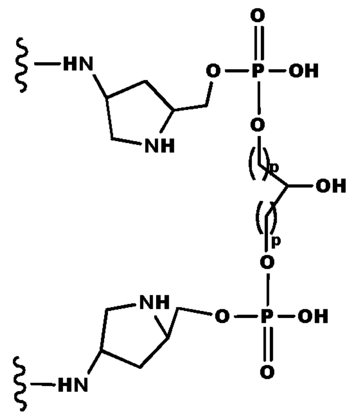 ; 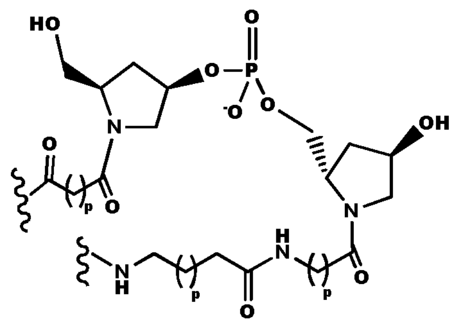 ;

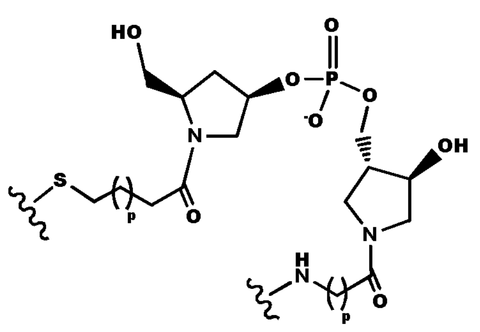 ; 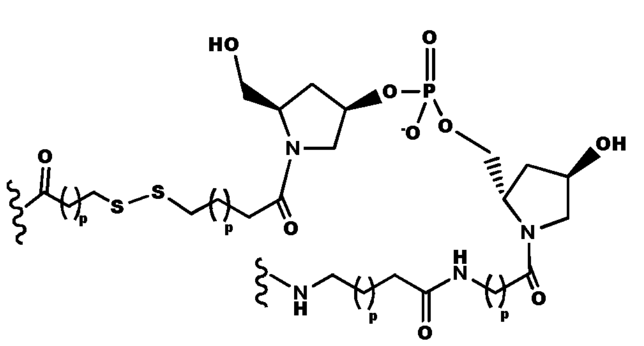 ;

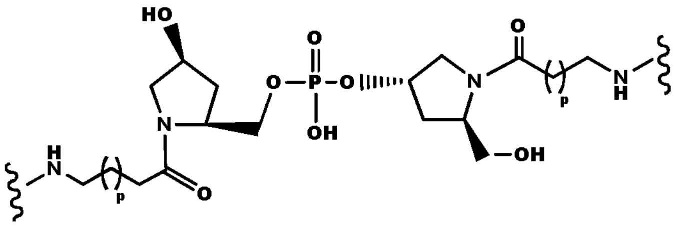

;

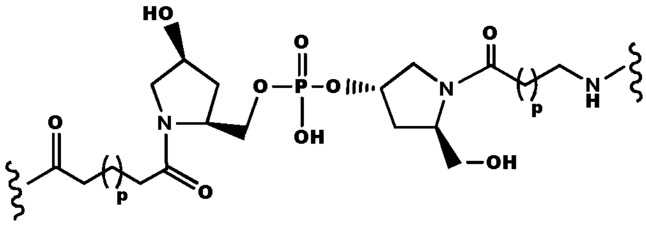

;

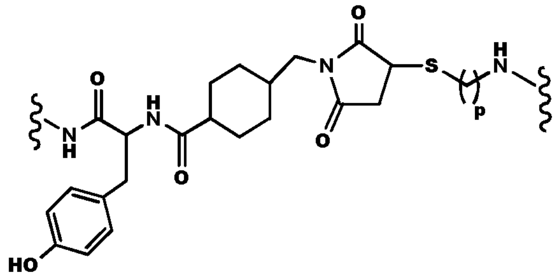

;

wherein, each p is independently any integer of 1-20; s is any integer of 2-13; $Z_1$ and $Z_2$ are the same or different substituent groups.

It should be noted that, the above structures are not exhaustive. As long as it is a carrier connected to the sequence of the present invention, regardless of the disclosed or undisclosed delivery method, targeting any receptor or any structure is within the scope of the present invention.

The published delivery methods include: conjugated carrier delivery method and lipid encapsulation delivery method. The conjugated carrier delivery method includes: cholesterol conjugate, antibody conjugate, folic acid conjugate and GalNac conjugate, etc.; the lipid encapsulation delivery method includes: lipid nanoparticles (LNP), polymer nanoparticles and extracellular vesicles, etc.

More preferably, in the RNA inhibitor or a pharmaceutically acceptable salt thereof of the present invention, the carrier structure at the 5' end of the sense strand is 5'MVIP17, the carrier structure at the 3' end of the sense strand is 3'MVIP17, the combination of sense strand 5'MVIP and antisense strand 3'MVIP is 5'MVIP01/3'MVIP01, 5'MVIP01/3'MVIP17, 5'MVIP17/3'MVIP01 or 5'MVIP09/3'MVIP09, or the combination of sense strand 5'MVIP and sense strand 3'MVIP is 5'MVIP01/3'MVIP09, 5'MVIP09/3'MVIP01 or 5'MVIP01/3'MVIP01。

Further preferably, in the RNA inhibitor or a pharmaceutically acceptable salt thereof of the present invention, the RNA inhibitor is selected from: Ky-12-DS23001, Ky-12-DS25001, Ky-12-DS25401, Ky-12-DS29701, Ky-12-DS29801, Ky-12-DS31701, Ky-12-DS31702, Ky-12-DS31703, Ky-12-DS31704, Ky-12-DS31705, Ky-12-DS31706, Ky-12-DS31707, Ky-12-DS31708, Ky-12-DS31709, Ky-12-DS31711, Ky-12-DS31712, Ky-12-DS31713, Ky-12-DS33001, Ky-12-DS33006, Kylo-12-DS1071, Kylo-12-DS1081, Kylo-12-DS1131, Kylo-12-DS1141, Kylo-12-DS1241, Kylo-12-DS1311, Kylo-12-DS1321, Kylo-12-DS5911, Kylo-12-DS2911, Kylo-12-DS2611, Kylo-12-DS2311, Kylo-12-DS3111 and Kylo-12-DS5411。

In another aspect, the present invention also provides the use of the above-mentioned RNA inhibitor or a pharmaceutically acceptable salt thereof in the preparation of a drug for treating and/or preventing a disease associated with an elevated APOC3 level, wherein the disease associated with an elevated APOC3 level includes hepatic diseases, which comprise inflammatory, cardiovascular and cerebrovascular and metabolic diseases, wherein the cardiovascular and cerebrovascular diseases include hyperlipidemia, stroke, atherosclerosis, thrombosis, coronary heart disease, aortic valve stenosis, hypertriglyceridemia (HTG), severe hypertriglyceridemia (sHTG) or familial chylomicronemia syndrome (FCS)。

In yet another aspect, the present invention provides a pharmaceutical composition, which comprises the above-mentioned RNA inhibitor or a pharmaceutically acceptable salt thereof for inhibiting APOC3 gene expression and optional pharmaceutically acceptable excipients, wherein the pharmaceutically acceptable excipients may be pharmaceutically

acceptable excipients, vehicles and/or diluents, and the dosage form of the pharmaceutical composition is an oral preparation, an intravenous injection, or a subcutaneous or intramuscular injection, preferably a subcutaneous injection.

In yet another aspect, the present invention also provides a method for treating and/or preventing a disease, condition or syndrome associated with an elevated level of APOC3, the method comprising administering to a subject in need thereof a therapeutically effective amount of an RNA inhibitor or a pharmaceutically acceptable salt thereof that inhibits APOC3 gene expression, or a pharmaceutical composition comprising the RNA inhibitor or pharmaceutically acceptable salt thereof and optional pharmaceutically acceptable excipients. The mode of administration to the subject (administration method) includes oral administration, intravenous injection, subcutaneous or intramuscular injection, rectal or intraperitoneal application, and aerosol inhalation. Those skilled in the art will readily appreciate other aspects and advantages of the present invention from the detailed description below. The detailed description below only shows and describes exemplary embodiments of the present invention. As those skilled in the art will realize, the content of the present invention enables those skilled in the art to modify the specific embodiments disclosed without departing from the spirit and scope of the invention involved in the present invention. Accordingly, the drawings and descriptions in the specification of the present invention are merely exemplary and not restrictive.

**Description of drawings**

The specific features of the invention involved in the present invention are shown in the appended claims. The features and advantages of the inventions of the present invention can be better understood by referring to the exemplary embodiments and drawings described in detail below. A brief description of the drawings is as follows:

Figure 1 is a schematic diagram of the average hAPOC3 level in the serum of hAPOC3 Tg mice after intervention with RNA inhibitor in Example 9 of the present invention;
Figure 2 is a schematic diagram of the inhibitory effect on APOC3 mRNA level after intervention with RNA inhibitor in Example 10 of the present invention;
Figure 3 is a schematic diagram of the inhibitory effect on APOC3 mRNA level in HepG 2 cells after intervention with RNA inhibitor in Example 11 of the present invention;
Figure 4 is a schematic diagram of the inhibitory effect on APOC3 mRNA level in PHH cells after intervention with RNA inhibitor in Example 12 of the present invention;
Figure 5 is a schematic diagram of the average hAPOC3 level in the serum of hAPOC3 Tg mice after intervention with RNA inhibitor in Example 13 of the present invention;
Figure 6 is a schematic diagram of the average TG level in the serum of hAPOC3 Tg mice after intervention with RNA inhibitor in Example 13 of the present invention;
Figure 7 is a schematic diagram of the inhibitory effect on hAPOC3 in the serum of hAPOC3 Tg mice after intervention with RNA inhibitor in Example 14 of the present invention;
Figure 8 is a schematic diagram of the reduction effect of TC in the serum of hAPOC3 Tg mice after intervention with RNA inhibitor in Example 14 of the present invention;
Figure 9 is a schematic diagram of the reduction effect of TG in the serum of hAPOC3 Tg mice after intervention with RNA inhibitor in Example 14 of the present invention;
Figure 10 is a schematic diagram of the reduction effect of LDL-c in the serum of hAPOC3 Tg mice after intervention with RNA inhibitor in Example 14 of the present invention;
Figure 11 is a schematic diagram of the inhibition effect on APOC3 in the serum of hyperlipidemia cynomolgus monkey after intervention with RNA inhibitor in Example 15 of the present invention;
Figure 12 is a schematic diagram of the reduction effect of TG in the serum of hyperlipidemia cynomolgus monkey after intervention with RNA inhibitor in Example 15 of the present invention;
Figure 13 is a schematic diagram of the change in HDL-c level in the serum of hyperlipidemia cynomolgus monkey after intervention with RNA inhibitor in Example 15 of the present invention;
Figure 14 is a schematic diagram of the reduction effect of LDL-c in the serum of hyperlipidemia cynomolgus monkey after intervention with RNA inhibitor in Example 15 of the present invention;
Figure 15 is a schematic diagram of the reduction effect of TC in the serum of hyperlipidemia cynomolgus monkey after intervention with RNA inhibitor in Example 15 of the present invention.

**Mode of carring out the invention**

The implementation of the present invention will be described below with specific examples. Those familiar with this technology can easily understand other advantages and effects of the present invention from the contents disclosed in the specification.

## Definition of terms

In the present invention, examples of APOC3 mRNA sequences are easily obtained using public databases such as GenBank, UniProt and OMIM. The term "APOC3" includes human APOC3, cynomolgus monkey APOC3, macaque APOC3, mouse APOC3, rat (Rattus norvegicus) APOC3, rabbit (Oryctolagus) APOC3, etc. As long as the organisms actually exist and have the APOC3 gene, they are within the scope of the present invention. The mRNA sequence of human APOC3 can be found, for example, in GenBank NM_000040.3. The amino acid sequence and complete coding sequence of cynomolgus monkey APOC3 can be found, for example, in GenBank accession number GI:544489959(XM_05579730 .1). The amino acid sequence and complete coding sequence of macaque APOC3 can be found, for example, in GenBank accession number GI:297269260(XM_001090312 .2); the amino acid sequence and complete coding sequence of mouse APOC3 can be found, for example, in GenBank accession number GI:577019555(NM_023114 .4); the amino acid sequence and complete coding sequence of rat (Rattus norvegicus) APOC3 can be found, for example, in GenBank accession number GI:402534545 (NM_012501.2); and rabbit (Oryctolagus) APOC3 , GenBank accession number GI:655601498(XM_002708371 .2).

In the present invention, the terms "iRNA", "RNA inhibitor", "iRNA agent" and "RNA interfering agent" can be used interchangeably, and generally refer to agents containing RNA as defined by the terms herein, which can mediate the targeted cleavage of RNA transcripts through the RNA-induced silencing complex (RISC) pathway. iRNA guides the sequence specific degradation of mRNA via a process called RNA interference (RNA inhibitor). iRNA regulates (e.g., inhibits) the expression of APOC3 gene in cells (e.g., cells in a subject such as a mammalian subject).

In some embodiments, RNA inhibitor may be single stranded siRNAs (ssRNA inhibitors) introduced into a cell or organism to inhibit target mRNA. Single stranded RNA inhibitors bind the RISC endonuclease Argonaute 2, which then cleaves the target mRNA. Single stranded siRNAs are generally 15 to 30 nucleotides and chemically modified.

In some embodiments, the "iRNA" used in the present invention is a double stranded RNA, and is referred to herein as "double stranded RNA inhibitor", "double stranded RNA (dsRNA) molecule", "dsRNA agent" or "dsRNA". The term "dsRNA" refers to a complex of ribonucleic acid molecules with a duplex structure containing two antiparallel and essentially complementary nucleic acid strands, known as having a "sense" and "antisense" orientation relative to the target RNA (i.e., APOC3 gene). In some embodiments of the present invention, double stranded RNA (dsRNA) triggers the degradation of target RNA (e.g., mRNA) through a post transcriptional gene silencing mechanism (herein referred to as RNA interference or RNA inhibitor).

The duplex structure can be any length that allows the required target RNA to be specifically degraded through the RISC pathway, and can have a length in range of about 19 to 36 base pairs, for example, the length of about 19-30 base pairs, for example, the length of about 19, 20, 21, 22, 23, 24, 25, 26, 27, 28, 29, 30, 31, 32, 33, 34, 35 or 36 base pairs. The ranges and lengths within the above ranges and lengths are also included as part of the present invention. In some embodiments, the iRNA agent of the present invention is a dsRNA containing 15-23 nucleotides in each strand, which interacts with the target RNA sequence (e.g., APOC3 gene) to guide the cleavage of the target RNA. In some embodiments, the iRNA of the present invention is a dsRNA containing 24-30 nucleotide, which interacts with the target RNA sequence (e.g., APOC3 target mRNA sequence) to guide the cleavage of the target RNA.

Generally, most nucleotides of each chain of dsRNA molecule are ribonucleotides, but as detailed herein, each chain or both chains can also include one or more non-ribonucleotides, such as deoxyribonucleotides or modified nucleotides. In addition, the "iRNA" used herein may include ribonucleotides with chemical modifications; iRNA can include substantial modifications at multiple nucleotides. The term "modified nucleotide" used herein refers to a nucleotide that independently has a modified sugar moiety, a modified internucleotide linkage or a modified nucleobase, or any combination thereof. Therefore, the term "modified nucleotide" covers the substitution, addition, or removal of, for example, functional groups or atoms of internucleotide linkages, sugar moieties, or nucleobases. The modifications applicable to the agent of the present invention include all types of modifications disclosed herein or known in the art.

In the present invention, the terms "nucleic acid" and "polynucleotide" can be used interchangeably and refer to the polymeric form of nucleotides (deoxyribonucleotides or ribonucleotides or analogues thereof) of any length. Polynucleotides can have any three-dimensional structure and can perform any function. The following are non-limiting examples of polynucleotides: genes or gene segments (such as probes, primers, EST or SAGE tags), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, siRNA, miRNA, shRNA, RNA inhibitor reagents and primers. Polynucleotides may be modified or substituted at one or more bases, sugars, and/or phosphates with any of the various modifications or substitutions described in the present invention or known in the art. Polynucleotides can contain modified nucleotides, such as methylated nucleotides and nucleotide analogues. If present, the nucleotide structure can be modified before or after polymer assembly. Nucleotide sequences can be blocked by non-nucleotide components. Polynucleotides can be modified after polymerization, for example by conjugating with labeled components. The term can be double stranded and single stranded molecules. Unless otherwise stated or required, any embodiment as a polynucleotide of the present invention includes a double

stranded form and each of the two complementary single stranded forms known or predicted to constitute the double stranded form.

In the present invention, the term "target nucleic acid" or "target sequence" generally refers to the consecutive part of the nucleotide sequence of the mRNA molecule formed during the transcription of the APOC3 gene, including mRNA as the main transcription product of RNA processing. The target part of the sequence should be at least long enough to serve as a substrate for iRNA-guided cleavage at or near that portion of the nucleotide sequence of mRNA molecule formed during APOC3 gene transcription. In one embodiment, the target sequence is within the protein coding region of APOC3. The target sequence can be about 19-36 nucleotides in length, for example, it is preferred to be about 19-30 nucleotides in length. The ranges and lengths within the above ranges and lengths are also included as part of the present invention.

In the present invention, the term "nucleotide sequence" usually refers to a series or a certain sequence of nucleobases, nucleotides and/or nucleosides, whether modified or unmodified, described by a series of letters using standard nucleotide nomenclature and a list of symbols for modified nucleotides described in the present invention.

In the present invention, the term "oligonucleotide" generally refers to a polymer composed of multiple nucleotide residues (deoxyribonucleotide or ribonucleotide, or related structural variant or synthetic analog thereof) connected by phosphodiester bond (or related structural variant or synthetic analog thereof). Therefore, although the term "oligonucleotide" generally refers to the nucleotide polymer in which the nucleotide residues and the linkage between them are naturally occurred, it should be understood that the scope of the term also includes various analogues, including but not limited to: peptide nucleic acid (PNA), aminophosphate, thiophosphate, methyl phosphonate, 2-O-methyl ribonucleic acid, etc. The exact size of the molecule may depend on the specific application. Oligonucleotides are generally short in length, usually about 10-30 nucleotide residues, but the term can also refer to molecules of any length, although the term "polynucleotide" or "nucleic acid" is generally used for larger oligonucleotides.

In some embodiments, the oligonucleotides comprise one or more unmodified ribonucleosides (RNA) and/or unmodified deoxyribonucleosides (DNA) and/or one or more modified nucleosides. The term "modified oligonucleotide" generally refers to an oligonucleotide containing at least one modified nucleoside and/or at least one modified internucleoside linkage.

In the present invention, the term "modified nucleoside" generally means a nucleoside containing at least one chemical modification compared with a naturally occurring RNA or DNA nucleoside. Modified nucleosides contain modified sugar moieties and/or modified nucleobases.

In the present invention, the term "nucleobase" generally refers to a heterocyclic pyrimidine or purine compound, which is a component of all nucleic acids and includes adenine, guanine, cytosine, thymine and uracil. Nucleotides may include modified nucleotides or nucleotide mimics, abasic sites (Ab or X), or the part substituted by the substituent. As used herein, "nucleobase sequence" generally means a sequence of consecutive nucleobases that does not depend on any sugar, linkage or nucleobase modification. The term "unmodified nucleobase" or "naturally occurring nucleobase" generally means naturally occurring heterocyclic nucleobases of RNA or DNA: purine bases adenine and guanine; and the pyrimidine bases thymine, cytosine and uracil. "Modified nucleobase" usually means any nucleobase that is not a naturally occurring nucleobase.

The "g", "c", "a", "t" and "u" each generally represent nucleotides containing guanine, cytosine, adenine, thymine and uracil as bases, respectively. However, it should be understood that, the term "ribonucleotide" or "nucleotide" may also refer to modified nucleotides, as shown in Tables 1 and 2. It is well known to those skilled in the art that guanine, cytosine, adenine, thymidine and uracil can be replaced by other moieties without significantly changing the base pairing properties of the oligonucleotide containing the nucleotides bearing such substituted moieties. For example, but not limited to, i (inosinic acid), also called hypoxanthine, can be base paired with a, c, and u. In some cases, the i can also be paired with the g. This phenomenon is called the wobble phenomenon. Thus, a nucleotide comprising the a, c or u can be replaced by a nucleotide comprising, for example, the i in the nucleotide sequence of a dsRNA characterized in the present invention. In another example, the a and c anywhere in the oligonucleotide can be replaced by the g and u respectively, to form a g-u wobble base pairing with the target mRNA. Sequences containing such replacement moieties are suitable for use in the compositions and methods of the present invention.

In the present invention, the term "sugar moiety" generally refers to the naturally occurring sugar moiety or modified sugar moiety of a nucleoside. The term "naturally occurring sugar moiety" generally refers to a ribofuranosyl as found in naturally occurring RNA or a deoxyribofuranosyl as found in naturally occurring DNA. "Modified sugar moiety" means a substituted sugar moiety or sugar substitute.

In the present invention, the term "internucleoside linkage" generally refers to the covalent linkage between adjacent nucleosides in oligonucleotides. "Naturally occurring internucleoside linkage" means 3' to 5' phosphodiester linkage. "Modified internucleoside linkage" means any internucleoside linkage other than the naturally occurring internucleoside linkage.

In the present invention, the term "antisense oligonucleotide" refers to a single stranded oligonucleotide molecule with a nucleobase sequence complementary to the corresponding segment of the target nucleic acid (e.g.,

the target genome sequence, mRNA precursor, or mRNA molecule). In some embodiments, the antisense oligonucleotide is 12 to 30 nucleobases in length. In some embodiments, the antisense oligonucleotide is an unmodified or modified nucleic acid with a nucleotide sequence complementary to the sequence of target nucleic acid (such as APOC3 polynucleotide).

In the present invention, the term "antisense strand" generally refers to the strand of RNA inhibitor (such as dsRNA) that includes a region that are substantially complementary to the target sequence. When used herein, the term "complementary region" generally refers to a region on the antisense strand that is substantially complementary to the sequence defined in the present invention (e.g., target sequence). When the complementary region is not fully complementary to the target sequence, the mismatch can be in the inner or terminal region of the molecule. In general, the most tolerated mismatches are in the terminal region, for example, within 5, 4, 3, or 2 nucleotides at the 5' and/or 3' ends.

In the present invention, the term "sense strand" (S) generally refers to such a strand of RNA inhibitor, which includes a region that is substantially complementary to a region of the antisense strand as the term defined herein. The "sense" strand is sometimes referred to as the "passenger" strand or "antiguide" strand. With their sequences, the antisense strand targets the desired mRNA, while the sense strand targets different targets. Therefore, if the antisense strand is incorporated into RISC, the correct target is targeted. The incorporation of sense strand can lead to off-target effects. These off-target effects can be limited by the use of modifications or 5' end caps on the sense strand.

In the present invention, the term "complementary" when used to describe the first nucleotide sequence (such as RNA inhibitor sense strand or APOC3 mRNA) in terms of the second nucleotide sequence (such as RNA inhibitor antisense strand) refers to the ability of oligonucleotides or polynucleotides containing the first nucleotide sequence to hybridize (form base pair hydrogen bonds) with oligonucleotides or polynucleotides containing the second nucleotide sequence and form duplex or double helix structures under certain conditions. Complementary sequences include Watson-Crick base pairs or non-Watson-Crick base pairs, and include natural or modified nucleotides or nucleotide mimics, as long as the above requirements for their hybridization ability are realized. "Complementary" does not require nucleobase complementarity on each nucleoside. On the contrary, some mismatches can be tolerated.

In the present invention, the term "fully complementary" generally means that all (100%) bases in the consecutive sequence of the first polynucleotide will hybridize with the same number of bases in the consecutive sequence of the second polynucleotide. The consecutive sequence may comprise all or part of the first or second nucleotide sequence. As used herein, "partially complementary" generally means that in the hybridized nucleobase sequence pairs, at least about 70% of the bases in the consecutive sequence of the first polynucleotide will hybridize with the same number of bases in the consecutive sequence of the second polynucleotide. As used herein, "substantially complementary" generally means that in the hybridized nucleobase sequence pairs, at least about 80% of the bases in the consecutive sequence of the first polynucleotide will hybridize with the same number of bases in the consecutive sequence of the second polynucleotide. The terms "complementary", "fully complementary" and "substantially complementary" as used herein can be used in terms of base matching between the sense strand and antisense strand of RNA inhibitor or between the antisense strand of RNA inhibitor and the sequence of APOC3 mRNA. Sequence identity or complementarity does not depend on modification. For the purpose of determining identity or complementarity, for example, A and fA are complementary to U (or T) and identical to a.

In the present invention, the term "homologous" or "homology" generally refers to the number of nucleotides of the subject nucleic acid sequence that have matched the same nucleotide of the reference nucleic acid sequence, which is usually determined by sequence analysis programs (for example, Karlin and Altschul, 1990, PNAS 87:2264-2268; Karlin and Altschul, 1993, PNAS 90:5873-5877), or by visual inspection. As used herein, the term "complete homology" or "fully homologous" generally refers to complete (100%) homology or "identity" between the reference sequence and the subject nucleic acid sequence. As used herein, the term "substantially homologous" or "substantial homology" generally refers to the fact that nucleotides at the same nucleotide positions in the subject sequence and the reference sequence are at least 50% (e.g., at least 55%, 60%, 65%, 70%, 75%, 80%, 85%, 90%, 95%, 96%, 97%, 98% or 99%) homologous.

**In** the present invention, the term "ligand" generally refers to any compound or molecule that can be covalently or otherwise chemically bound to bioactive substances (such as oligonucleotides). **In** some embodiments, the ligand is able to interact directly or indirectly with another compound such as a receptor, the receptor interacting with the ligand may exist on the cell surface, or alternatively may be an intracellular and/or intercellular receptor, the interaction of the ligand with the receptor may lead to a biochemical reaction, or may simply be a physical interaction or binding.

In the present invention, the terms "induce", "inhibit", "strengthen", "elevate", "increase", "decrease", "reduce" and the like usually indicate quantitative differences between two states. For example, "the amount that effectively inhibits the activity or expression of APOC3" means that the level of APOC3 activity or expression in the treated samples will be lower than that in the untreated samples. The terms described apply, for example, to expression levels and activity levels. The terms "decrease" and "reduce" are used interchangeably and usually indicate any changes less than the original. "Decrease" and "reduce" are relative terms that need to be compared before and after measurement. "Decrease" and "reduce" include complete depletion.

In some embodiments, the term "reduce" may refer to an overall reduction of about 5%, 10%, 20%, 25%, 30%, 40%, 50%, 60%, 70%, 80%, 85%, 90%, 95%, or 100% in the expression level/amount of a gene/gene product such as protein or biomarker in a first sample compared to the expression level/amount of the corresponding gene/gene product such as protein or biomarker in a second sample, as detected by standard methods known in the art, such as those described in the present invention. In some embodiments, the term "reduce" refers to a reduction in the expression level / amount of a gene or biomarker in the first sample, wherein the reduction is at least about 0.9 times, 0.8 times, 0.7 times, 0.6 times, 0.5 times, 0.4 times, 0.3 times, 0.2 times, 0.1 times, 0.05 times, or 0.01 times the expression level / amount of the corresponding gene or biomarker in the second sample. In some embodiments, the first sample is a sample obtained from a subject, while the second sample is a reference sample.

In the present invention, the term "expression" generally refers to the process by which a gene eventually produces a protein. The expression includes, but is not limited to, transcription, post transcriptional modifications (e.g., splicing, polyadenylation, addition of 5 ’- caps), and translation.

In the present invention, the term "pharmaceutically acceptable" generally refers to one or more non-toxic substances that do not interfere with the effectiveness of the biological activity of the active ingredient. Such preparations may generally contain salts, excipients, buffers, preservatives, compatible vehicles, and optionally other therapeutic agents. Such pharmaceutically acceptable preparations may also generally contain compatible solid or liquid fillers, diluents, or encapsulation materials suitable for administration to humans. When used in medicine, salts should be pharmaceutically acceptable salts, but non-pharmaceutically acceptable salts can be conveniently used to prepare pharmaceutically acceptable salts, which cannot be excluded from the scope of the present invention. Such pharmacologically and pharmaceutically acceptable salts include but are not limited to those prepared from the following acids: hydrochloric acid, hydrobromic acid, sulfuric acid, nitric acid, phosphoric acid, maleic acid, acetic acid, salicylic acid, citric acid, boric acid, formic acid, malonic acid, succinic acid, etc. The pharmaceutically acceptable salts can also be prepared into alkali metal salts or alkaline earth metal salts, such as sodium salt, potassium salt or calcium salt.

In the present invention, the term "lipid nanoparticles" or "LNP" generally refers to nanoparticles containing encapsulated pharmacologically active molecules, such as nucleic acid molecules, for example, iRNA or plasmid from which iRNA is transcribed. The iRNA may contain no ligand or may contain a ligand, such as a GalNAc derivative. LNP is described in, for example, Chinese patent No. CN103189057B, the complete contents of which are incorporated herein by reference.

In the present invention, the term "prevent and/or treat" not only includes the prevention and/or treatment of disease, but also generally includes the prevention of the onset of disease, slowing or reversing the progression of disease, preventing or slowing the onset of one or more symptoms related to disease, reducing and/or alleviating one or more symptoms related to disease, reducing the severity and/or duration of the disease and/or any symptom associated with it and/or preventing further increases in the severity of the disease and/or any symptom associated with it, preventing, reducing or reversing any physiological damage caused by the disease, and any pharmacological effects that are generally beneficial to the patient being treated. The RNA inhibitor or pharmaceutical composition of the present invention does not need to achieve complete cure or eradication of any symptoms or manifestations of the disease to form a feasible therapeutic agent. As recognized in relevant fields, drugs used as therapeutic agents can reduce the severity of a given disease state, but do not need to eliminate every manifestation of the disease to be considered as useful therapeutic agents. Similarly, treatments administered prophylactically to constitute viable prophylactic agents do not need to be completely effective in preventing the onset of the condition. Simply reducing the impact of disease in subjects (for example, by reducing the numbers or severity of their symptoms, or by improving the effectiveness of another treatment, or by producing another beneficial effect), or reducing the likelihood of disease occurrence or exacerbation is sufficient.

In the present invention, the term "disease" or "condition" can be used interchangeably, and generally refers to any deviation from the normal state of the subject, such as any change in the state of the body or some organs, which hinders or disturbs the performance of the function, and/or causes symptoms such as discomfort, dysfunction, pain or even death in the person who is ill or in contact with him. Disease or condition can also be called distemper, ailing, ailment, malady, disorder, sicknesses, illnesses and complaints.

In the present invention, the term "administration" generally refers to the introduction of the pharmaceutical preparation of the present invention into the subject’s body through any route of introduction or delivery. Any method known to those skilled in the art for contacting a cell, organ or tissue with the drug can be adopted. The administration may include, but is not limited to, intravenous, intra-arterial, intranasal, intra-abdominal, intramuscular, subcutaneous transdermal or oral administration. The daily dose can be divided into one, two or more suitable forms of dose to be administered at one, two or more times during a certain time period.

In the present invention, the term "contact" generally refers to the contact of two or more different types of substances in any order, in any way and for any duration. Contact can occur in vivo, ex vivo, or in vitro. In some embodiments, it may mean that the RNA inhibitor or composition of the present invention directly contacts cell or tissue. In other embodiments, the term refers to making the RNA inhibitor or composition of the present invention contact cell or tissue indirectly. For example, the method of the present invention includes a method in which the subject is exposed to the

RNA inhibitor or composition of the present invention, and then the RNA inhibitor or composition contacts the cell or tissue through diffusion or any other active or passive transport process known in the art through which the compound circulates in the body.

In the present invention, the term "effective amount" or "effective dose" generally refers to an amount sufficient to achieve or at least partially achieve the desired effect. The "therapeutically effective amount" or "therapeutically effective dose" of a drug or therapeutic agent is generally any amount of drug that, when used alone or in combination with another therapeutic agent, promotes disease regression (as evidenced by a decrease in the severity of disease symptoms, an increase in the frequency and duration of asymptomatic periods of disease, or prevention of impairment or disability due to the disease). The "preventive effective amount" or "preventive effective dose" of a drug usually refers to the amount of drug that inhibits the development or recurrence of disease when administered alone or in combination with another therapeutic agent to subjects at risk of disease development or disease recurrence. A variety of methods known to those skilled in the art can be used to evaluate the ability of therapeutic or preventive agents to promote disease regression or inhibit disease development or recurrence, such as predicting efficacy in humans in human subjects during clinical trials and in animal model systems, or determining the activity of the agent in in vitro assays. In some embodiments, "effective amount" refers to the amount of RNA inhibitor that produces the expected pharmacological, therapeutic or preventive results.

In the present invention, the term "subject" generally refers to humans or non-human animals (including mammals) that need diagnosis, prognosis, improvement, prevention and/or treatment of diseases, such as humans, non-human primates (apes, gibbons, gorillas, chimpanzees, orangutans, macaques), domestic animals (dogs and cats), farm animals (poultry such as chickens and ducks, horses, cattle, goats, sheep, pigs) and experimental animals (mice, rats, rabbits, guinea pigs). Human subjects include fetal, newborn, infant, adolescent and adult subjects. Subjects include animal disease models.

In the present invention, the terms "include", "comprise", "have", "may/can", "contain" and their variants are generally intended to be open-ended transitional phrases, terms or words, which do not exclude the possibility of additional actions or structures. The term "consisting of/composed of" usually means that no other component (or similarly, feature, integer, step, etc.) can exist.

In the present invention, the term "about" usually means approximately, in the region of, roughly, or around. When the term "about" is used to refer to the range of values, the cut-off value or specific value is used to indicate that the stated value may differ by up to 10% from the enumerated value. Therefore, the term "about" can be used to cover variation from a specific value of $\pm$ 10% or less, $\pm$ 5% or less, $\pm$ 1% or less, $\pm$ 0.5% or less, or $\pm$ 0.1% or less.

It should be understood that, the term "at least" before a number or series of numbers includes the number adjacent to the term "at least", and all subsequent numbers or integers logically included, that is, "greater than or equal to". For example, the number of nucleotides in a nucleic acid molecule must be an integer. For example, "at least 15 nucleotides " means 15,16,17,18,19,20, 21 or more nucleotides, "at least 16 nucleotides" means 16, 17, 18, 19, 20, 21 or more nucleotides; "at least 17 nucleotides" means 17, 18, 19, 20, 21 or more nucleotides; and so on. When "at least" appears before a series of numbers or ranges, it should be understood that "at least" can modify each number in the series or ranges.

It should be understood that "no more than" used herein refers to a value or integer that is adjacent to the value and logically lower than that value, that is, "less than or equal to", such as from the context logic, to zero. For example, having "no more than 3 nucleotides" means having 3, 2 or 1 nucleotide(s). When "no more than" appears before a series of numbers or ranges, it should be understood that "no more than" can modify each number in the series or ranges. The range used herein includes both upper and lower limits.

**Detail of invention**

In one aspect, the present invention provides an RNA inhibitor or a pharmaceutically acceptable salt thereof for inhibiting the expression of APOC3 gene.

In some embodiments, the RNA inhibitor comprises a single-stranded oligonucleotide or doublestranded ribonucleic acid (dsRNA) molecule for inhibiting the expression of APOC3 gene in cells, such as cells of a subject (e.g., a mammal, such as a person susceptible to APOC3-related disorders such as hyperlipidemia), wherein the dsRNA comprises an antisense strand having a complementary region complementary to at least a portion of mRNA formed in the expression of APOC3 gene. The complementary region is about 15-30 nucleotides in length (e.g., about 30, 29, 28, 27, 26, 25, 24, 23, 22, 21, 20, 19, 18, 17, 16 or 15 nucleotides in length).

The dsRNA includes two RNA strands, which can be complementary and hybridize to form a duplex structure (complementary region) under the conditions in which the dsRNA uses them. One strand (antisense strand) of the dsRNA includes a complementary region that is substantially, and often completely, complementary to the target sequence. The target sequence can be derived from the sequence of mRNA formed during the expression of APOC3 gene. The other strand (sense strand) includes a region complementary to the antisense strand, so that when combined under appropriate conditions, the two strands can hybridize and form a duplex structure. Typically, duplex structures are 15 to 30 base pairs in

length. Similarly, the length of the region complementary to the target sequence is 15 to 30 nucleotides.

In some embodiments, the dsRNA is about 19 to about 23 nucleotides in length, or about 24 to about 30 nucleotides in length. In general, the dsRNA is long enough to be used as a substrate for Dicer. For example, it is known in the art that the dsRNA with a length greater than about 21-23 nucleotides can be used as a substrate for Dicer. Those skilled in the art also understand that the region of RNA targeted for cleavage is usually part of larger RNA molecules (usually mRNA molecules). A "part" of the target is consecutive nucleotides of the mRNA target, which is long enough to allow it to be a substrate for RNA inhibitor-guided cleavage (i.e., cleavage via RISC pathway).

Those skilled in the art should also understand that the duplex region is the main functional part of dsRNA, for example, the duplex region of about 19 to about 30 base pairs, such as, about 19-30, 19-29, 19-28, 19-27, 19-26, 19-25, 19-24, 19-23, 19-22, 19-21, 19-20 base pairs. Therefore, in one embodiment, in order to achieve the extent of becoming a functional duplex (for example, 15-30 base pairs) that targets the required RNA for cleavage, RNA molecules or complexes of RNA molecules with duplex regions of more than 30 base pairs are dsRNA.

In one aspect of the present invention, the RNA inhibitor or pharmaceutically acceptable salt thereof of the present invention comprises an antisense strand, wherein the antisense strand comprises at least 15 consecutive nucleotides that are substantially complementary to the nucleotides at the corresponding positions of APOC3 mRNA NM_000040.3 (SEQ ID NO. 1 : ctgctcagttcatccctagaggcagctgctccaggaacagaggtgccatgcagccccgggtactccttgttgttgccctc ctggcgctcctggcctct gcccgagcttcagaggccgaggatgcctcccttctcagcttcatgcagggttacatgaagcacgccaccaagaccgccaaggatgcac tgagcag cgtgcaggagtcccaggtggcccagcaggccaggggctgggtgaccgatggcttcagttccctgaaagactactggagcaccgttaaggacaag ttc tctgagttctgggatttggaccctgaggtcagaccaacttcagccgtggctgcctgagacctcaataccccaagtccacctgcctatccatcctgc gagctccttgggtc ctgcaatctccagggctgcccctgtaggttgcttaaaagggacagtattctcagtgctctcctaccccacctcatgcctggcccc cctccaggcatgctggcctcccaat aaagctggacaagaagctgctatga) or a sequence that differs from it by no more than 3 nucleotides.

In some embodiments, the antisense strand and the sense strand form a duplex structure (complementary region), and the complementary region includes at least 15, 16, 17, 18, 19, 20, 21, 22, or 23 consecutive nucleotides.

In some embodiments, the sense strand of the RNA inhibitor is substantially homologous to the target sequences in Table 1.

Table 1 target sequence of the RNA inhibitor

| SEQ ID NO. | target sequence 5'→3' |
|---|---|
| 2 | gcttaaaagggacagtattct |
| 3 | ggagcaccgttaaggacaagt |
| 4 | gagcaccgttaaggacaagtt |
| 5 | gcaccgttaaggacaagttct |
| 6 | gcatgctggcctcccaataaa |
| 7 | gttctctgagttctgggattt |
| 8 | ttacatgaagcacgccaccaa |
| 9 | gctgcctgagacctcaatacc |
| 10 | gtccacctgcctatccatcct |
| 11 | gatggcttcagttccctgaaa |
| 12 | tctcagcttcatgcagggtta |
| 13 | tcagcttcatgcagggttaca |
| 14 | gcttcatgcagggttacatga |
| 15 | ctctgagttctgggatttgga |
| 16 | gcttcagttccctgaaagact |
| 17 | cagttccctgaaagactactg |
| 18 | ttccctgaaagactactggag |
| 19 | aaagactactggagcaccgtt |
| 20 | agactactggagcaccgttaa |
| 21 | ccgttaaggacaagttctctg |
| 22 | ggacaagttctctgagttctg |

(continued)

| SEQ ID NO. | target sequence 5'→3' |
|---|---|
| 23 | tctgagttctgggatttggac |
| 24 | tgaggtcagaccaacttcagc |
| 25 | tgagacctcaataccccaagt |
| 26 | tatccatcctgcgagctcctt |
| 27 | ctgcccctgtaggttgcttaa |
| 28 | tgcccctgtaggttgcttaaa |
| 29 | cccctgtaggttgcttaaaag |
| 30 | agaccgccaaggatgcactga |
| 31 | taggttgcttaaaagggacag |
| 32 | cagtattctcagtgctctcct |
| 33 | tattctcagtgctctcctacc |
| 34 | tgctctcctaccccacctcat |
| 35 | gctccttgggtcctgcaatct |
| 36 | tcagaccaacttcagccgtgg |
| 37 | tggctgcctgagacctcaata |
| 38 | ttctgggatttggaccctgag |
| 39 | tgggatttggaccctgaggtc |
| 40 | atttggaccctgaggtcagac |
| 41 | agaccaacttcagccgtggct |
| 42 | ttgggtcctgcaatctccagg |
| 43 | acctgcctatccatcctgcga |
| 44 | tgcctatccatcctgcgagct |
| 45 | gagctccttgggtcctgcaat |
| 46 | acagtattctcagtgctctcc |
| 47 | acagtattctcagtgctctcc |
| 48 | tccttgggtcctgcaatctcc |
| 49 | tggaccctgaggtcagaccaa |
| 50 | accctgaggtcagaccaactt |
| 51 | cctgaggtcagaccaacttca |
| 52 | aagggacagtattctcagtgc |

where, g=guanylate, a=adenylate, c=cytidylate, t=thymidylate.

In some embodiments, the sense strand of the RNA inhibitor is substantially homologous to target sequences in Table 1, and is selected from a) sequences in Table 2, b) sequences having at least 15 consecutive nucleotides identical to those in a), and c) sequences having no more than 3 nucleotides different from those in a) and b).

Table 2 sense strand sequences of the RNA inhibitor

| SEQ ID NO. | single strand code | sense strand 5'→3' |
|---|---|---|
| 53 | S1 | gcuuaaaagggacaguauucu |
| 54 | S2 | ggagcaccguuaaggacaagu |
| 55 | S3 | gagcaccguuaaggacaaguu |

(continued)

| SEQ ID NO. | single strand code | sense strand 5'→3' |
|---|---|---|
| 56 | S4 | gcaccguuaaggacaaguucu |
| 57 | S5 | gcaugcuggccucccaauaaa |
| 58 | S6 | guucucugaguucugggauuu |
| 59 | S7 | uuacaugaagcacgccaccaa |
| 60 | S8 | gcugccugagaccucaauacc |
| 61 | S9 | guccaccugccuauccauccu |
| 62 | S10 | gauggcuucaguucccugaaa |
| 63 | S11 | ucucagcuucaugcaggguua |
| 64 | S12 | ucagcuucaugcaggguuaca |
| 65 | S13 | gcuucaugcaggguuacauga |
| 66 | S14 | cucugaguucugggauuugga |
| 67 | S15 | gcuucaguucccugaaagacu |
| 68 | S16 | caguucccugaaagacuacug |
| 69 | S17 | uucccugaaagacuacuggag |
| 70 | S18 | aaagacuacuggagcaccguu |
| 71 | S19 | agacuacuggagcaccguuaa |
| 72 | S20 | ccguuaaggacaaguucucug |
| 73 | S21 | ggacaaguucucugaguucug |
| 74 | S22 | ucugaguucugggauuuggac |
| 75 | S23 | ugaggucagaccaacuucagc |
| 76 | S24 | ugagaccucaauaccccaagu |
| 77 | S25 | uauccauccugcgagcuccuu |
| 78 | S26 | cugccccuguagguugcuuaa |
| 79 | S27 | ugccccuguagguugcuuaaa |
| 80 | S28 | ccccuguagguugcuuaaaag |
| 81 | S29 | agaccgccaaggaugcacuga |
| 82 | S30 | uagguugcuuaaaagggacag |
| 83 | S31 | caguauucucagugcucuccu |
| 84 | S32 | uauucucagugcucuccuacc |
| 85 | S33 | ugcucuccuaccccaccucau |
| 86 | S34 | gcuccuugggguccugcaaucu |
| 87 | S35 | ucagaccaacuucagccgugg |
| 88 | S36 | uggcugccugagaccucaaua |
| 89 | S37 | uucugggauuuggacccugag |
| 90 | S38 | ugggauuuggacccugagguc |
| 91 | S39 | auuuggacccugaggucagac |
| 92 | S40 | agaccaacuucagccguggcu |
| 93 | S41 | uuggguccugcaaucuccagg |
| 94 | S42 | accugccuauccauccugcga |

(continued)

| SEQ ID NO. | single strand code | sense strand 5'→3' |
|---|---|---|
| 95 | S43 | ugccuauccauccugcgagcu |
| 96 | S44 | gagcuccuugggucccugcaau |
| 97 | S45 | acaguauucucagugcucucc |
| 98 | S46 | ucucagugcucuccuacccca |
| 99 | S47 | uccuuggguccugcaaucucc |
| 100 | S48 | uggacccugaggucagaccaa |
| 101 | S49 | acccugaggucagaccaacuu |
| 102 | S50 | ccugaggucagaccaacuuca |
| 103 | S51 | aagggacaguauucucagugc |
| 701 | S193 | agggacaguauucucagugcu |
| 702 | S194 | gcuuaaaagggacaguauuca |
| 703 | S195 | cugcccgagcuucagaggcuu |
| 704 | S196 | cccgagcuucagaggccgauu |
| 705 | S197 | ccgagcuucagaggccgagga |
| 706 | S198 | cgagcuucagaggccgaggau |
| 707 | S199 | gagcuucagaggccgaggaua |
| 708 | S200 | agcuucagaggccgaggauaa |
| 709 | S201 | gcuucagaggccgaggaugaa |
| 710 | S202 | cuucagaggccgaggaugccu |
| 711 | S203 | ucagaggccgaggaugccucu |
| 712 | S204 | aggccgaggaugccucccuuc |
| 713 | S205 | gccgaggaugccucccuucuc |
| 714 | S206 | cgaggaugccucccuucucag |
| 715 | S207 | gaggaugccucccuucucaga |
| 716 | S208 | aggaugccucccuucucagcu |
| 717 | S209 | gaugccucccuucucagcuua |
| 718 | S210 | augccucccuucucagcuuca |
| 719 | S211 | gccucccuucucagcuucaug |
| 720 | S212 | ccucccuucucagcuucauaa |
| 721 | S213 | cucccuucucagcuucaugca |
| 722 | S214 | ucccuucucagcuucaugcag |
| 723 | S215 | cccuucucagcuucaugcauu |
| 724 | S216 | ccuucucagcuucaugcagaa |
| 725 | S217 | cuucucagcuucaugcagggu |
| 726 | S218 | uucucagcuucaugcaggguu |
| 727 | S219 | ucucagcuucaugcaggguuc |
| 728 | S220 | cucagcuucaugcaggguuac |
| 729 | S221 | cagcuucaugcaggguuacag |
| 730 | S222 | agcuucaugcaggguuacaug |

(continued)

| SEQ ID NO. | single strand code | sense strand 5'→3' |
|---|---|---|
| 731 | S223 | cuucaugcaggguuacaugaa |
| 732 | S224 | uucaugcaggguuacaugaag |
| 733 | S225 | ucaugcaggguuacaugaaga |
| 734 | S226 | caugcaggguuacaugaagca |
| 735 | S227 | augcaggguuacaugaagcac |
| 736 | S228 | ugcaggguuacaugaagcauu |
| 737 | S229 | gcaggguuacaugaagcacuu |
| 738 | S230 | caggguuacaugaagcacgaa |
| 739 | S231 | aggguuacaugaagcacgcaa |
| 740 | S232 | ggguuacaugaagcacgccac |
| 741 | S233 | gguuacaugaagcacgccaca |
| 742 | S234 | guuacaugaagcacgccacca |
| 743 | S235 | uacaugaagcacgccaccaag |
| 744 | S236 | caugaagcacgccaccaagac |
| 745 | S237 | augaagcacgccaccaagaca |
| 746 | S238 | ugaagcacgccaccaagacca |
| 747 | S239 | gaagcacgccaccaagaccuu |
| 748 | S240 | cacgccaccaagaccgccaau |
| 749 | S241 | acgccaccaagaccgccaauu |
| 750 | S242 | cgccaccaagaccgccaagga |
| 751 | S243 | gccaccaagaccgccaaggau |
| 752 | S244 | ccaccaagaccgccaaggaug |
| 753 | S245 | caccaagaccgccaaggauaa |
| 754 | S246 | accaagaccgccaaggaugca |
| 755 | S247 | ccaagaccgccaaggaugcac |
| 756 | S248 | caagaccgccaaggaugcaaa |
| 757 | S249 | aagaccgccaaggaugcacua |
| 758 | S250 | gaccgccaaggaugcacugag |
| 759 | S251 | accgccaaggaugcacugagu |
| 760 | S252 | ccgccaaggaugcacugagca |
| 761 | S253 | cgccaaggaugcacugagcag |
| 762 | S254 | gccaaggaugcacugagcauu |
| 763 | S255 | ccaaggaugcacugagcagcg |
| 764 | S256 | caaggaugcacugagcagcgu |
| 765 | S257 | aaggaugcacugagcagcgug |
| 766 | S258 | aggaugcacugagcagcguuu |
| 767 | S259 | ggaugcacugagcagcgugca |
| 768 | S260 | gaugcacugagcagcgugcag |
| 769 | S261 | augcacugagcagcgugcauu |

(continued)

| SEQ ID NO. | single strand code | sense strand 5'→3' |
|---|---|---|
| 770 | S262 | ugcacugagcagcgugcagga |
| 771 | S263 | gcacugagcagcgugcaggau |
| 772 | S264 | cacugagcagcgugcaggagu |
| 773 | S265 | acugagcagcgugcaggaguc |
| 774 | S266 | cugagcagcgugcaggaguaa |
| 775 | S267 | ugagcagcgugcaggagucaa |
| 776 | S268 | gagcagcgugcaggaguccaa |
| 777 | S269 | gcagcgugcaggagucccagu |
| 778 | S270 | cagcgugcaggagucccaggu |
| 779 | S271 | gcgugcaggagucccagguga |
| 780 | S272 | cgugcaggagucccaggugaa |
| 781 | S273 | aagggacaguauucucaguga |
| 782 | S274 | gcaggagucccagguggccua |
| 783 | S275 | aggagucccagguggcccauu |
| 784 | S276 | agucccagguggcccagcauu |
| 785 | S277 | agcaggccaggggcuggguaa |
| 786 | S278 | caggccaggggcuggggugaca |
| 787 | S279 | ccaggggcugggugaccgauc |
| 788 | S280 | caggggcugggugaccgaucu |
| 789 | S281 | gggcugggugaccgauggcuu |
| 790 | S282 | gcugggugaccgauggcuuca |
| 791 | S283 | ugggugaccgauggcuucagu |
| 792 | S284 | ggugaccgauggcuucagugu |
| 793 | S285 | gugaccgauggcuucaguuaa |
| 794 | S286 | ugaccgauggcuucaguucau |
| 795 | S287 | gaccgauggcuucaguuccau |
| 796 | S288 | ccgauggcuucaguucccuga |
| 797 | S289 | cgauggcuucaguucccugaa |
| 798 | S290 | gauggcuucaguucccugaaa |
| 799 | S291 | auggcuucaguucccugaaag |
| 800 | S292 | uggcuucaguucccugaaauu |
| 801 | S293 | ggcuucaguucccugaaagac |
| 802 | S294 | cuucaguucccugaaagacua |
| 803 | S295 | uucaguucccugaaagacuau |
| 804 | S296 | ucaguucccugaaagacuacu |
| 805 | S297 | aguucccugaaagacuacuga |
| 806 | S298 | guucccugaaagacuacugga |
| 807 | S299 | ucccugaaagacuacuggauu |
| 808 | S300 | cccugaaagacuacuggagca |

(continued)

| SEQ ID NO. | single strand code | sense strand 5'→3' |
|---|---|---|
| 809 | S301 | ccugaaagacuacuggagcac |
| 810 | S302 | cugaaagacuacuggagcauu |
| 811 | S303 | ugaaagacuacuggagcacua |
| 812 | S304 | gaaagacuacuggagcaccgu |
| 813 | S305 | aagacuacuggagcaccguua |
| 814 | S306 | gacuacuggagcaccguuaau |
| 815 | S307 | acuacuggagcaccguuaagu |
| 816 | S308 | cuacuggagcaccguuaagga |
| 817 | S309 | uacuggagcaccguuaaggau |
| 818 | S310 | acuggagcaccguuaaggaaa |
| 819 | S311 | cuggagcaccguuaaggacau |
| 820 | S312 | uggagcaccguuaaggacaag |
| 821 | S313 | agcaccguuaaggacaaguua |
| 822 | S314 | gcaccguuaaggacaaguucu |
| 823 | S315 | caccguuaaggacaaguucuu |
| 824 | S316 | accguuaaggacaaguucucu |
| 825 | S317 | cguuaaggacaaguucucuga |
| 826 | S318 | guuaaggacaaguucucugau |
| 827 | S319 | uuaaggacaaguucucugagu |
| 828 | S320 | uaaggacaaguucucugaguu |
| 829 | S321 | aaggacaaguucucugaguuc |
| 830 | S322 | ggacaaguucucugaguucua |
| 831 | S323 | gacaaguucucugaguucuga |
| 832 | S324 | acaaguucucugaguucugaa |
| 833 | S325 | caaguucucugaguucuggga |
| 834 | S326 | aaguucucugaguucugggau |
| 835 | S327 | aguucucugaguucugggauu |
| 836 | S328 | uucucugaguucugggauuuc |
| 837 | S329 | ucucugaguucugggauuuaa |
| 838 | S330 | ugaguucugggauuuggacaa |
| 839 | S331 | gaguucugggauuuggaccau |
| 840 | S332 | aguucugggauuuggacccua |
| 841 | S333 | guucugggauuuggacccuaa |
| 842 | S334 | ucugggauuuggacccugaau |
| 843 | S335 | cugggauuuggacccugagau |
| 844 | S336 | gggauuuggacccugagguca |
| 845 | S337 | ggauuuggacccugaggucaa |
| 846 | S338 | gauuuggacccugaggucaga |
| 847 | S339 | uuuggacccugaggucagaaa |

(continued)

| SEQ ID NO. | single strand code | sense strand 5'→3' |
|---|---|---|
| 848 | S340 | uuggacccugaggucagacua |
| 849 | S341 | gacccugaggucagaccaacu |
| 850 | S342 | cccugaggucagaccaacuuc |
| 851 | S343 | cugaggucagaccaacuucag |
| 852 | S344 | gaggucagaccaacuucagaa |
| 853 | S345 | aggucagaccaacuucagcaa |
| 854 | S346 | ggucagaccaacuucagccuu |
| 855 | S347 | gucagaccaacuucagccguu |
| 856 | S348 | ucagaccaacuucagccguaa |
| 857 | S349 | cagaccaacuucagccgugaa |
| 858 | S350 | gaccaacuucagccguggcua |
| 859 | S351 | accaacuucagccguggcuuu |
| 860 | S352 | ccaacuucagccguggcugaa |
| 861 | S353 | caacuucagccguggcugcua |
| 862 | S354 | acuucagccguggcugccuga |
| 863 | S355 | uucagccguggcugccugaga |
| 864 | S356 | guggcugccugagaccucaau |
| 865 | S357 | ggcugccugagaccucaauac |
| 866 | S358 | cugccugagaccucaauacuu |
| 867 | S359 | ugccugagaccucaauaccaa |
| 868 | S360 | gccugagaccucaauacccaa |
| 869 | S361 | ccugagaccucaauaccccau |
| 870 | S362 | cugagaccucaauaccccaau |
| 871 | S363 | gagaccucaauaccccaaguc |
| 872 | S364 | agaccucaauaccccaaguuu |
| 873 | S365 | gaccucaauaccccaagucca |
| 874 | S366 | accucaauaccccaaguccac |
| 875 | S367 | ccucaauaccccaaguccauu |
| 876 | S368 | cucaauaccccaaguccaccu |
| 877 | S369 | ucaauaccccaaguccaccua |
| 878 | S370 | aauaccccaaguccaccugaa |
| 879 | S371 | uaccccaaguccaccugccua |
| 880 | S372 | accccaaguccaccugccuau |
| 881 | S373 | cccaaguccaccugccuauaa |
| 882 | S374 | ccaaguccaccugccuaucca |
| 883 | S375 | caaguccaccugccuauccau |
| 884 | S376 | aaguccaccugccuauccauc |
| 885 | S377 | aguccaccugccuauccauaa |
| 886 | S378 | uccaccugccuauccauccuu |

(continued)

| SEQ ID NO. | single strand code | sense strand 5'→3' |
|---|---|---|
| 887 | S379 | ccaccugccuauccauccugu |
| 888 | S380 | caccugccuauccauccugaa |
| 889 | S381 | ccugccuauccauccugcgag |
| 890 | S382 | cugccuauccauccugcgauu |
| 891 | S383 | ugccuauccauccugcgagcu |
| 892 | S384 | ccuauccauccugcgagcucu |
| 893 | S385 | cuauccauccugcgagcuccu |
| 894 | S386 | auccauccugcgagcuccuua |
| 895 | S387 | uccauccugcgagcuccuuga |
| 896 | S388 | ccauccugcgagcuccuuguu |
| 897 | S389 | cauccugcgagcuccuuggau |
| 898 | S390 | auccugcgagcuccuugggUc |
| 899 | S391 | uccugcgagcuccuuggguaa |
| 900 | S392 | ccugcgagcuccuuggguccu |
| 901 | S393 | cugcgagcuccuuggguccua |
| 902 | S394 | ugcgagcuccuuggguccuaa |
| 903 | S395 | agcuccuuggguccugcaauu |
| 904 | S396 | gcuccuuggguccugcaauaa |
| 905 | S397 | cuccuuggguccugcaaucua |
| 906 | S398 | ccuuggguccugcaaucucuu |
| 907 | S399 | cuuggguccugcaaucuccau |
| 908 | S400 | uggguccugcaaucuccagaa |
| 909 | S401 | ggguccugcaaucuccaggaa |
| 910 | S402 | gguccugcaaucuccaggguu |
| 911 | S403 | guccugcaaucuccagggcua |
| 912 | S404 | uccugcaaucuccagggcuaa |
| 913 | S405 | ccugcaaucuccagggcugaa |
| 914 | S406 | cugcaaucuccagggcugcau |
| 915 | S407 | ugcaaucuccagggcugccaa |
| 916 | S408 | aaucuccagggcugccccuua |
| 917 | S409 | agggcugccccuguagguuga |
| 918 | S410 | gcugccccuguagguugcuua |
| 919 | S411 | ugccccuguagguugcuuaaa |
| 920 | S412 | cuguagguugcuuaaaaggga |
| 921 | S413 | uguagguugcuuaaaagggaa |
| 922 | S414 | agguugcuuaaaagggacauu |
| 923 | S415 | guugcuuaaaagggacaguau |
| 924 | S416 | uugcuuaaaagggacaguauc |
| 925 | S417 | ugcuuaaaagggacaguaucu |

(continued)

| SEQ ID NO. | single strand code | sense strand 5'→3' |
|---|---|---|
| 926 | S418 | cuuaaaagggacaguauucua |
| 927 | S419 | uaaaagggacaguauucucaa |
| 928 | S420 | aaaagggacaguauucucauu |
| 929 | S421 | aaagggacaguauucucagaa |
| 930 | S422 | agggacaguauucucaguguu |
| 931 | S423 | gggacaguauucucagugcuu |
| 932 | S424 | gacaguauucucagugcucuu |
| 933 | S425 | aguauucucagugcucuccaa |
| 934 | S426 | guauucucagugcucuccuaa |
| 935 | S427 | auucucagugcucuccuacuu |
| 936 | S428 | cucagugcucuccuaccccaa |
| 937 | S429 | ucagugcucuccuaccccauu |
| 938 | S430 | agugcucuccuaccccaccua |
| 939 | S431 | gugcucuccuaccccaccuaa |
| 940 | S432 | gcucuccuaccccaccucauu |
| 941 | S433 | cucuccuaccccaccucauau |
| 942 | S434 | cuccuaccccaccucaugcau |
| 943 | S435 | ccuaccccaccucaugccuaa |
| 944 | S436 | uaccccaccucaugccugguc |
| 945 | S437 | ccuccaggcaugcuggccuaa |
| 946 | S438 | uccaggcaugcuggccuccua |
| 947 | S439 | aagggacaguauuctcagugu |
| 948 | S440 | gagggacaguauucucagugu |
| 949 | S441 | cagggacaguauucucagugu |
| 950 | S442 | gggacaguauucucagugcua |
| 951 | S443 | gagcgacaguauucucagugu |
| 952 | S444 | uagggacaguauucucagugu |
| 953 | S445 | augggacaguauucucagugu |
| 954 | S446 | agggacaguauucucagugua |
| 955 | S447 | aagggacaguauucucagu |
| 956 | S448 | gcuuaaaagggacaguauuca |
| 957 | S449 | gcuuaaaagggacaguauucu |
| 958 | S450 | cuuaaaagggacaguauucuc |
| 959 | S451 | uuaaaagggacaguauucuca |
| 960 | S452 | uaaaagggacaguauucucag |
| 961 | S453 | aaaagggacaguauucucagc |
| 962 | S454 | aaagggacaguauucucagug |
| 963 | S455 | uaaagggacaguauucucagu |
| 964 | S456 | aagcgacaguauucucagugu |

where, g=guanylate, a=adenylate, u=uridylate, c=cytidylate.

In some embodiments, the antisense strand of the RNA inhibitor is selected from a) the following sequences, b) sequences having at least 15 consecutive nucleotides identical to those in a), and c) sequences having no more than 3 nucleotides different from those in a) and b).

Table3 antisense strand sequences of the RNA inhibitor

| SEQ ID NO. | single strand code | antisense strand sequence 5'→3' |
|---|---|---|
| 104 | AS1 | agaauacugucccuuuuaagc |
| 105 | AS2 | acuuguccuuaacggugcucc |
| 106 | AS3 | aacuuguccuuaacggugcuc |
| 107 | AS4 | agaacuuguccuuaacggugc |
| 108 | AS5 | uuuauugggaggccagcaugc |
| 109 | AS6 | aaaucccagaacucagagaac |
| 110 | AS7 | uugguggcgugcuucauguaa |
| 111 | AS8 | gguauugaggucucaggcagc |
| 112 | AS9 | aggauggauaggcagguggac |
| 113 | AS10 | uuucagggaacugaagccauc |
| 114 | AS11 | uaacccugcaugaagcugaga |
| 115 | AS12 | uguaacccugcaugaagcuga |
| 116 | AS13 | ucauguaacccugcaugaagc |
| 117 | AS14 | uccaaaucccagaacucagag |
| 118 | AS15 | agucuuucagggaacugaagc |
| 119 | AS16 | caguagucuuucagggaacug |
| 120 | AS17 | cuccaguagucuuucagggaa |
| 121 | AS18 | aacggugcuccaguagucuuu |
| 122 | AS19 | uuaacggugcuccaguagucu |
| 123 | AS20 | cagagaacuuguccuuaacgg |
| 124 | AS21 | cagaacucagagaacuugucc |
| 125 | AS22 | guccaaaucccagaacucaga |
| 126 | AS23 | gcugaaguuggucugaccuca |
| 127 | AS24 | acuuggggguauugaggucuca |
| 128 | AS25 | aaggagcucgcaggauggaua |
| 129 | AS26 | uuaagcaaccuacaggggcag |
| 130 | AS27 | uuuaagcaaccuacaggggca |
| 131 | AS28 | cuuuuaagcaaccuacagggg |
| 132 | AS29 | ucagugcauccuuggcggucu |
| 133 | AS30 | cugucccuuuuaagcaaccua |
| 134 | AS31 | aggagagcacugagaauacug |
| 135 | AS32 | gguaggagagcacugagaaua |
| 136 | AS33 | augaggugggguaggagagca |
| 137 | AS34 | agauugcaggacccaaggagc |
| 138 | AS35 | ccacggcugaaguuggucuga |
| 139 | AS36 | uauugaggucucaggcagcca |

(continued)

| SEQ ID NO. | single strand code | antisense strand sequence 5'→3' |
|---|---|---|
| 140 | AS37 | cucaggguccaaaucccagaa |
| 141 | AS38 | gaccucaggguccaaauccca |
| 142 | AS39 | gucugaccucaggguccaaau |
| 143 | AS40 | agccacggcugaaguuggucu |
| 144 | AS41 | ccuggagauugcaggacccaa |
| 145 | AS42 | ucgcaggauggauaggcaggu |
| 146 | AS43 | agcucgcaggauggauaggca |
| 147 | AS44 | auugcaggacccaaggagcuc |
| 148 | AS45 | ggagagcacugagaauacugu |
| 149 | AS46 | ugggguaggagagcacugaga |
| 150 | AS47 | ggagauugcaggacccaagga |
| 151 | AS48 | uuggucugaccucaggucca |
| 152 | AS49 | aaguuggucugaccucagggu |
| 153 | AS50 | ugaaguuggucugaccucagg |
| 154 | AS51 | gcacugagaauacugucccuuuu |
| 437 | AS193 | cacugagaauacugucccuua |
| 438 | AS194 | uaauacugucccuuuuaagcaa |
| 439 | AS195 | gccucugaagcucgggcagag |
| 440 | AS196 | ucggccucugaagcucgggca |
| 441 | AS197 | cucggccucugaagcucgggc |
| 442 | AS198 | ccucggccucugaagcucggg |
| 443 | AS199 | uccucggccucugaagcucgg |
| 444 | AS200 | auccucggccucugaagcucg |
| 445 | AS201 | cauccucggccucugaagcuc |
| 446 | AS202 | gcauccucggccucugaagcu |
| 447 | AS203 | aggcauccucggccucugaag |
| 448 | AS204 | agggaggcauccucggccucu |
| 449 | AS205 | gaagggaggcauccucggccu |
| 450 | AS206 | gagaagggaggcauccucggc |
| 451 | AS207 | ugagaagggaggcauccucgg |
| 452 | AS208 | cugagaagggaggcauccucg |
| 453 | AS209 | agcugagaagggaggcauccu |
| 454 | AS210 | aagcugagaagggaggcaucc |
| 455 | AS211 | ugaagcugagaagggaggcau |
| 456 | AS212 | augaagcugagaagggaggca |
| 457 | AS213 | caugaagcugagaagggaggc |
| 458 | AS214 | gcaugaagcugagaagggagg |
| 459 | AS215 | ugcaugaagcugagaagggag |
| 460 | AS216 | cugcaugaagcugagaaggga |

(continued)

| SEQ ID NO. | single strand code | antisense strand sequence 5'→3' |
|---|---|---|
| 461 | AS217 | ccugcaugaagcugagaaggg |
| 462 | AS218 | cccugcaugaagcugagaagg |
| 463 | AS219 | acccugcaugaagcugagaag |
| 464 | AS220 | aacccugcaugaagcugagaa |
| 465 | AS221 | guaacccugcaugaagcugag |
| 466 | AS222 | uguaacccugcaugaagcuga |
| 467 | AS223 | cauguaacccugcaugaagcu |
| 468 | AS224 | ucauguaacccugcaugaagc |
| 469 | AS225 | uucauguaacccugcaugaag |
| 470 | AS226 | cuucauguaacccugcaugaa |
| 471 | AS227 | gcuucauguaacccugcauga |
| 472 | AS228 | ugcuucauguaacccugcaug |
| 473 | AS229 | gugcuucauguaacccugcau |
| 474 | AS230 | cgugcuucauguaacccugca |
| 475 | AS231 | gcgugcuucauguaacccugc |
| 476 | AS232 | ggcgugcuucauguaacccug |
| 477 | AS233 | uggcgugcuucauguaacccu |
| 478 | AS234 | guggcgugcuucauguaaccc |
| 479 | AS235 | ugguggcgugcuucauguaac |
| 480 | AS236 | cuugguggcgugcuucaugua |
| 481 | AS237 | ucuugguggcgugcuucaugu |
| 482 | AS238 | gucuugguggcgugcuucaug |
| 483 | AS239 | ggucuugguggcgugcuucau |
| 484 | AS240 | uggcggucuugguggcgugcu |
| 485 | AS241 | uuggcggucuugguggcgugc |
| 486 | AS242 | cuuggcggucuugguggcgug |
| 487 | AS243 | ccuuggcggucuugguggcgu |
| 488 | AS244 | uccuuggcggucuugguggcg |
| 489 | AS245 | auccuuggcggucuugguggc |
| 490 | AS246 | cauccuuggcggucuuggugg |
| 491 | AS247 | gcauccuuggcggucuuggug |
| 492 | AS248 | ugcauccuuggcggucuuggu |
| 493 | AS249 | gugcauccuuggcggucuugg |
| 494 | AS250 | cagugcauccuuggcggucuu |
| 495 | AS251 | ucagugcauccuuggcggucu |
| 496 | AS252 | cucagugcauccuuggcgguc |
| 497 | AS253 | gcucagugcauccuuggcggu |
| 498 | AS254 | ugcucagugcauccuuggcgg |
| 499 | AS255 | cugcucagugcauccuuggcg |

(continued)

| SEQ ID NO. | single strand code | antisense strand sequence 5'→3' |
|---|---|---|
| 500 | AS256 | gcugcucagugcauccuuggc |
| 501 | AS257 | cgcugcucagugcauccuugg |
| 502 | AS258 | acgcugcucagugcauccuug |
| 503 | AS259 | cacgcugcucagugcauccuu |
| 504 | AS260 | gcacgcugcucagugcauccu |
| 505 | AS261 | ugcacgcugcucagugcaucc |
| 506 | AS262 | cugcacgcugcucagugcauc |
| 507 | AS263 | ccugcacgcugcucagugcau |
| 508 | AS264 | uccugcacgcugcucagugca |
| 509 | AS265 | cuccugcacgcugcucagugc |
| 510 | AS266 | acuccugcacgcugcucagug |
| 511 | AS267 | gacuccugcacgcugcucagu |
| 512 | AS268 | ggacuccugcacgcugcucag |
| 513 | AS269 | ugggacuccugcacgcugcuc |
| 514 | AS270 | cugggacuccugcacgcugcu |
| 515 | AS271 | accugggacuccugcacgcug |
| 516 | AS272 | caccugggacuccugcacgcu |
| 517 | AS273 | ccaccugggacuccugcacgc |
| 518 | AS274 | ggccaccugggacuccugcac |
| 519 | AS275 | ugggccaccugggacuccugc |
| 520 | AS276 | ugcugggccaccugggacucc |
| 521 | AS277 | acccagccccuggccugcugg |
| 522 | AS278 | ucacccagccccuggccugcu |
| 523 | AS279 | ucggucacccagccccuggcc |
| 524 | AS280 | aucggucacccagccccuggc |
| 525 | AS281 | gccaucggucacccagccccu |
| 526 | AS282 | aagccaucggucacccagccc |
| 527 | AS283 | ugaagccaucggucacccagc |
| 528 | AS284 | acugaagccaucggucaccca |
| 529 | AS285 | aacugaagccaucggucaccc |
| 530 | AS286 | gaacugaagccaucggucacc |
| 531 | AS287 | ggaacugaagccaucggucac |
| 532 | AS288 | agggaacugaagccaucgguc |
| 533 | AS289 | cagggaacugaagccaucggu |
| 534 | AS290 | ucagggaacugaagccaucgg |
| 535 | AS291 | uucagggaacugaagccaucg |
| 536 | AS292 | uuucagggaacugaagccauc |
| 537 | AS293 | cuuucagggaacugaagccau |
| 538 | AS294 | gucuuucagggaacugaagcc |

(continued)

| SEQ ID NO. | single strand code | antisense strand sequence 5'→3' |
|---|---|---|
| 539 | AS295 | agucuuucagggaacugaagc |
| 540 | AS296 | uagucuuucagggaacugaag |
| 541 | AS297 | aguagucuuucagggaacuga |
| 542 | AS298 | caguagucuuucagggaacug |
| 543 | AS299 | uccaguagucuuucagggaac |
| 544 | AS300 | cuccaguagucuuucagggaa |
| 545 | AS301 | gcuccaguagucuuucaggga |
| 546 | AS302 | ugcuccaguagucuuucaggg |
| 547 | AS303 | gugcuccaguagucuuucagg |
| 548 | AS304 | ggugcuccaguagucuuucag |
| 549 | AS305 | acggugcuccaguagucuuuc |
| 550 | AS306 | uaacggugcuccaguagucuu |
| 551 | AS307 | uuaacggugcuccaguagucu |
| 552 | AS308 | cuuaacggugcuccaguaguc |
| 553 | AS309 | ccuuaacggugcuccaguagu |
| 554 | AS310 | uccuuaacggugcuccaguag |
| 555 | AS311 | guccuuaacggugcuccagua |
| 556 | AS312 | uguccuuaacggugcuccagu |
| 557 | AS313 | acuuguccuuaacggugcucc |
| 558 | AS314 | aacuuguccuuaacggugcuc |
| 559 | AS315 | gaacuuguccuuaacggugcu |
| 560 | AS316 | agaacuuguccuuaacggugc |
| 561 | AS317 | agagaacuuguccuuaacggu |
| 562 | AS318 | cagagaacuuguccuuaacgg |
| 563 | AS319 | ucagagaacuuguccuuaacg |
| 564 | AS320 | cucagagaacuuguccuuaac |
| 565 | AS321 | acucagagaacuuguccuuaa |
| 566 | AS322 | gaacucagagaacuuguccuu |
| 567 | AS323 | agaacucagagaacuuguccu |
| 568 | AS324 | cagaacucagagaacuugucc |
| 569 | AS325 | ccagaacucagagaacuuguc |
| 570 | AS326 | cccagaacucagagaacuugu |
| 571 | AS327 | ucccagaacucagagaacuug |
| 572 | AS328 | aaucccagaacucagagaacu |
| 573 | AS329 | aaaucccagaacucagagaac |
| 574 | AS330 | guccaaaucccagaacucaga |
| 575 | AS331 | gguccaaaucccagaacucag |
| 576 | AS332 | ggguccaaaucccagaacuca |
| 577 | AS333 | aggguccaaaucccagaacuc |

(continued)

| SEQ ID NO. | single strand code | antisense strand sequence 5'→3' |
|---|---|---|
| 578 | AS334 | ucaggguccaaaucccagaac |
| 579 | AS335 | cucaggguccaaaucccagaa |
| 580 | AS336 | accucaggguccaaaucccag |
| 581 | AS337 | gaccucaggguccaaauccca |
| 582 | AS338 | ugaccucaggguccaaauccc |
| 583 | AS339 | ucugaccucaggguccaaauc |
| 584 | AS340 | gucugaccucaggguccaaau |
| 585 | AS341 | uuggucugaccucaggaucca |
| 586 | AS342 | aguuggucugaccucaggguc |
| 587 | AS343 | gaaguuggucugaccucaggg |
| 588 | AS344 | cugaaguuggucugaccucag |
| 589 | AS345 | gcugaaguuggucugaccuca |
| 590 | AS346 | ggcugaaguuggucugaccuc |
| 591 | AS347 | cggcugaaguuggucugaccu |
| 592 | AS348 | acggcugaaguuggucugacc |
| 593 | AS349 | cacggcugaaguuggucugac |
| 594 | AS350 | gccacggcugaaguuggucug |
| 595 | AS351 | agccacggcugaaguugglucu |
| 596 | AS352 | cagccacggcugaaguugguc |
| 597 | AS353 | gcagccacggcugaaguuggu |
| 598 | AS354 | aggcagccacggcugaaguug |
| 599 | AS355 | ucaggcagccacggcugaagu |
| 600 | AS356 | ugaggucucaggcagccacgg |
| 601 | AS357 | auugaggucucaggcagccac |
| 602 | AS358 | guauugaggucucaggcagcc |
| 603 | AS359 | gguauugaggucucaggcagc |
| 604 | AS360 | ggguauugaggucucaggcag |
| 605 | AS361 | gggguauugaggucucaggca |
| 606 | AS362 | ugggguauugaggucucaggc |
| 607 | AS363 | cuugggguauugaggucucag |
| 608 | AS364 | acuugggguauugaggucuca |
| 609 | AS365 | gacuugggguauugaggucuc |
| 610 | AS366 | ggacuugggguauugaggucu |
| 611 | AS367 | uggacuugggguauugagguc |
| 612 | AS368 | guggacuugggguauugaggu |
| 613 | AS369 | gguggacuugggguauugagg |
| 614 | AS370 | cagguggacuugggguauuga |
| 615 | AS371 | ggcagguggacuugggguauu |
| 616 | AS372 | aggcagguggacuugggguau |

(continued)

| SEQ ID NO. | single strand code | antisense strand sequence 5'→3' |
|---|---|---|
| 617 | AS373 | auaggcagguggacuuggggu |
| 618 | AS374 | gauaggcagguggacuugggg |
| 619 | AS375 | ggauaggcagguggacuuggg |
| 620 | AS376 | uggauaggcagguggacuugg |
| 621 | AS377 | auggauaggcagguggacuug |
| 622 | AS378 | ggauggauaggcagguggacu |
| 623 | AS379 | aggauggauaggcagguggac |
| 624 | AS380 | caggauggauaggcaggugga |
| 625 | AS381 | cgcaggauggauaggcaggug |
| 626 | AS382 | ucgcaggauggauaggcaggu |
| 627 | AS383 | cucgcaggauggauaggcagg |
| 628 | AS384 | agcucgcaggauggauaggca |
| 629 | AS385 | gagcucgcaggauggauaggc |
| 630 | AS386 | aggagcucgcaggauggauag |
| 631 | AS387 | aaggagcucgcaggauggaua |
| 632 | AS388 | caaggagcucgcaggauggau |
| 633 | AS389 | ccaaggagcucgcaggaugga |
| 634 | AS390 | cccaaggagcucgcaggaugg |
| 635 | AS391 | acccaaggagcucgcaggaug |
| 636 | AS392 | gacccaaggagcucgcaggau |
| 637 | AS393 | ggacccaaggagcucgcagga |
| 638 | AS394 | aggacccaaggagcucgcagg |
| 639 | AS395 | uugcaggacccaaggagcucg |
| 640 | AS396 | auugcaggacccaaggagcuc |
| 641 | AS397 | gauugcaggacccaaggagcu |
| 642 | AS398 | gagauugcaggacccaaggag |
| 643 | AS399 | ggagauugcaggacccaagga |
| 644 | AS400 | cuggagauugcaggacccaag |
| 645 | AS401 | ccuggagauugcaggacccaa |
| 646 | AS402 | cccuggagauugcaggaccca |
| 647 | AS403 | gcccuggagauugcaggaccc |
| 648 | AS404 | agcccuggagauugcaggacc |
| 649 | AS405 | cagcccuggagauugcaggac |
| 650 | AS406 | gcagcccuggagauugcagga |
| 651 | AS407 | ggcagcccuggagauugcagg |
| 652 | AS408 | aggggcagcccuggagauugc |
| 653 | AS409 | aaccuacaggggcagcccugg |
| 654 | AS410 | agcaaccuacaggggcagccc |
| 655 | AS411 | uaagcaaccuacaggggcagc |

(continued)

| SEQ ID NO. | single strand code | antisense strand sequence 5'→3' |
|---|---|---|
| 656 | AS412 | ccuuuuaagcaaccuacaggg |
| 657 | AS413 | cccuuuuaagcaaccuacagg |
| 658 | AS414 | ugucccuuuuaagcaaccuac |
| 659 | AS415 | acugucccuuuuaagcaaccu |
| 660 | AS416 | uacugucccuuuuaagcaacc |
| 661 | AS417 | auacugucccuuuuaagcaac |
| 662 | AS418 | gaauacugucccuuuuaagca |
| 663 | AS419 | gagaauacugucccuuuuaag |
| 664 | AS420 | ugagaauacugucccuuuuaa |
| 665 | AS421 | cugagaauacugucccuuuua |
| 666 | AS422 | cacugagaauacugucccuuu |
| 667 | AS423 | gcacugagaauacugucccuu |
| 668 | AS424 | gagcacugagaauacugucc c |
| 669 | AS425 | ggagagcacugagaauacugu |
| 670 | AS426 | aggagagcacugagaauacug |
| 671 | AS427 | guaggagagcacugagaauac |
| 672 | AS428 | gggguaggagagcacugagaa |
| 673 | AS429 | uggggguaggagagcacugaga |
| 674 | AS430 | ggugggguaggagagcacuga |
| 675 | AS431 | aggugggguaggagagcacug |
| 676 | AS432 | ugaggugggguaggagagcac |
| 677 | AS433 | augaggugggguaggagagca |
| 678 | AS434 | gcaugaggugggguaggagag |
| 679 | AS435 | aggcaugaggugggguaggag |
| 680 | AS436 | ccaggcaugaggugggguagg |
| 681 | AS437 | aggccagcaugccuggagggg |
| 682 | AS438 | ggaggccagcaugccuggagg |
| 683 | AS439 | acacugagaauacugucccuu |
| 684 | AS440 | acacugagaauacugucccuc |
| 685 | AS441 | acacugagaauacugucccug |
| 686 | AS442 | gcacugagaauacugucccuu |
| 687 | AS443 | acacugagaauacugucgcuc |
| 688 | AS444 | acacugagaauacugucccua |
| 689 | AS445 | acacugagaauacugucccau |
| 690 | AS446 | ucacugagaauacugucccuu |
| 691 | AS447 | ucacugagaauacugucccuu |
| 692 | AS448 | ugaauacugucccuuuuaagc |
| 693 | AS449 | aauacugucccuuuuaagcaa |
| 694 | AS450 | gaauacugucccuuuuaagca |

(continued)

| SEQ ID NO. | single strand code | antisense strand sequence 5'→3' |
|---|---|---|
| 695 | AS451 | agaauacugucccuuuuaagc |
| 696 | AS452 | gagaauacugucccuuuuaag |
| 697 | AS453 | ugagaauacugucccuuuuaa |
| 698 | AS454 | cugagaauacugucccuuuua |
| 699 | AS455 | acugagaauacugucccuuua |
| 700 | AS456 | acacugagaauacugucgcuu |

where, g=guanylate, a=adenylate, u=uridylate, c=cytidylate.

In some screened embodiments, the RNA inhibitor is selected from a) a combination of a sense strand in Table 2 and an antisense strand in Table 3, b) sequences having at least 15 consecutive nucleotides identical to those in a), and c) sequences having no more than 3 nucleotides different from those in a) and b).

In some embodiments, the RNA inhibitor is selected from a) sequences in Table 5, b) sequences having at least 15 consecutive nucleotides identical to those in a), and c) sequences having no more than 3 nucleotides different from those in a) and b).

Table 5 RNA inhibitors

| RNA inhibitor | sense strand code | sense strand SEQ ID NO. | antisense strand code | antisense strand SEQ ID NO. |
|---|---|---|---|---|
| Ky-12-DS67 | S193 | 701 | AS 193 | 437 |
| Ky-12-DS68 | S194 | 702 | AS 194 | 438 |
| Ky-12-DS69 | S195 | 703 | AS195 | 439 |
| Ky-12-DS70 | S196 | 704 | AS 196 | 440 |
| Ky-12-DS71 | S197 | 705 | AS 197 | 441 |
| Ky-12-DS72 | S198 | 706 | AS198 | 442 |
| Ky-12-DS73 | S199 | 707 | AS 199 | 443 |
| Ky-12-DS74 | S200 | 708 | AS200 | 444 |
| Ky-12-DS75 | S201 | 709 | AS201 | 445 |
| Ky-12-DS76 | S202 | 710 | AS202 | 446 |
| Ky-12-DS77 | S203 | 711 | AS203 | 447 |
| Ky-12-DS78 | S204 | 712 | AS204 | 448 |
| Ky-12-DS79 | S205 | 713 | AS205 | 449 |
| Ky-12-DS80 | S206 | 714 | AS206 | 450 |
| Ky-12-DS81 | S207 | 715 | AS207 | 451 |
| Ky-12-DS82 | S208 | 716 | AS208 | 452 |
| Ky-12-DS83 | S209 | 717 | AS209 | 453 |
| Ky-12-DS84 | S210 | 718 | AS210 | 454 |
| Ky-12-DS85 | S211 | 719 | AS211 | 455 |
| Ky-12-DS86 | S212 | 720 | AS212 | 456 |
| Ky-12-DS87 | S213 | 721 | AS213 | 457 |
| Ky-12-DS88 | S214 | 722 | AS214 | 458 |
| Ky-12-DS89 | S215 | 723 | AS215 | 459 |
| Ky-12-DS90 | S216 | 724 | AS216 | 460 |

(continued)

| RNA inhibitor | sense strand code | sense strand SEQ ID NO. | antisense strand code | antisense strand SEQ ID NO. |
|---|---|---|---|---|
| Ky-12-DS91 | S217 | 725 | AS217 | 461 |
| Ky-12-DS92 | S218 | 726 | AS218 | 462 |
| Ky-12-DS93 | S219 | 727 | AS219 | 463 |
| Ky-12-DS94 | S220 | 728 | AS220 | 464 |
| Ky-12-DS95 | S221 | 729 | AS221 | 465 |
| Ky-12-DS96 | S222 | 730 | AS222 | 466 |
| Ky-12-DS97 | S223 | 731 | AS223 | 467 |
| Ky-12-DS98 | S224 | 732 | AS224 | 468 |
| Ky-12-DS99 | S225 | 733 | AS225 | 469 |
| Ky-12-DS100 | S226 | 734 | AS226 | 470 |
| Ky-12-DS101 | S227 | 735 | AS227 | 471 |
| Ky-12-DS102 | S228 | 736 | AS228 | 472 |
| Ky-12-DS103 | S229 | 737 | AS229 | 473 |
| Ky-12-DS104 | S230 | 738 | AS230 | 474 |
| Ky-12-DS105 | S231 | 739 | AS231 | 475 |
| Ky-12-DS106 | S232 | 740 | AS232 | 476 |
| Ky-12-DS107 | S233 | 741 | AS233 | 477 |
| Ky-12-DS108 | S234 | 742 | AS234 | 478 |
| Ky-12-DS109 | S235 | 743 | AS235 | 479 |
| Ky-12-DS110 | S236 | 744 | AS236 | 480 |
| Ky-12-DS111 | S237 | 745 | AS237 | 481 |
| Ky-12-DS112 | S238 | 746 | AS238 | 482 |
| Ky-12-DS113 | S239 | 747 | AS239 | 483 |
| Ky-12-DS114 | S240 | 748 | AS240 | 484 |
| Ky-12-DS115 | S241 | 749 | AS241 | 485 |
| Ky-12-DS116 | S242 | 750 | AS242 | 486 |

(continued)

| RNA inhibitor | sense strand code | sense strand SEQ ID NO. | antisense strand code | antisense strand SEQ ID NO. |
|---|---|---|---|---|
| Ky-12-DS117 | S243 | 751 | AS243 | 487 |
| Ky-12-DS118 | S244 | 752 | AS244 | 488 |
| Ky-12-DS119 | S245 | 753 | AS245 | 489 |
| Ky-12-DS120 | S246 | 754 | AS246 | 490 |
| Ky-12-DS121 | S247 | 755 | AS247 | 491 |
| Ky-12-DS122 | S248 | 756 | AS248 | 492 |
| Ky-12-DS123 | S249 | 757 | AS249 | 493 |
| Ky-12-DS124 | S250 | 758 | AS250 | 494 |
| Ky-12-DS125 | S251 | 759 | AS251 | 495 |
| Ky-12-DS126 | S252 | 760 | AS252 | 496 |
| Ky-12-DS127 | S253 | 761 | AS253 | 497 |
| Ky-12-DS128 | S254 | 762 | AS254 | 498 |
| Ky-12-DS129 | S255 | 763 | AS255 | 499 |
| Ky-12-DS130 | S256 | 764 | AS256 | 500 |
| Ky-12-DS131 | S257 | 765 | AS257 | 501 |
| Ky-12-DS132 | S258 | 766 | AS258 | 502 |
| Ky-12-DS133 | S259 | 767 | AS259 | 503 |
| Ky-12-DS134 | S260 | 768 | AS260 | 504 |
| Ky-12-DS135 | S261 | 769 | AS261 | 505 |
| Ky-12-DS136 | S262 | 770 | AS262 | 506 |
| Ky-12-DS137 | S263 | 771 | AS263 | 507 |
| Ky-12-DS138 | S264 | 772 | AS264 | 508 |

(continued)

| RNA inhibitor | sense strand code | sense strand SEQ ID NO. | antisense strand code | antisense strand SEQ ID NO. |
|---|---|---|---|---|
| Ky-12-DS139 | S265 | 773 | AS265 | 509 |
| Ky-12-DS140 | S266 | 774 | AS266 | 510 |
| Ky-12-DS141 | S267 | 775 | AS267 | 511 |
| Ky-12-DS142 | S268 | 776 | AS268 | 512 |
| Ky-12-DS143 | S269 | 777 | AS269 | 513 |
| Ky-12-DS144 | S270 | 778 | AS270 | 514 |
| Ky-12-DS145 | S271 | 779 | AS271 | 515 |
| Ky-12-DS146 | S272 | 780 | AS272 | 516 |
| Ky-12-DS147 | S273 | 781 | AS273 | 517 |
| Ky-12-DS148 | S274 | 782 | AS274 | 518 |
| Ky-12-DS149 | S275 | 783 | AS275 | 519 |
| Ky-12-DS150 | S276 | 784 | AS276 | 520 |
| Ky-12-DS151 | S277 | 785 | AS277 | 521 |
| Ky-12-DS152 | S278 | 786 | AS278 | 522 |
| Ky-12-DS153 | S279 | 787 | AS279 | 523 |
| Ky-12-DS154 | S280 | 788 | AS280 | 524 |
| Ky-12-DS155 | S281 | 789 | AS281 | 525 |
| Ky-12-DS156 | S282 | 790 | AS282 | 526 |
| Ky-12-DS157 | S283 | 791 | AS283 | 527 |
| Ky-12-DS158 | S284 | 792 | AS284 | 528 |
| Ky-12-DS159 | S285 | 793 | AS285 | 529 |
| Ky-12-DS160 | S286 | 794 | AS286 | 530 |

(continued)

| RNA inhibitor | sense strand code | sense strand SEQ ID NO. | antisense strand code | antisense strand SEQ ID NO. |
|---|---|---|---|---|
| Ky-12-DS161 | S287 | 795 | AS287 | 531 |
| Ky-12-DS162 | S288 | 796 | AS288 | 532 |
| Ky-12-DS163 | S289 | 797 | AS289 | 533 |
| Ky-12-DS164 | S290 | 798 | AS290 | 534 |
| Ky-12-DS165 | S291 | 799 | AS291 | 535 |
| Ky-12-DS166 | S292 | 800 | AS292 | 536 |
| Ky-12-DS167 | S293 | 801 | AS293 | 537 |
| Ky-12-DS168 | S294 | 802 | AS294 | 538 |
| Ky-12-DS169 | S295 | 803 | AS295 | 539 |
| Ky-12-DS170 | S296 | 804 | AS296 | 540 |
| Ky-12-DS171 | S297 | 805 | AS297 | 541 |
| Ky-12-DS172 | S298 | 806 | AS298 | 542 |
| Ky-12-DS173 | S299 | 807 | AS299 | 543 |
| Ky-12-DS174 | S300 | 808 | AS300 | 544 |
| Ky-12-DS175 | S301 | 809 | AS301 | 545 |
| Ky-12-DS176 | S302 | 810 | AS302 | 546 |
| Ky-12-DS177 | S303 | 811 | AS303 | 547 |
| Ky-12-DS178 | S304 | 812 | AS304 | 548 |
| Ky-12-DS179 | S305 | 813 | AS305 | 549 |
| Ky-12-DS180 | S306 | 814 | AS306 | 550 |
| Ky-12-DS181 | S307 | 815 | AS307 | 551 |
| Ky-12-DS182 | S308 | 816 | AS308 | 552 |

(continued)

| RNA inhibitor | sense strand code | sense strand SEQ ID NO. | antisense strand code | antisense strand SEQ ID NO. |
|---|---|---|---|---|
| Ky-12-DS183 | S309 | 817 | AS309 | 553 |
| Ky-12-DS184 | S310 | 818 | AS310 | 554 |
| Ky-12-DS185 | S311 | 819 | AS311 | 555 |
| Ky-12-DS186 | S312 | 820 | AS312 | 556 |
| Ky-12-DS187 | S313 | 821 | AS313 | 557 |
| Ky-12-DS188 | S314 | 822 | AS314 | 558 |
| Ky-12-DS189 | S315 | 823 | AS315 | 559 |
| Ky-12-DS190 | S316 | 824 | AS316 | 560 |
| Ky-12-DS191 | S317 | 825 | AS317 | 561 |
| Ky-12-DS192 | S318 | 826 | AS318 | 562 |
| Ky-12-DS193 | S319 | 827 | AS319 | 563 |
| Ky-12-DS194 | S320 | 828 | AS320 | 564 |
| Ky-12-DS195 | S321 | 829 | AS321 | 565 |
| Ky-12-DS196 | S322 | 830 | AS322 | 566 |
| Ky-12-DS197 | S323 | 831 | AS323 | 567 |
| Ky-12-DS198 | S324 | 832 | AS324 | 568 |
| Ky-12-DS199 | S325 | 833 | AS325 | 569 |
| Ky-12-DS200 | S326 | 834 | AS326 | 570 |
| Ky-12-DS201 | S327 | 835 | AS327 | 571 |
| Ky-12-DS202 | S328 | 836 | AS328 | 572 |
| Ky-12-DS203 | S329 | 837 | AS329 | 573 |
| Ky-12-DS204 | S330 | 838 | AS330 | 574 |

(continued)

| RNA inhibitor | sense strand code | sense strand SEQ ID NO. | antisense strand code | antisense strand SEQ ID NO. |
|---|---|---|---|---|
| Ky-12-DS205 | S331 | 839 | AS331 | 575 |
| Ky-12-DS206 | S332 | 840 | AS332 | 576 |
| Ky-12-DS207 | S333 | 841 | AS333 | 577 |
| Ky-12-DS208 | S334 | 842 | AS334 | 578 |
| Ky-12-DS209 | S335 | 843 | AS335 | 579 |
| Ky-12-DS210 | S336 | 844 | AS336 | 580 |
| Ky-12-DS211 | S337 | 845 | AS337 | 581 |
| Ky-12-DS212 | S338 | 846 | AS338 | 582 |
| Ky-12-DS213 | S339 | 847 | AS339 | 583 |
| Ky-12-DS214 | S340 | 848 | AS340 | 584 |
| Ky-12-DS215 | S341 | 849 | AS341 | 585 |
| Ky-12-DS216 | S342 | 850 | AS342 | 586 |
| Ky-12-DS217 | S343 | 851 | AS343 | 587 |
| Ky-12-DS218 | S344 | 852 | AS344 | 588 |
| Ky-12-DS219 | S345 | 853 | AS345 | 589 |
| Ky-12-DS220 | S346 | 854 | AS346 | 590 |
| Ky-12-DS221 | S347 | 855 | AS347 | 591 |
| Ky-12-DS222 | S348 | 856 | AS348 | 592 |
| Ky-12-DS223 | S349 | 857 | AS349 | 593 |
| Ky-12-DS224 | S350 | 858 | AS350 | 594 |
| Ky-12-DS225 | S351 | 859 | AS351 | 595 |
| Ky-12-DS226 | S352 | 860 | AS352 | 596 |

(continued)

| RNA inhibitor | sense strand code | sense strand SEQ ID NO. | antisense strand code | antisense strand SEQ ID NO. |
|---|---|---|---|---|
| Ky-12-DS227 | S353 | 861 | AS353 | 597 |
| Ky-12-DS228 | S354 | 862 | AS354 | 598 |
| Ky-12-DS229 | S355 | 863 | AS355 | 599 |
| Ky-12-DS230 | S356 | 864 | AS356 | 600 |
| Ky-12-DS231 | S357 | 865 | AS357 | 601 |
| Ky-12-DS232 | S358 | 866 | AS358 | 602 |
| Ky-12-DS233 | S359 | 867 | AS359 | 603 |
| Ky-12-DS234 | S360 | 868 | AS360 | 604 |
| Ky-12-DS235 | S361 | 869 | AS361 | 605 |
| Ky-12-DS236 | S362 | 870 | AS362 | 606 |
| Ky-12-DS237 | S363 | 871 | AS363 | 607 |
| Ky-12-DS238 | S364 | 872 | AS364 | 608 |
| Ky-12-DS239 | S365 | 873 | AS365 | 609 |
| Ky-12-DS240 | S366 | 874 | AS366 | 610 |
| Ky-12-DS241 | S367 | 875 | AS367 | 611 |
| Ky-12-DS242 | S368 | 876 | AS368 | 612 |
| Ky-12-DS243 | S369 | 877 | AS369 | 613 |
| Ky-12-DS244 | S370 | 878 | AS370 | 614 |
| Ky-12-DS245 | S371 | 879 | AS371 | 615 |
| Ky-12-DS246 | S372 | 880 | AS372 | 616 |
| Ky-12-DS247 | S373 | 881 | AS373 | 617 |
| Ky-12-DS248 | S374 | 882 | AS374 | 618 |

(continued)

| RNA inhibitor | sense strand code | sense strand SEQ ID NO. | antisense strand code | antisense strand SEQ ID NO. |
|---|---|---|---|---|
| Ky-12-DS249 | S375 | 883 | AS375 | 619 |
| Ky-12-DS250 | S376 | 884 | AS376 | 620 |
| Ky-12-DS251 | S377 | 885 | AS377 | 621 |
| Ky-12-DS252 | S378 | 886 | AS378 | 622 |
| Ky-12-DS253 | S379 | 887 | AS379 | 623 |
| Ky-12-DS254 | S380 | 888 | AS380 | 624 |
| Ky-12-DS255 | S381 | 889 | AS381 | 625 |
| Ky-12-DS256 | S382 | 890 | AS382 | 626 |
| Ky-12-DS257 | S383 | 891 | AS383 | 627 |
| Ky-12-DS258 | S384 | 892 | AS384 | 628 |
| Ky-12-DS259 | S385 | 893 | AS385 | 629 |
| Ky-12-DS260 | S386 | 894 | AS386 | 630 |
| Ky-12-DS261 | S387 | 895 | AS387 | 631 |
| Ky-12-DS262 | S388 | 896 | AS388 | 632 |
| Ky-12-DS263 | S389 | 897 | AS389 | 633 |
| Ky-12-DS264 | S390 | 898 | AS390 | 634 |
| Ky-12-DS265 | S391 | 899 | AS391 | 635 |
| Ky-12-DS266 | S392 | 900 | AS392 | 636 |
| Ky-12-DS267 | S393 | 901 | AS393 | 637 |
| Ky-12-DS268 | S394 | 902 | AS394 | 638 |
| Ky-12-DS269 | S395 | 903 | AS395 | 639 |
| Ky-12-DS270 | S396 | 904 | AS396 | 640 |

(continued)

| RNA inhibitor | sense strand code | sense strand SEQ ID NO. | antisense strand code | antisense strand SEQ ID NO. |
|---|---|---|---|---|
| Ky-12-DS271 | S397 | 905 | AS397 | 641 |
| Ky-12-DS272 | S398 | 906 | AS398 | 642 |
| Ky-12-DS273 | S399 | 907 | AS399 | 643 |
| Ky-12-DS274 | S400 | 908 | AS400 | 644 |
| Ky-12-DS275 | S401 | 909 | AS401 | 645 |
| Ky-12-DS276 | S402 | 910 | AS402 | 646 |
| Ky-12-DS277 | S403 | 911 | AS403 | 647 |
| Ky-12-DS278 | S404 | 912 | AS404 | 648 |
| Ky-12-DS279 | S405 | 913 | AS405 | 649 |
| Ky-12-DS280 | S406 | 914 | AS406 | 650 |
| Ky-12-DS281 | S407 | 915 | AS407 | 651 |
| Ky-12-DS282 | S408 | 916 | AS408 | 652 |
| Ky-12-DS283 | S409 | 917 | AS409 | 653 |
| Ky-12-DS284 | S410 | 918 | AS410 | 654 |
| Ky-12-DS285 | S411 | 919 | AS411 | 655 |
| Ky-12-DS286 | S412 | 920 | AS412 | 656 |
| Ky-12-DS287 | S413 | 921 | AS413 | 657 |
| Ky-12-DS288 | S414 | 922 | AS414 | 658 |
| Ky-12-DS289 | S415 | 923 | AS415 | 659 |
| Ky-12-DS290 | S416 | 924 | AS416 | 660 |
| Ky-12-DS291 | S417 | 925 | AS417 | 661 |
| Ky-12-DS292 | S418 | 926 | AS418 | 662 |

(continued)

| RNA inhibitor | sense strand code | sense strand SEQ ID NO. | antisense strand code | antisense strand SEQ ID NO. |
|---|---|---|---|---|
| Ky-12-DS293 | S419 | 927 | AS419 | 663 |
| Ky-12-DS294 | S420 | 928 | AS420 | 664 |
| Ky-12-DS295 | S421 | 929 | AS421 | 665 |
| Ky-12-DS296 | S422 | 930 | AS422 | 666 |
| Ky-12-DS297 | S423 | 931 | AS423 | 667 |
| Ky-12-DS298 | S424 | 932 | AS424 | 668 |
| Ky-12-DS299 | S425 | 933 | AS425 | 669 |
| Ky-12-DS300 | S426 | 934 | AS426 | 670 |
| Ky-12-DS301 | S427 | 935 | AS427 | 671 |
| Ky-12-DS302 | S428 | 936 | AS428 | 672 |
| Ky-12-DS303 | S429 | 937 | AS429 | 673 |
| Ky-12-DS304 | S430 | 938 | AS430 | 674 |
| Ky-12-DS305 | S431 | 939 | AS431 | 675 |
| Ky-12-DS306 | S432 | 940 | AS432 | 676 |
| Ky-12-DS307 | S433 | 941 | AS433 | 677 |
| Ky-12-DS308 | S434 | 942 | AS434 | 678 |
| Ky-12-DS309 | S435 | 943 | AS435 | 679 |
| Ky-12-DS310 | S436 | 944 | AS436 | 680 |
| Ky-12-DS311 | S437 | 945 | AS437 | 681 |
| Ky-12-DS312 | S438 | 946 | AS438 | 682 |
| Ky-12-DS313 | S439 | 947 | AS439 | 683 |
| Ky-12-DS314 | S440 | 948 | AS440 | 684 |

(continued)

| RNA inhibitor | sense strand code | sense strand SEQ ID NO. | antisense strand code | antisense strand SEQ ID NO. |
|---|---|---|---|---|
| Ky-12-DS315 | S441 | 949 | AS441 | 685 |
| Ky-12-DS316 | S442 | 950 | AS442 | 686 |
| Ky-12-DS317 | S443 | 951 | AS443 | 687 |
| Ky-12-DS318 | S444 | 952 | AS444 | 688 |
| Ky-12-DS319 | S445 | 953 | AS445 | 689 |
| Ky-12-DS320 | S446 | 954 | AS446 | 690 |
| Ky-12-DS321 | S447 | 955 | AS447 | 691 |
| Ky-12-DS322 | S448 | 956 | AS448 | 692 |
| Ky-12-DS323 | S449 | 957 | AS449 | 693 |
| Ky-12-DS324 | S450 | 958 | AS450 | 694 |
| Ky-12-DS325 | S451 | 959 | AS451 | 695 |
| Ky-12-DS326 | S452 | 960 | AS452 | 696 |
| Ky-12-DS327 | S453 | 961 | AS453 | 697 |
| Ky-12-DS328 | S454 | 962 | AS454 | 698 |
| Ky-12-DS329 | S455 | 963 | AS455 | 699 |
| Ky-12-DS330 | S456 | 964 | AS456 | 700 |

In some embodiments, the RNA inhibitor can be added into the cell line through cell transfection methods or liposome-nucleic acid nanoparticle methods well known in the art for sequence screening. Patents US9233971B2, US9080186B2, CN102985548B and CN103189057B related to methods for preparing lipid compounds and liposome-nucleic acid nanoparticles are incorporated into this specification in their entirety.

In some embodiments, the amphoteric lipids in the lipid compounds described therein are preferably macrocyclic lipids D1C1, T1C1, T1C6, T4C4, B2C1, B2C6, B2C7 and M10C1.

The person skilled in the art knows that dsRNA with a duplex structure of about 19 to 23 base pairs, for example, 21 base pairs, has been considered to be particularly effective in inducing RNAinterference (Elbashir et al., EMBO 2001, 20:6877-6888). However, others have found that shorter or longer RNA duplex structures are also effective (Chu and Rana (2007) RNA14:1714-1719; Kim et al (2005) Nat Biotech 23:222-226). It is reasonable to expect that new sequences designed by those skilled in the art by subtracting or adding a few nucleotides at one or both ends of a sequence in Tables 1-3 and 5 can be similarly effective compared with the sequences of the present invention. Therefore, sequences containing at least 15, 16, 17, 18, 19, 20, 21 consecutive nucleotides identical to sequences in Tables 1-3 and 5 should all be within the scope of protection of the present invention. At least 15 consecutive nucleic acids refer to: sequences having

15, 16, 17, 18, 19, 20, 21, 22, 23 or more consecutive nucleotides and having an inhibitory effect that differs from the sequence of the present invention by no more than about 5, 10, 15, 20, 25 or 30% in terms of the ability to inhibit APOC3 gene expression are included in the scope of protection of the present invention.

The dsRNA described in the present invention may further include one or more single stranded nucleotide protruding end, for example, 1, 2, 3 or 4 nucleotides. The nucleotide protruding end may comprise nucleotide/nucleoside analogues or their combination, including deoxynucleotides/ nucleosides. The protruding end may be on the sense strand, the antisense strand, or any combination thereof. In addition, the nucleotides on protruding end can exist at the 5'- end, 3'- end or both ends of the antisense or sense strand of dsRNA. The protruding end can be caused by one strand being longer than the other, or by two strands of the same length crosslinking. The protruding end may form a mismatch with the target mRNA or it may be complementary to the targeted gene sequence or may be another sequence.

The dsRNA can also contain only a single protruding end, which can enhance the interference activity of RNA inhibitors without affecting their overall stability. For example, the single-stranded protruding end can be located at the 3'- end of the sense strand, or alternatively, at the 3'- end of the antisense strand. The RNA inhibitors can also have blunt end, located at the 5'- end of the antisense strand (or the 3'- end of the sense strand), and vice versa. Generally, the antisense strand of RNA inhibitors has a nucleotide protruding end at the 3'- end and a blunt end at the 5'- end.

In some embodiments, the protruding end exists at the 3'-end of the sense strand, the antisense strand, or both strands. In some embodiments, such 3'-protruding end is present in the antisense strand. In some embodiments, such 3'- protruding end exists in the sense strand.

In some embodiments, the dsRNA has a length of 21 nucleotide and is blunt ended on both ends.

In some embodiments, the dsRNA has a length of 21 nucleotides, and both the sense strand and antisense strand have protruding ends of 2 nucleotide at their 3 'ends.

In some embodiments, the sense strand of the dsRNA has a length of 19 nucleotides, the antisense strand has a length of 21 nucleotides, and the antisense strand has a protruding end of 2 nucleotides at its 3' end.

In some embodiments, the sense strand of the dsRNA has a length of 21 nucleotides, the antisense strand has a length of 23 nucleotides, and the antisense strand has a protruding end of 2 nucleotides at its 3' end.

In order to enhance the stability of the RNA inhibitor described in the present invention in vivo, the sense strand and antisense strand of the RNA inhibitor can be modified without affecting its activity or even enhancing its activity, and the nucleotide therein can have a modification group to modify the whole strand or part of it. In some embodiments, one or more nucleotides on the sense strand and/or antisense strand are modified to form modified nucleotides.

In some embodiments, the RNA of the RNA inhibitor (e.g., dsRNA) of the present invention is unmodified and does not include, for example, chemical modification or conjugations known in the art and described herein. In other embodiments, the RNA of the RNA inhibitor (e.g., dsRNA) of the present invention is chemically modified to enhance stability or other favorable properties. In other embodiments of the application, all nucleotides or basically all nucleotides of the RNA inhibitor of the application are modified, that is, no more than 5, 4, 3, 2 or 1 unmodified nucleotide exists in the strand of the RNA inhibitor.

Nucleic acids described in the present invention can be synthesized and/or modified by methods well known in the art, such as those described in "Current protocols in nucleic acid chemistry", Beaucage, S.L. et al. (eds.), JohnWiley & Sons, Inc, New York, NY, USA, which is incorporated herein by reference. Modification includes, for example, end modification, such as, 5'-end modification (phosphorylation, conjugation, reverse ligation) or 3'-end modification (conjugation, DNA nucleotide, reverse ligation, etc.); base modification, such as replacing with stabilized bases, destabilized bases or bases paired with an expanded repertoire of partners, removing bases (abasic nucleotides) or conjugating bases; sugar modification (e.g., 2'- or 4' -position) or sugar substitution; or backbone modification, including modification or substitution of phosphodiester linkages. In the RNA inhibitor provided by the present invention, both the sense strand and antisense strand of the RNA inhibitor do not need uniform modification, and one or more modifications can be incorporated into a single nucleotide therein.

In some embodiments, the modified nucleotides comprise deoxyribonucleotides, nucleotide mimics, abasic nucleotides, 2'- modified nucleotides, 3'- to 3'-linkage (inverted) nucleotides, nucleotides containing unnatural bases, bridged nucleotides, peptide nucleic acids (PNA), unlocked nucleobase analogues, locked nucleotides, 3'-O-methoxy (2' inter-nucleoside linkage) nucleotides, 2'-F-arabinose nucleotides, 5'-Me/2'-Fluoro-nucleotides, morpholino nucleotides, vinyl phosphonate deoxyribonucleotides, nucleotides containing vinyl phosphonate and nucleotides containing cyclopropyl phosphonate.

In some embodiments, the 2'-modified nucleotides include: 2'-O-methyl nucleotide, 2'-deoxy-2'-fluoronucleotide, 2'-deoxynucleotide, 2'-methoxyethyl nucleotide, 2'-amino nucleotide and/or 2'-alkyl nucleotide.

In some embodiments, some or all of the 2' positions of the nucleotide glycosyls at odd-numbered positions from the 5' end of the sense strand are fluorine.

In some embodiments, the 2' positions of the glycosyls of at least 3 or 4 nucleotides on the sense strand are fluorine.

In some embodiments, some or all of the 2' positions of the nucleotide glycosyls at even-numbered positions from

the 5' end of the antisense strand are fluorine.

In some embodiments, the 2' positions of the glycosyls of at least 2 or 4 nucleotides on the antisense strand are fluorine.

In some embodiments, at least one 2' positions of the nucleotides glycosyls at positions 2, 4, 6, 8, 14 and 16 from the 5' end of the antisense strand is fluorine, for example, 2' positions of the nucleotides glycosyls at positions 2, 4, 6, 8, 14 and 16 from the 5' end of the antisense strand are all fluorine.

In some embodiments, except for the nucleotides at positions 2, 4, 6, 8, 14 and 16 starting from the 5' end of the antisense strand, at least one 2' positions of the remaining nucleotide glycosyls is methoxy.

In some embodiments, at least one 2' positions of the nucleotides glycosyls at positions 9,10 and 11 from the 5' end of the sense strand is fluorine, for example, 2' positions of the nucleotides glycosyls at positions 9,10 and 11 from the 5' end of the sense strand are all fluorine.

In some embodiments, except for the nucleotides at positions 9,10 and 11 starting from the 5' end of the sense strand, at least one 2' positions of the remaining nucleotide glycosyls is methoxy.

In some embodiments, except for the nucleotides at positions 7, 9,10 and 11 starting from the 5' end of the sense strand, at least one 2' positions of the remaining nucleotide glycosyls is methoxy.

For example, the -OH at 2' positions of some or all of the nucleotide glycosyls of the sense strand and/or antisense strand could be substituted, the substituent group is fluorine or methoxy, preferably 2' positions of the nucleotides glycosyls at positions 9,10 and 11 from the 5' end of the sense strand are fluorine, 2' positions of the nucleotides glycosyls at positions 2, 4, 6, 8, 14, 16,18 and 20 from the 5' end of the antisense strand are fluorine, and 2' positions of the remaining nucleotide glycosyls are methoxy, or preferably 2' positions of the nucleotides glycosyls at positions 5, 7, 8 and 9 from the 5' end of the sense strand are fluorine, 2' positions of the nucleotides glycosyls at positions 7,12 and 14 from the 5' end of the antisense strand are fluorine, and 2' positions of the remaining nucleotide glycosyls are methoxy.

In some embodiments, there are at least two consecutive phosphorothioate linkages among the nucleotides of the sense strand and/or antisense strand.

In some embodiments, there are at least two consecutive phosphorothioate linkages among three consecutive nucleotides at the end of the sense strand and/or the end of the antisense strand.

For example, there are at least two consecutive phosphorothioate linkages among three consecutive nucleotides at the 5' and 3' ends of the sense and antisense strands.

For another example, the 2' positions of nucleotide glycosyls at positions 9, 10 and 11 starting from the 5' end of the sense strand are fluorine, and the 2' positions of nucleotide glycosyls at positions 2, 4, 6, 8, 14, 16, 18 and 20 starting from the 5' end of the antisense strand are fluorine, and the 2' positions of the remaining nucleotide glycosyls are methoxy, and there are at least two consecutive phosphorothioate linkages among the 3 consecutive nucleotides at the 5' end and the 3 'end of the sense strand and the antisense strand.

In some embodiments, the 2' positions of some nucleotides in the sense strand are fluorine or methoxy, and the phosphate bonds among at least three adjacent nucleotides at the end of the antisense strand can be thiolated. The 2' positions of nucleotides at positions 5, 7, 8 and 9 or positions 3, 5, 7, 9, 11, 13, and15 starting from the 5' end of the sense strand are fluorine, the 2' positions of the remaining nucleotides are methoxy, the phosphate bonds among at least three adjacent nucleotides at the end of the antisense strand can be thiolated.

In some embodiments, the 2' positions of some nucleotides in the sense strand are fluorine or methoxy, and the phosphate bonds among at least three adjacent nucleotides at the end of the antisense strand can be thiolated. The 2' positions of nucleotides at positions 9, 10 and 11 or positions 3, 5, 7, 9, 11, 13, 15 and/or 17 starting from the 5' end of the sense strand are fluorine, the 2' positions of the remaining nucleotides are methoxy, the phosphate bonds among at least three adjacent nucleotides at the end of the antisense strand can be thiolated.

In some screened embodiments, the 3' ends of the sense strand and the antisense strand can each have two tt hangings. The sense strand and the antisense strand of the RNA inhibitor are selected from a) sequences in Table 6, b) sequences having at least 15 consecutive nucleotides identical to those in a), and c) sequences having no more than 3 nucleotides different from those in a) and b).

Table 6 RNA inhibitors

| RNA inhibitor | sense strand | | | antisense strand | | |
|---|---|---|---|---|---|---|
| | SEQ ID NO. | single strand code | Sequence 5'→3' | SEQ ID NO. | single strand code | antisense strand 5'→3' |
| DS52 | 155 | S52 | gcuuaaaagggacaguauucutt | 206 | AS52 | agaauacugucccuuuuaagctt |
| DS53 | 156 | S53 | ggagcaccguuaaggacaagutt | 207 | AS53 | acuuguccuuaacggugcucctt |
| DS54 | 157 | S54 | gagcaccguuaaggacaaguutt | 208 | AS54 | aacuuguccuuaacggugcuctt |
| DS55 | 158 | S55 | gcaccguuaaggacaaguucutt | 209 | AS55 | agaacuuguccuuaacggugctt |
| DS56 | 159 | S56 | gcaugcuggccucccaauaaatt | 210 | AS56 | uuuauugggaggccagcaugctt |
| DS57 | 160 | S57 | guucucugaguucugggauuutt | 211 | AS57 | aaaucccagaacucagagaactt |
| DS58 | 161 | S58 | uuacaugaagcacgccaccaatt | 212 | AS58 | uugguggcgugcuucauguaatt |
| DS59 | 162 | S59 | gcugccugagaccucaauaccttt | 213 | AS59 | gguauugaggucucaggcagctt |
| DS60 | 163 | S60 | guccaccugccuauccauccutt | 214 | AS60 | aggauggauaggcagguggactt |
| DS61 | 164 | S61 | gauggcuucaguucccugaaatt | 215 | AS61 | uuucagggaacugaagccaucttt |
| DS62 | 165 | S62 | ucucagcuucaugcaggguuatt | 216 | AS62 | uaacccugcaugaagcugagatt |
| DS63 | 166 | S63 | ucagcuucaugcaggguuacatt | 217 | AS63 | uguaacccugcaugaagcugatt |
| DS64 | 167 | S64 | gcuucaugcaggguuacaugatt | 218 | AS64 | ucauguaacccugcaugaagctt |

(continued)

| RNA inhibitor | sense strand | | | antisense strand | | |
|---|---|---|---|---|---|---|
| | SEQ ID NO. | single strand code | Sequence 5'→3' | SEQ ID NO. | single strand code | antisense strand 5'→3' |
| DS65 | 168 | S65 | cucugaguucugggauuugg att | 219 | AS65 | uccaaaucccagaacucagagtt |
| DS66 | 169 | S66 | gcuucaguucccugaaagacu tt | 220 | AS66 | agucuuucagggaacugaagct t |
| DS67 | 170 | S67 | caguucccugaaagacuacug tt | 221 | AS67 | caguagucuuucagggaacug tt |
| DS68 | 171 | S68 | uucccugaaagacuacuggag tt | 222 | AS68 | cuccaguagucuuucagggaat t |
| DS69 | 172 | S69 | aaagacuacuggagcaccguu tt | 223 | AS69 | aacggugcuccaguagucuuu tt |
| DS70 | 173 | S70 | agacuacuggagcaccguuaa tt | 224 | AS70 | uuaacggugcuccaguagucu tt |
| DS71 | 174 | S71 | ccguuaaggacaaguucucug tt | 225 | AS71 | cagagaacuuguccuuaacggt t |
| DS72 | 175 | S72 | ggacaaguucucugaguucug tt | 226 | AS72 | cagaacucagagaacuugucct t |
| DS73 | 176 | S73 | ucugaguucugggauuugga ctt | 227 | AS73 | guccaaaucccagaacucagatt |
| DS74 | 177 | S74 | ugaggucagaccaacuucagc tt | 228 | AS74 | gcugaaguuggucugaccuca tt |
| DS75 | 178 | S75 | ugagaccucaauaccccaagut t | 229 | AS75 | acuuggggguauugaggucuca tt |
| DS76 | 179 | S76 | uauccauccugcgagcuccuu tt | 230 | AS76 | aaggagcucgcaggauggauat t |
| DS77 | 180 | S77 | cugccccuguagguugcuuaa tt | 231 | AS77 | uuaagcaaccuacaggggcagt t |

(continued)

| RNA inhibitor | sense strand | | | antisense strand | | |
|---|---|---|---|---|---|---|
| | SEQ ID NO. | single strand code | Sequence 5'→3' | SEQ ID NO. | single strand code | antisense strand 5'→3' |
| DS78 | 181 | S78 | ugccccuguagguugcuuaaatt | 232 | AS78 | uuuaagcaaccuacaggggcatt |
| DS79 | 182 | S79 | ccccuguagguugcuuaaaagtt | 233 | AS79 | cuuuuaagcaaccuacaggggtt |
| DS80 | 183 | S80 | agaccgccaaggaugcacugatt | 234 | AS80 | ucagugcauccuuggcggucutt |
| DS81 | 184 | S81 | uagguugcuuaaaagggacagtt | 235 | AS81 | cugucccuuuuaagcaaccuatt |
| DS82 | 185 | S82 | caguauucucagugcucuccutt | 236 | AS82 | aggagagcacugagaauacugtt |
| DS83 | 186 | S83 | uauucucagugcucuccuacctt | 237 | AS83 | gguaggagagcacugagaauatt |
| DS84 | 187 | S84 | ugcucuccuaccccaccucautt | 238 | AS84 | augaggugggguaggagagcatt |
| DS85 | 188 | S85 | gcuccuugggguccugcaaucutt | 239 | AS85 | agauugcaggacccaaggagctt |
| DS86 | 189 | S86 | ucagaccaacuucagccguggtt | 240 | AS86 | ccacggcugaaguuggucugatt |
| DS87 | 190 | S87 | uggcugccugagaccucaauatt | 241 | AS87 | uauugaggucucaggcagccatt |
| DS88 | 191 | S88 | uucugggauuuggacccugagtt | 242 | AS88 | cucaggguccaaaucccagaatt |
| DS89 | 192 | S89 | ugggauuuggacccugagguctt | 243 | AS89 | gaccucaggguccaaaucccatt |
| DS90 | 193 | S90 | auuuggacccugaggucagactt | 244 | AS90 | gucugaccucaggguccaaautt |

(continued)

| RNA inhibitor | sense strand | | | antisense strand | | |
|---|---|---|---|---|---|---|
| | SEQ ID NO. | single strand code | Sequence 5'→3' | SEQ ID NO. | single strand code | antisense strand 5'→3' |
| DS91 | 194 | S91 | agaccaacuucagccguggcu tt | 245 | AS91 | agccacggcugaaguuggucu tt |
| DS92 | 195 | S92 | uugggguccugcaaucuccagg tt | 246 | AS92 | ccuggagauugcaggacccaat t |
| DS93 | 196 | S93 | accugccuauccauccugcgat t | 247 | AS93 | ucgcaggauggauaggcaggu tt |
| DS94 | 197 | S94 | ugccuauccauccugcgagcu tt | 248 | AS94 | agcucgcaggauggauaggcat t |
| DS95 | 198 | S95 | gagcuccuuggguccugcaau tt | 249 | AS95 | auugcaggacccaaggagcuct t |
| DS96 | 199 | S96 | acaguauucucagugcucucc tt | 250 | AS96 | ggagagcacugagaauacugut t |
| DS97 | 200 | S97 | ucucagugcucuccuaccccat t | 251 | AS97 | ugggguaggagagcacugaga tt |
| DS98 | 201 | S98 | uccuuggguccugcaaucucc tt | 252 | AS98 | ggagauugcaggacccaaggat t |
| DS99 | 202 | S99 | uggacccugaggucagaccaa tt | 253 | AS99 | uuggucugaccucagggucca tt |
| DS100 | 203 | S100 | acccugaggucagaccaacuut t | 254 | AS10 0 | aaguuggucugaccucagggu tt |
| DS101 | 204 | S101 | ccugaggucagaccaacuucat t | 255 | AS10 1 | ugaaguuggucugaccucagg tt |
| DS102 | 205 | S102 | aagggacaguauucucagugc tt | 256 | AS10 2 | gcacugagaauacugucccuu uu |

where g=guanylate, a=adenylate, u=uridylate, c=cytidylate, t=thymidylate.

In some embodiments, the 2' positions of some nucleotides in the sense strand are fluorine, and the phosphate bonds among at least three adjacent nucleotides at the end of the antisense strand can be thiolated.

In some embodiments, the sense strand in the RNA inhibitor is preferably selected from the sense strand

sequences in Tables 6-1 and 6-2 below.

Table 6-1 modified sequence of the sense strand

| SEQ ID NO. | sense strand code | sense strand sequence 5'→3' |
|---|---|---|
| 257 | S103 | GsGsAGfCAfCfCfGUUAAGGACAAsGsU |
| 258 | S104 | GsAsGCfACfCfGfUUAAGGACAAGsUsU |
| 259 | S105 | GsCsACfCGfUfUfAAGGACAAGUUsCsU |
| 260 | S106 | GsCsAUfGCfUfGfGCCUCCCAAUAsAsA |
| 261 | S107 | UsUsACfAUfGfAfAGCACGCCACCsAsA |
| 262 | S108 | UsCsUCfAGfCfUfUCAUGCAGGGUsUsA |
| 263 | S109 | GsCsUUfCAfGfUfUCCCUGAAAGAsCsU |
| 264 | S110 | CsAsGUfUCfCfCfUGAAAGACUACsUsG |
| 265 | S111 | UsUsCCfCUfGfAfAAGACUACUGGsAsG |
| 266 | S112 | AsAsAGfACfUfAfCUGGAGCACCGsUsU |
| 267 | S113 | AsGsACfUAfCfUfGGAGCACCGUUsAsA |
| 268 | S114 | CsCsGUfUAfAfGfGACAAGUUCUCsUsG |
| 269 | S115 | GsGsACfAAfGfUfUCUCUGAGUUCsUsG |
| 270 | S116 | UsCsUGfAGfUfUfCUGGGAUUUGGsAsC |
| 271 | S117 | UsGsAGfACfCfUfCAAUACCCCAAsGsU |
| 272 | S118 | CsUsGCfCCfCfUfGUAGGUUGCUUsAsA |
| 273 | S119 | UsGsCCfCCfUfGfUAGGUUGCUUAsAsA |
| 274 | S120 | CsCsCCfUGfUfAfGGUUGCUUAAAsAsG |
| 275 | S121 | UsAsGGfUUfGfCfUUAAAAGGGACsAsG |
| 276 | S122 | CsAsGUfAUfUfCfUCAGUGCUCUCsCsU |
| 277 | S123 | UsAsUUfCUfCfAfGUGCUCUCCUAsCsC |
| 278 | S124 | UsGsGCfUGfCfCfUGAGACCUCAAsUsA |
| 279 | S125 | UsUsGGfGUfCfCfUGCAAUCUCCAsGsG |
| 280 | S126 | UsGsCCtUAfUfCfCAUCCUGCGAGsCsU |
| 281 | S127 | GsAsGCfUCfCfUfUGGGUCCUGCAsAsU |
| 282 | S128 | AsCsAGfUAfUfUfCUCAGUGCUCUsCsC |
| 283 | S129 | UsCsUCfAGfUfGfCUCUCCUACCCsCsA |
| 284 | S130 | AsCsCCfUGfAfGfGUCAGACCAACsUsU |
| 285 | S131 | CsCsUGfAGfGfUfCAGACCAACUUsCsA |
| 286 | S132 | AsAsGGfGAfCfAfGUAUUCUCAGUsGsC |
| 287 | S133 | GsGsfAGfCAfCfCfGUfUAfAGfGACAAsGsU |
| 288 | S134 | GsAsfGCfACfCfGfUUfAAfGGfACAAGsUsU |
| 289 | S135 | GsCsfACfCGfUfUfAAfGGfACfAAGUUsCsU |
| 290 | S136 | GsCsfAUfGCfUfGfGCfCUfCCfCAAUAsAsA |
| 291 | S137 | UsUsfACfAUfGfAfAGfCAfCGfCCACCsAsA |
| 292 | S138 | UsCsfUCfAGfCfUfUCfAUfGCfAGGGUsUsA |
| 293 | S139 | GsCsfUUfCAfGfUfUCfCCfUGfAAAGAsCsU |
| 294 | S140 | CsAsfGUfUCfCfCfUGfAAfAGfACUACsUsG |

(continued)

| SEQ ID NO. | sense strand code | sense strand sequence 5'→3' |
|---|---|---|
| 295 | S141 | UsUsfCCfCUfGfAfAAfGAfCUfACUGGsAsG |
| 296 | S142 | AsAsfAGfACfUfAfCUfGGfAGCfACCGsUsU |
| 297 | S143 | AsGsfACfUAfCfUfGGfAGfCAfCCGUUsAsA |
| 298 | S144 | CsCsfGUfUAfAfGfGAfCAfAGfUUCUCsUsG |
| 299 | S145 | GsGsfACfAAfGfUfUCfUCfUGfAGUUCsUsG |
| 300 | S146 | UsCsfUGfAGfUfUfCUfGGfGAfUUUGGsAsC |
| 301 | S147 | UsGsfAGfACfCfUfCAfAUfACfCCCAAsGsU |
| 302 | S148 | CsUsfGCfCCfCfUfGUfAGfGUfUGCUUsAsA |
| 303 | S149 | UsGsfCCfCCfUfGfUAfGGfUUfGCUUAsAsA |
| 304 | S150 | CsCsfCCfUGfUfAfGGfUUfGCfUUAAAsAsG |
| 305 | S151 | UsAsfGGfUUfGfCfUUfAAfAAfGGGACsAsG |
| 306 | S152 | CsAsfGUfAUfUfCfUCfAGfUGfCUCUCsCsU |
| 307 | S153 | UsAsfUUfCUfCfAfGUfGCfUCfUCCUAsCsC |
| 308 | S154 | UsGsfGCfUGfCfCfUGfAGfACfCUCAAsUsA |
| 309 | S155 | UsUsfGGfGUfCfCfUGfCAfAUfCUCCAsGsG |
| 310 | S156 | UsGsfCCfUAfUfCfCAfUCfCUfGCGAGsCsU |
| 311 | S157 | GsAsfGCfUCfCfUfUGfGGfUCfCUGCAsAsU |
| 312 | S158 | AsCsfAGfUAfUfUfCUfCAfGUfGCUCUsCsC |
| 313 | S159 | UsCsfUCfAGfUfGfCUfCUfCCfUACCCsCsA |
| 314 | S160 | AsCsfCCfUGfAfGfGUfCAfGAfCCAACsUsU |
| 315 | S161 | CsCsfUGfAGfGfUfCAfGAfCCfAACUUsCsA |
| 316 | S162 | AsAsfGGfGAfCfAfGUfAUfUCfUCAGUsGsC |
| 407 | S163 | GsGsAGCACCfGfUfUAAGGACAAsGsU |
| 408 | S164 | GsAsGCACCGfUfUfAAGGACAAGsUsU |
| 409 | S165 | GsCsACCGUUfAfAfGGACAAGUUsCsU |
| 410 | S166 | GsCsAUGCUGfGfCfCUCCCAAUAsAsA |
| 411 | S167 | UsUsACAUGAfAfGfCACGCCACCsAsA |
| 412 | S168 | UsCsUCAGCUfUfCfAUGCAGGGUsUsA |
| 413 | S169 | GsCsUUCAGUfUfCfCCUGAAAGAsCsU |
| 414 | S170 | CsAsGUUCCCfUfGfAAAGACUACsUsG |
| 415 | S171 | UsUsCCCUGAfAfAfGACUACUGGsAsG |
| 416 | S172 | AsAsAGACUAfCfUfGGAGCACCGsUsU |
| 417 | S173 | AsGsACUACUfGfGfAGCACCGUUsAsA |
| 418 | S174 | CsCsGUUAAGfGfAfCAAGUUCUCsUsG |
| 419 | S175 | GsGsACAAGUfUfCfUCUGAGUUCsUsG |
| 420 | S176 | UsCsUGAGUUfCfUfGGGAUUUGGsAsC |
| 421 | S177 | UsGsAGACCUfCfAfAUACCCCAAsGsU |
| 422 | S178 | CsUsGCCCCUfGfUfAGGUUGCUUsAsA |
| 423 | S179 | UsGsCCCCUGfUfAfGGUUGCUUAsAsA |

(continued)

| SEQ ID NO. | sense strand code | sense strand sequence 5'→3' |
|---|---|---|
| 424 | S180 | CsCsCCUGUAfGfGfUUGCUUAAAsAsG |
| 425 | S181 | UsAsGGUUGCfUfUfAAAAGGGACsAsG |
| 426 | S182 | CsAsGUAUUCfUfCfAGUGCUCUCsCsU |
| 427 | S183 | UsAsUUCUCAfGfUfGCUCUCCUAsCsC |
| 428 | S184 | UsGsGCUGCCfUfGfAGACCUCAAsUsA |
| 429 | S185 | UsUsGGGUCCfUfGfCAAUCUCCAsGsG |
| 430 | S186 | UsGsCCUAUCfCfAfUCCUGCGAGsCsU |
| 431 | S187 | GsAsGCUCCUfUfGfGGUCCUGCAsAsU |
| 432 | S188 | AsCsAGUAUUfCfUfCAGUGCUCUsCsC |
| 433 | S189 | UsCsUCAGUGfCfUfCUCCUACCCsCsA |
| 434 | S190 | AsCsCCUGAGfGfUfCAGACCAACsUsU |
| 435 | S191 | CsCsUGAGGUfCfAfGACCAACUUsCsA |
| 436 | S192 | AsAsGGGACAfGfUfAUUCUCAGUsGsC |

Table 6-2 modified sequence of sense strand

| SEQ ID NO. | sense strand code | sense strand 5'→3' |
|---|---|---|
| 1028 | S457 | GsUsGGCUGCfCfUfGAGACCUCAsAsU |
| 1029 | S458 | AsAsGUCCACfCfUfGCCUAUCCAsUsC |
| 1030 | S459 | CsAsCCUGCCfUfAfUCCAUCCUGsAsA |
| 1031 | S460 | GsGsGACAGUfAfUfUCUCAGUGCsUsU |
| 1032 | S461 | GsAsCAGUAUfUfCfUCAGUGCUCsUsU |
| 1033 | S462 | GsAsGCGACAfGfUfAUUCUCAGUsGsU |
| 1034 | S463 | GsUsGGCUfGCfCfUfGAGACCUCAsAsU |
| 1035 | S464 | AsAsGUCCfACfCfUfGCCUAUCCAsUsC |
| 1036 | S465 | CsAsCCUGfCCfUfAfUCCAUCCUGsAsA |
| 1037 | S466 | GsGsGACAfGUfAfUfUCUCAGUGCsUsU |
| 1038 | S467 | GsAsCAGUfAUfUfCfUCAGUGCUCsUsU |
| 1039 | S468 | GsAsGCGAfCAfGfUfAUUCUCAGUsGsU |
| 1040 | S469 | GUGGCUGCfCfUfGAGACCUCAsAsU |
| 1041 | S470 | AAGUCCACfCfUfGCCUAUCCAsUsC |
| 1042 | S471 | CACCUGCCfUfAfUCCAUCCUGsAsA |
| 1043 | S472 | GGGACAGUfAfUfUCUCAGUGCsUsU |
| 1044 | S473 | GACAGUAUfUfCfUCAGUGCUCsUsU |
| 1045 | S474 | GUGGCUfGCfCfUfGAGACCUCAsAsU |
| 1046 | S475 | AAGUCCfACfCfUfGCCUAUCCAsUsC |
| 1047 | S476 | CACCUGfCCfUfAfUCCAUCCUGsAsA |
| 1048 | S477 | GGGACAfGUfAfUfUCUCAGUGCsUsU |
| 1049 | S478 | GACAGUfAUfUfCfUCAGUGCUCsUsU |
| 1050 | S479 | GAGCGACAfGfUfAUUCUCAGUsGsU |

(continued)

| SEQ ID NO. | sense strand code | sense strand 5'→3' |
|---|---|---|
| 1051 | S480 | GAGCGAfCAfGfUfAUUCUCAGUsGsU |
| 1052 | S481 | GAGCGACAfGfUfAUUCUCAsGsU |
| 1053 | S482 | GsUsGGCUGCfCfUfGAGACCUCAsAsA |
| 1054 | S483 | AsAsGUCCACfCfUfGCCUAUCCAsUsA |
| 1055 | S484 | CsGsCCUGCCfUfAfUCCAUCCUGsAsA |
| 1056 | S485 | GsGsGACAGUfAfUfUCUCAGUGCsUsA |
| 1057 | S486 | GsAsCAGUAUfUfCfUCAGUGCUCsUsA |
| 1058 | S487 | GsUsGGCUfGCfCfUfGAGACCUCAsAsA |
| 1059 | S488 | AsAsGUCCfACfCfUfGCCUAUCCAsUsA |
| 1060 | S489 | CsGsCCUGfCCfUfAfUCCAUCCUGsAsA |
| 1061 | S490 | GsGsGACAfGUfAfUfUCUCAGUGCsUsA |
| 1062 | S491 | GsAsCAGUfAUfUfCfUCAGUGCUCsUsA |
| 1063 | S492 | AsAsGCGACAfGfUfAUUCUCAGUsGsA |
| 1064 | S493 | AsAsGCGAfCAfGfUfAUUCUCAGUsGsA |
| 1065 | S494 | AsAsGCGACAfGfUfAUUCUCAsGsA |
| 1066 | S495 | GUGGCUGCfCfUfGAGACCUCAsAsA |
| 1067 | S496 | AAGUCCACfCfUfGCCUAUCCAsUsA |
| 1068 | S497 | CGCCUGCCfUfAfUCCAUCCUGsAsA |
| 1069 | S498 | GGGACAGUfAfUfUCUCAGUGCsUsA |
| 1070 | S499 | GACAGUAUfUfCfUCAGUGCUCsUsA |
| 1071 | S500 | GUGGCUfGCfCfUfGAGACCUCAsAsA |
| 1072 | S501 | AAGUCCfACfCfUfGCCUAUCCAsUsA |
| 1073 | S502 | CGCCUGfCCfUfAfUCCAUCCUGsAsA |
| 1074 | S503 | GGGACAfGUfAfUfUCUCAGUGCsUsA |
| 1075 | S504 | GACAGUfAUfUfCfUCAGUGCUCsUsA |
| 1076 | S505 | AAGCGACAfGfUfAUUCUCAGUsGsA |
| 1077 | S506 | AAGCGAfCAfGfUfAUUCUCAGUsGsA |
| 1078 | S507 | AAGCGACAfGfUfAUUCUCAsGsA |
| 1079 | S508 | GsUsGGCUGCfCfUfGAGACCUsCsA |
| 1080 | S509 | GsAsGUCCACfCfUfGCCUAUCsCsA |
| 1081 | S510 | CsAsCCUGCCfUfAfUCCAUCCsUsA |
| 1082 | S511 | GsGsGACAGUfAfUfUCUCAGUsGsA |
| 1083 | S512 | GsAsCAGUAUfUfCfUCAGUGCsUsA |
| 1084 | S513 | GsTsGGCUfGCfCfUfGAGACCUsCsA |
| 1085 | S514 | AsAsGUCCfACfCfUfGCCUAUCsCsA |
| 1086 | S515 | CsAsCCUGfCCfUfAfUCCAUCCsUsA |
| 1087 | S516 | GsGsGACAfGUfAfUfUCUCAGUsGsA |
| 1088 | S517 | GsAsCAGUfAUfUfCfUCAGUGCsUsA |
| 1089 | S518 | GsAsGCGACAfGfUfAUUCUCAsGsU |

(continued)

| SEQ ID NO. | sense strand code | sense strand 5'→3' |
|---|---|---|
| 1090 | S519 | GsCsGCGAfCAfGfUfAUUCUCAsGsU |
| 1091 | S520 | GsCsGCGACAfGfUfAUUCTCAsGsA |
| 1092 | S521 | GsUsGGCUfGCfCfUfGAGACCUsCsA |
| 1093 | S522 | AsAsGUCCACfCfUfGCCUAUCsCsA |
| 1094 | S523 | CsAsCCUGCCfUfAfUCCAUCCUGsCsG |
| 1095 | S524 | CsAsCCUGfCCfUfAfUCCAUCCUGsCsG |
| 1096 | S525 | CsAsCCUGCCfUfAfUCCAUCCsUsG |
| 1097 | S526 | CsAsCCUGfCCfUfAfUCCAUCCsUsG |
| 1098 | S527 | GsGsGACAGUfAfUfUCUCAGUGCsUsC |
| 1099 | S528 | GsGsGACAfGUfAfUfUCUCAGUGCsUsC |
| 1100 | S529 | GsGsGACAGUfAfUfUCUCAGUsGsC |
| 1101 | S530 | GsGsGACAfGUfAfUfUCUCAGUsGsC |
| 1102 | S531 | GsAsCAGUAUfUfCfUCAGUGCUCsUsC |
| 1103 | S532 | GsAsCAGUfAUfUfCfUCAGUGCUCsUsC |
| 1104 | S533 | GsAsCAGUAUfUfCfUCAGUGCsUsC |
| 1105 | S534 | GsAsCAGUfAUfUfCfUCAGUGCsUsC |
| 1106 | S535 | AsAsGGGACAfGfUfAUUCUCAGUsGsC |
| 1107 | S536 | AsAsGGGAfCAfGfUfAUUCUCAGUsGsC |
| 1108 | S537 | AsAsGGGACAfGfUfAUUCUCAsGsU |
| 1109 | S538 | AsAsGGGAfCAfGfUfAUUCUCAsGsU |
| 1110 | S539 | AsAsGCGACAfGfUfAUUCUCAGUsGsU |
| 1111 | S540 | AsAsGCGfACAfGfUfAUUCUCAGUsGsU |
| 1112 | S541 | AsAsGGfGAfCfAfGUAUUCTCAGUsGsU |
| 1113 | S542 | GsAsGGfGAfCAfGUAUUCUCAGUsGsU |
| 1114 | S543 | CsAsGGfGAfCfAfGUAUUCUCAGUsGsU |
| 1115 | S544 | GsGsGAfCAfGfUfAUUCUCAGUGCsUsA |
| 1116 | S545 | GsAsGGfGAfCfAfGUAUUCUCAGUsGsU |
| 1117 | S546 | GsAsGCfGAfCfAfGUAUUCTCAGUsGsU |
| 1118 | S547 | UsAsGGfGAfCfAfGUAUUCTCAGUsGsU |
| 1119 | S548 | AsUsGGfGAfCfAfGUAUUCTCAGUsGsU |
| 1120 | S549 | AsGsGGfACfAfGfUAUUCUCAGUGsUsA |
| 1121 | S550 | AsAsGGfGAfCfAfGUAUUCUCAGUsGsA |
| 1122 | S551 | GsCsUUfAAfAfAfGGGACAGUAUUsCsA |
| 1123 | S552 | GsCsUUAAAAfGfGfGACAGUAUUsCsA |
| 1124 | S553 | GsCsUUfAAfAfAfGGGACAGUAUUsCsU |
| 1125 | S554 | GsCsfUUfAAfAfAfGGfGAfCAfGUAUUsCsU |
| 1126 | S555 | CsUsUAfAAfAfGfGGACAGUAUUCsUsC |
| 1127 | S556 | CsUsfUAfAAfAfGfGGfACfAGfUAUUCsUsC |
| 1128 | S557 | UsUsAAfAAfGfGfGACAGUAUUCUsCsA |

(continued)

| SEQ ID NO. | sense strand code | sense strand 5'→3' |
|---|---|---|
| 1129 | S558 | UsUsfAAfAAfGfGfGAfCAfGUfAUUCUsCsA |
| 1130 | S559 | UsAsAAfAGfGfGfACAGUAUUCUCsAsG |
| 1131 | S560 | UsAsfAAfAGfGfGfACfAGfUAfUUCUCsAsG |
| 1132 | S561 | AsAsAAfGGfGfAfCAGUAUUCUCAsGsC |
| 1133 | S562 | AsAsfAAfGGfGfAfCAfGUfAUfUCUCAsGsC |
| 1134 | S563 | AsAsAGfGGfAfCfAGUAUUCUCAGsUsG |
| 1135 | S564 | AsAsAfGGfGAfCfAfGUfAUfUCfUCAGsUsG |
| 1136 | S565 | AsAsGGfGAfCfAfGUAUUCUCAGUsGsC |
| 1137 | S566 | AsAsfGGfGAfCfAfGUfAUfUCfUCAGUsGsC |
| 1138 | S567 | AsGsGGfACfAfGfUAUUCUCAGUGsCsU |
| 1139 | S568 | AsGsfGGfACfAfGfUAfUUfCUfCAGUGsCsU |
| 1140 | S569 | GsCsUUfAAfAfAfGGGACAGUAUUsCsA |
| 1141 | S570 | GsCsUUAAAAfGfGfGACAGUAUUsCsA |

where, G=2'-O-methylguanylate, A=2'-O-methyladenylate, U=2'-O-methyluridylate, C=2'-O-methylcytidylate; fG=2'-fluoroguanylate, fA=2'-fluoroadenylate, fU=2'-fluorouridylate, fC=2'-fluorocytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate, fGs=2'-fluoro-3'-thioguanylate, fAs=2'-fluoro-3'-thioadenylate, fUs=2'-fluoro-3'-thiouridylate, fCs=2'-fluoro-3'-thiocytidylate, T=thymidylate, Ts=3'-thiothymidylate.

In some embodiments, the sense strand of the RNA inhibitor of the present invention is selected from a) sequences in Tables 6-1 and 6-2, b) sequences having at least 15 consecutive nucleotides identical to those in a), and c) sequences having no more than 3 nucleotides different from those in a) and b).

In some embodiments, the 2' positions of some nucleotides in the antisense strand are fluorine, and the phosphate bonds among at least three adjacent nucleotides at the end of the antisense strand can be thiolated. The antisense strand of the RNA inhibitor is preferably selected from a) sequences in Tables 7-1 and 7-2, b) sequences having at least 15 consecutive nucleotides identical to those in a), and c) sequences having no more than 3 nucleotides different from those in a) and b).

Table 7-1 modified sequences of antisense strand

| SEQ ID NO. | antisense strand code | antisense strand 5'→3' |
|---|---|---|
| 317 | AS103 | AsCsUUGUfCCUUAfACfGGUGCUsCsC |
| 318 | AS104 | AsAsCUUGfUCCUUfAAfCGGUGCsUsC |
| 319 | AS105 | AsGsAACUfUGUCCfUUfAACGGUsGsC |
| 320 | AS106 | UsUsUAUUfGGGAGfGCfCAGCAUsGsC |
| 321 | AS107 | UsUsGGUGfGCGUGfCUfUCAUGUsAsA |
| 322 | AS108 | UsAsACCCfUGCAUfGAfAGCUGAsGsA |
| 323 | AS109 | AsGsUCUUfUCAGGfGAfACUGAAsGsC |
| 324 | AS110 | CsAsGUAGfUCUUUfCAfGGGAACsUsG |
| 325 | AS111 | CsUsCCAGfUAGUCfUUfUCAGGGsAsA |
| 326 | AS112 | AsAsCGGUfGCUCCfAGfUAGUCUsUsU |
| 327 | AS113 | UsUsAACGfGUGCUfCCfAGUAGUsCsU |
| 328 | AS114 | CsAsGAGAfACUUGfUCfCUUAACsGsG |
| 329 | AS115 | CsAsGAACfUCAGAfGAfACUUGUsCsC |

(continued)

| SEQ ID NO. | antisense strand code | antisense strand 5'→3' |
|---|---|---|
| 330 | AS116 | GsUsCCAAfAUCCCfAGfAACUCAsGsA |
| 331 | AS117 | AsCsUUGGfGGUAUfUGfAGGUCUsCsA |
| 332 | AS118 | UsUsAAGCfAACCUfACfAGGGGCsAsG |
| 333 | AS119 | UsUsUAAGfCAACCfUAfCAGGGGsCsA |
| 334 | AS120 | CsUsUUUAfAGCAAfCCfUACAGGsGsG |
| 335 | AS121 | CsUsGUCCfCUUUUfAAfGCAACCsUsA |
| 336 | AS122 | AsGsGAGAfGCACUfGAfGAAUACsUsG |
| 337 | AS123 | GsGsUAGGfAGAGCfACfUGAGAAsUsA |
| 338 | AS124 | UsAsUUGAfGGUCUfCAfGGCAGCsCsA |
| 339 | AS125 | CsCsUGGAfGAUUGfCAfGGACCCsAsA |
| 340 | AS126 | AsGsCUCGfCAGGAfUGfGAUAGGsCsA |
| 341 | AS127 | AsUsUGCAfGGACCfCAfAGGAGCsUsC |
| 342 | AS128 | GsGsAGAGfCACUGfAGfAAUACUsGsU |
| 343 | AS129 | UsGsGGGUfAGGAGfAGfCACUGAsGsA |
| 344 | AS130 | AsAsGUUGfGUCUGfACfCUCAGGsGsU |
| 345 | AS131 | UsGsAAGUfUGGUCfUGfACCUCAsGsG |
| 346 | AS132 | GsCsACUGfAGAAUfACfUGUCCCUUsUsU |
| 347 | AS133 | AsfCsUUGfUCfCfUfUAACfGfGfUGCUsCsC |
| 348 | AS134 | AsfAsCUUfGUfCfCfUUAAfCfGfGUGCsUsC |
| 349 | AS135 | AsfGsAACfUUfGfUfCCUUfAfAfCGGUsGsC |
| 350 | AS136 | UsfUsUAUfUGfGfGfAGGCfCfAfGCAUsGsC |
| 351 | AS137 | UsfUsGGUfGGfCfGfUGCUfUfCfAUGUsAsA |
| 352 | AS138 | UsfAsACCfCUfGfCfAUGAfAfGfCUGAsGsA |
| 353 | AS139 | AsfGsUCUfUUfCfAfGGGAfAfCfUGAAsGsC |
| 354 | AS140 | CsfAsGUAfGUfCfUfUUCAfGfGfGAACsUsG |
| 355 | AS141 | CsfUsCCAfGUfAfGfUCUUfUfCfAGGGsAsA |
| 356 | AS142 | AsfAsCGGfUGfCfUfCCAGfUfAfGUCUsUsU |
| 357 | AS143 | UsfUsAACfGGfUfGfCUCCfAfGfUAGUsCsU |
| 358 | AS144 | CsfAsGAGfAAfCfUfUGUCfCfUfUAACsGsG |
| 359 | AS145 | CsfAsGAAfCUfCfAfGAGAfAfCfUUGUsCsC |
| 360 | AS146 | GsfUsCCAfAAfUfCfCCAGfAfAfCUCAsGsA |
| 361 | AS147 | AsfCsUUGfGGfGfUfAUUGfAfGfGUCUsCsA |
| 362 | AS148 | UsfUsAAGfCAfAfCfCUACfAfGfGGGCsAsG |
| 363 | AS149 | UsfUsUAAfGCfAfAfCCUAfCfAfGGGGsCsA |
| 364 | AS150 | CsfUsUUUfAAfGfCfAACCfUfAfCAGGsGsG |
| 365 | AS151 | CsfUsGUCfCCfUfUfUUAAfGfCfAACCsUsA |
| 366 | AS152 | AsfGsGAGfAGfCfAfCUGAfGfAfAUACsUsG |
| 367 | AS153 | GsfGsUAGfGAfGfAfGCACfUfGfAGAAsUsA |
| 368 | AS154 | UsfAsUUGfAGfGfUfCUCAfGfGfCAGCsCsA |

(continued)

| SEQ ID NO. | antisense strand code | antisense strand 5'→3' |
|---|---|---|
| 369 | AS155 | CsfCsUGGfAGfAfUfUGCAfGfGfACCCsAsA |
| 370 | AS156 | AsfGsCUCfGCfAfGfGAUGfGfAfUAGGsCsA |
| 371 | AS157 | AsfUsUGCfAGfGfAfCCCAfAfGfGAGCsUsC |
| 372 | AS158 | GsfGsAGAfGCfAfCfUGAGfAfAfUACUsGsU |
| 373 | AS159 | UsfGsGGGfUAfGfGfAGAGfCfAfCUGAsGsA |
| 374 | AS160 | AsfAsGUUfGGfUfCfUGACfCfUfCAGGsGsU |
| 375 | AS161 | UsfGsAAGfUUfGfGfUCUGfAfCfCUCAsGsG |
| 376 | AS162 | GsfCsACUfGAfGfAfAUACfUfGfUCCCUUsUsU |
| 377 | AS163 | AsfCsUfUGfUCfCUUAACfGGfUGCUsCsC |
| 378 | AS164 | AsfAsCfUUfGUfCCUUAAfCGfGUGCsUsC |
| 379 | AS165 | AsfGsAfACfUUfGUCCUUfAAfCGGUsGsC |
| 380 | AS166 | UsfUsUfAUfUGfGGAGGCfCAfGCAUsGsC |
| 381 | AS167 | UsfUsGfGUfGGfCGUGCUfUCfAUGUsAsA |
| 382 | AS168 | UsfAsAfCCfCUfGCAUGAfAGfCUGAsGsA |
| 383 | AS169 | AsfGsUfCUfUUfCAGGGAfACfUGAAsGsC |
| 384 | AS170 | CsfAsGfUAfGUfCUUUCAfGGfGAACsUsG |
| 385 | AS171 | CsfUsCfCAfGUfAGUCUUfUCfAGGGsAsA |
| 386 | AS172 | AsfAsCfGGfUGfCUCCAGfUAfGUCUsUsU |
| 387 | AS173 | UsfUsAfACfGGfUGCUCCfAGfUAGUsCsU |
| 388 | AS174 | CsfAsGfAGfAAfCUUGUCfCUfUAACsGsG |
| 389 | AS175 | CsfAsGfAAfCUfCAGAGAfACfUUGUsCsC |
| 390 | AS176 | GsfUsCfCAfAAfUCCCAGfAAfCUCAsGsA |
| 391 | AS177 | AsfCsUfUGfGGfGUAUUGfAGfGUCUsCsA |
| 392 | AS178 | UsfUsAfAGfCAfACCUACfAGfGGGCsAsG |
| 393 | AS179 | UsfUsUfAAfGCfAACCUAfCAfGGGGsCsA |
| 394 | AS180 | CsfUsUfUUfAAfGCAACCfUAfCAGGsGsG |
| 395 | AS181 | CsfUsGfUCfCCfUUUUAAfGCfAACCsUsA |
| 396 | AS182 | AsfGsGfAGfAGfCACUGAfGAfAUACsUsG |
| 397 | AS183 | GsfGsUfAGfGAfGAGCACfUGfAGAAsUsA |
| 398 | AS184 | UsfAsUfUGfAGfGUCUCAfGGfCAGCsCsA |
| 399 | AS185 | CsfCsUfGGfAGfAUUGCAfGGfACCCsAsA |
| 400 | AS186 | AsfGsCfUCfGCfAGGAUGfGAfUAGGsCsA |
| 401 | AS187 | AsfUsUfGCfAGfGACCCAfAGfGAGCsUsC |
| 402 | AS188 | GsfGsAfGAfGCfACUGAGfAAfUACUsGsU |
| 403 | AS189 | UsfGsGfGGfUAfGGAGAGfCAfCUGAsGsA |
| 404 | AS190 | AsfAsGfUUfGGfUCUGACfCUfCAGGsGsU |
| 405 | AS191 | UsfGsAfAGfUUfGGUCUGfACfCUCAsGsG |
| 406 | AS192 | GsfCsAfCUfGAfGAAUACfUGfUCCCUUsUsU |

Table 7-2 modified sequences of antisense strand

| SEQ ID NO. | antisense strand code | antisense strand 5'-3' |
|---|---|---|
| 965 | AS457 | UsfGsAfGGfUCfUCAGGCfAGfCCfACsfGsG |
| 966 | AS458 | UsfGsGfAUfAGfGCAGGUfGGfACfUUsfGsG |
| 967 | AS459 | CsfAsGfGAfUGfGAUAGGfCAfGGfUGsfGsA |
| 968 | AS460 | GsfCsAfCUfGAfGAAUACfUGfUCfCCsfUsU |
| 969 | AS461 | GsfAsGfCAfCUfGAGAAUfACfUGfUCsfCsC |
| 970 | AS462 | AsfCsAfCUfGAfGAAUACfUGfUCfGCsfUsC |
| 971 | AS463 | UsfGsAfGGfUCfUCfAGGCfAGfCCfACsGsG |
| 972 | AS464 | UsfGsGfAUfAGfGCfAGGUfGGfACfUUsGsG |
| 973 | AS465 | CsfAsGfGAfUGfGAfUAGGfCAfGGfUGsGsA |
| 974 | AS466 | GsfCsAfCUfGAfGAfAUACfUGfUCfCCsUsU |
| 975 | AS467 | GsfAsGfCAfCUfGAfGAAUfACfUGfUCsCsC |
| 976 | AS468 | AsfCsAfCUfGAfGAfAUACfUGfUCfGCsUsC |
| 977 | AS469 | AsfCsAfCUfGAfGAAUACfUGfUCfGCfUCsUsU |
| 978 | AS470 | UsfGsAfGGfUCfUCAGGCfAGfCCfACfGG |
| 979 | AS471 | UsfGsGfAUfAGfGCAGGUfGGfACfUUfGG |
| 980 | AS472 | CsfAsGfGAfUGfGAUAGGfCAfGGfUGfGA |
| 981 | AS473 | GsfCsAfCUfGAfGAAUACfUGfUCfCCfUU |
| 982 | AS474 | GsfAsGfCAfCUfGAGAAUfACfUGfUCfCC |
| 983 | AS475 | UsfGsAfGGfUCfUCfAGGCfAGfCCfACGG |
| 984 | AS476 | UsfGsGfAUfAGfGCfAGGUfGGfACfUUGG |
| 985 | AS477 | CsfAsGfGAfUGfGAfUAGGfCAfGGfUGGA |
| 986 | AS478 | GsfCsAfCUfGAfGAfAUACfUGfUCfCCUU |
| 987 | AS479 | GsfAsGfCAfCUfGAfGAAUfACfUGfUCCC |
| 988 | AS480 | AsfCsAfCUfGAfGAAUACfUGfUCfGCfUC |
| 989 | AS481 | AsfCsAfCUfGAfGAfAUACfUGfUCfGCUC |
| 990 | AS482 | AsfCsAfCUfGAfGAAUACfUGfUCfGCfUCUU |
| 991 | AS483 | UsfGsAfGGfUCfUCAGGCfAGfCCfACfGsG |
| 992 | AS484 | UsfGsGfAUfAGfGCAGGUfGGfACfUUfGsG |
| 993 | AS485 | CsfAsGfGAfUGfGAUAGGfCAfGGfUGfGsA |
| 994 | AS486 | GsfCsAfCUfGAfGAAUACfUGfUCfCCfUsU |
| 995 | AS487 | GsfAsGfCAfCUfGAGAAUfACfUGfUCfCsC |
| 996 | AS488 | UsfGsAfGGfUCfUCfAGGCfAGfCCfACGsG |
| 997 | AS489 | UsfGsGfAUfAGfGCfAGGUfGGfACfUUGsG |
| 998 | AS490 | CsfAsGfGAfUGfGAfUAGGfCAfGGfUGGsA |
| 999 | AS491 | GsfCsAfCUfGAfGAfAUACfUGfUCfCCUsU |
| 1000 | AS492 | GsfAsGfCAfCUfGAfGAAUfACfUGfUCCsC |
| 1001 | AS493 | AsfCsAfCUfGAfGAAUACfUGfUCfGCfUsC |
| 1002 | AS494 | AsfCsAfCUfGAfGAfAUACfUGfUCfGCUsC |
| 1003 | AS495 | AsfCsAfCUfGAfGAAUACfUGfUCfGCfUCUsU |

(continued)

| SEQ ID NO. | antisense strand code | antisense strand 5'-3' |
|---|---|---|
| 1004 | AS496 | AsfUsUfGAfGGfUCUCAGfGCfAGfCCsfAsC |
| 1005 | AS497 | AsfUsUfGAfGGfUCfUCAGfGCfAGfCCsAsC |
| 1006 | AS498 | AsfUsUfGAfGGfUCUCAGfGCfAGfCCfACsGsG |
| 1007 | AS499 | AsfUsUfGAfGGfUCfUCAGfGCfAGfCCACsGsG |
| 1008 | AS500 | GsfAsUfGGfAUfAGGCAGfGUfGGfACsfUsU |
| 1009 | AS501 | GsfAsUfGGfAUfAGfGCAGfGUfGGfACsUsU |
| 1010 | AS502 | GsfAsUfGGfAUfAGGCAGfGUfGGfACfUUsGsG |
| 1011 | AS503 | GsfAsUfGGfAUfAGfGCAGfGUfGGfACUUsGsG |
| 1012 | AS504 | CsfGsCfAGfGAfUGGAUAfGGfCAfGGsfUsG |
| 1013 | AS505 | CsfGsCfAGfGAfUGfGAUAfGGfCAfGGsUsG |
| 1014 | AS506 | CsfGsCfAGfGAfUGGAUAfGGfCAfGGfUGsGsA |
| 1015 | AS507 | CsfGsCfAGfGAfUGfGAUAfGGfCAfGGUGsGsA |
| 1016 | AS508 | GsfAsGfCAfCUfGAGAAUfACfUGfUCfCCsUsU |
| 1017 | AS509 | GsfAsGfCAfCUfGAfGAAUfACfUGfUCCCsUsU |
| 1018 | AS510 | GsfAsGfAGfCAfCUGAGAfAUfACfUGsfUsC |
| 1019 | AS511 | GsfAsGfAGfCAfCUfGAGAfAUfACfUGsUsC |
| 1020 | AS512 | GsfAsGfAGfCAfCUGAGAfAUfACfUGfUCsCsC |
| 1021 | AS513 | GsfAsGfAGfCAfCUfGAGAfAUfACfUGUCsCsC |
| 1022 | AS514 | GsfCsAfCUfGAfGAAUACfUGfUCfCCfUUsUsU |
| 1023 | AS515 | GsfCsAfCUfGAfGAfAUACfUGfUCfCCUUsUsU |
| 1024 | AS516 | AsfCsAfCUfGAfGAAUACfUGfUCfGCsfUsU |
| 1025 | AS517 | AsfCsAfCUfGAfGAAUACfUGfUCfGCfUUsUsU |
| 1026 | AS518 | AsfCsAfCUfGAfGAfAUACfUGfUCfGCsUsU |
| 1027 | AS519 | AsfCsAfCUfGAfGAfAUACfUGfUCfGCUUsUsU |
| 1142 | AS520 | AsCsACUGfAGAAUfACfUGUCCCsUsU |
| 1143 | AS521 | AsCsACUGfAGAAUfACfUGUCCCsUsC |
| 1144 | AS522 | AsCsACUGfAGAAUfACfUGUCCCsUsG |
| 1145 | AS523 | GsCsACUGfAGAAUfACfUGUCCCsUsU |
| 1146 | AS524 | AsCsACUGfAGAAUfACfUGUCCCsUsC |
| 1147 | AS525 | AsCsACUGfAGAAUfACfUGUCGCsUsC |
| 1148 | AS526 | AsCsACUGfAGAAUfACfUGUCCCsUsA |
| 1149 | AS527 | AsCsACUGfAGAAUfACfUGUCCCsAsU |
| 1150 | AS528 | UsCsACUGfAGAAUfACfUGUCCCsUsU |
| 1151 | AS529 | UsCsACUGfAGAAUfACfUGUCCCsUsU |
| 1152 | AS530 | UsGsAAUAfCUGUCfCCfUUUUAAsGsC |
| 1153 | AS531 | UsfGsAfAUfACfUGfUCfCCfUUfUUfAAsGsC |
| 1154 | AS532 | AsAsUACUfGUCCCfUUfUUAAGCsAsA |
| 1155 | AS533 | AsfAsUfACfUGfUCCCUUfUUfAAGCsAsA |
| 1156 | AS534 | GsAsAUACfUGUCCfCUfUUUAAGsCsA |

(continued)

| SEQ ID NO. | antisense strand code | antisense strand 5'-3' |
|---|---|---|
| 1157 | AS535 | GsfAsAfUAfCUfGUCCCUfUUfUAAGsCsA |
| 1158 | AS536 | AsGsAAUAfCUGUCfCCfUUUUAAsGsC |
| 1159 | AS537 | AsfGsAfAUfACfUGUCCCfUUfUUAAsGsC |
| 1160 | AS538 | GsAsGAAUfACUGUfCCfCUUUUAsAsG |
| 1161 | AS539 | GsfAsGfAAfUAfCUGUCCfCUfUUUAsAsG |
| 1162 | AS540 | UsGsAGAAfUACUGfUCfCCUUUUsAsA |
| 1163 | AS541 | UsfGsAfGAfAUfACUGUCfCCfUUUUsAsA |
| 1164 | AS542 | CsfUsGfAGfAAfUACUGUfCCfCUUUsUsA |
| 1165 | AS543 | CsfUsGfAGfAAfUACUGUfCCfCUUUsUsA |
| 1166 | AS544 | AsCsUGAGfAAUACfUGfUCCCUUsUsA |
| 1167 | AS545 | AsfCsUfGAfGAfAUACUGfUCfCCUUsUsA |
| 1168 | AS546 | CsAsCUGAfGAAUAfCUfGUCCCUsUsA |
| 1169 | AS547 | CsfAsCfUGfAGfAAUACUfGUfCCCUsUsA |
| 1170 | AS548 | UsAsAUAfCUGUCfCCfUUUUAAGCsAsA |
| 1171 | AS549 | UsAsfAUfACfUGfUCfCCfUUfUUfAAGCsAsA |

where, G=2'-O-methylguanylate, A=2'-O-methyladenylate, U=2'-O-methyluridylate, C=2'-O-methylcytidylate, fG=2'-fluoroguanylate, fA=2'-fluoroadenylate, fU=2'-fluorouridylate, fC=2'-fluorocytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate, fGs=2'-fluoro-3'-thioguanylate, fAs=2'-fluoro-3'-thioadenylate, fUs=2'-fluoro-3'-thiouridylate, fCs=2'-fluoro-3'-thiocytidylate, T=thymidylate.

In some embodiments, the antisense strand of the RNA inhibitor of the present invention is selected from a) sequences in Tables 7-1 and 7-2, b) sequences having at least 15 consecutive nucleotides identical to those in a), and c) sequences having no more than 3 nucleotides different from those in a) and b).

In some embodiments, the sense strand and antisense strand of the RNA inhibitor is selected from the following Table 8.

Table 8 RNA inhibitors with modified sequences

| RNA inhibitor | sense strand SEQ ID NO. | sense strand code | antisense strand SEQ ID NO. | antisense strand code |
|---|---|---|---|---|
| DS103 | 407 | S163 | 377 | AS163 |
| DS104 | 408 | S164 | 378 | AS164 |
| DS105 | 409 | S165 | 379 | AS165 |
| DS106 | 410 | S166 | 380 | AS166 |
| DS107 | 411 | S167 | 381 | AS167 |
| DS108 | 412 | S168 | 382 | AS168 |
| DS109 | 413 | S169 | 383 | AS169 |
| DS110 | 414 | S170 | 384 | AS170 |
| DS111 | 415 | S171 | 385 | AS171 |
| DS112 | 416 | S172 | 386 | AS172 |
| DS113 | 417 | S173 | 387 | AS173 |
| DS114 | 418 | S174 | 388 | AS174 |
| DS115 | 419 | S175 | 389 | AS175 |

(continued)

| RNA inhibitor | sense strand SEQ ID NO. | sense strand code | antisense strand SEQ ID NO. | antisense strand code |
|---|---|---|---|---|
| DS116 | 420 | S176 | 390 | AS176 |
| DS117 | 421 | S177 | 391 | AS177 |
| DS118 | 422 | S178 | 392 | AS178 |
| DS119 | 423 | S179 | 393 | AS179 |
| DS120 | 424 | S180 | 394 | AS180 |
| DS121 | 425 | S181 | 395 | AS181 |
| DS122 | 426 | S182 | 396 | AS182 |
| DS123 | 427 | S183 | 397 | AS183 |
| DS124 | 428 | S184 | 398 | AS184 |
| DS125 | 429 | S185 | 399 | AS185 |
| DS126 | 430 | S186 | 400 | AS186 |
| DS127 | 431 | S187 | 401 | AS187 |
| DS128 | 432 | S188 | 402 | AS188 |
| DS129 | 433 | S189 | 403 | AS189 |
| DS130 | 434 | S190 | 404 | AS190 |
| DS131 | 435 | S191 | 405 | AS191 |
| DS132 | 436 | S192 | 406 | AS192 |

In some embodiments, the distribution, targeting or stability of the RNA inhibitor can be altered by introducing ligands of target tissue receptors into the vector. For example, a specific ligand can provide an enhanced affinity for a selected target (e.g., molecule, cell, or cell type, compartment (e.g., cell or organ compartment, body tissue, organ, or region) compared to species where no ligand is present.

Ligands can include naturally occurring substances, such as proteins (such as human serum albumin (HSA), low-density lipoprotein (LDL), or globulin); carbohydrates (such as glucan, pullulan, chitin, chitosan, inulin, cyclodextrin, N-acetylglucosamine, N-acetylgalactosamine, or hyaluronic acid); or lipids. Ligands can also be recombinant or synthetic molecules, such as synthetic polymers, such as synthetic polyamino acids.

Ligands may also include targeting groups, such as cell- or tissue-targeting agents that bind to a specific cell type such as kidney cells, for example, lectins, glycoproteins, lipids or proteins, such as antibodies. The targeting groups can be thyrotropin, melanotropin, lectin, glycoprotein, surfactant protein A, mucin carbohydrate, multivalent lactose, multivalent galactose, N-acetyl-galactosamine, N-acetyl-glucosamine multivalent mannose, multivalent fucose, glycosylated polyamino acids, multivalent galactose, transferrin, bisphosphonate, polyglutamic acid, polyaspartic acid, lipid, cholesterol, steroid, cholic acid, folic acid, vitamin B12, vitamin A, biotin, or RGD peptide or RGD peptide mimetics. In some embodiments, the ligand is a multivalent galactose, for example, N-acetyl-galactosamine.

The sense strand and antisense strand contained in the RNA inhibitors of the present invention can be conveniently and routinely prepared by the well-known technique of solid-phase synthesis. Additionally or alternatively, any other method known in the art for such synthesis, such as liquid-phase synthesis or fermentation, may be used. It is also known to use similar techniques to prepare other oligonucleotides, such as phosphorothioates and alkylated derivatives.

In some embodiments, in addition to standard nucleoside phosphoramidite monomers and non-standard nucleoside phosphoramidite monomers that are commercially available and routinely used in oligonucleotide synthesis, the oligonucleotides or linked nucleotides of the present invention can be synthesized by an automated synthesizer using phosphoramidite method derived from the carrier-nucleoside phosphoramidite monomer.

In some embodiments, the ligand of the present invention is conjugated by conjugating to the 5' end and/or 3' end of the antisense strand, and/or the 5' end and/or 3' end of the sense strand through a carrier structure.

For example, the carrier structure can be conjugated to the 5' end and/or 3' end of the sense strand; or the carrier structure can be conjugated to the 5' end of the antisense strand, and the carrier structure is conjugated to the 3' end of the sense strand; or the carrier structure can be conjugated to the 3' end of the antisense strand, and the carrier structure is

conjugated to the 5' end of the sense strand.

In some embodiments, the carrier structure includes 5'MVIP and 3'MVIP, wherein the 5'MVIP is conjugated at the 5' end of the sense strand and/or antisense strand, the 3'MVIP is conjugated at the 3' end of the antisense strand and/or sense strand, the structure of the 5'MVIP is shown in formula I, the structure of the 3'MVIP is shown in formula II,

$$\mathbf{(X\text{-}L)_n\text{-}B\text{-}D\text{-}R_1\text{-},} \qquad \text{I}$$

$$\mathbf{(X\text{-}L)_m\text{-}B\text{-}D\text{-}R_2\text{-},} \qquad \text{II}$$

wherein,

X is a liver targeting specific ligand;
L is a branched chain;
B is a linker;
D is a linking chain;
$R_1$ and $R_2$ are transition points;
The 5'MVIP is connected with the 5' end of the sense strand or the 5' end of the antisense strand through the transition point $R_1$, and the 3'MVIP is connected with the 3' end of the sense strand or the 3' end of the antisense strand through the transition point $R_2$. n and m are each independently any integer of 0-4, and n+m=an integer of 2-6, preferably n+m=2, 3 or 4, more preferably 4.

In some embodiments, the $R_1$ or $R_2$ is connected to the sense or antisense strand through a phosphate or modified phosphate, preferably through a phosphate or phosphorothioate.

In some embodiments, m or n can be 0, that is, there is no 3'MVIP or 5'MVIP.

In some embodiments, when n=0 (that is, there is no 5'MVIP), the structure of the 3'MVIP can be

m=2, 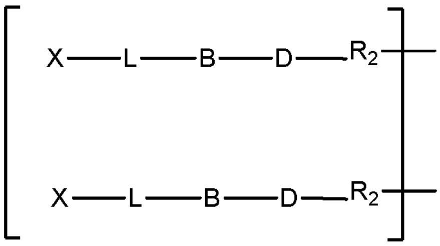

m=2, 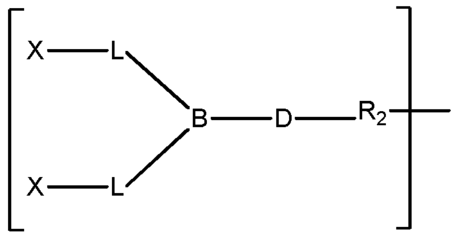

m=3, 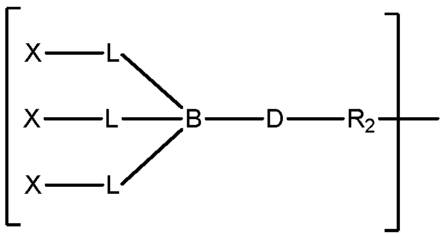

m=3, 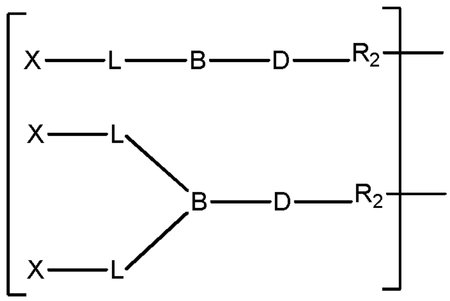

m=4, 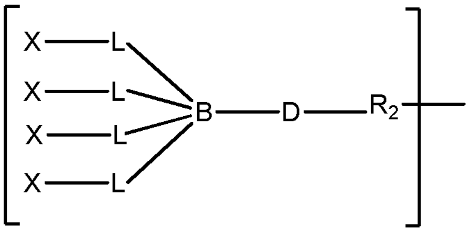

m=4, 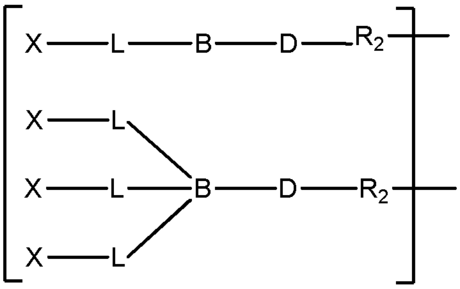

m=4, 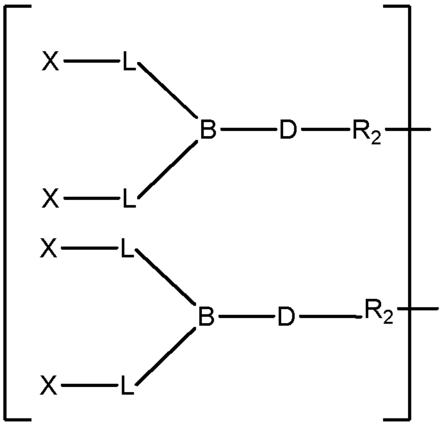

In some embodiments, when n=1, the structure of the 3'MVIP can be

m=1, 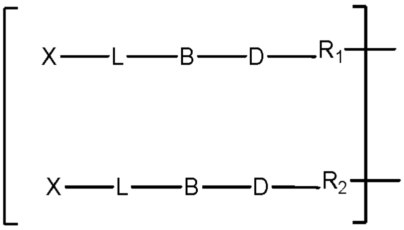

m=3, 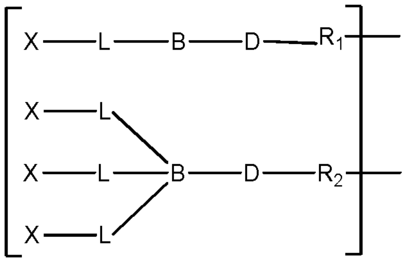

m=2, 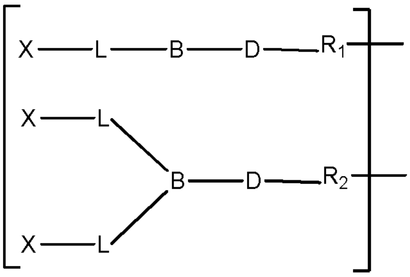

m=4, 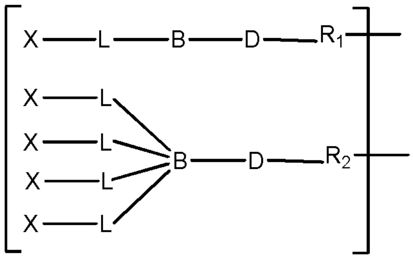

In some embodiments, when n=2, the structure of the 3'MVIP can be

m=0, 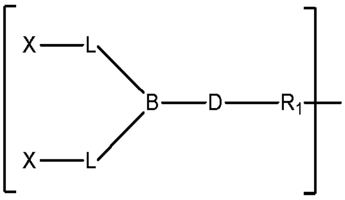

m=0, 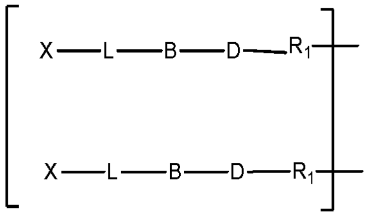

m=1, 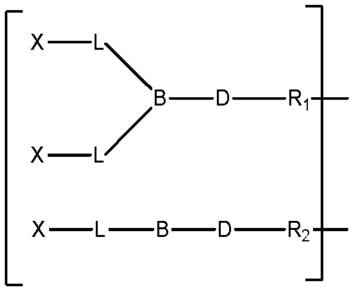

m=2, 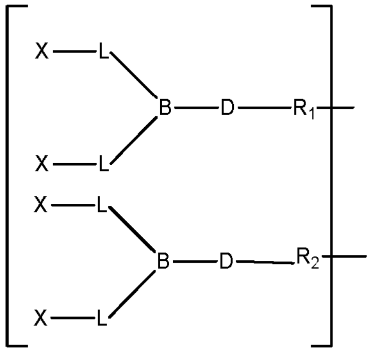

m=3, 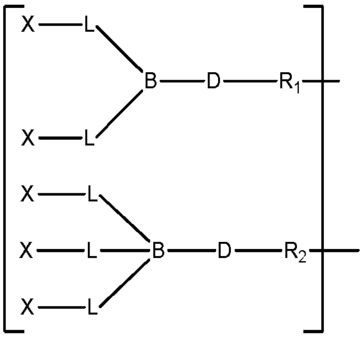

m=4, 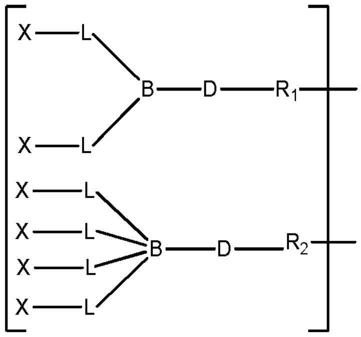

。

In some embodiments, when n=3, the structure of the 3'MVIP can be:

m=0, 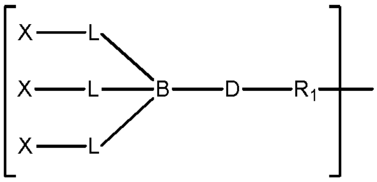

m=0, 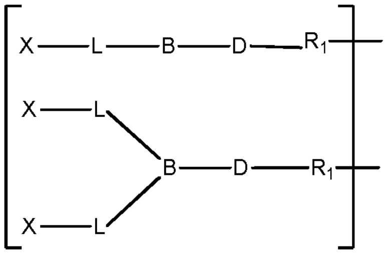

m=1, 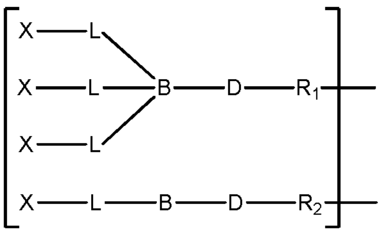

m=2, 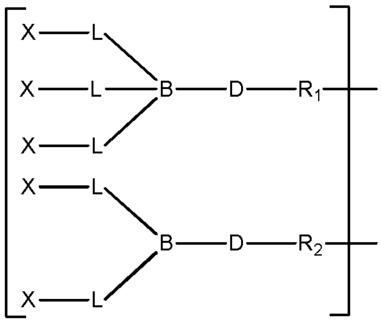

m=3, 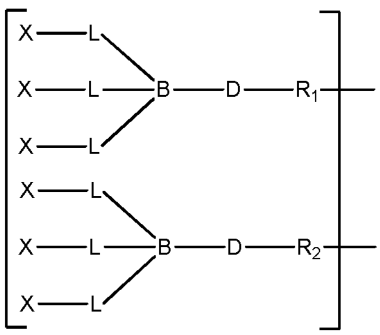

。

In some embodiments, when n=4, the structure of the 3'MVIP can be:

m=0, 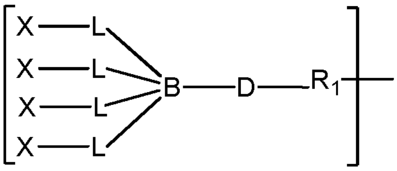

m=0, 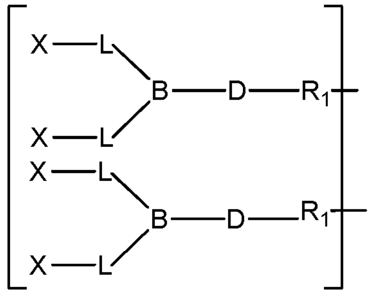

m=0, 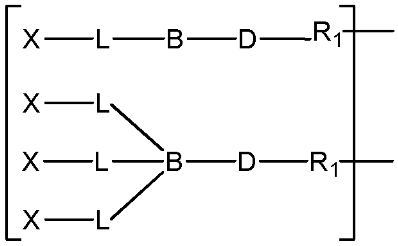

m=1, 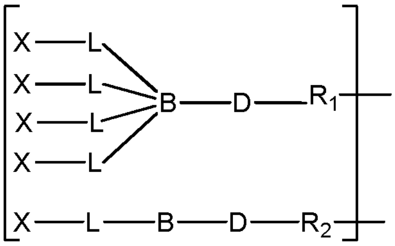

m=2, 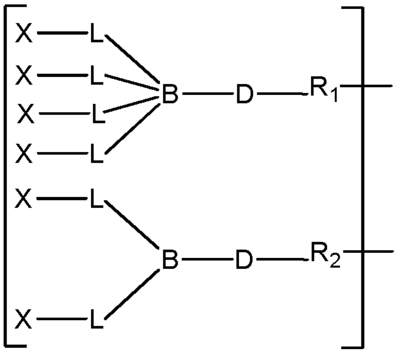

。

In some embodiments, the n refers to the sum of n in the 5'MVIP at 5' ends of the sense strand and antisense strand of the RNAinhibitor respectively, and the m refers to the sum of m in the 3'MVIP at 3' ends of the sense strand and antisense strand of the RNA inhibitor respectively.

In some embodiments, the transition points $R_1$ and $R_2$ contain -NH-, -S-, and/or -O- in their structures, $R_1$ and $R_2$ are respectively connected with the linking chain D and the 5' and 3' ends of the sense strand and/or antisense strand through -NH-, -S-, or -O- in the structure., and $R_1$ and $R_2$ are the same or different.

In some embodiments, the $R_1$ and $R_2$ are optionally straight chain, or straight chain or cyclic structure with amido, carboxyl, or alkyl branched chain, and the cyclic structure includes saturated or unsaturated aliphatic carbocyclyl, or five or six membered heterocyclyl or aromatic hydrocarbyl containing sulfur, oxygen, or nitrogen atom.

In some embodiments, the $R_1$ and/or R2 are $-E_1(CH_2)_xCH_2E_2-$, wherein x is any integer of 3-12, and the groups $E_1$ and $E_2$ can be -NH-, -S-, or -O-, respectively.

In some embodiments, the $R_1$ and/or $R_2$ are $-E_1(CH_2)_{x1}CH(OH)(CH_2)_{x2}E_2-$, wherein x1 or x2 are each independently any integer of 3-10, and $E_1$ and $E_2$ can be -NH-, -S-, or -O-, respectively.

In some embodiments, the $R_1$ is a heterocyclic or carbocyclic structure containing N, S or O as shown below.

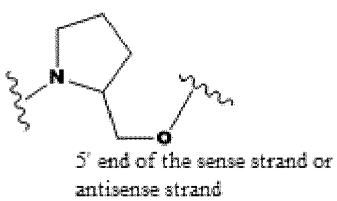


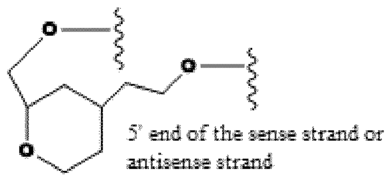

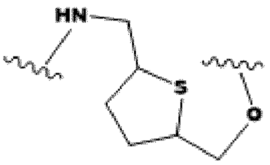

5' end of the sense strand or antisense strand

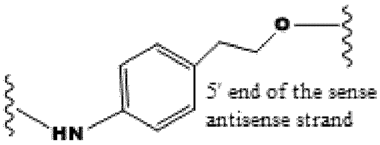

5' end of the sense strand or antisense strand

In some embodiments, the transition point $R_1$ is $-NH(CH_2)_xCH_2O-$, wherein x is any integer of 3-12, preferably any integer of 4-6, which can be introduced by the following two phosphoramidite monomers:

i. One of -O- or -S- in structure of $R_1$ is used for the synthesis of $R_1$ phosphoramidite monomer, and is connected to the 5' end of the sense strand or antisense strand of RNA inhibitor by solid-phase synthesis. In the structure, -NH-, -S-, or -O- is used to connect with the linking chain D in the 5'MVIP, thereby introducing the liver targeting specific ligand X at the 5' end of the sense strand or antisense strand of the RNA inhibitor. An exemplary structure of a monomer introduced to the 5' end of the sense or antisense strand of the RNA inhibitor is as follows.

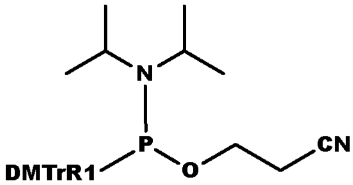

In some embodiments, the following structure is preferred:

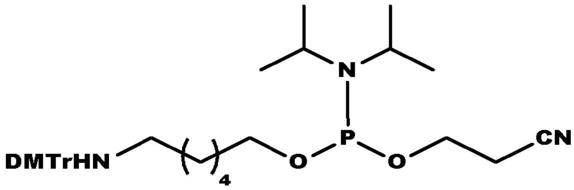

ii. One of -NH-, -S-, or -O- in structure of $R_1$ is first connected with the linking chain D, and another -NH-, -S-, or -O- is used to form ester with phosphoramidite in the synthesis of 5'MVIP phosphoramidite monomer. The exemplary structure of 5'MVIP phosphoramidite monomer of sense strand or antisense strand is as follows:

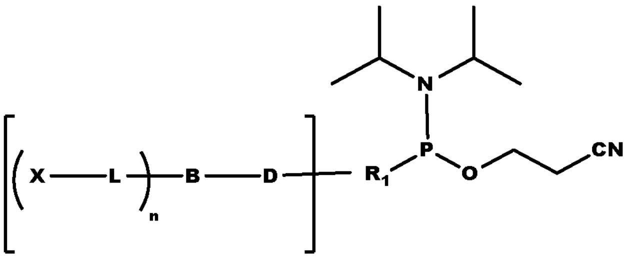

In some embodiments, the 5'MVIP phosphoramidite monomer of the sense strand or antisense strand preferably has the following structure:

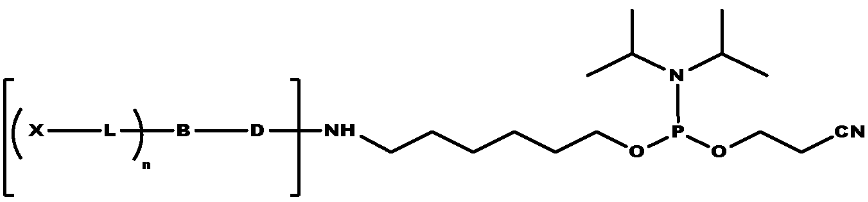

When n in the general formula is 1-4, the linker B part of the above monomer is branched for 1 to 4 times respectively to obtain the corresponding monomer compounds. With the help of the above monomer compounds, the liver targeting specific ligand X is introduced to the 5' end of the sense or antisense strand through solid-phase synthesis.

In some embodiments, the transition point $R_1$ is $-NH(CH_2)_xCH_2O-$, wherein x can be an integer of 3-12, preferably an integer of 4-6.

In some embodiments, the 5'MVIP phosphoramidite monomer has the structure selected from the following structures:

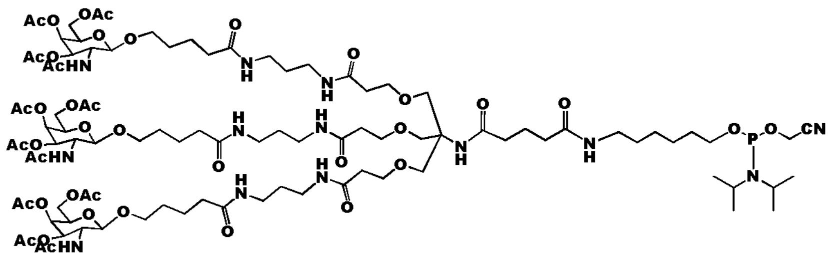

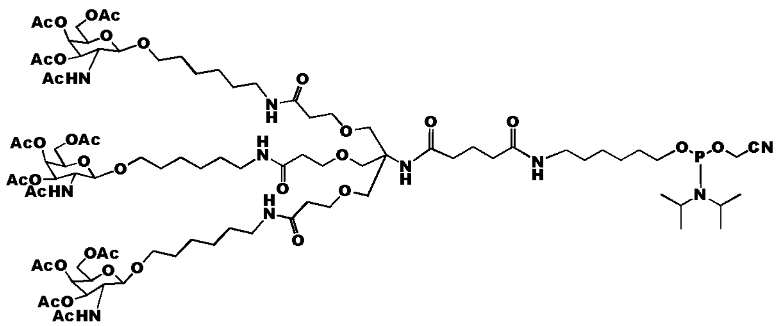

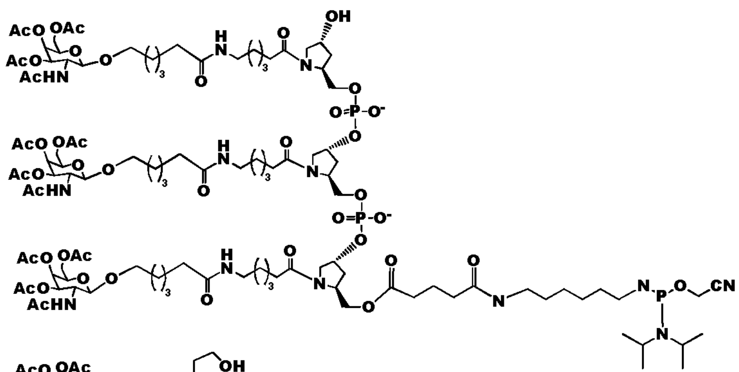

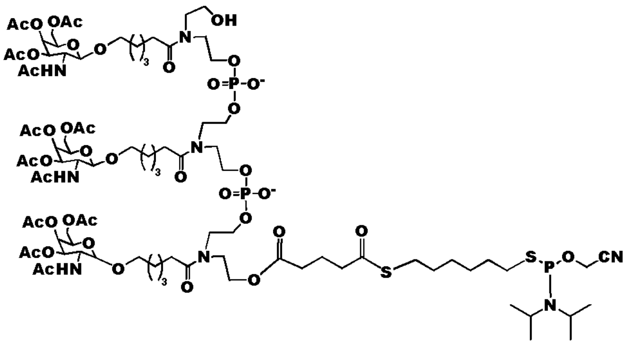

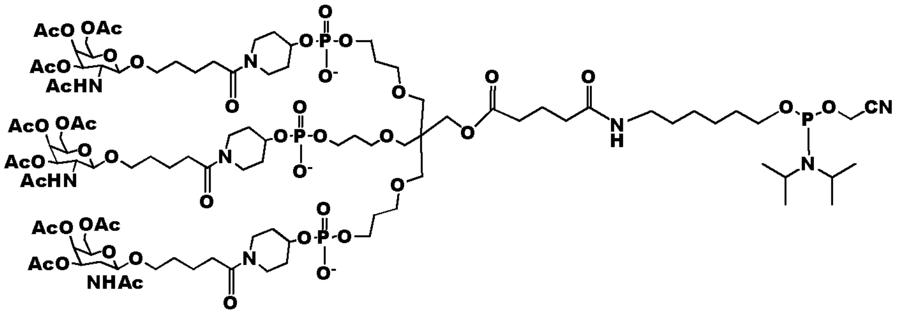

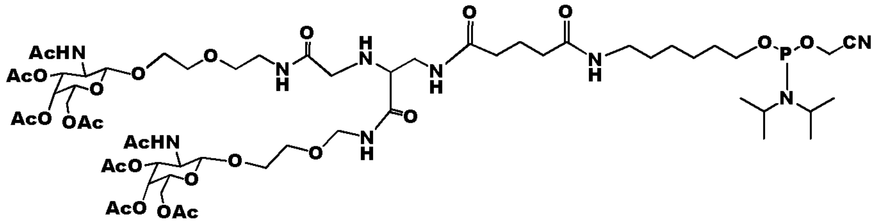

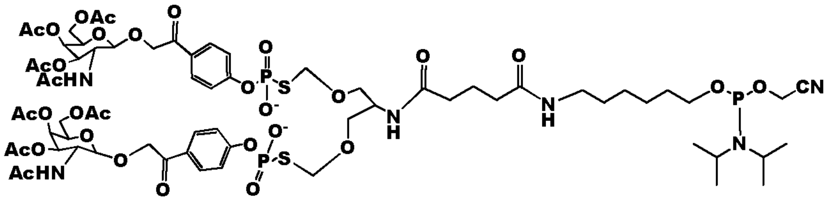

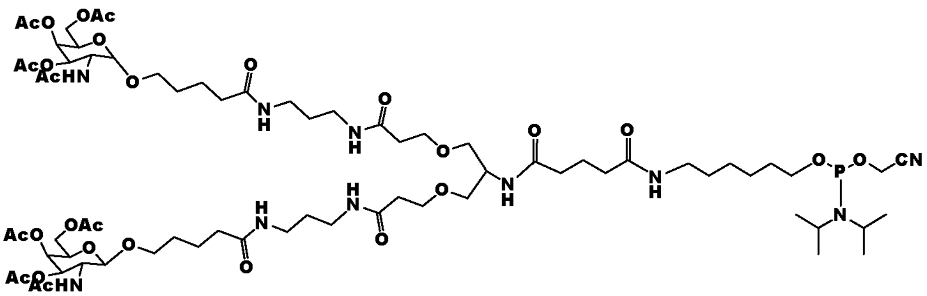

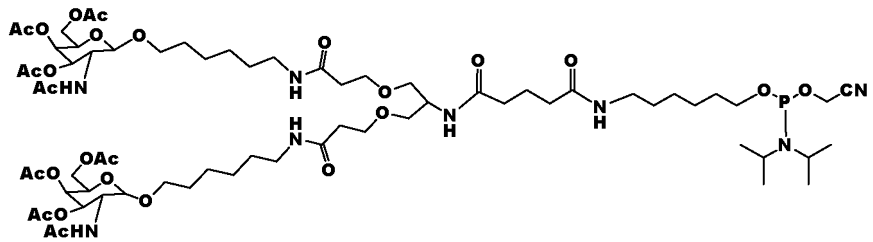

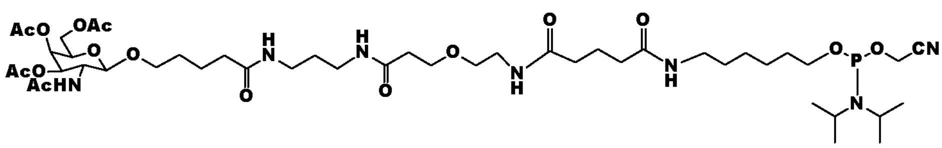

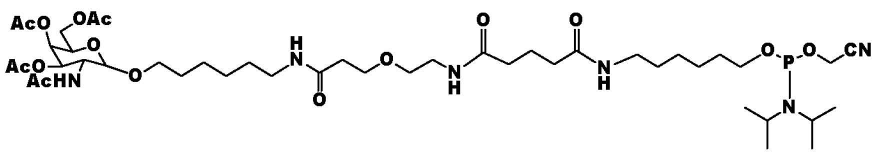

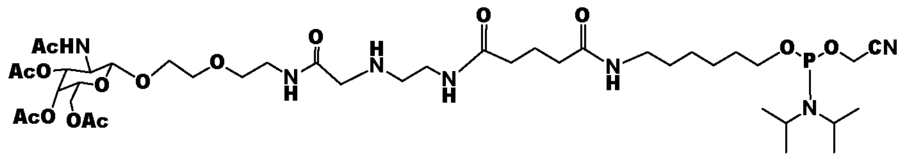

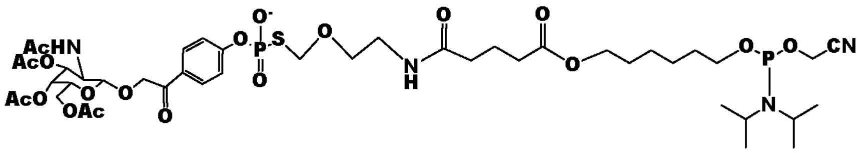

。

In some embodiments, the transition point $R_2$ is a heterocyclic or carbocyclic structure containing N, S or O as shown below:

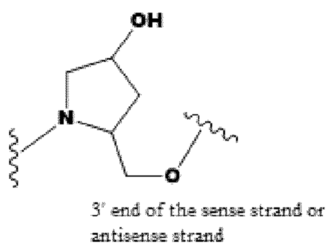


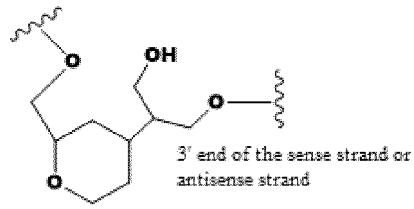


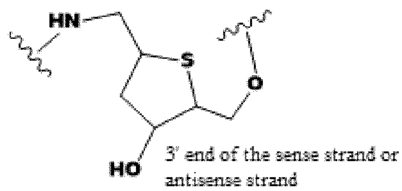


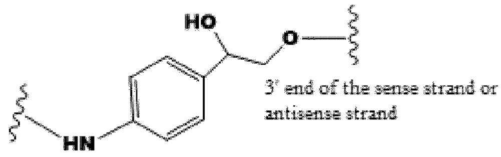


.

In some embodiments, the transition point $R_2$ is $-NH(CH_2)_{x1}CH(OH)(CH_2)_{x2}CH_2O-$, wherein x1 is any integer of 1-4, x2 is any integer of 0-4.

The transition point $R_2$ described in the present invention forms ester or amide with -NH-, -S-, or - O- in the structure of $R_2$ through succinic anhydride, and at the same time conjugates with -NH- in blank solid support to form 3'MVIP solid support, and then 3'MVIP is introduced to the 3' end of the sense strand or antisense strand by phosphoramidite solid-phase synthesis.

In some embodiments, the heterocycle in the structure of transition point $R_2$ is a pyrrole ring or a piperidine ring, which is connected with the linking chain D of 3'MVIP through the nitrogen heteroatom in the ring. The exemplary structures of the introduced 3'MVIP solid support are as follows:

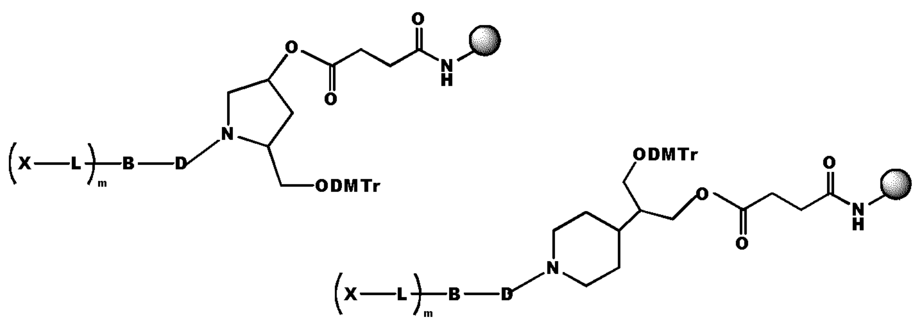

When m in the general formula is 1-4, the linker B part in the monomer above is branched for 1 to 4 times respectively to obtain the corresponding Solid Supports.

In some embodiments, the transition point $R_2$ is $-B_4(CH_2)_{x1}CH(OH)(CH_2)_{x2}CH_2B_5-$, wherein x1 is any integer of 1-4, x2 is any integer of 0-4, and $B_4$ and $B_5$ are -NH-, -S-, or -O- respectively, the exemplary formula of the introduced 3

'MVIP solid spport is as follows:

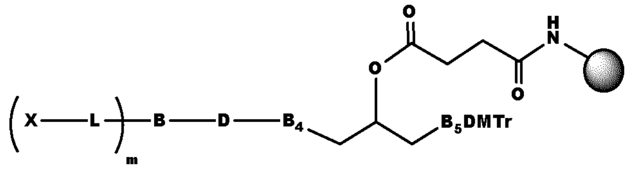

When m in the general formula is 1-4, the linker B part in the monomer above is branched for 1 to 4 times respectively to obtain the corresponding Solid Supports.

In some embodiments, $R_2$ is $-NHCH_2CH(OH)CH_2O-$, the exemplary formula of the introduced 3 'MVIP solid spport is as follows:

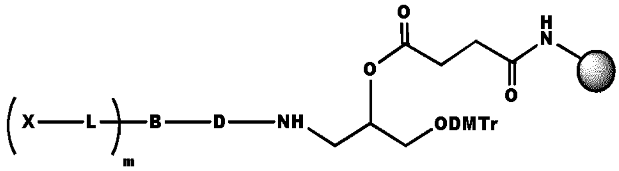

When m in the general formula is 1-4, the linker B part in the monomer above is branched for 1 to 4 times respectively to obtain the corresponding Solid Supports.

In some embodiments, the structure of 3'MVIP solid support is as follows:

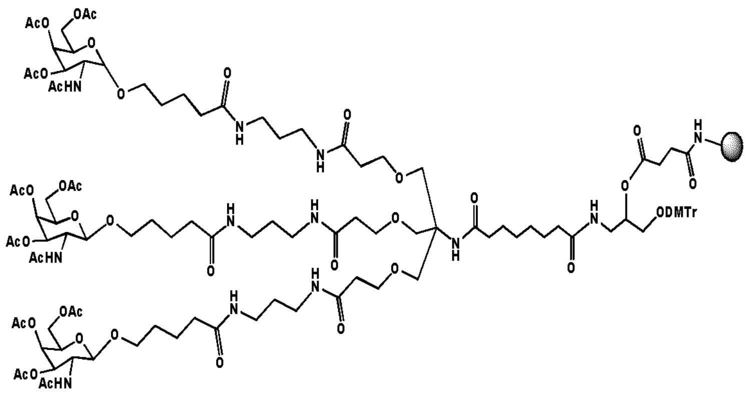

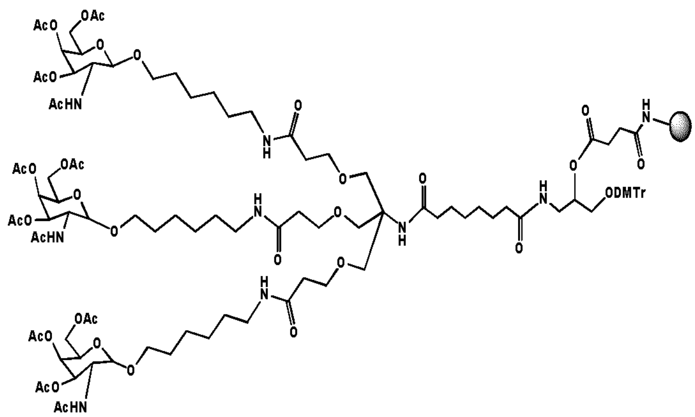

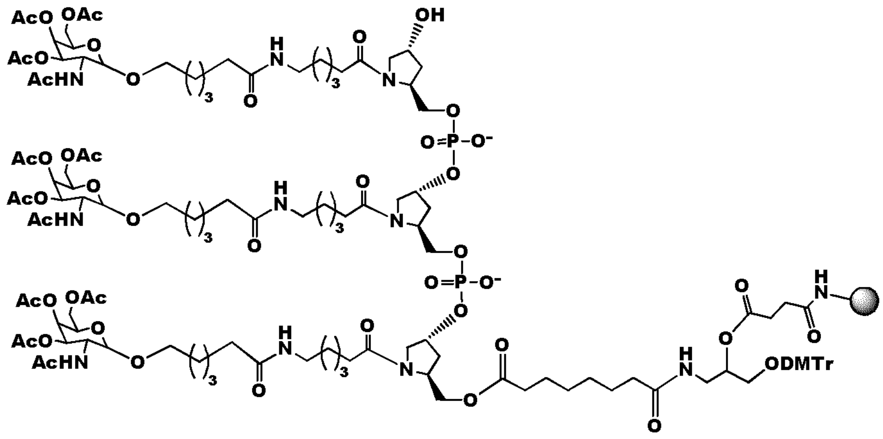

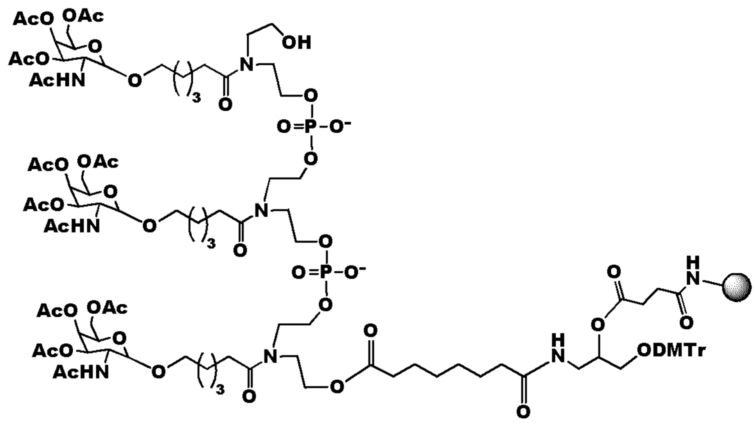

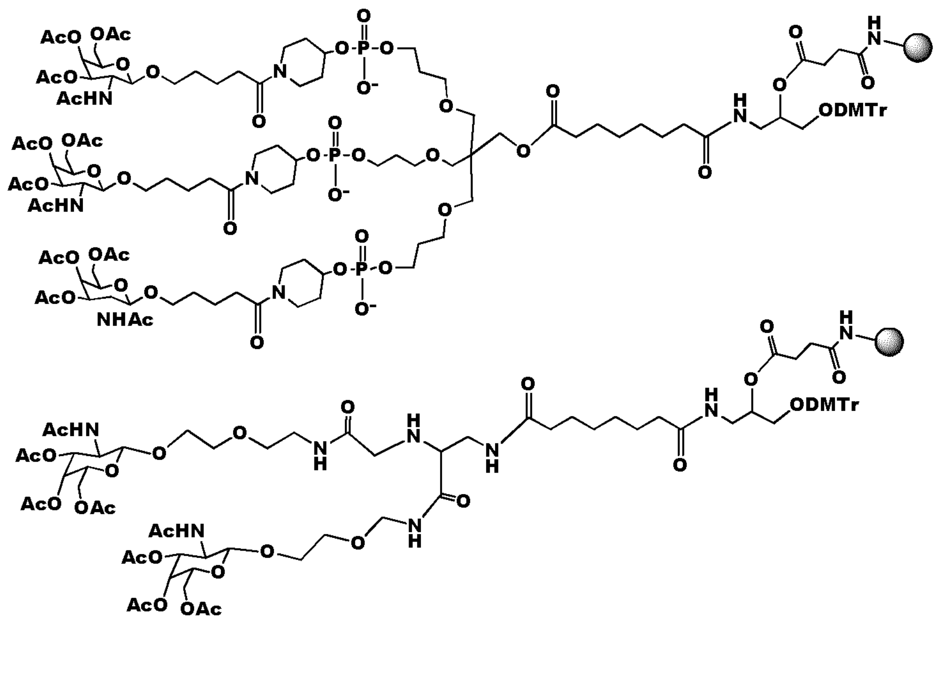

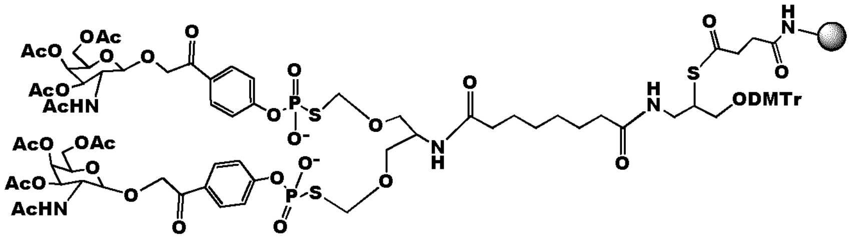

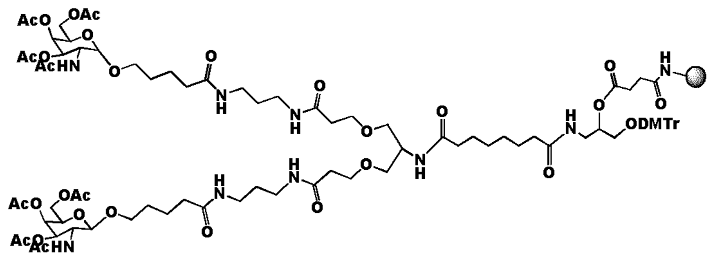

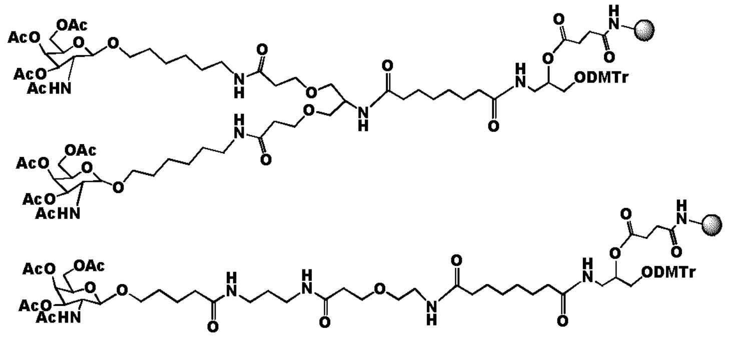

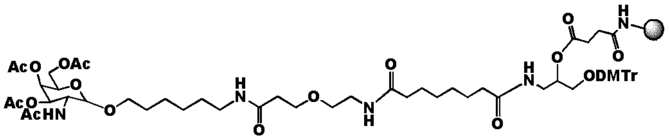

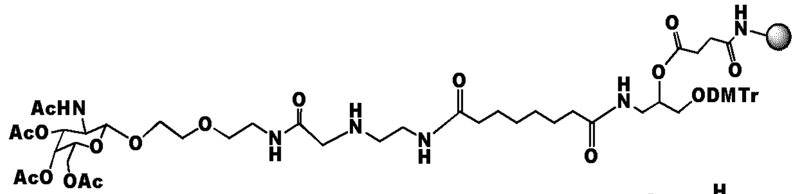

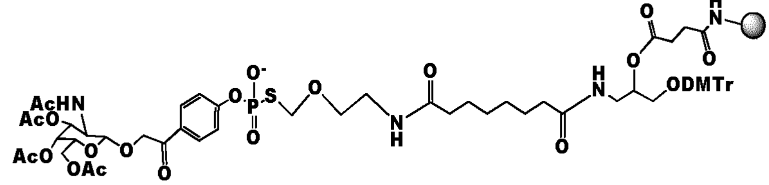

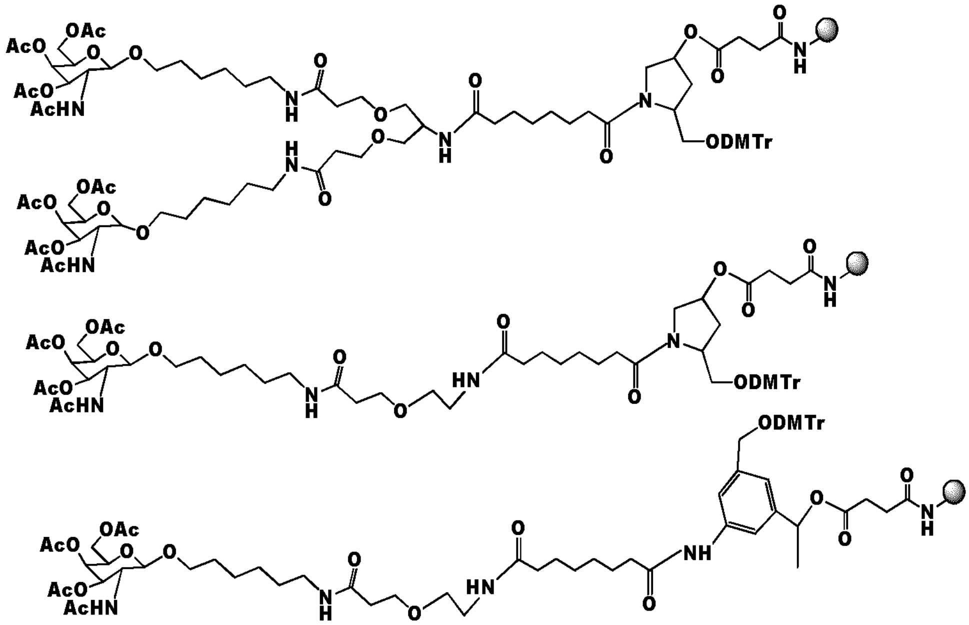

In some embodiments, the liver targeting specific ligand X is selected from structures used to enhance the uptake of RNA inhibitor by hepatocytes, which can be lipids, steroids, vitamins, sugars, proteins, peptides, polyamines and peptide mimetic structures. In the RNA inhibitor provided by the present invention, the liver targeting specific ligand X introduced to the end of the sense strand or antisense strand of the RNA inhibitor can be the same or different. For example, in terms of characteristics, some can enhance liver targeting, some can be the structure that regulates the pharmacokinetics of the RNA inhibitor in vivo, and some can be the structure with dissolution activity in vivo. In some embodiments, the liver targeting specific ligand X is selected from one or more monosaccharides and derivatives thereof in the following structures.

In some embodiments, the monosaccharide is selected from one or more of the following structures: mannose, galactose, D-arabinose, glucose, fructose, xylose, glucosamine, ribose. The monosaccharide derivatives is selected from mannose derivatives, galactose derivatives, glucose derivatives, ribose derivatives, and other derivatives.

In some embodiments, the liver targeting specific ligand X is selected from galactose, galactosamine, N-acetylgalactosamine and their derivatives, and its general structural formula is as follows:

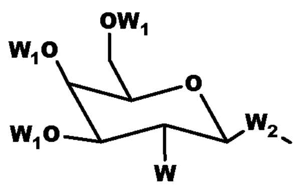

wherein, $W_1$ is a hydrogen or hydroxyl protecting group, which can be the same or different; W is -OH, -NHCOOH or $-NHCO(CH_2)qCH_3$, wherein q is an integer of 0-4; $W_2$ is -NH-, O, S or C.

In some embodiments, the liver targeting specific ligand X is N-acetylgalactosamine and its derivatives.

In some embodiments, the liver targeting specific ligand X is selected from the following structures:

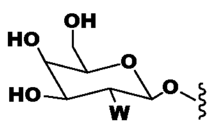 ; 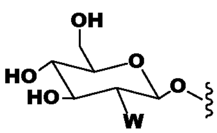 ; 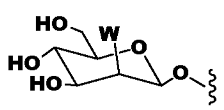 ;

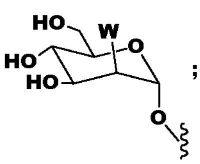 ; 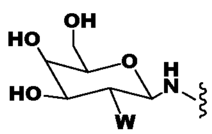 ; 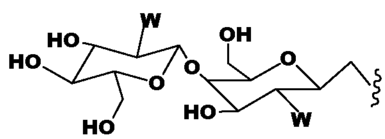 ;

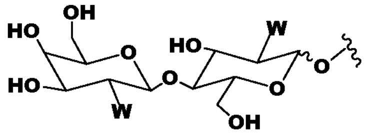 ; 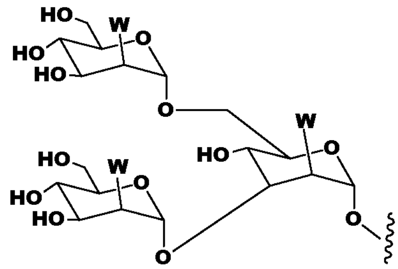 ;

wherein W is selected from one or two of -OH, -NHCOOH or $-NHCO(CH_2)_qCH_3$, wherein q is an integer of 0-4.

In some embodiments, the liver targeting specific ligand X may be the same or different in the same 5'MVIP or 3'MVIP structure.

In some embodiments, the X between 5'MVIP and 3'MVIP can be the same or different.

In some embodiments, the branched chain L is a $C_4$-$C_{18}$ carbon chain containing -NH-, -C(=O)-,-O-, -S-, amido, phosphoryl, thiophosphoryl, $C_4$-$C_{10}$ aliphatic carbocyclyl, phenyl, or a combination of these groups.

In some embodiments, the branched chain L also has a side chain of hydroxyethyl or carboxylic acids group.

In some embodiments, the branched chain L is a $C_7$-$C_{18}$ carbon chain containing amido group or six-membered aliphatic carbocyclyl group.

In some embodiments, the branched chain L is selected from one or more of the following structures:

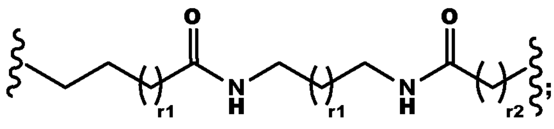 ; 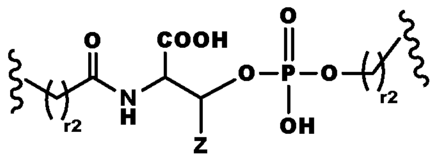 ;

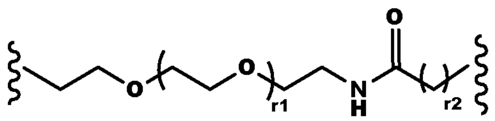 ; 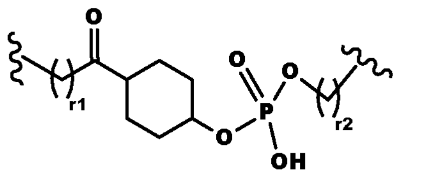 ;

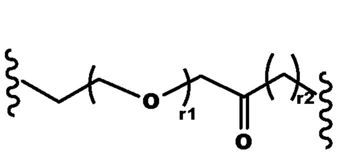 ; 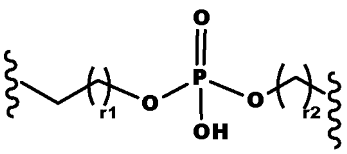 ; 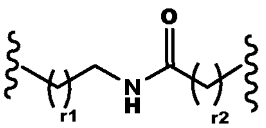 .

wherein, r1 is any integer of 1-12, r2 is any integer of 0-20, and Z is H, alkyl or amido group, the alkyl is such as $C_1$-$C_5$ alkyl.

In some embodiments, the structure of the linker B is related to the number of X that can be introduced. The linker B contains -NH-, C, O, S, amido, phosphoryl, thiophosphoryl. When n or m is 1, it is a straight chain, and when n or m is 2, 3, or 4, the number of branches is 2, 3, or 4 respectively.

In some embodiments, the linker B is selected from the following structures:

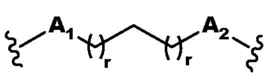 ; 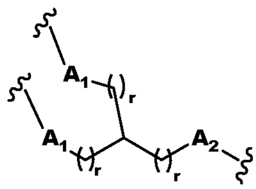 ; 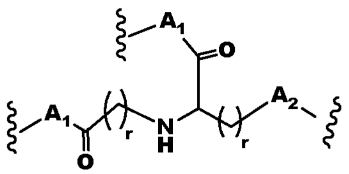 ;

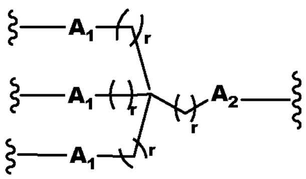 ; 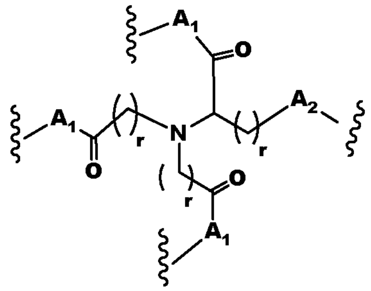 ; 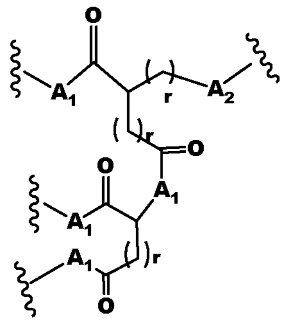 ;

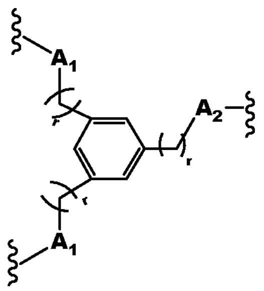 ; 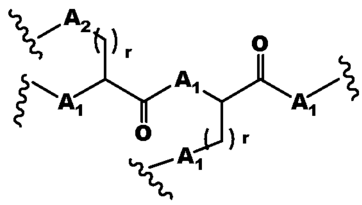 ;

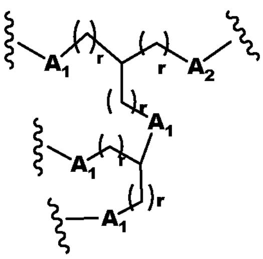 ; 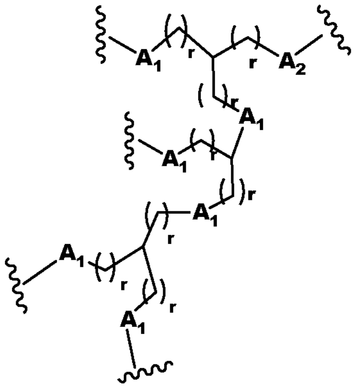 ; 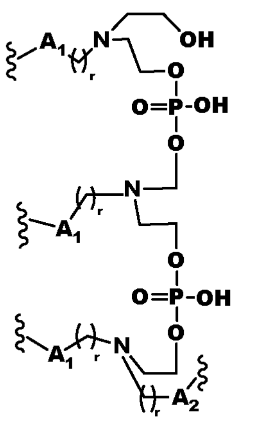 .

wherein, $A_1$ and $A_2$ are each independently C, O, S, -NH-, carbonyl, amido, phosphoryl or thiophosphoryl, and r is an integer of 0-4.

In some embodiments, the linker B is selected from the following structures:

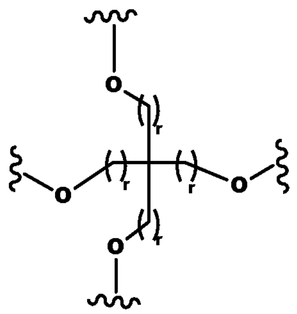 ; 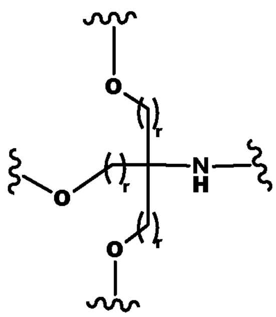 ; 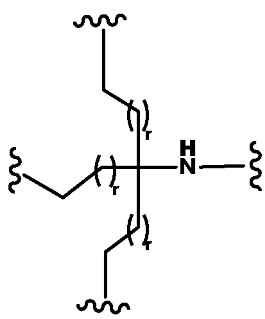 ;

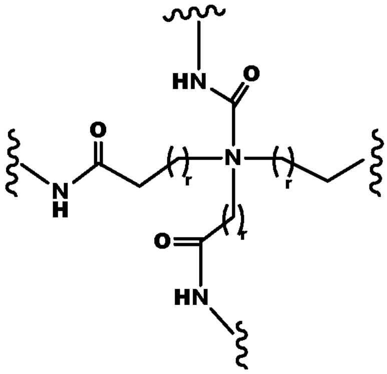 ; 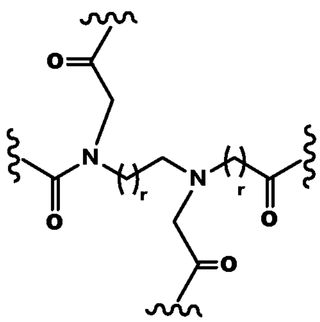 ; 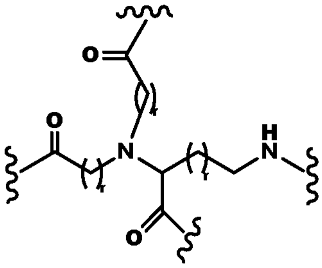 ;

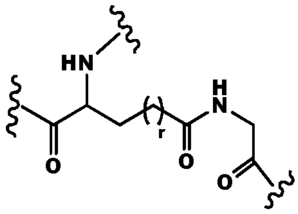 ; 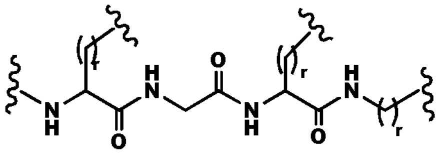 ; 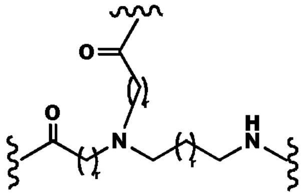 ;

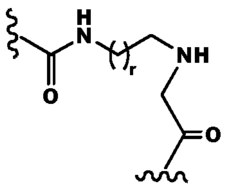 ; 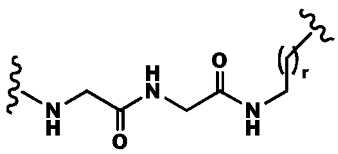 ; 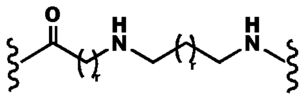 ;

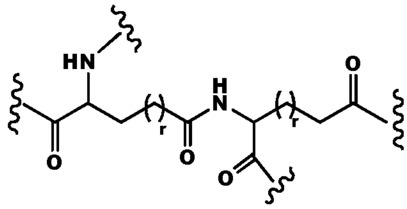 ; 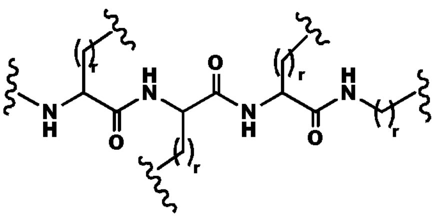 ;

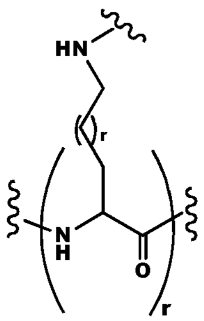 ; 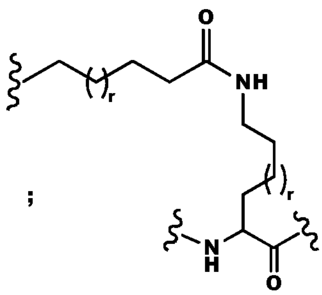 ; 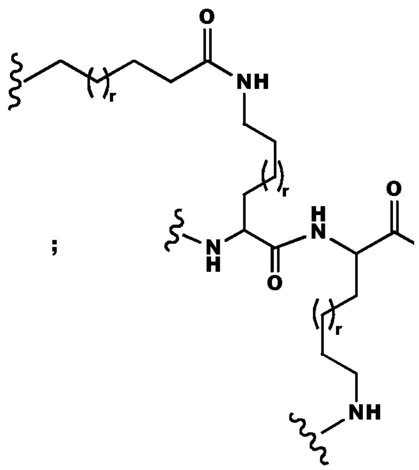

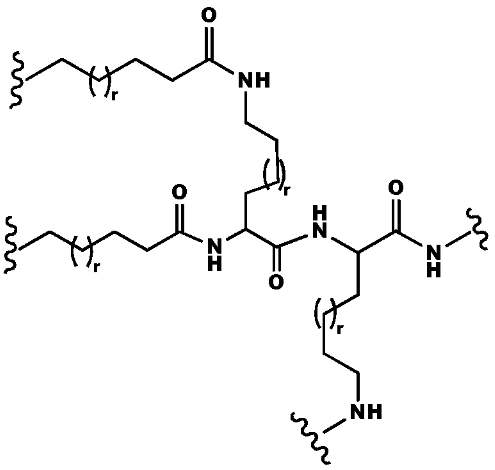 ; 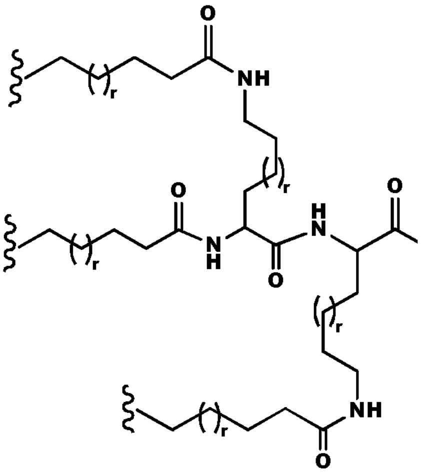

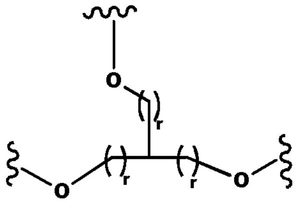 ; 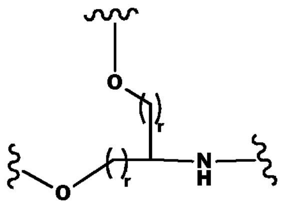 ; 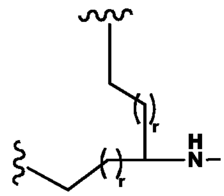

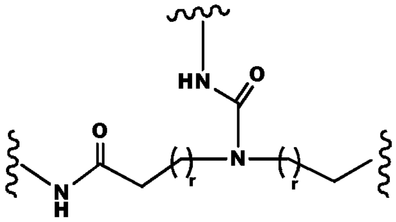 ; 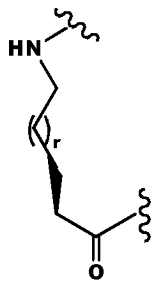 ; 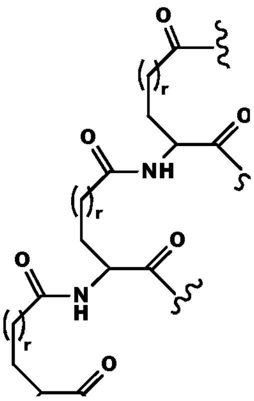

wherein, r is any integer of 0-4.

In some embodiments, the linker B is selected from the following structures:

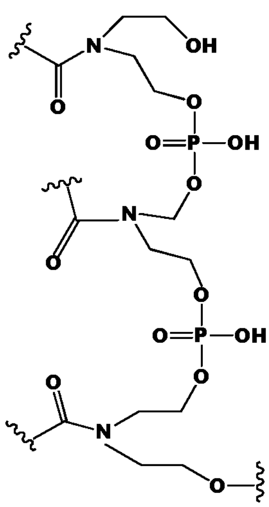 ; 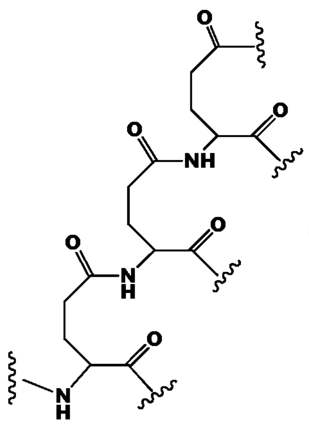 ; 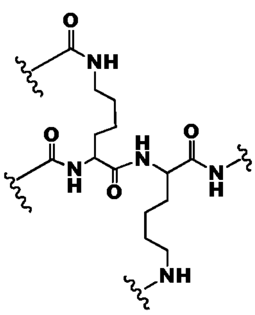,

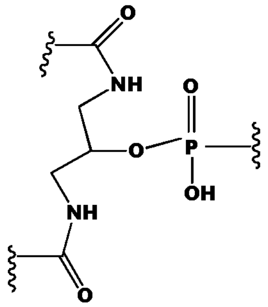 ; 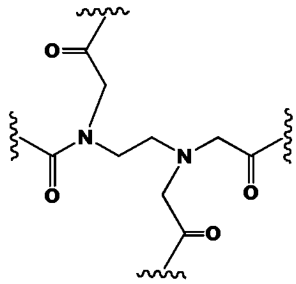 ; 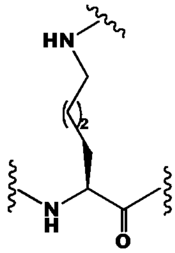 ;

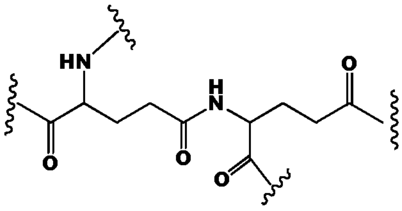 ; 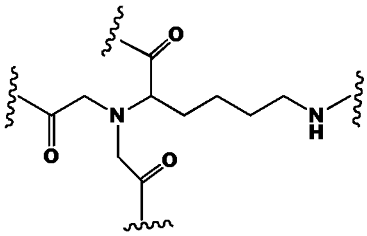 ; 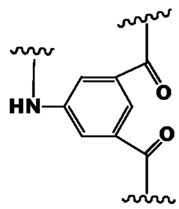 ;

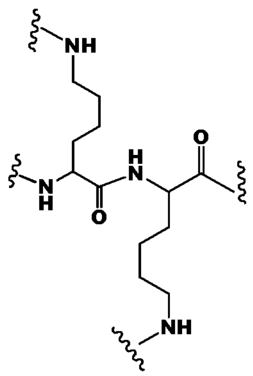 ; 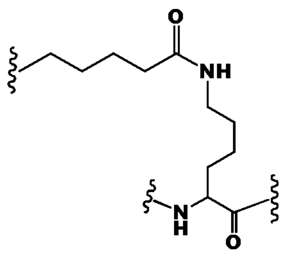 ; 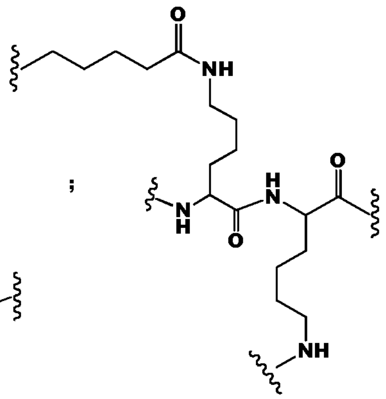 ;

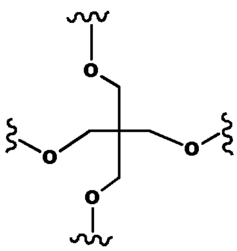 ; 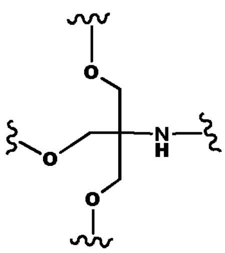 ; 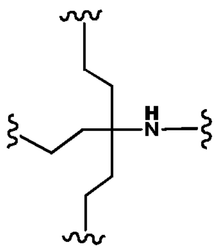 ; 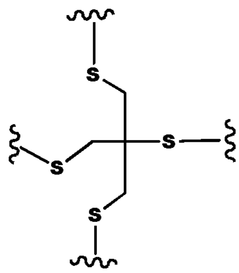 ;

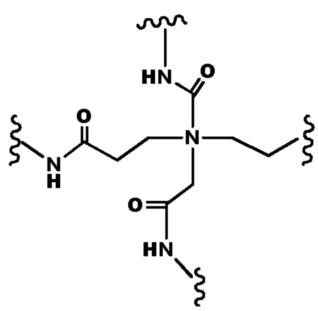 ; 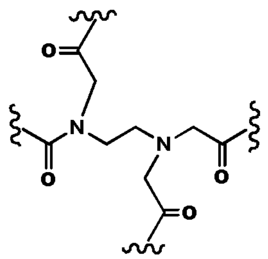 ; 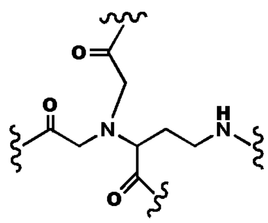 ;

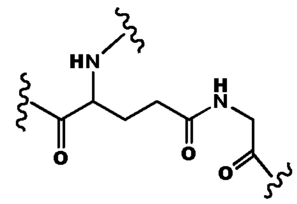 ; 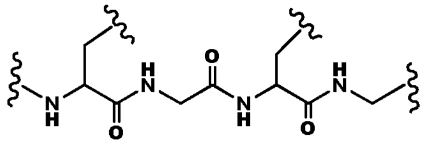 ; 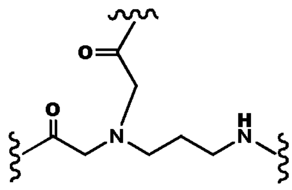 ;

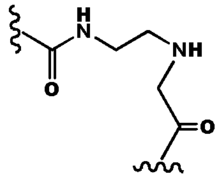 ; 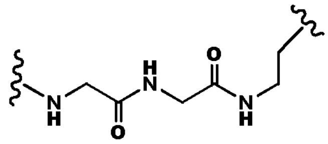 ; 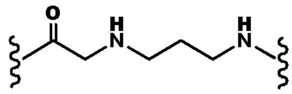 ;

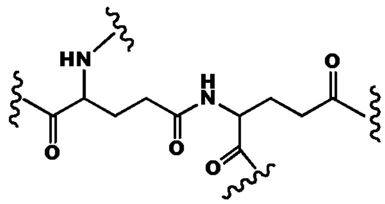 ; 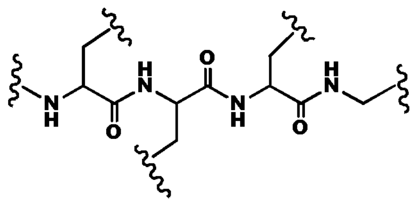 ;

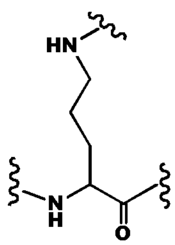 ; 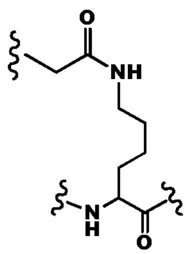 ; 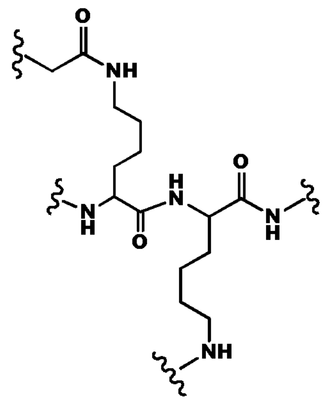 ;

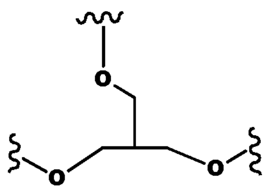 ; 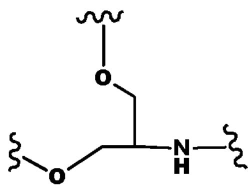 ; 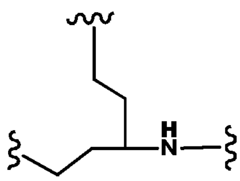 ; 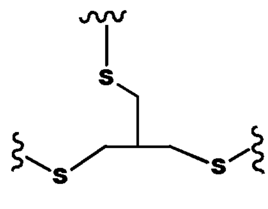 ;

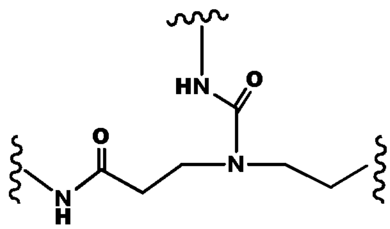 ; 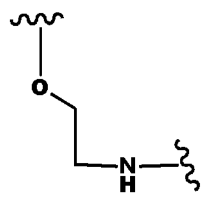 ; 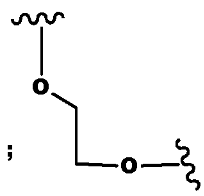 ; 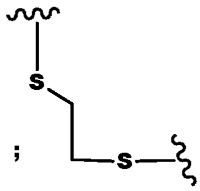

In some embodiments, the linker B is selected from the following structures:

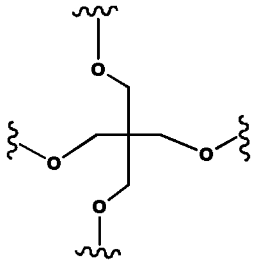 ; 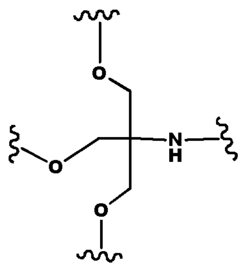 ; 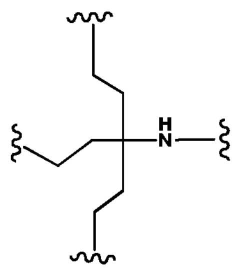 ; 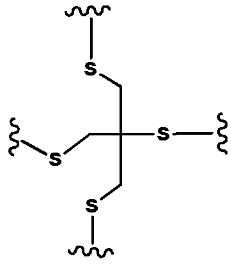

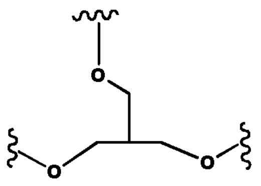 ; 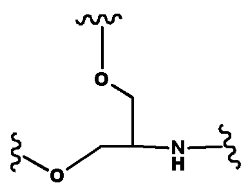 ; 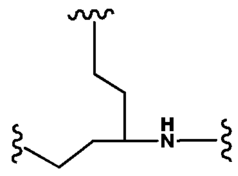 ; 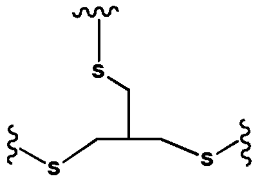

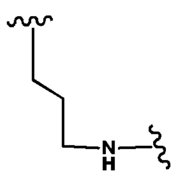 ; 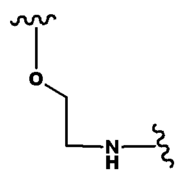 ; 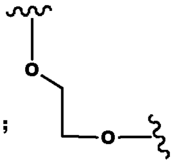 ; 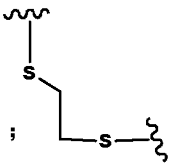

In some embodiments, the linking chain D is a $C_3$-$C_{18}$ carbon chain containing -NH-, C=O, O, S, amido, phosphoryl, thiophosphoryl, aromatic hydrocarbyl, $C_4$-$C_{10}$ aliphatic carbocyclyl, five- or six-membered heterocyclyl containing 1-3 nitrogen, or a combination of these groups.

In some embodiments, the linking chain D also has side chains of hydroxymethyl, methyl tert-butyl, methylphenoyl, and $C_5$-$C_6$ aliphatic cyclic groups.

In some embodiments, the linking chain D is a $C_3$-$C_{10}$ carbon chain containing two C=O, six-membered aliphatic carbocyclyl or phenyl groups.

In some embodiments, the linking chain D is a $C_3$-$C_{10}$ carbon chain containing two C=O.

In some embodiments, the linking chain D is selected from the following structures:

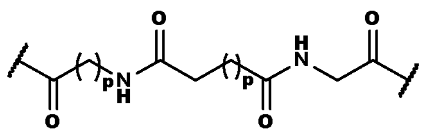 ; 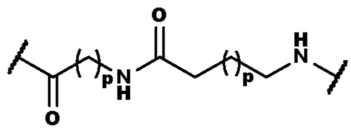 ; 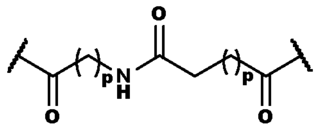 ;

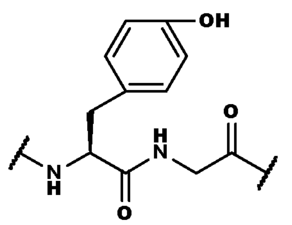 ; 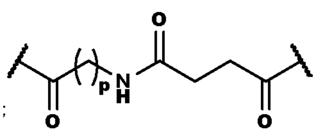 ; 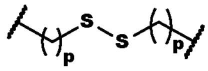 ; 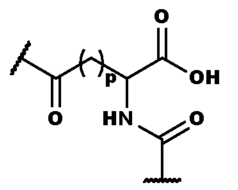 ;

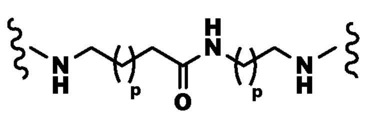 ; 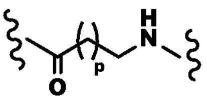 ; 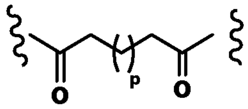 ;

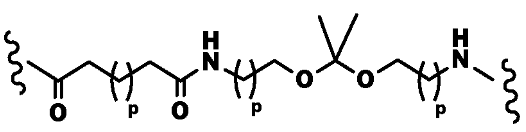 ; 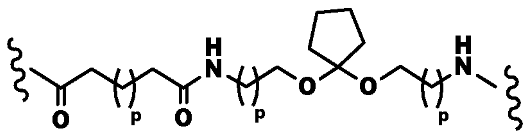 ;

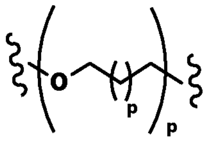 ; 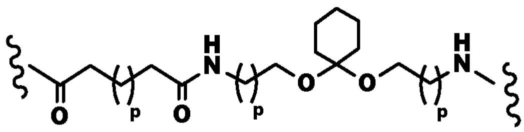 ; 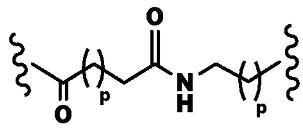 ;

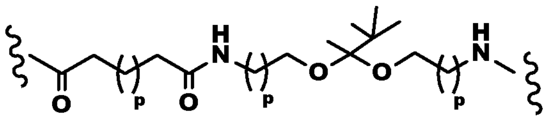 ; 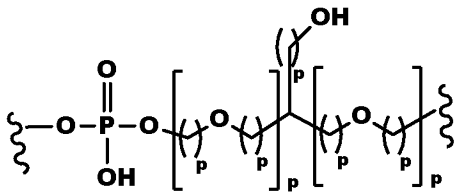 ;

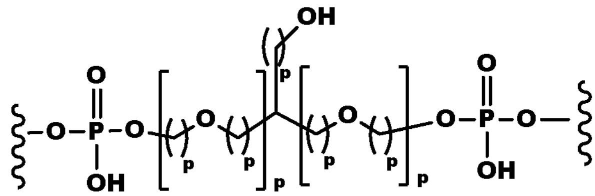 ; 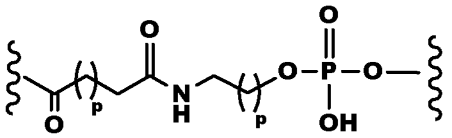 ;

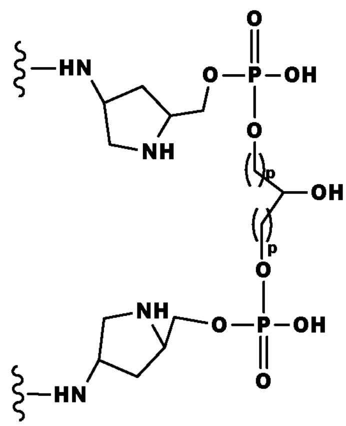 ; 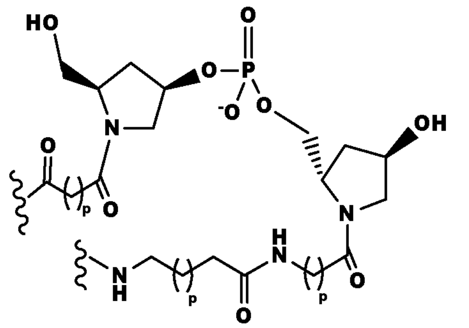 ;

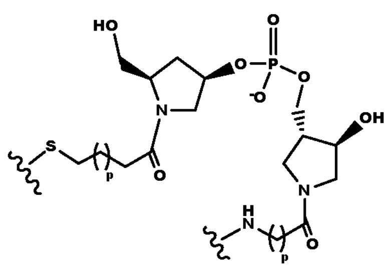 ; 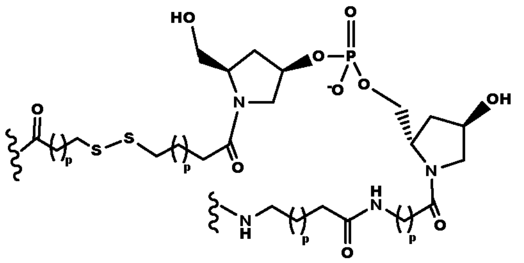 ;

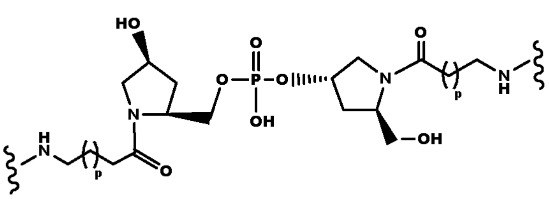 ;

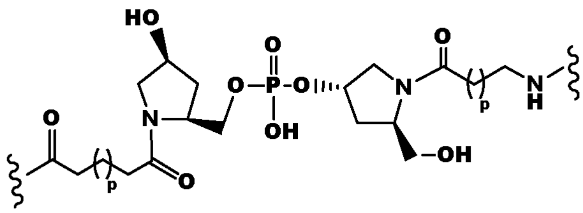
;

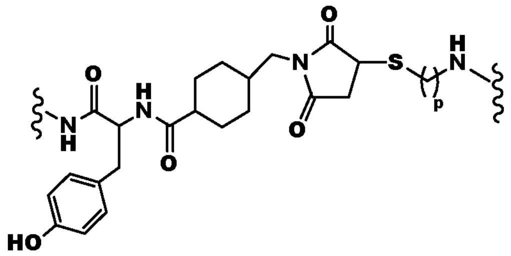
;

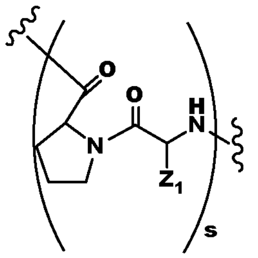
;
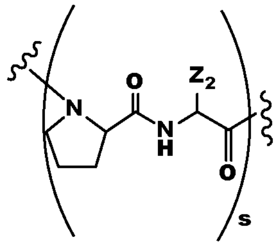
;
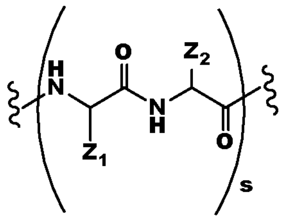
;

wherein, each p is independently any integer of 1-20; s is an integer of 2-13; $Z_1$ and $Z_2$ are the same or different substituents, such as $C_3$-$C_{10}$ alkyl.

In some embodiments, the linking chain D is selected from the following structures:

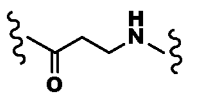
;
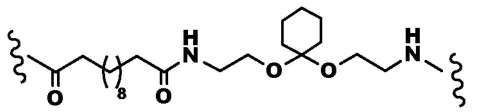
;
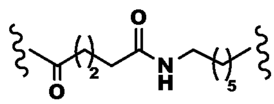
;

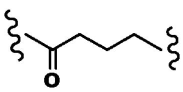
;
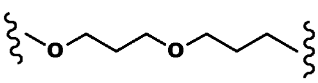
;
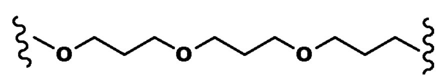
;

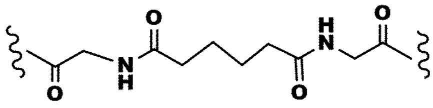
;
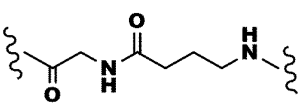
;
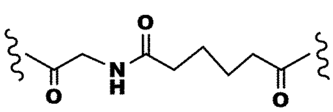
;

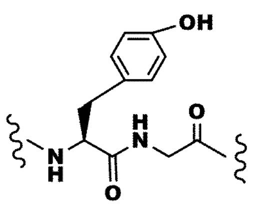
;
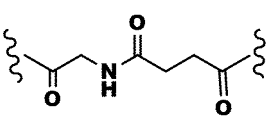
;
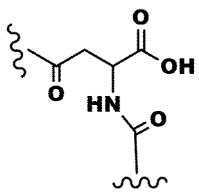
;
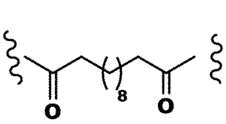
;

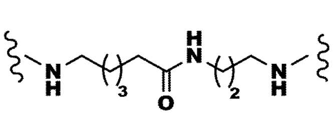 ; 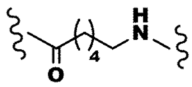 ; 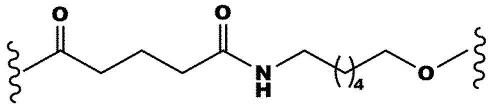 ;

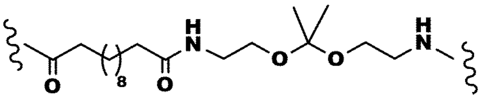 ; 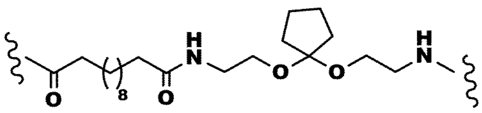 ;

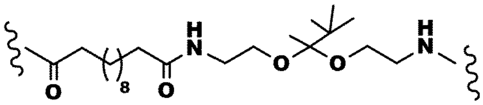 ; 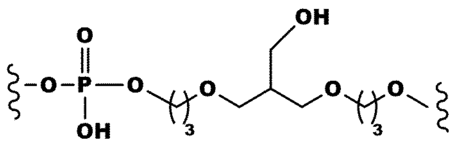 ;

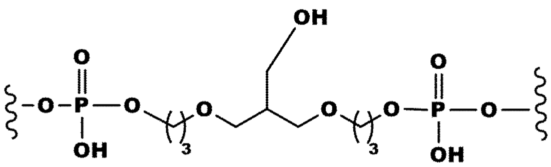 ; 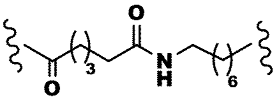 ;

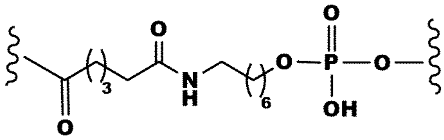 ; 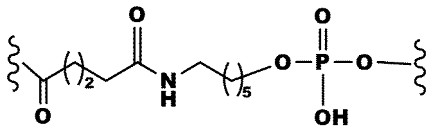 ;

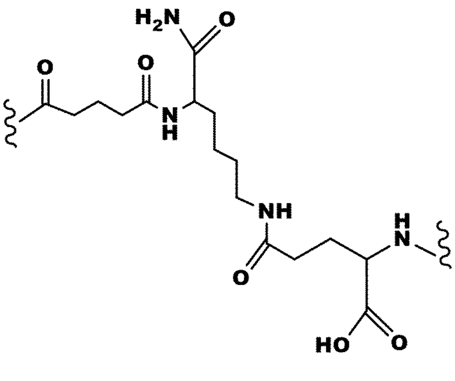 ; 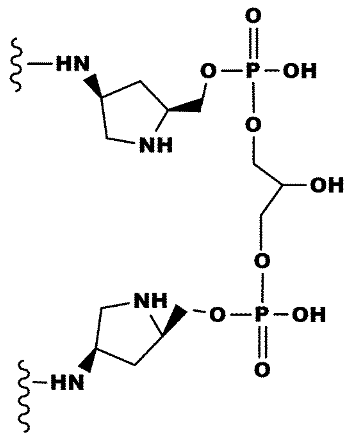 ;

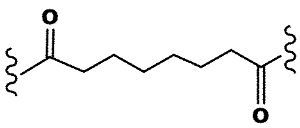 ; 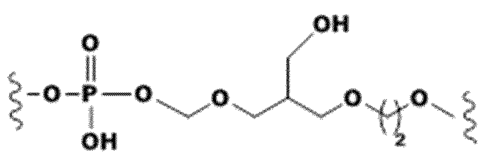 ;

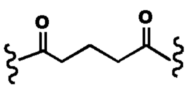 ; 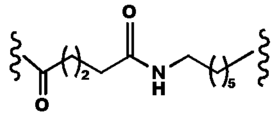 ; 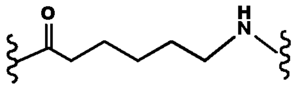 ;

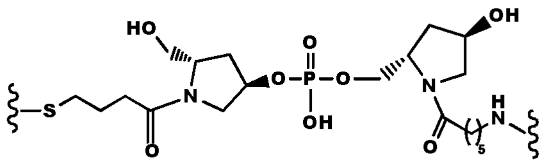 ; 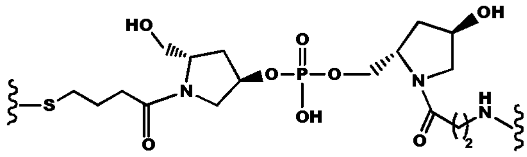 ;

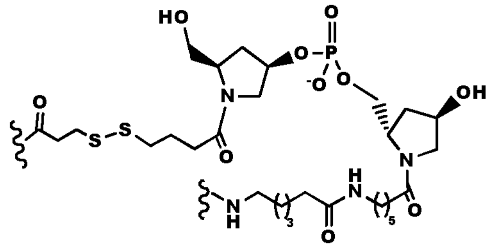 ; 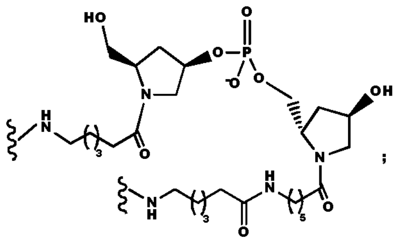 ;

.

In some embodiments, the linking chain D is selected from the following structures:

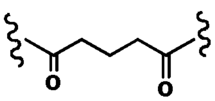 ; 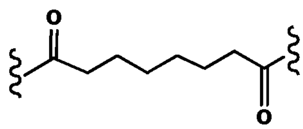 ; 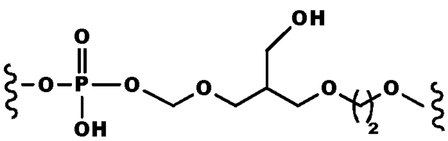 ;

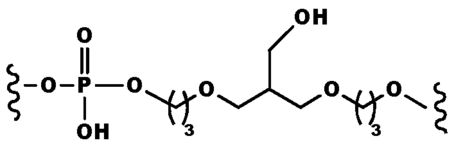 ; 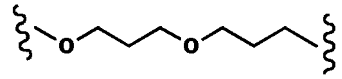 ;

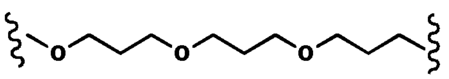 ; 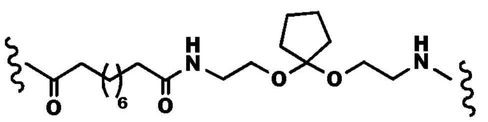 ;

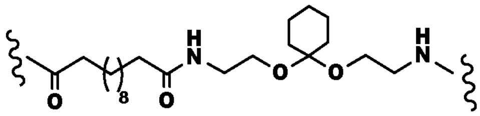

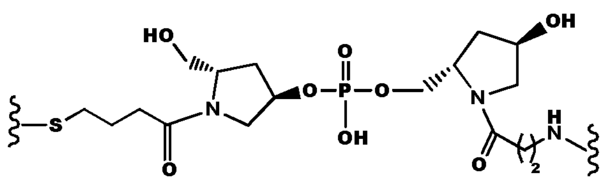 ;

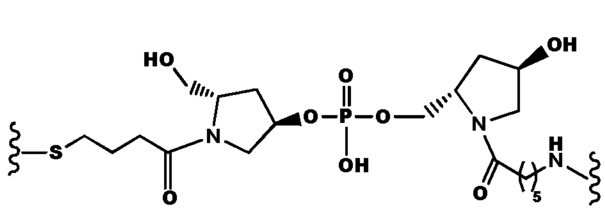 ; 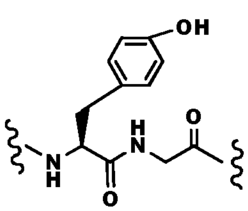

In some embodiments, **(X-L)$_n$-B-D-** in the structure of 5'MVIP and **(X-L)$_m$-B-D-** in the structure of 3'MVIP are

selected from one or more of the following structures:

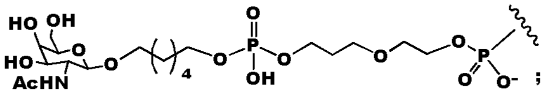 ; 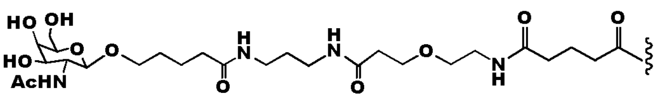 ;

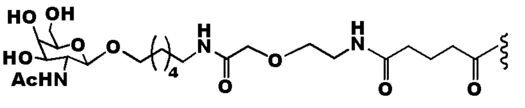 ; 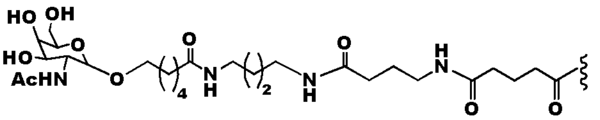 ;

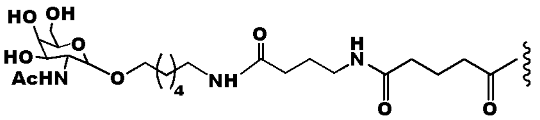 ; 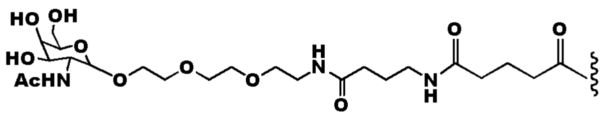 ;

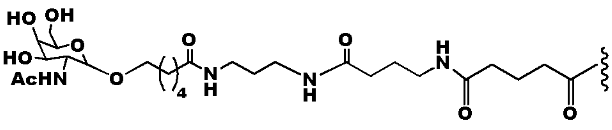 ; 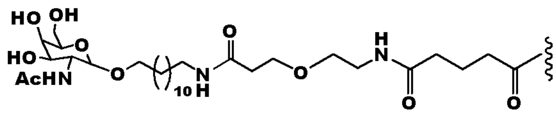 ;

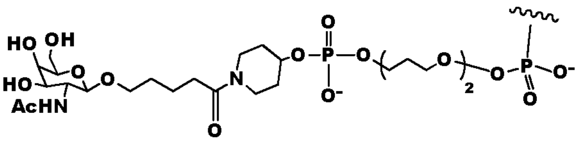 ; 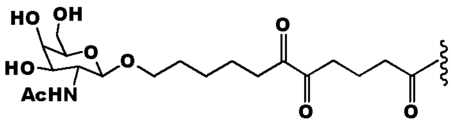 ;

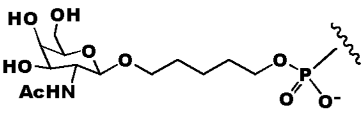 ; 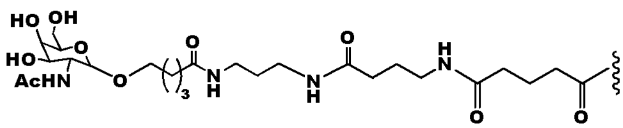 ;

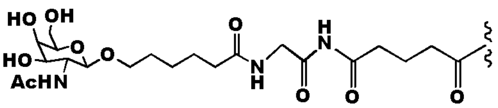 ; 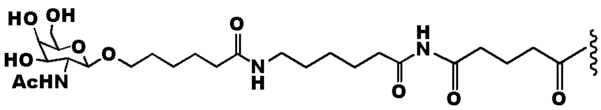 ;

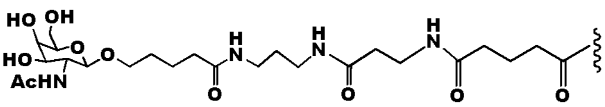 ; 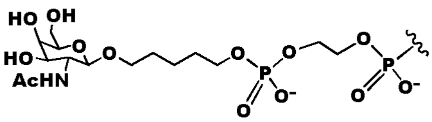 ;

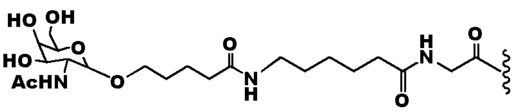 ; 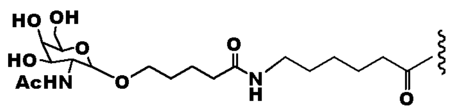 ;

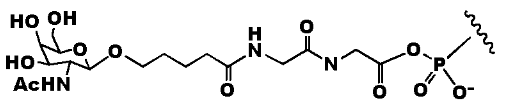 ; 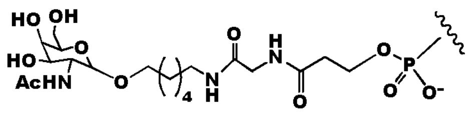 ;

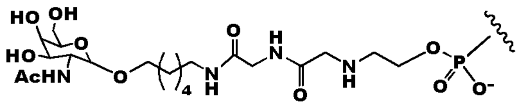 ; 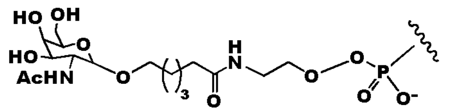 ;

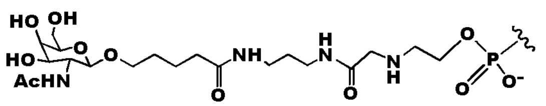 ; 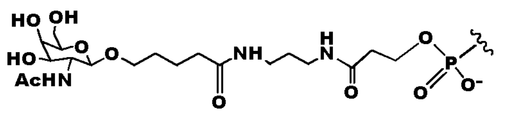 ;

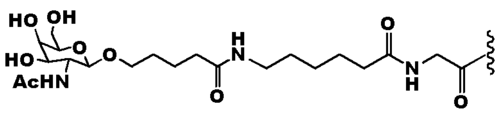 ; 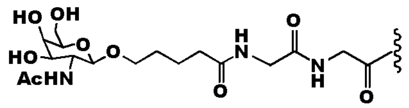 ;

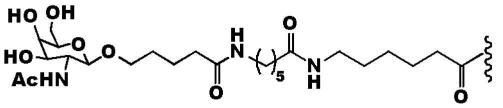 ; 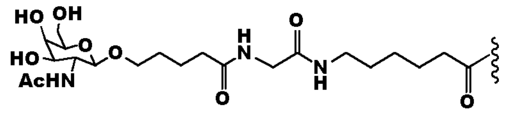 ;

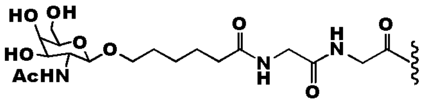 ; 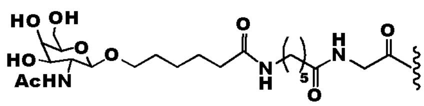 ;

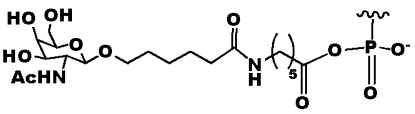 ; 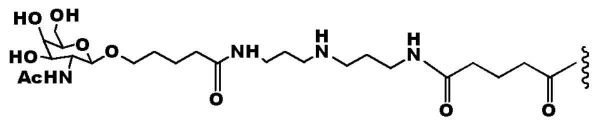 ;

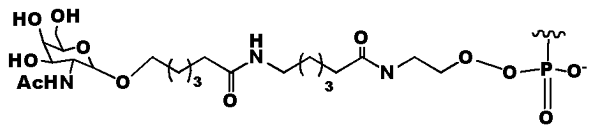 ;

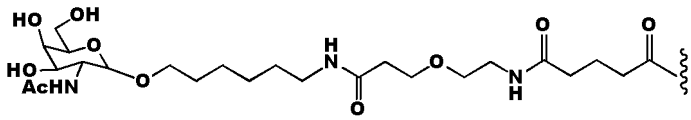 ;

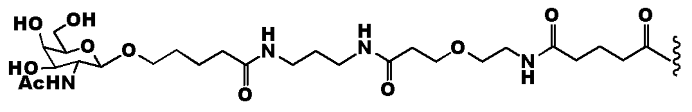 ;

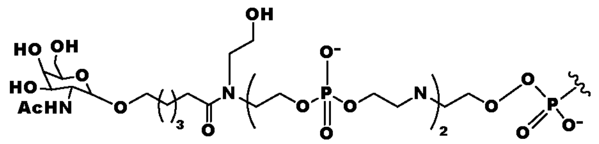 ;

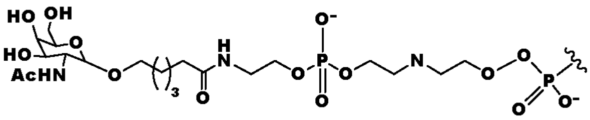 ;

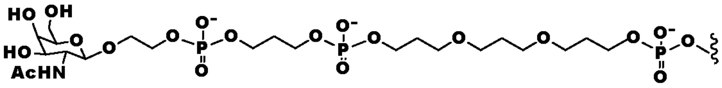 ;

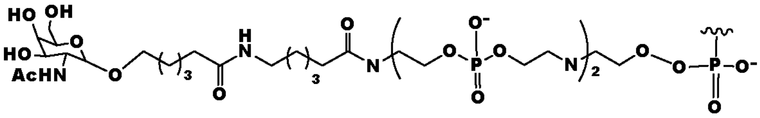 ;

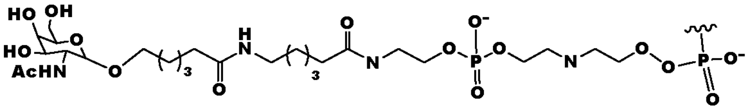 ;

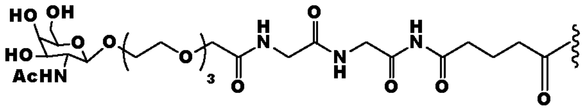 ;

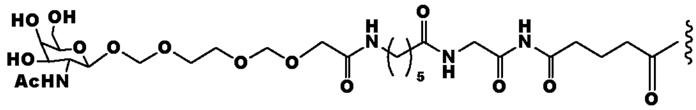 ;

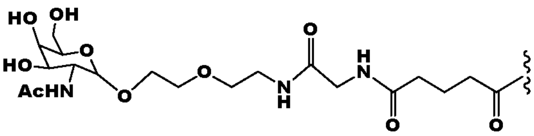 ; 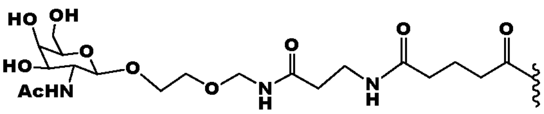 ;

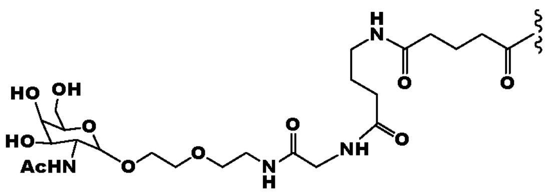 ; 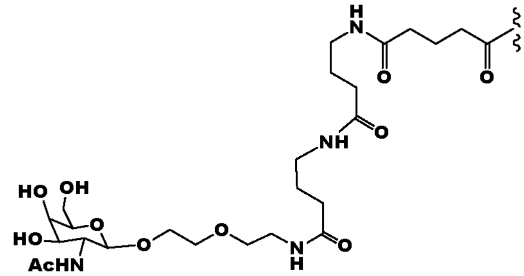 ;

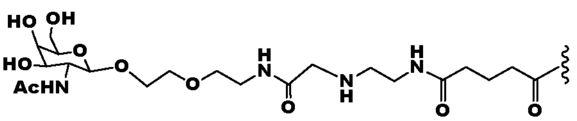 ; 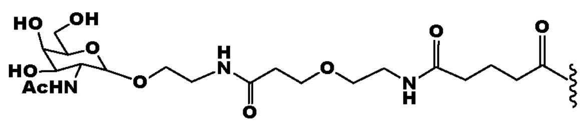 ;

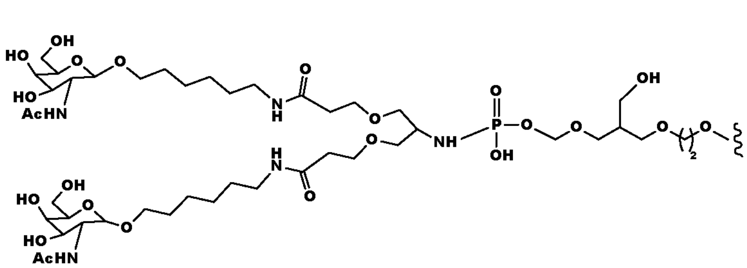

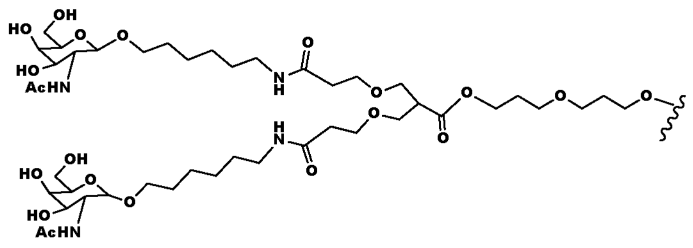

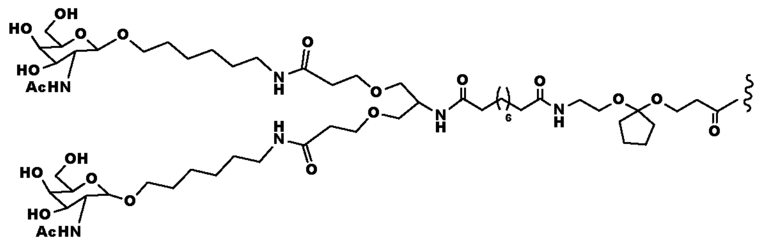

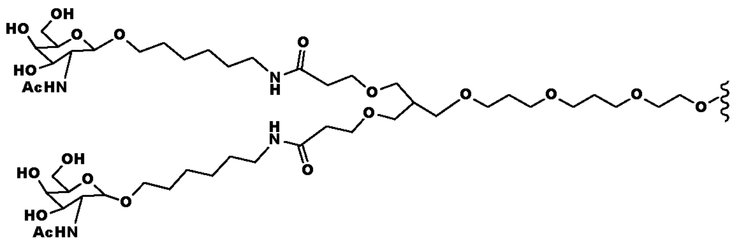

;

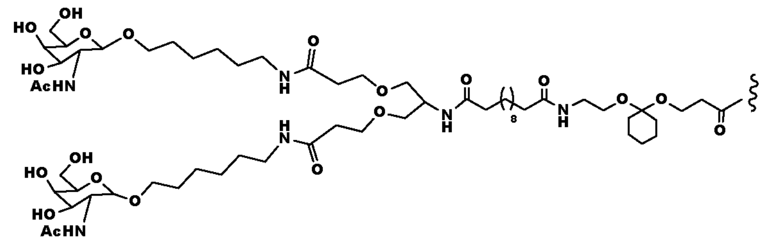

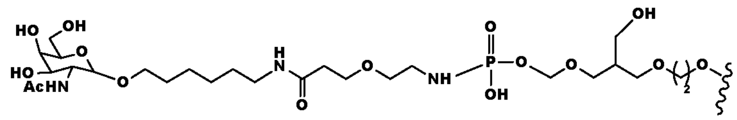

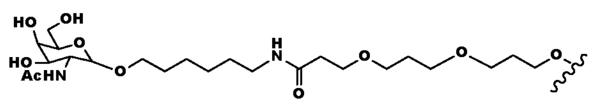

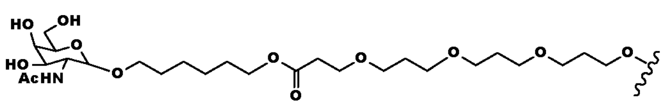

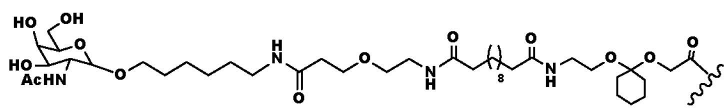

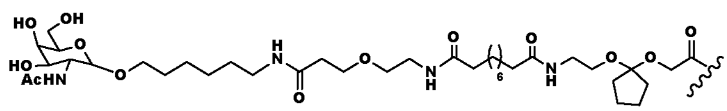

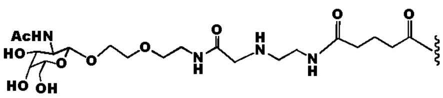

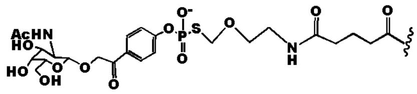

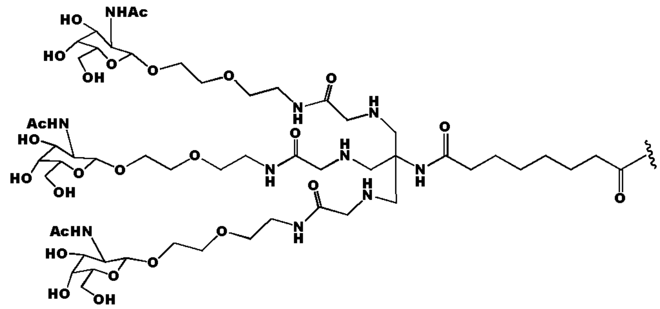

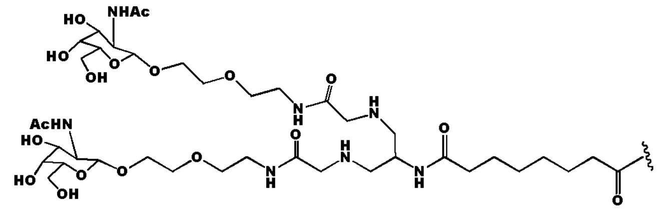

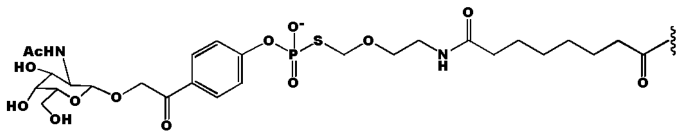

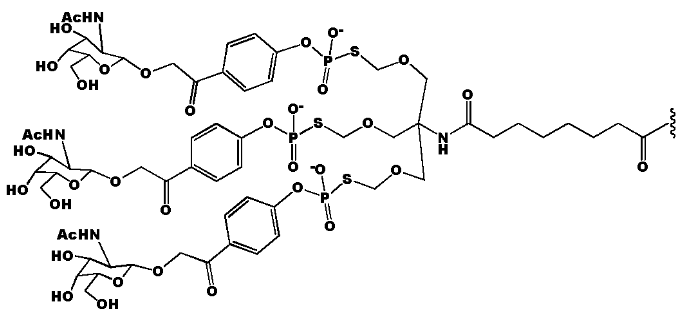

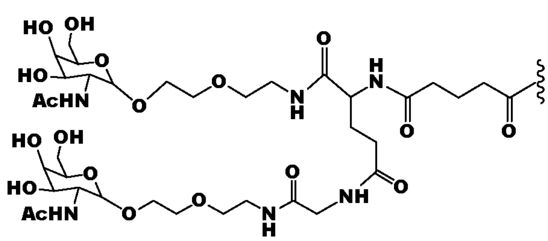

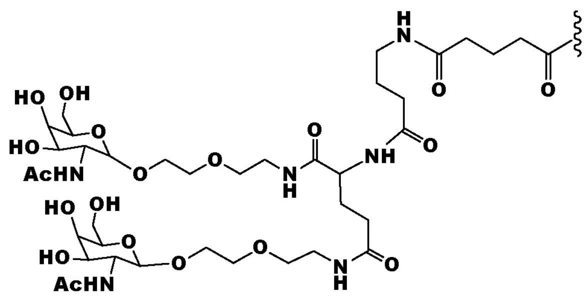

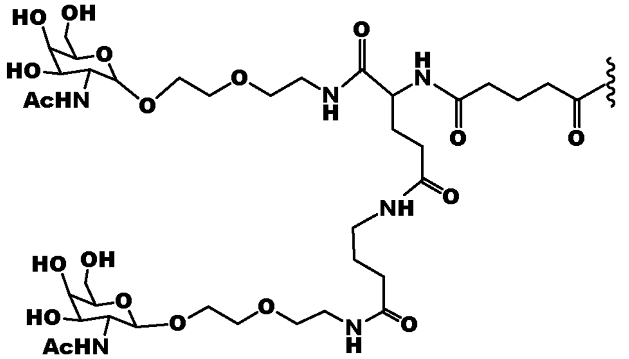

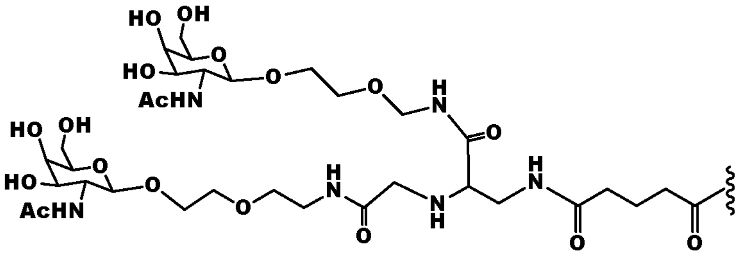

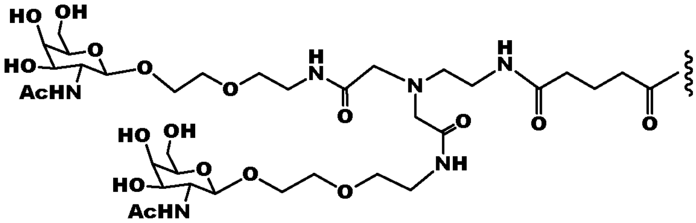

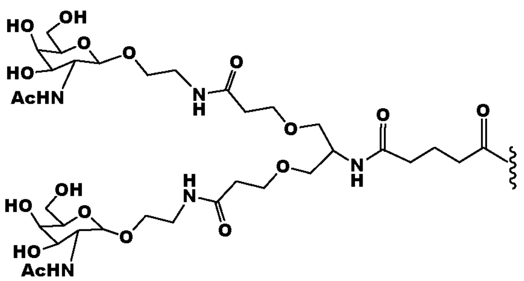

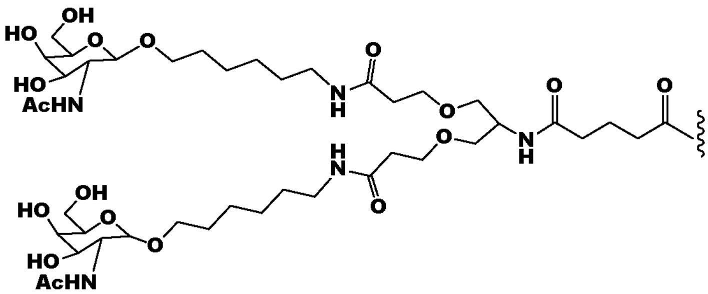

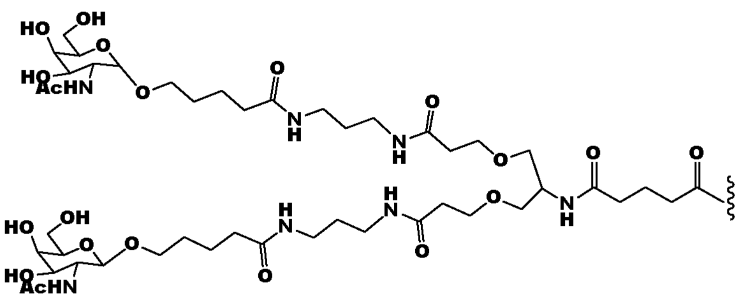

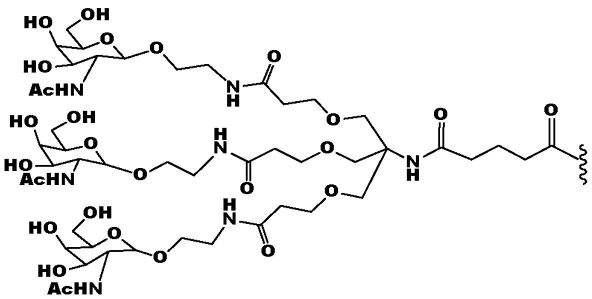

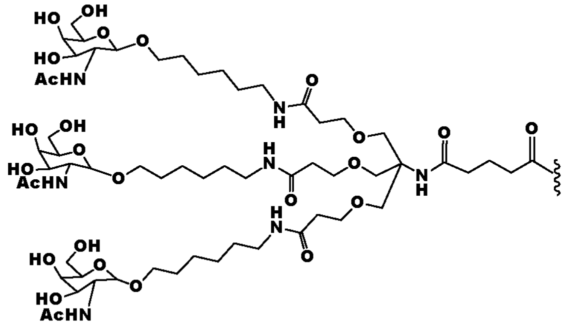

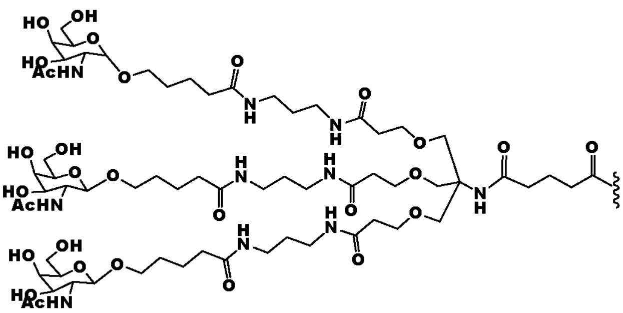
;

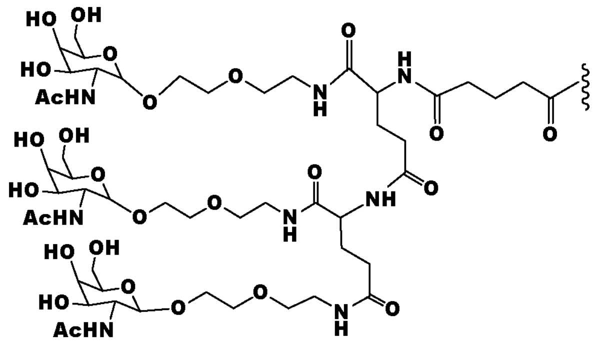

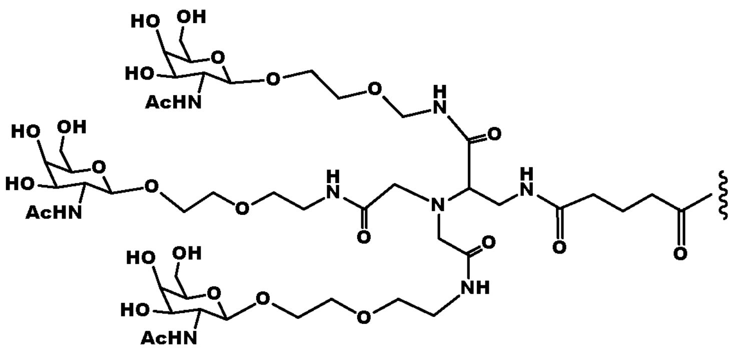

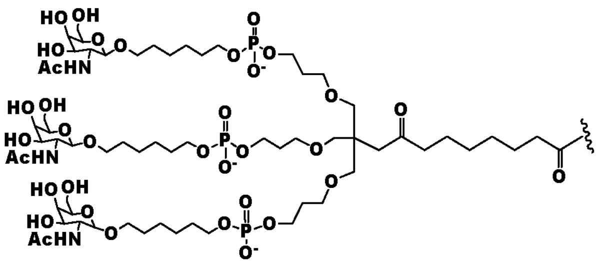
;

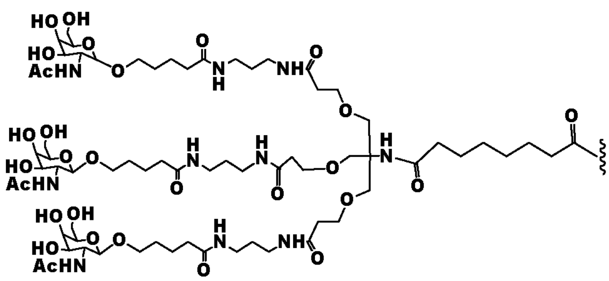
;

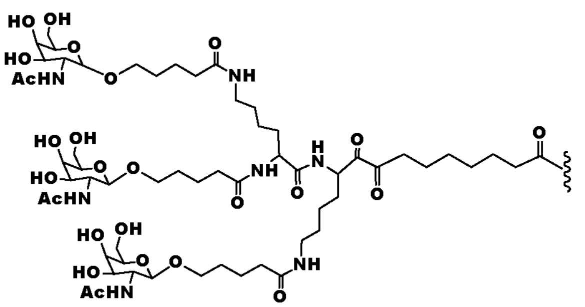
;

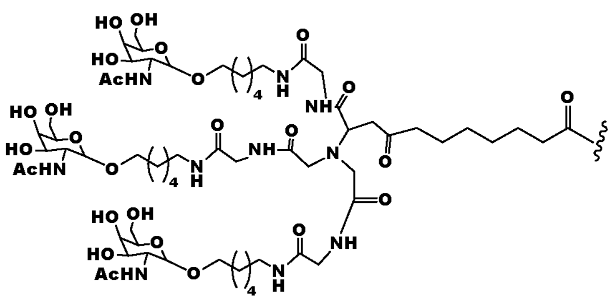
;

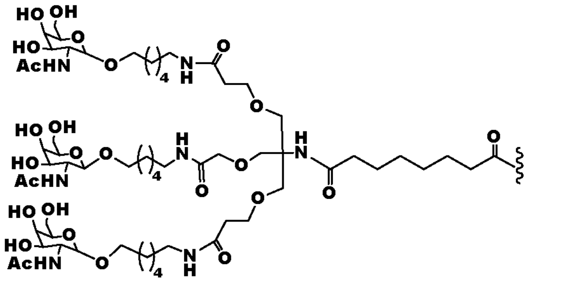
;

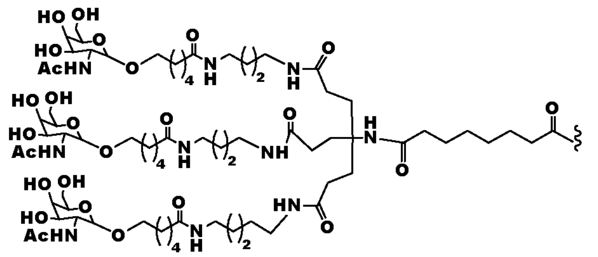
;

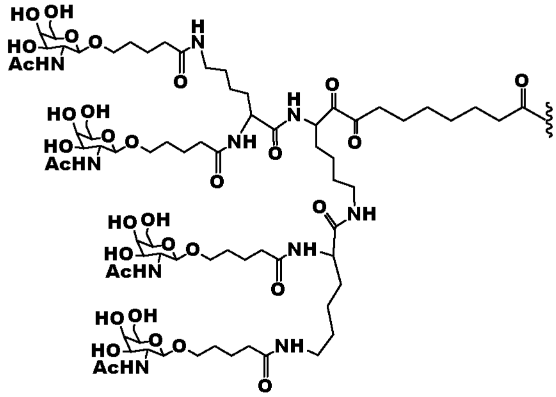
;

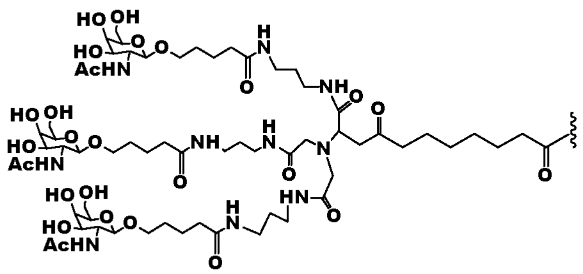
;

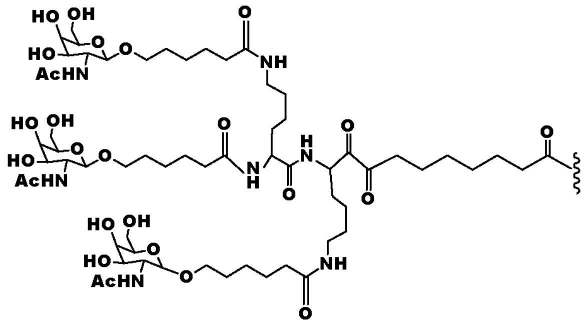
;

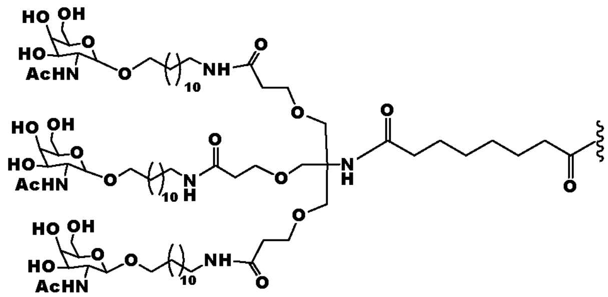
;

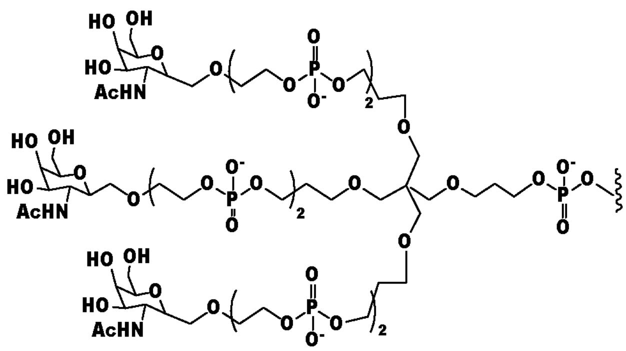
;

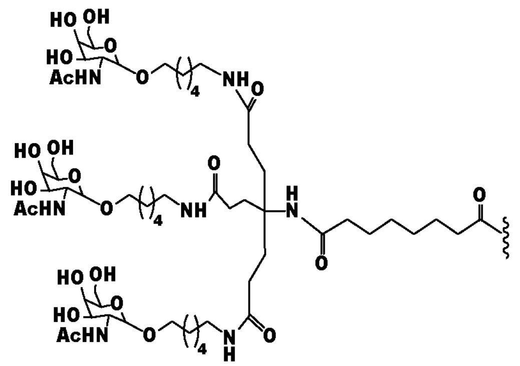
.

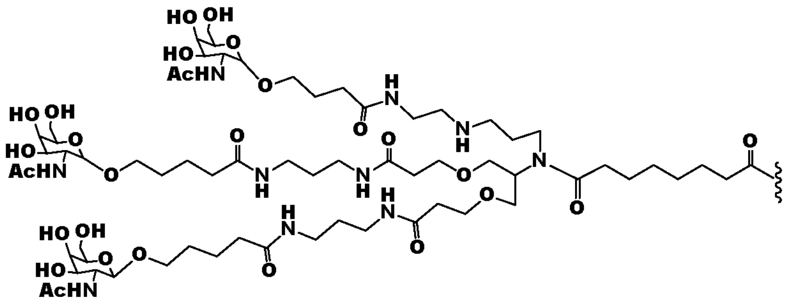

;

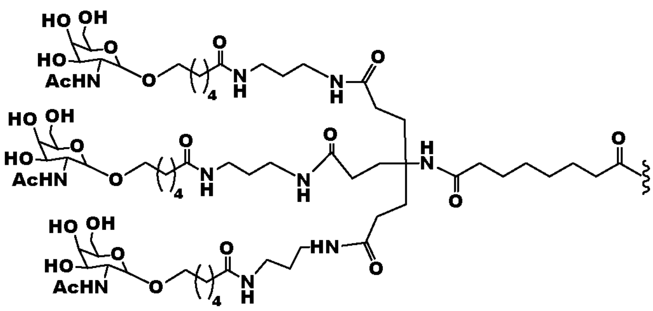

;

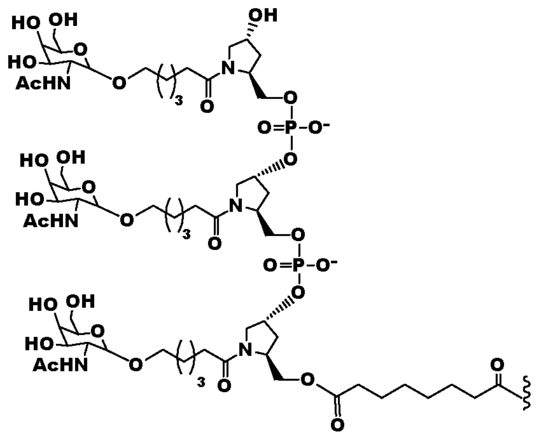

;

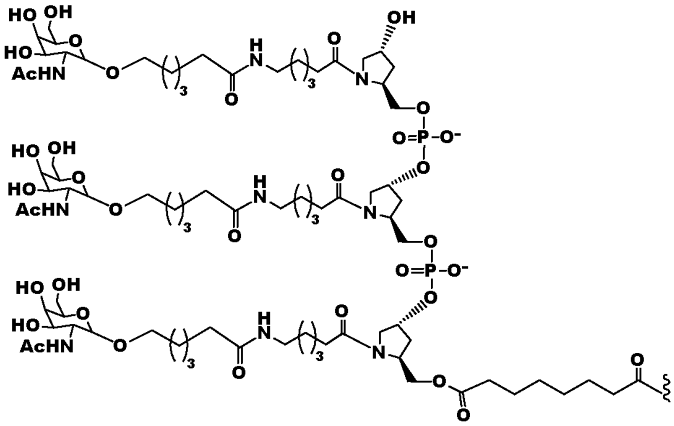

;

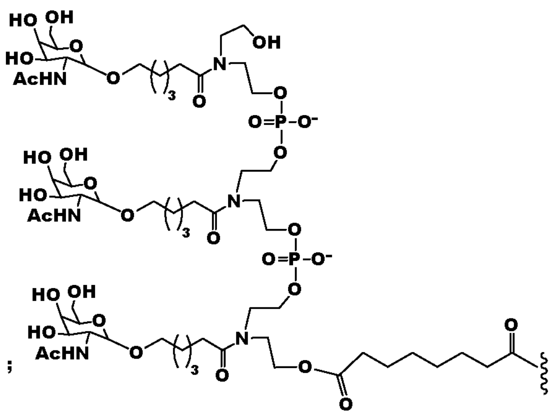

;

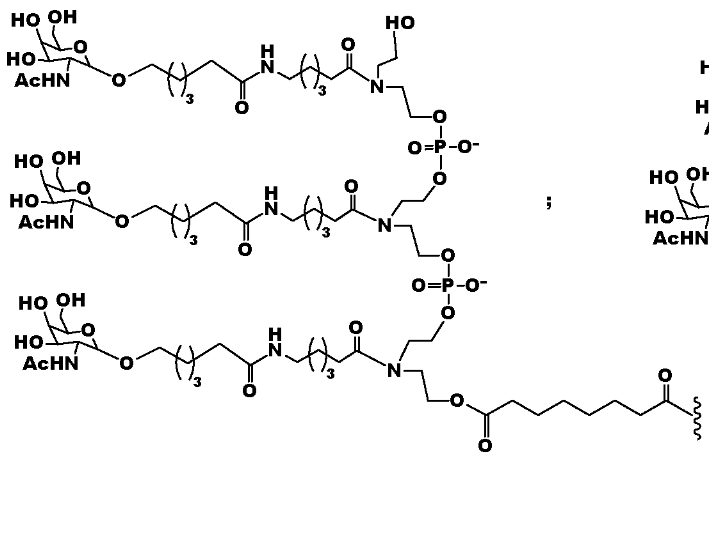

;

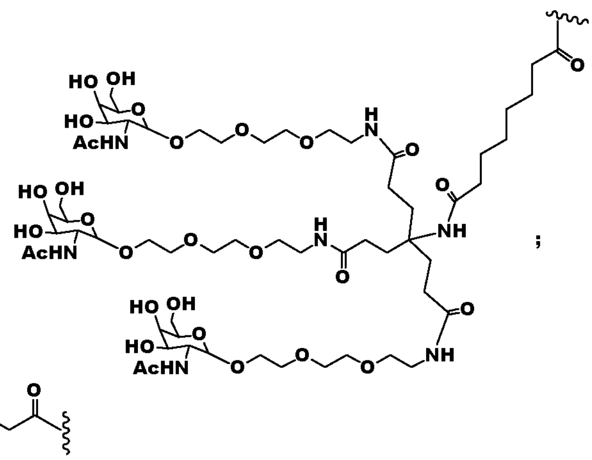

;

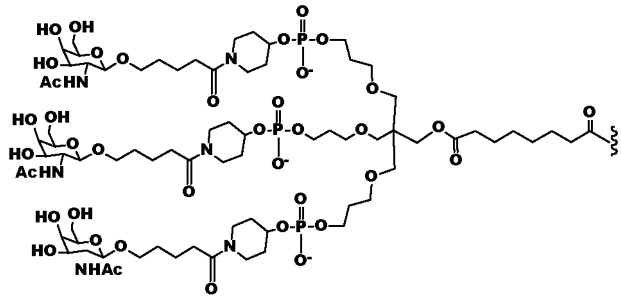 ; 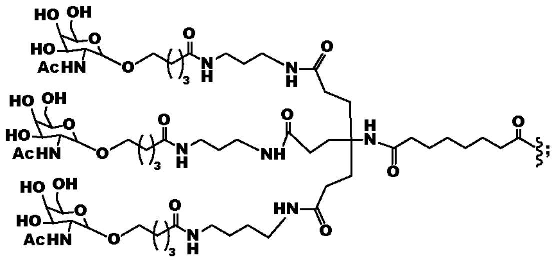 ;

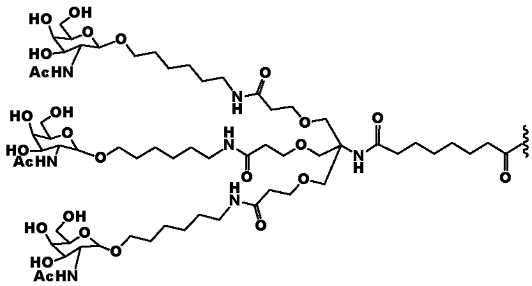 ; 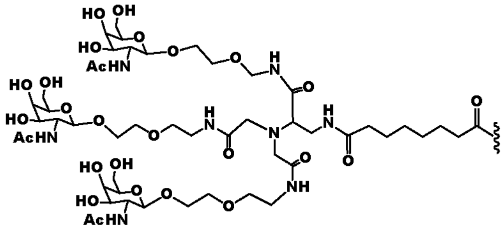 ;

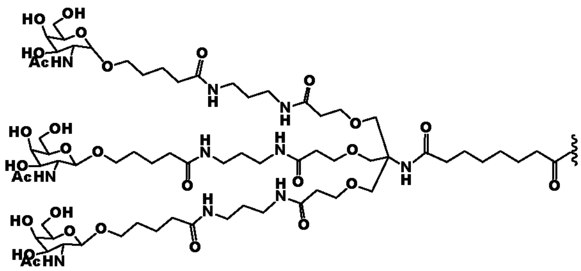 ; 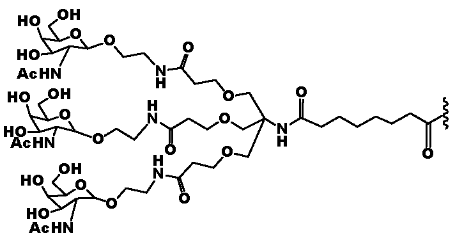 ;

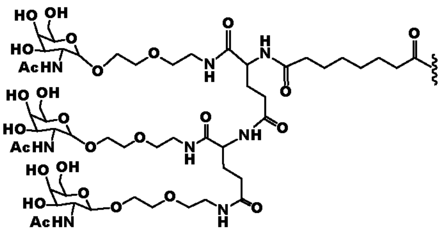 ; 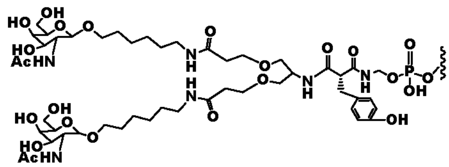

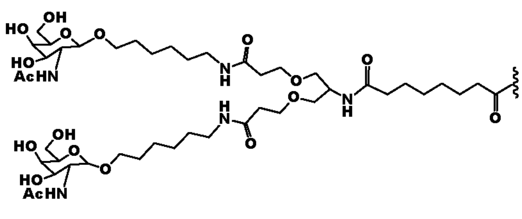 ; 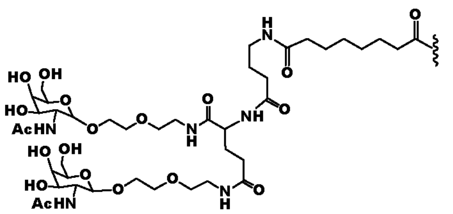 ;

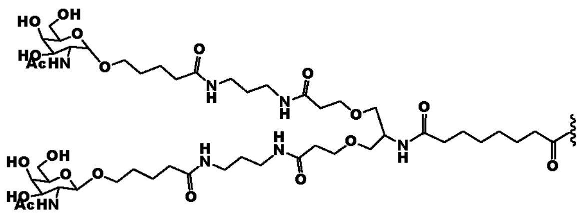 ; 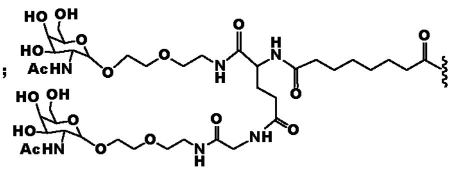 ;

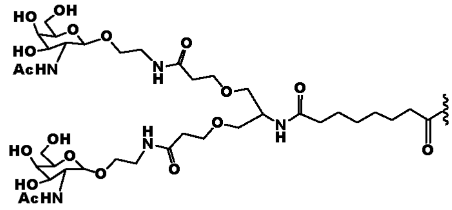 ;

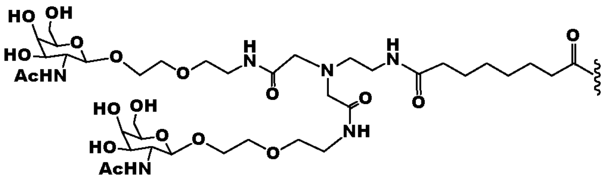 ; 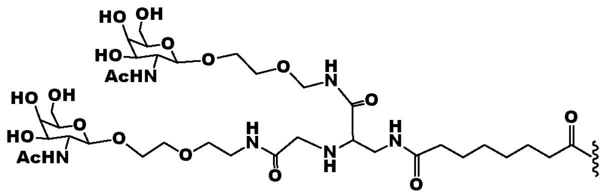 ;

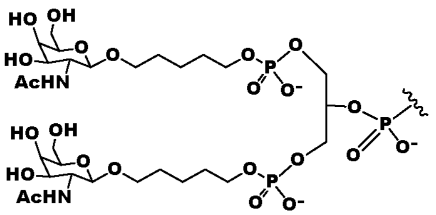 ; 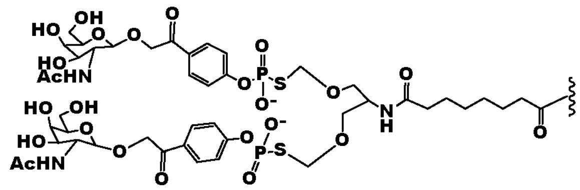 ;

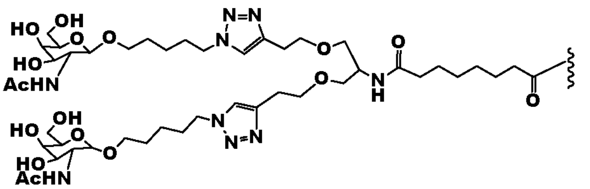 ; 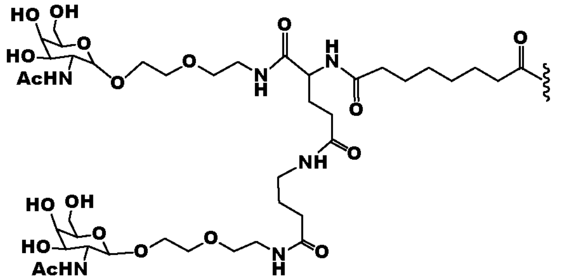 ;

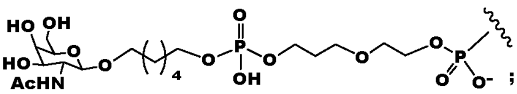 ; 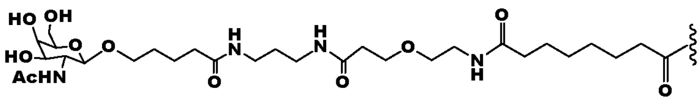 ;

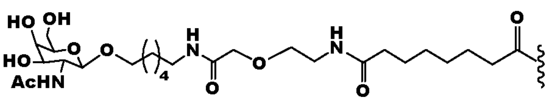 ; 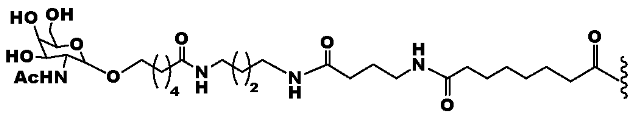 ;

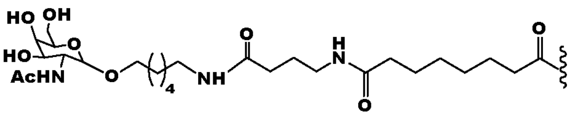 ; 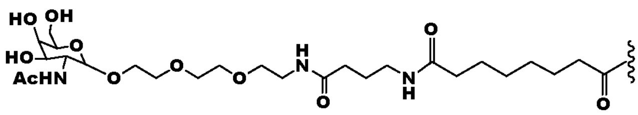 ;

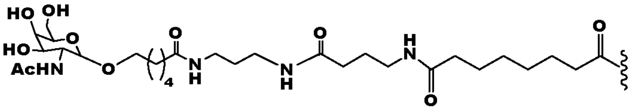 ; 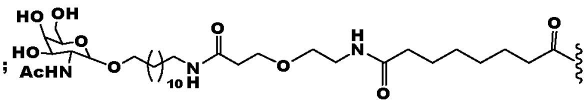 ;

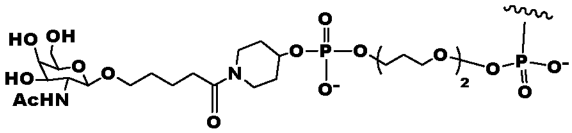 ; 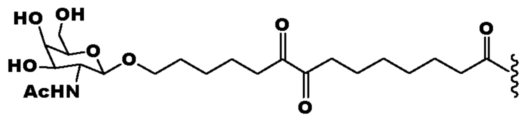 ;

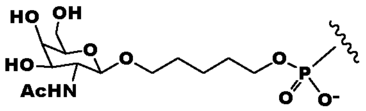 ; 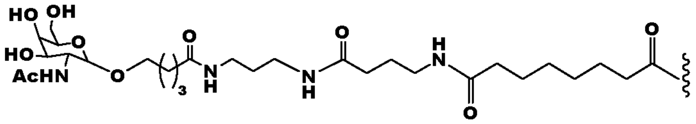 ;

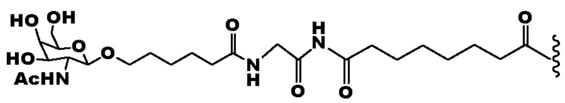 ; 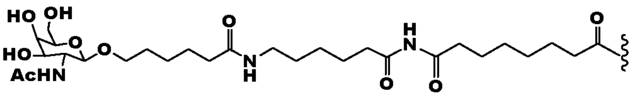 ;

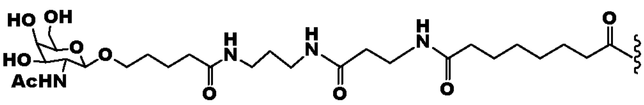 ; 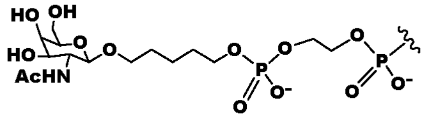 ;

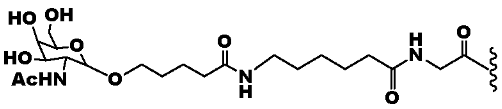 ; 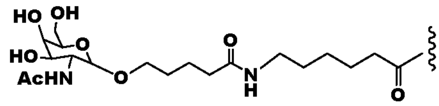 ;

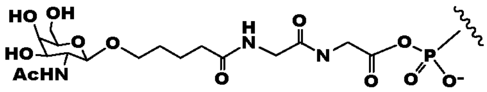 ; 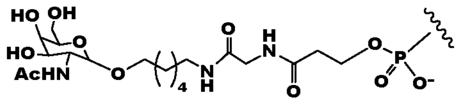 ;

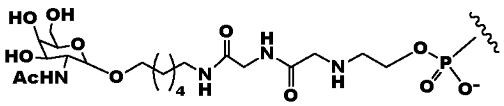 ; 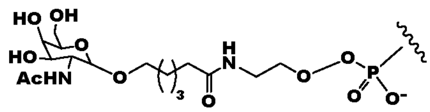 ;

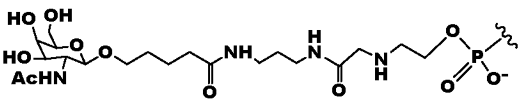 ; 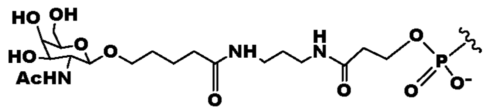 ;

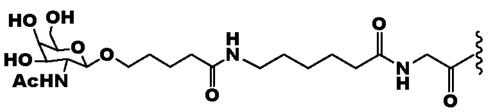 ; 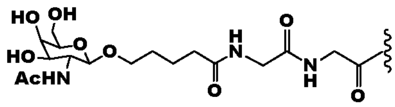 ;

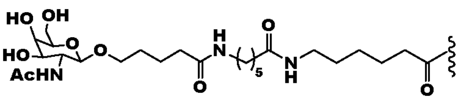 ; 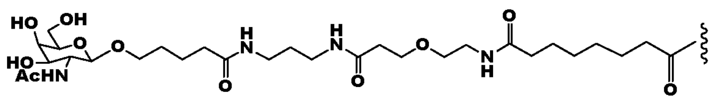 ;

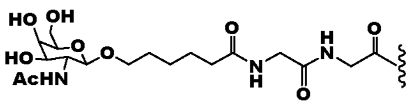 ; 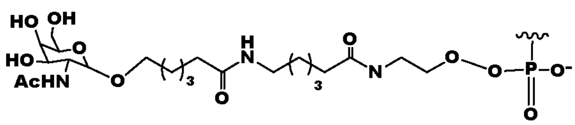 ;

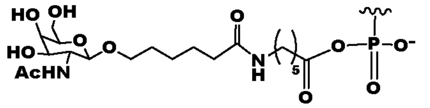 ; 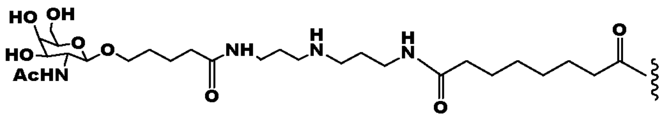 ;

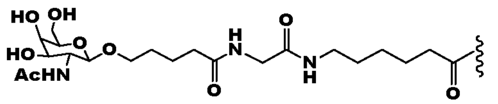 ; 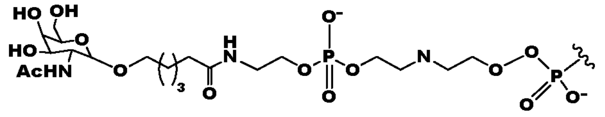 ;

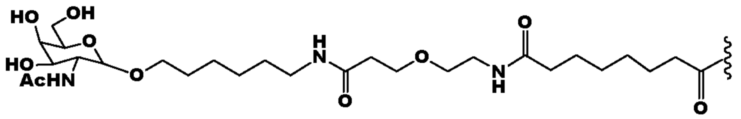 ; 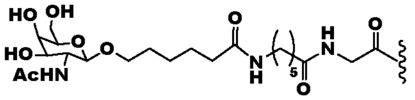 ;

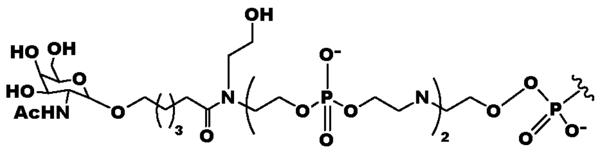 ;

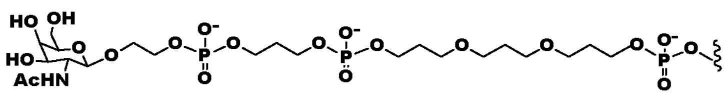 ;

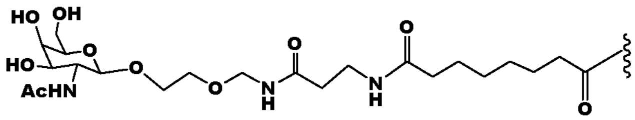 ;

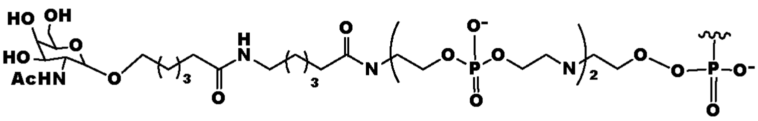 ;

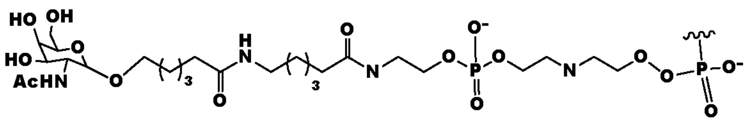 ;

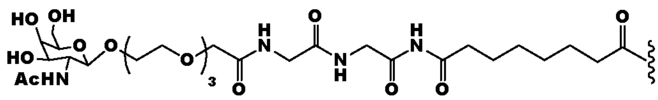 ;

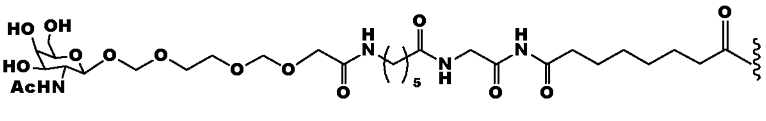 ;

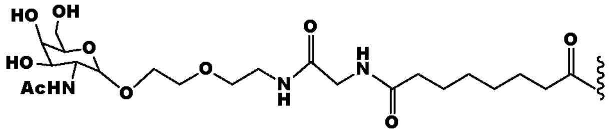 ;

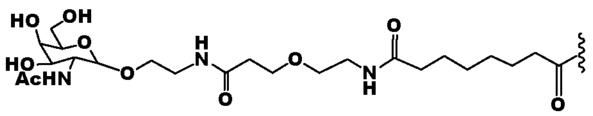 ;

In some embodiments, the X, L, B and D are the same or different within the respective 5 'MVIP and 3' MVIP or between the 5 'MVIP and 3' MVIP.

In some embodiments, **$(X-L)_n$-B-D** in the structure of 5'MVIP is selected from the structures shown in Table 9:

Table 9 **$(X-L)_n$-B-D-** in structures of 5'MVIP

| No. | code | Structural formula |
|---|---|---|
| 1 | 5'YICdd-01 | 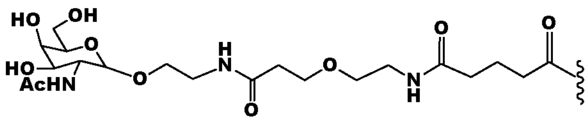 |
| 2 | 5'YICd-01 | 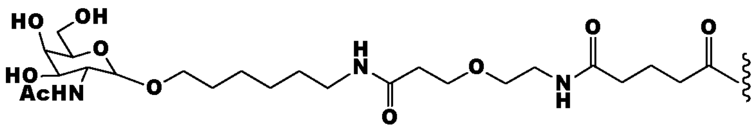 |
| 3 | 5'YICc-01 | 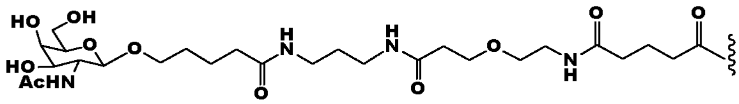 |
| 4 | 5'YICa-01 | 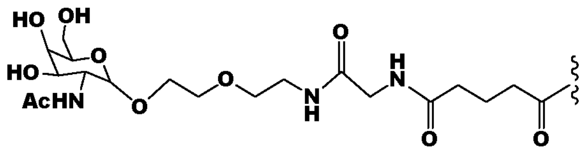 |
| 5 | 5'YICa-02 | 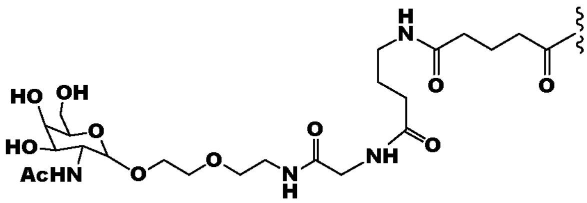 |
| 6 | 5'YICa-03 | 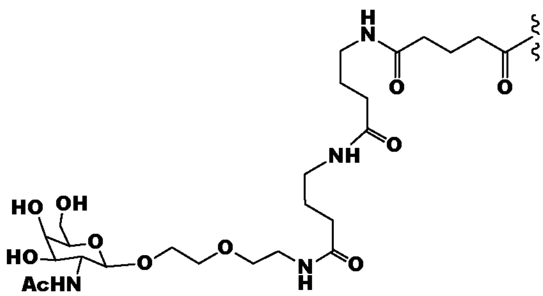 |
| 7 | 5'YICa-04 | 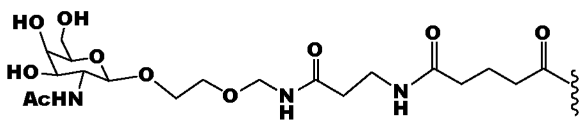 |
| 8 | 5'YICa-05 | 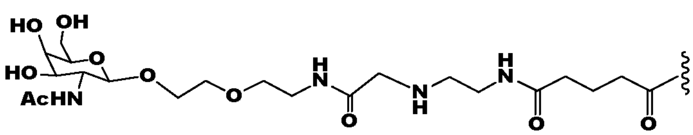 |

(continued)

| No. | code | Structural formula |
|---|---|---|
| 9 | 5'ERCa-01 | 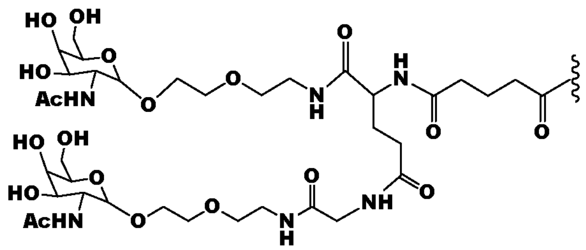 |
| 10 | 5'ERCa-02 | 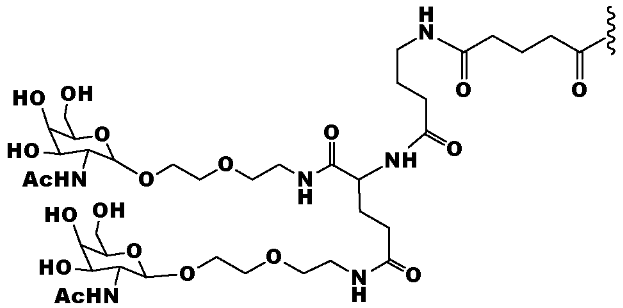 |
| 11 | 5'ERCa-03 | 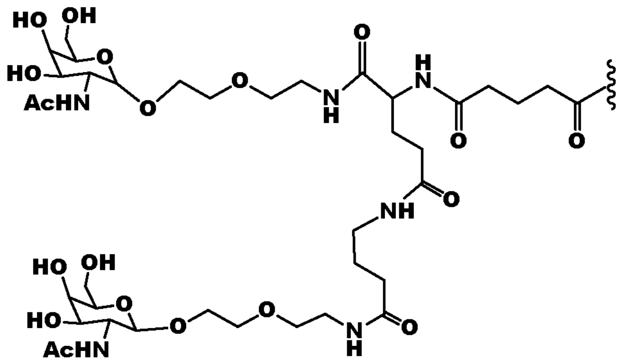 |
| 12 | 5'ERCa-04 | 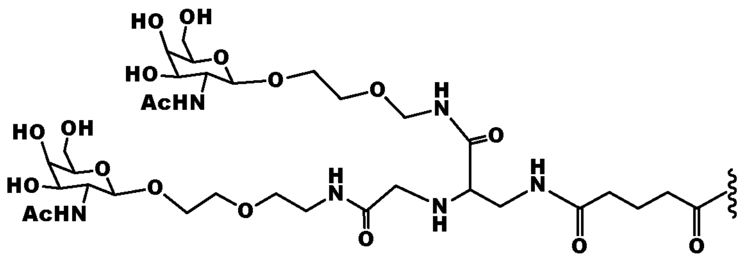 |
| 13 | 5'ERCa-05 | 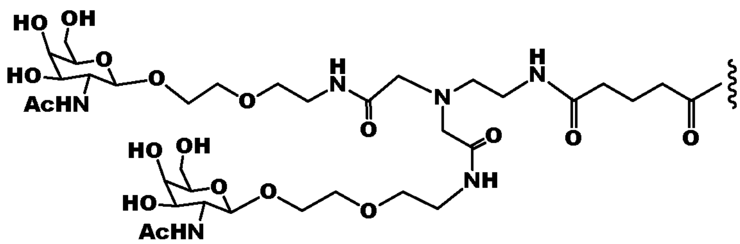 |

(continued)

| No. | code | Structural formula |
|---|---|---|
| 14 | 5'ERCdd-01 | 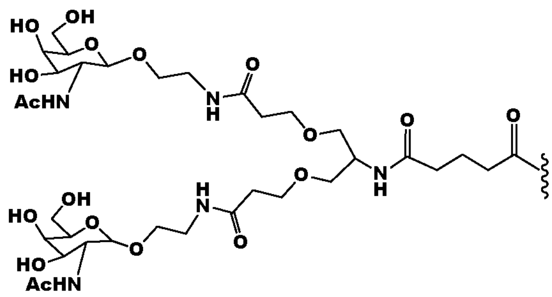 |
| 15 | 5'ERCd-01 | 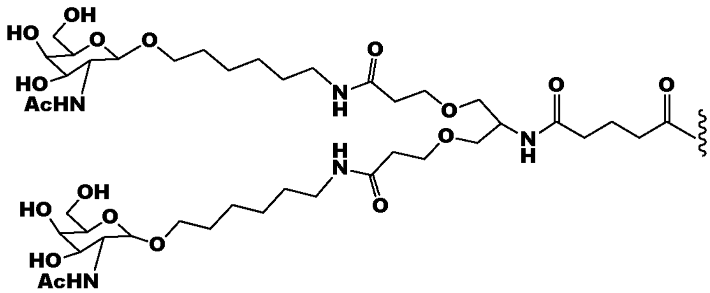 |
| 16 | 5'ERCc-01 | 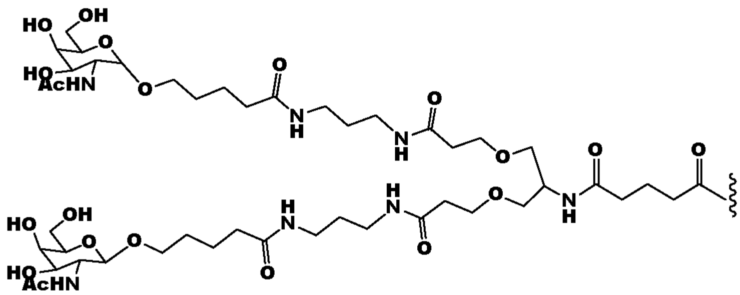 |
| 17 | 5'SANCdd-01 | 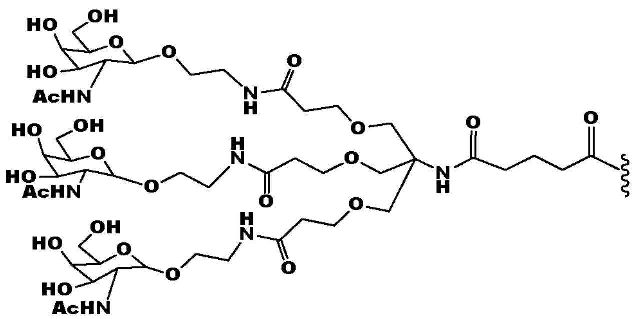 |
| 18 | 5'SANCd-01 | 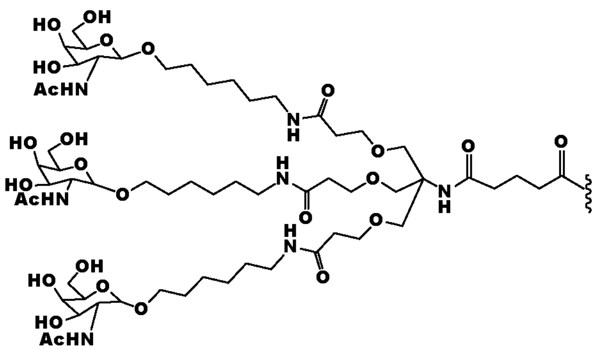 |

(continued)

| No. | code | Structural formula |
|---|---|---|
| 19 | 5'SANCc-01 | 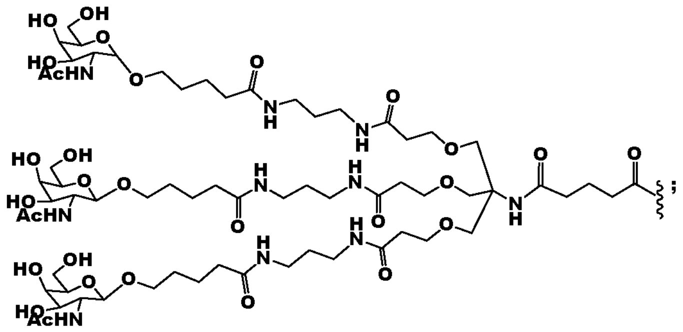 |
| 20 | 5'SANCa-01 | 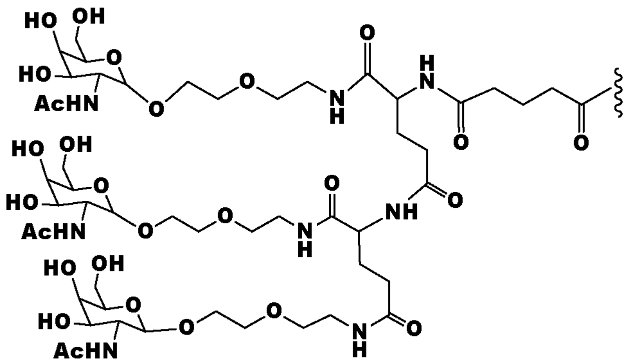 |
| 21 | 5'SANCa-02 | 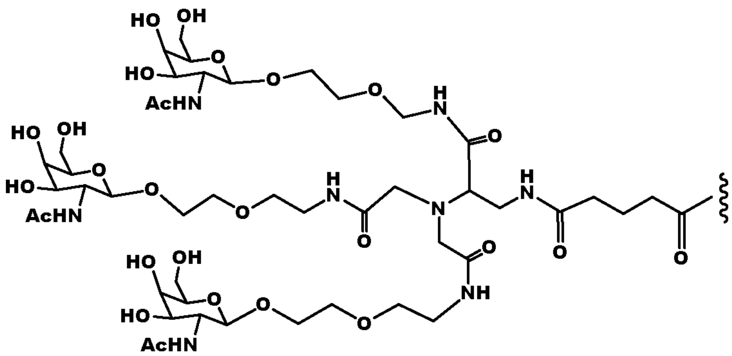 |
| 22 | 5'SANCa-03 | 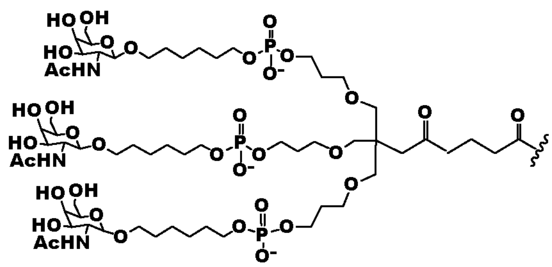 |

In some embodiments, 5'MVIP may not exist, in which case m may be any integer of 2-4.

In some embodiments, **(X-L)$_m$-B-D-** in the structure of 3'MVIP is selected from the structures shown in Table 10:

Table 10 **(X-L)$_m$-B-D-** in structure of 3'MVIP

| No. | code | structure |
|---|---|---|
| 1 | 3'SANCdd-01 | 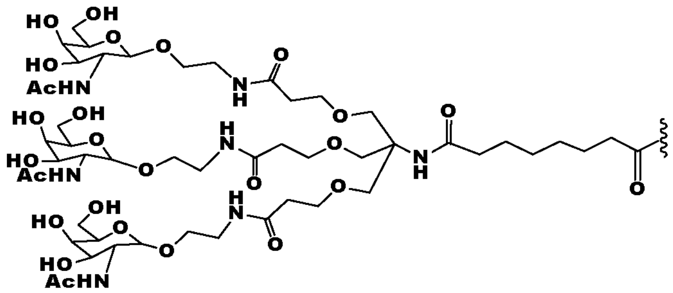 |
| 2 | 3'SANCd-01 | 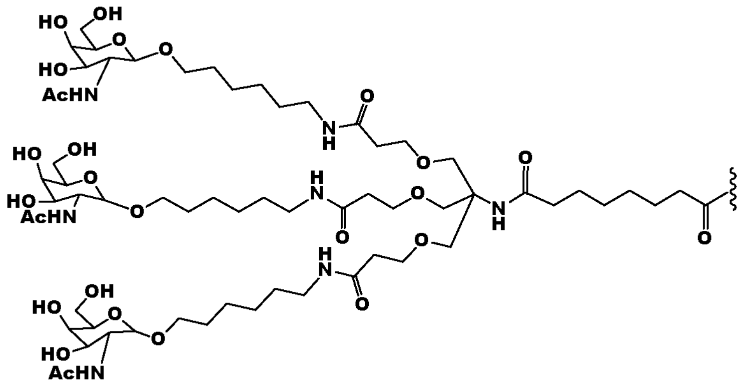 |
| 3 | 3'SANCc-01 | 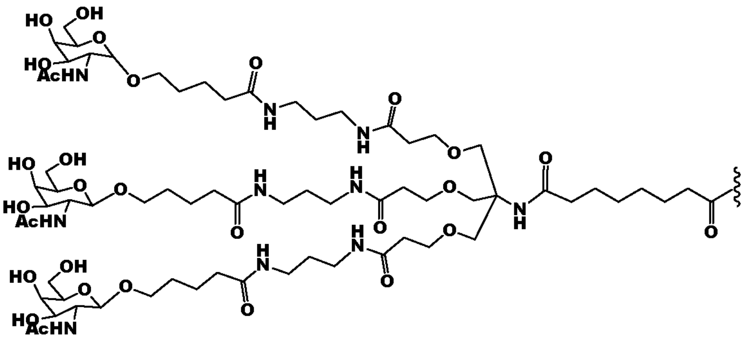 |
| 4 | 3'SANCa-01 | 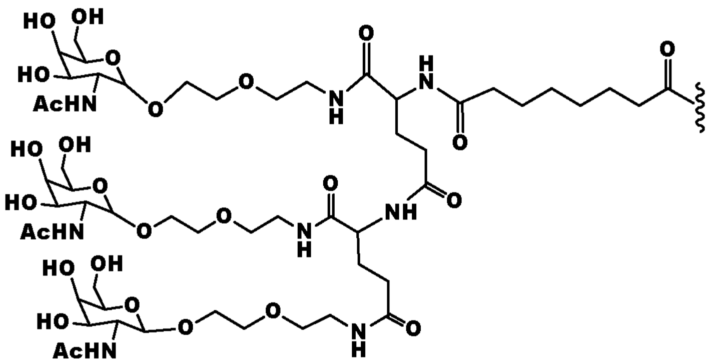 |

(continued)

| No. | code | structure |
|---|---|---|
| 5 | 3'SANCa-02 | 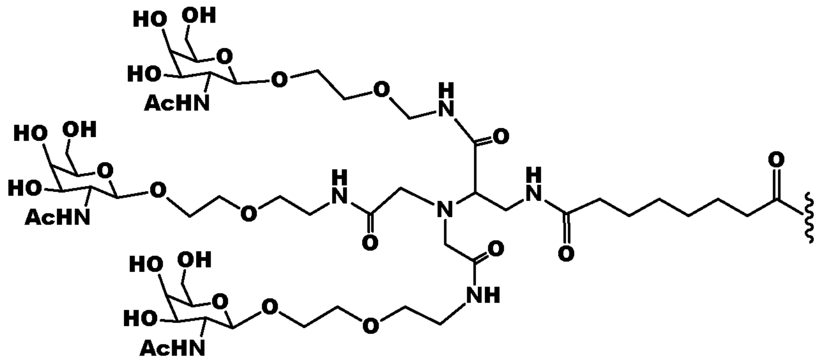 |
| 6 | 3'SANCa-03 | 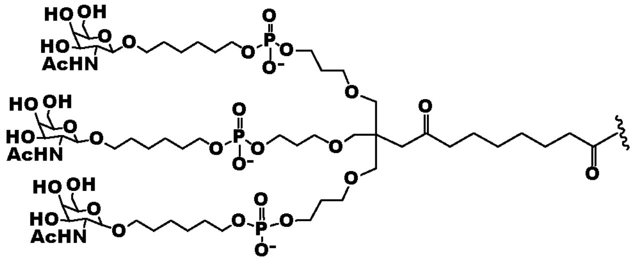 |
| 7 | 3'ERCdd-01 | 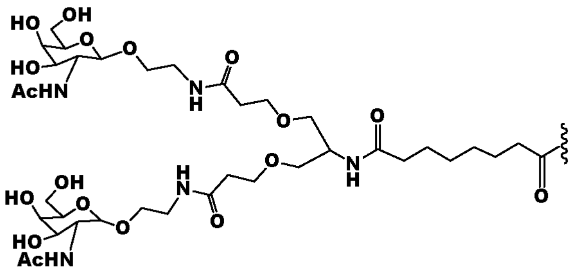 ; |
| 8 | 3'ERCd-01 | 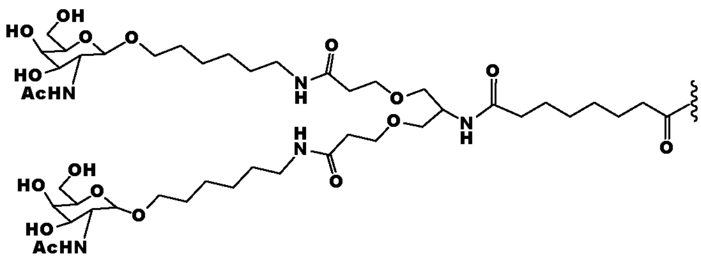 |
| 9 | 3'ERCc-01 | 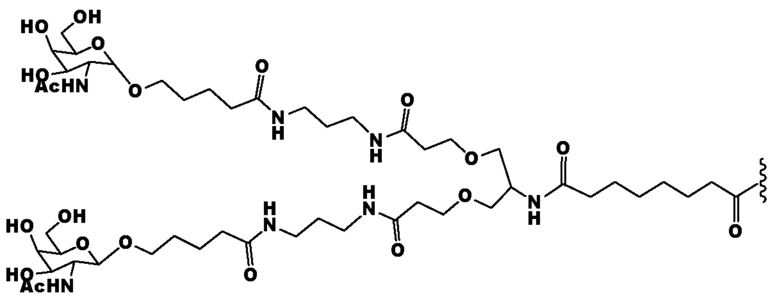 |

(continued)

| No. | code | structure |
|---|---|---|
| 10 | 3'ERCa-01 | 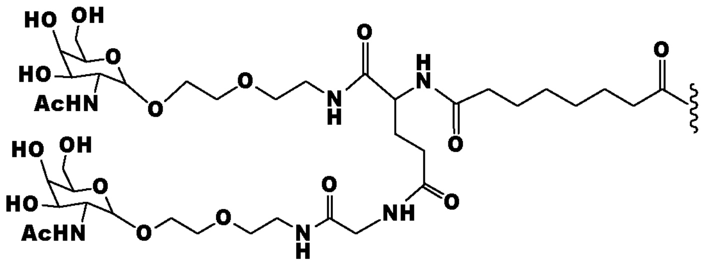 |
| 11 | 3'ERCa-02 | 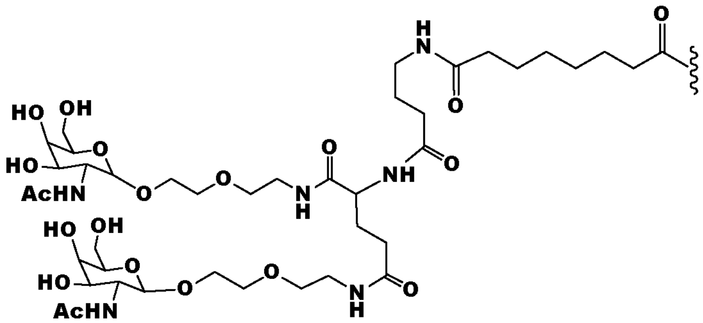 |
| 12 | 3'ERCa-03 | 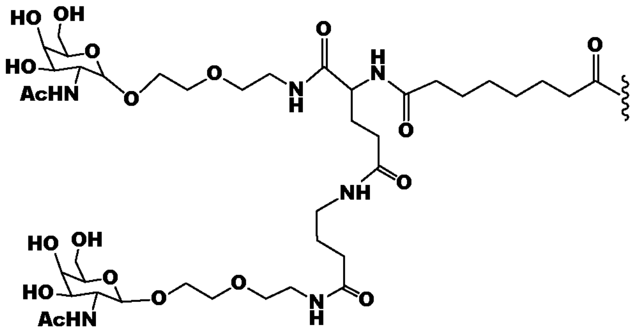 |
| 13 | 3'ERCa-04 | 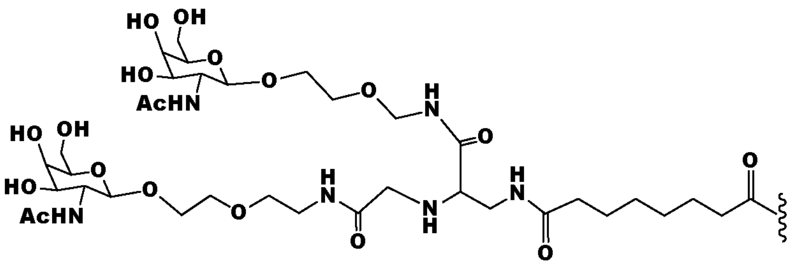 |
| 14 | 3'ERCa-05 | 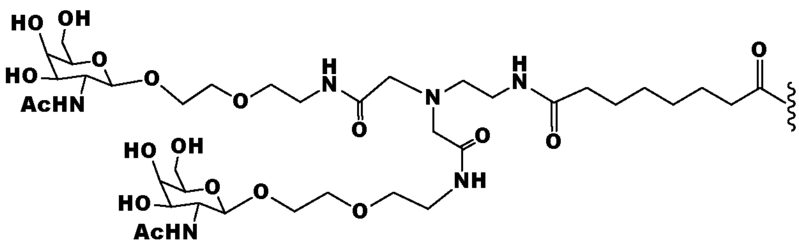 |
| 15 | 3'YICa-01 | 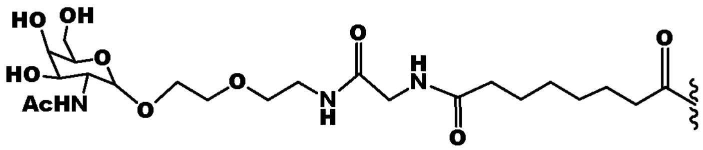 |

(continued)

| No. | code | structure |
|---|---|---|
| 16 | 3'YICa-02 | 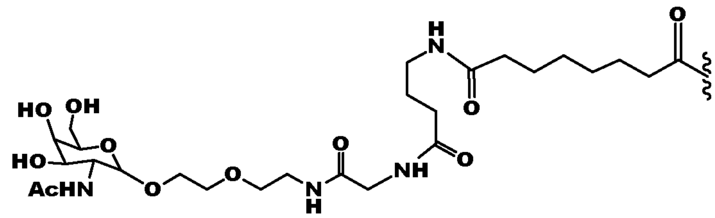 |
| 17 | 3'YICa-03 | 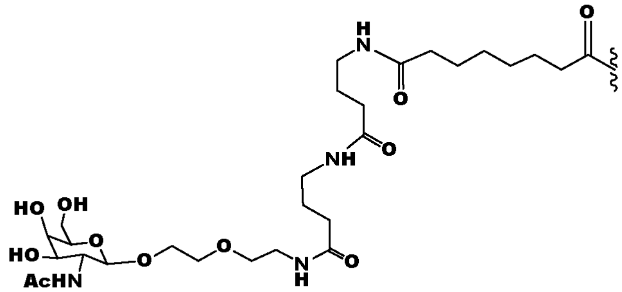 |
| 18 | 3'YICa-04 | 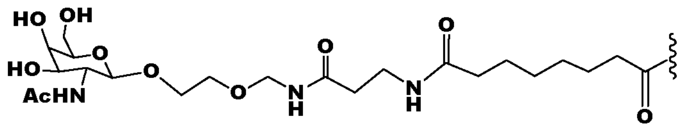 |
| 19 | 3'YICa-05 | 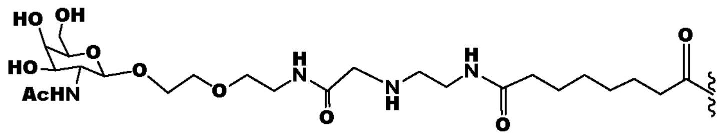 |
| 20 | 3'YICdd-01 | 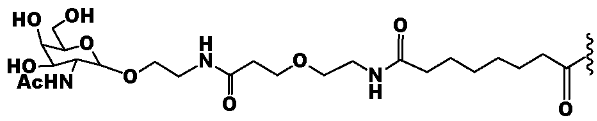 |
| 21 | 3'YICd-01 | 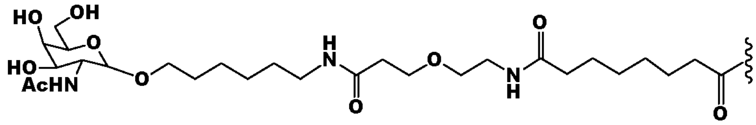 |
| 22 | 3'YICc-01 | 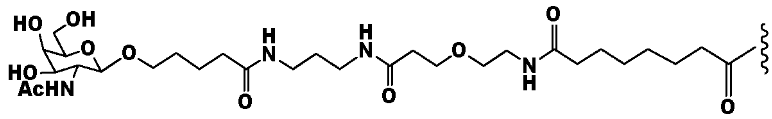 |

In some embodiments, the combinations of **(X-L)$_n$-B-D-** and $R_1$ in the carrier structure of 5'MVIP are shown in Table 11.

Table 11 combinations of **(X-L)$_n$-B-D-** and $R_1$ in 5'MVIP

| No. | **(X-L)$_n$-B-D-** code | $R_1$ | 5'MVIP code |
|---|---|---|---|
| 1 | 5'YICd-01 | $-NH(CH_2)_6O-$ | 5'MVIP01 |
| 2 | 5'YICc-01 | $-NH(CH_2)_6O-$ | 5'MVIP02 |
| 3 | 5'YICa-01 | $-O(CH_2)_6O-$ | 5'MVIP03 |
| 4 | 5'YICa-02 | $-O(CH_2)_6O-$ | 5'MVIP04 |
| 5 | 5'YICa-03 | $-S(CH_2)_6O-$ | 5'MVIP05 |

(continued)

| No. | **(X-L)$_n$-B-D-** code | $R_1$ | 5'MVIP code |
|---|---|---|---|
| 6 | 5'YICa-04 | $-NH(CH_2)_6S-$ | 5'MVIP06 |
| 7 | 5'YICa-05 | $-NH(CH_2)_8$ O- | 5'MVIP07 |
| 8 | 5'YICr-06 | $-NH(CH_2)_8O-$ | 5'MVIP08 |
| 9 | 5'ERCd-01 | $-NH(CH_2)_6O-$ | 5'MVIP09 |
| 10 | 5'ERCc-01 | $-NH(CH_2)_6O-$ | 5'MVIP10 |
| 11 | 5'ERCa-01 | $-NH(CH_2)_5CH(CH_2CH_3)O-$ | 5'MVIP11 |
| 12 | 5'ERCa-02 | $-O(CH_2)_6O-$ | 5'MVIP12 |
| 13 | 5'ERCa-03 | $-S(CH_2)_6O-$ | 5'MVIP13 |
| 14 | 5'ERCa-04 | $-O(CH_2)_6O-$ | 5'MVIP14 |
| 15 | 5'ERCa-05 | $-O(CH_2)_6O-$ | 5'MVIP15 |
| 16 | 5'ERCr-06 | $-S(CH_2)_4CH(CH_3)O-$ | 5'MVIP16 |
| 17 | 5'SANCd-01 | $-NH(CH_2)_6O-$ | 5'MVIP17 |
| 18 | 5'SANCc-01 | $-NH(CH_2)_6O-$ | 5'MVIP18 |
| 19 | 5'ERCd-01 | 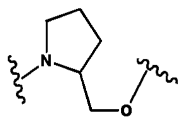 | 5'MVIP19 |
| 20 | 5'ERCd-01 | 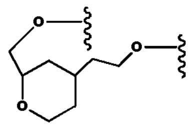 | 5'MVIP20 |
| 21 | 5'YICd-01 | 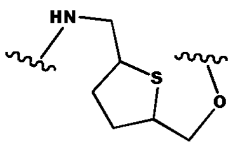 | 5'MVIP21 |
| 22 | 5'SANCd-01 | 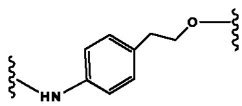 | 5'MVIP22 |

In some embodiments, 3'MVIP may not exist, in which case n may be any integer of 2-4.

In some embodiments, the combinations of **(X-L)$_m$-B-D-** and $R_2$ in the carrier structure of 3'MVIP are shown in Table 12.

Table 12 combinations of **(X-L)$_m$-B-D-** and $R_2$ in 3'MVIP

| No. | **(X-L)$_m$-B-D-** code | $R_2$ | 3'MVIP code |
|---|---|---|---|
| 1 | 3'YICd-01 | 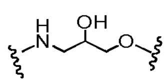 | 3'MVIP01 |
| 2 | 3'YICc-01 | 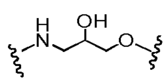 | 3'MVIP02 |
| 3 | 3'YICa-01 | 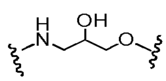 | 3'MVIP03 |

(continued)

| No. | **(X-L)$_m$-B-D-** code | $R_2$ | 3'MVIP code |
|---|---|---|---|
| 4 | 3'YICa-02 | 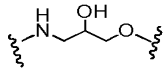 | 3'MVIP04 |
| 5 | 3'YICa-03 | 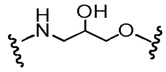 | 3'MVIP05 |
| 6 | 3'YICa-04 | 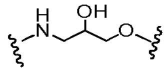 | 3'MVIP06 |
| 7 | 3'YICa-05 | 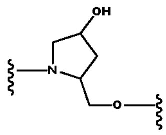 | 3'MVIP07 |
| 8 | 3'YICr-06 | 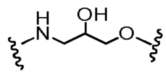 | 3'MVIP08 |
| 9 | 3'ERCd-01 | 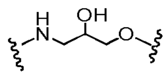 | 3'MVIP09 |
| 10 | 3'ERCc-01 | 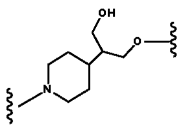 | 3'MVIP10 |
| 11 | 3'ERCa-01 | 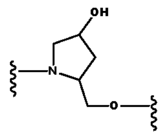 | 3'MVIP11 |
| 12 | 3'ERCa-02 | 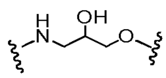 | 3'MVIP12 |
| 13 | 3'ERCa-03 | 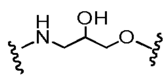 | 3'MVIP13 |
| 14 | 3'ERCa-04 | 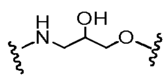 | 3'MVIP14 |
| 15 | 3'ERCa-05 | 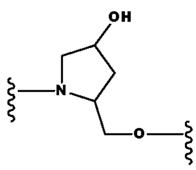 | 3'MVIP15 |
| 16 | 3'ERCr-06 | 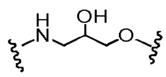 | 3'MVIP16 |
| 17 | 3'SANCd-01 | 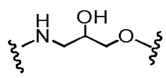 | 3'MVIP17 |
| 18 | 3'SANCc-01 | 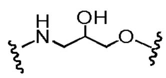 | 3'MVIP18 |

(continued)

| No. | **(X-L)$_m$-B-D-** code | $R_2$ | 3'MVIP code |
|---|---|---|---|
| 19 | 3'SANCa-01 | 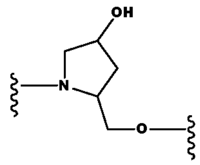 | 3'MVIP19 |
| 20 | 3'ERCd-01 | 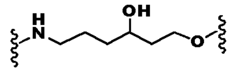 | 3'MVIP20 |
| 21 | 3'ERCd-01 | 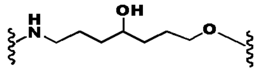 | 3'MVIP21 |
| 22 | 3'ERCd-01 | 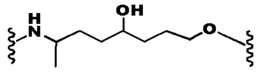 | 3'MVIP22 |
| 23 | 3'ERCd-01 | 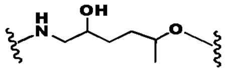 | 3'MVIP23 |
| 24 | 3'ERCd-01 | 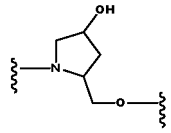 | 3'MVIP24 |
| 25 | 3'ERCd-01 | 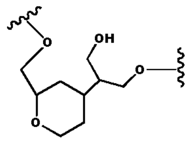 | 3'MVIP25 |
| 26 | 3'ERCd-01 | 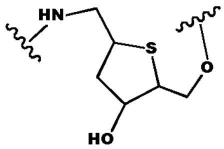 | 3'MVIP26 |
| 27 | 3'ERCd-01 | 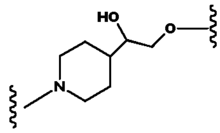 | 3'MVIP27 |

In some embodiments, the 5'MVIP is selected from any one or more of 5'MVIP01 to 5'MVIP22 in Table 11.

In some embodiments, the 3'MVIP is selected from any one or more of 3'MVIP01 to 3'MVIP27 in Table 12.

In some embodiments, there is a possibility of combining any of the 5'MVIP in Table 11 with any of the 3'MVIP in Table 12, where n+m=2, 3, 4, 5 or 6.

In some embodiments, the sense strand in the RNA inhibitor can be selected from the sequences in Table 13 below.

Table 13 sense strand conjugated to 5'MVIP09

| sense strand code | sense strand sequence 5'→3' |
|---|---|
| S19301 | 5'MVIP09-GsAsGCfACfCfGfUUAAGGACAAGsUsU |
| S19401 | 5'MVIP09-GsCsACfCGfUfUfAAGGACAAGUUsCsU |
| S19501 | 5'MVIP09-UsUsACAUGAfAfGfCACGCCACCsAsA |
| S19601 | 5'MVIP09-UsCsUCAGCUfUfCfAUGCAGGGUsUsA |

(continued)

| sense strand code | sense strand sequence 5'→3' |
|---|---|
| S19701 | 5'MVIP09-GsCsUUfCAfGfUfUCCCUGAAAGAsCsU |
| S19801 | 5'MVIP09-UsUsCCfCUfGfAfAAGACUACUGGsAsG |
| S19901 | 5'MVIP09-AsGsACUACUfGfGfAGCACCGUUsAsA |
| S20001 | 5'MVIP09-CsCsGUUAAGfGfAfCAAGUUCUCsUsG |
| S20101 | 5'MVIP09-CsUsGCfCCfCfUfGUAGGUUGCUUsAsA |
| S20201 | 5'MVIP09-CsAsGUfAUfUfCfUCAGUGCUCUCsCsU |
| S20301 | 5'MVIP09-UsGsGCUGCCfUfGfAGACCUCAAsUsA |
| S20401 | 5'MVIP09-GsAsGCfUCfCfUfUGGGUCCUGCAsAsU |
| S20501 | 5'MVIP09-CsCsUGAGGUfCfAfGACCAACUUsCsA |
| S20601 | 5'MVIP09-AsAsGGGACAfGfUfAUUCUCAGUsGsC |

In some embodiments, the sense strand of the RNA inhibitor described in the present invention differs from each sequence in Table 13 by 1, 2, or 3 nucleotides, or has at least 15, 16, 17, 18, 19, 20, 21 consecutive nucleotides identical to the sequences in Table 13.

In some embodiments, the antisense strand in the RNA inhibitor can be selected from the sequences in Table 14 below.

Table 14 antisense strand conjugated to 3'MVIP09

| antisense strand code | antisense strand sequence 5'→3' |
|---|---|
| AS19301 | AsAsCUUGfUCCUUfAAfCGGUGCsUsC-3'MVIP09 |
| AS19401 | AsGsAACUfUGUCCfUUfAACGGUsGsC-3'MVIP09 |
| AS19501 | UsfUsGfGUfGGfCGUGCUfUCfAUGUsAsA-3'MVIP09 |
| AS19601 | UsfAsAfCCfCUfGCAUGAfAGfCUGAsGsA-3'MVIP09 |
| AS19701 | AsGsUCUUfUCAGGfGAfACUGAAsGsC-3'MVIP09 |
| AS19801 | CsUsCCAGfUAGUCfUUfUCAGGGsAsA-3'MVIP09 |
| AS19901 | UsfUsAfACfGGfUGCUCCfAGfUAGUsCsU-3'MVIP09 |
| AS20001 | CsfAsGfAGfAAfCUUGUCfCUfUAACsGsG-3'MVIP09 |
| AS20101 | UsUsAAGCfAACCUfACfAGGGGCsAsG-3'MVIP09 |
| AS20201 | AsGsGAGAfGCACUfGAfGAAUACsUsG-3'MVIP09 |
| AS20301 | UsfAsUfUGfAGfGUCUCAfGGfCAGCsCsA-3'MVIP09 |
| AS20401 | AsUsUGCAfGGACCfCAfAGGAGCsUsC-3'MVIP09 |
| AS20501 | UsfGsAfAGfUUfGGUCUGfACfCUCAsGsG-3'MVIP09 |
| AS20601 | GsfCsAfCUfGAfGAAUACfUGfUCCCUUsUsU-3'MVIP09 |

In some embodiments, the antisense strand of the RNA inhibitor described in the present invention differs from each sequence in Table 14 by 1, 2, or 3 nucleotides, or has at least 15, 16, 17, 18, 19, 20, 21 consecutive nucleotides identical to the sequences in Table 14.

In some in vivo experimental embodiments, the RNA inhibitor described in the present invention is selected from the sequences in Table 15.

Table 15 RNA inhibitors containing 5'MVIP09/3'MVIP09 combination

| RNA inhibitor | sense strand code | antisense strand code |
|---|---|---|
| Kylo-12-DS1041 | S19301 | AS19301 |

(continued)

| RNA inhibitor | sense strand code | antisense strand code |
|---|---|---|
| Kylo-12-DS1051 | S19401 | AS19401 |
| Kylo-12-DS1071 | S19501 | AS19501 |
| Kylo-12-DS1081 | S19601 | AS19601 |
| Kylo-12-DS1091 | S19701 | AS19701 |
| Kylo-12-DS1111 | S19801 | AS19801 |
| Kylo-12-DS1131 | S19901 | AS19901 |
| Kylo-12-DS1141 | S20001 | AS20001 |
| Kylo-12-DS1181 | S20101 | AS20101 |
| Kylo-12-DS1221 | S20201 | AS20201 |
| Kylo-12-DS1241 | S20301 | AS20301 |
| Kylo-12-DS1261 | S20401 | AS20401 |
| Kylo-12-DS1311 | S20501 | AS20501 |
| Kylo-12-DS1321 | S20601 | AS20601 |

In some embodiments, the sense strand and antisense strand of the RNA inhibitor described in the present invention differs from each sequence in Table 15 by 1, 2, or 3 nucleotides, or has at least 15, 16, 17, 18, 19, 20, 21 consecutive nucleotides identical to the sequences in Table 15.

It should be noted that, the combinations in Tables 13, 14, and 15 are not exhaustive. The sequences of the present invention can be connected to MVIPs of any structure, and the connection sites and number of connections are not limited. The "connection site is not limited" means that the site can be at 5' end, 3' end, or not at the end. The "number of connections is not limited" means that the number can be one or more and there is no limit.

In some embodiments, the 5' end and/or 3' end of the antisense strand of the RNA inhibitor UsfAsAfCCfCUfGCAUGAfAGfCUGAsGsA (SEQ ID NO: 382) is connected to 5'MVIP and/or 3'MVIP of different structures, and the antisense strand and its carrier structure are selected from the following Table 16:

Table 16 antisense strand conjugated to 5'MVIP and/or 3'MVIP

| antisense strand code | antisense strand sequence 5'→3' |
|---|---|
| AS19601 | UsfAsAfCCfCUfGCAUGAfAGfCUGAsGsA-3'MVIP09 |
| AS20701 | 5' MVIP17-UsfAsAfCCfCUfGCAUGAfAGfCUGAsGsA |
| AS20801 | UsfAsAfCCfCUfGCAUGAfAGfCUGAsGsA -3' MVIP01 |
| AS20901 | UsfAsAfCCfCUfGCAUGAfAGfCUGAsGsA -3' MVIP17 |
| AS21001 | 5' MVIP01-UsfAsAfCCfCUfGCAUGAfAGfCUGAsGsA -3' MVIP01 |
| AS21101 | 5' MVIP09-UsfAsAfCCfCUfGCAUGAfAGfCUGAsGsA -3' MVIP09 |
| AS21201 | 5' MVIP17-UsfAsAfCCfCUfGCAUGAfAGfCUGAsGsA -3' MVIP17 |
| AS21301 | 5' MVIP01-UsfAsAfCCfCUfGCAUGAfAGfCUGAsGsA -3' MVIP17 |

(continued)

| antisense strand code | antisense strand sequence 5'→3' |
|---|---|
| AS21401 | 5' MVIP17-UsfAsAfCCfCUfGCAUGAfAGfCUGAsGsA -3' MVIP01 |
| AS21501 | 5' MVIP01-UsfAsAfCCfCUfGCAUGAfAGfCUGAsGsA -3' MVIP09 |
| AS21601 | 5' MVIP09-UsfAsAfCCfCUfGCAUGAfAGfCUGAsGsA -3' MVIP01 |
| AS21701 | 5' MVIP09-UsfAsAfCCfCUfGCAUGAfAGfCUGAsGsA -3' MVIP17 |
| AS21801 | 5' MVIP17-UsfAsAfCCfCUfGCAUGAfAGfCUGAsGsA -3' MVIP09 |
| AS21901 | 5' MVIP 12-UsfAsAfCCfCUfGCAUGAfAGfCUGAsGsA |
| AS22001 | UsfAsAfCCfCUfGCAUGAfAGfCUGAsGsA-3' MVIP19 |
| AS22101 | 5'MVIP16- UsfAsAfCCfCUfGCAUGAfAGfCUGAsGsA - 3'MVIP16 |
| AS22201 | UsfAsAfCCfCUfGCAUGAfAGfCUGAsGsA-3' MVIP17 |
| AS22301 | UsfAsAfCCfCUfGCAUGAfAGfCUGAsGsA-3' MVIP18 |
| AS22401 | 5' MVIP03-UsfAsAfCCfCUfGCAUGAfAGfCUGAsGsA |
| AS22501 | 5' MVIP08-UsfAsAfCCfCUfGCAUGAfAGfCUGAsGsA |
| AS22601 | 5'MVIP16-UsfAsAfCCfCUfGCAUGAfAGfCUGAsGsA |
| AS22701 | 5'MVIP13-UsfAsAfCCfCUfGCAUGAfAGfCUGAsGsA- 3'MVIP06 |
| AS22801 | 5'MVIP04-UsfAsAfCCfCUfGCAUGAfAGfCUGAsGsA- 3'MVIP06 |
| AS22901 | 5' MVIP11-UsfAsAfCCfCUfGCAUGAfAGfCUGAsGsA |
| AS23001 | 5' MVIP11-UsfAsAfCCfCUfGCAUGAfAGfCUGAsGsA-3' MVIP14 |
| AS23101 | 5' MVIP15-UsfAsAfCCfCUfGCAUGAfAGfCUGAsGsA |
| AS23201 | 5' MVIP02-UsfAsAfCCfCUfGCAUGAfAGfCUGAsGsA |
| AS23301 | 5' MVIP05-UsfAsAfCCfCUfGCAUGAfAGfCUGAsGsA |
| AS23401 | 5' MVIP06-UsfAsAfCCfCUfGCAUGAfAGfCUGAsGsA |
| AS23501 | 5' MVIP07-UsfAsAfCCfCUfGCAUGAfAGfCUGAsGsA |
| AS23601 | 5' MVIP10- UsfAsAfCCfCUfGCAUGAfAGfCUGAsGsA |
| AS23701 | 5' MVIP14- UsfAsAfCCfCUfGCAUGAfAGfCUGAsGsA |

(continued)

| antisense strand code | antisense strand sequence 5'→3' |
|---|---|
| AS23801 | 5' MVIP18- UsfAsAfCCfCUfGCAUGAfAGfCUGAsGsA |
| AS23901 | UsfAsAfCCfCUfGCAUGAfAGfCUGAsGsA -3' MVIP02 |
| AS24001 | UsfAsAfCCfCUfGCAUGAfAGfCUGAsGsA-3' MVIP03 |
| AS24101 | UsfAsAfCCfCUfGCAUGAfAGfCUGAsGsA -3' MVIP04 |
| AS24201 | 5' MVIP04-UsfAsAfCCfCUfGCAUGAfAGfCUGAsGsA-3' MVIP04 |
| AS24301 | 5' MVIP03-UsfAsAfCCfCUfGCAUGAfAGfCUGAsGsA -3' MVIP19 |
| AS24401 | 5' MVIP18- UsfAsAfCCfCUfGCAUGAfAGfCUGAsGsA-3' MVIP18 |
| AS24501 | 5' MVIP08- UsfAsAfCCfCUfGCAUGAfAGfCUGAsGsA-3' MVIP18 |
| AS24601 | UsfAsAfCCfCUfGCAUGAfAGfCUGAsGsA -3' MVIP05 |
| AS24701 | UsfAsAfCCfCUfGCAUGAfAGfCUGAsGsA -3' MVIP07 |
| AS24801 | UsfAsAfCCfCUfGCAUGAfAGfCUGAsGsA -3' MVIP10 |
| AS24901 | UsfAsAfCCfCUfGCAUGAfAGfCUGAsGsA -3' MVIP11 |
| AS25001 | 5' MVIP11- UsfAsAfCCfCUfGCAUGAfAGfCUGAsGsA-3' MVIP11 |
| AS25101 | 5' MVIP15- UsfAsAfCCfCUfGCAUGAfAGfCUGAsGsA-3' MVIP15 |
| AS25201 | UsfAsAfCCfCUfGCAUGAfAGfCUGAsGsA -3' MVIP06 |
| AS25301 | UsfAsAfCCfCUfGCAUGAfAGfCUGAsGsA -3' MVIP08 |
| AS25401 | UsfAsAfCCfCUfGCAUGAfAGfCUGAsGsA -3' MVIP12 |
| AS25501 | UsfAsAfCCfCUfGCAUGAfAGfCUGAsGsA -3' MVIP13 |
| AS25601 | UsfAsAfCCfCUfGCAUGAfAGfCUGAsGsA -3' MVIP14 |
| AS25701 | UsfAsAfCCfCUfGCAUGAfAGfCUGAsGsA -3' MVIP15 |
| AS25801 | UsfAsAfCCfCUfGCAUGAfAGfCUGAsGsA -3' MVIP16 |
| AS25901 | UsfAsAfCCfCUfGCAUGAfAGfCUGAsGsA -3' MVIP24 |
| AS26001 | UsfAsAfCCfCUfGCAUGAfAGfCUGAsGsA -3' MVIP27 |
| AS26101 | 5' MVIP19-UsfAsAfCCfCUfGCAUGAfAGfCUGAsGsA |
| AS26201 | 5' MVIP20-UsfAsAfCCfCUfGCAUGAfAGfCUGAsGsA |
| AS26301 | 5' MVIP21-UsfAsAfCCfCUfGCAUGAfAGfCUGAsGsA |
| AS26401 | 5' MVIP22-UsfAsAfCCfCUfGCAUGAfAGfCUGAsGsA |
| AS26501 | 5' MVIP01-UsfAsAfCCfCUfGCAUGAfAGfCUGAsGsA |
| AS26601 | 5' MVIP09-UsfAsAfCCfCUfGCAUGAfAGfCUGAsGsA |

In some embodiments, the antisense strand of the RNA inhibitor described in the present invention differs from each sequence in Table 16 by 1, 2, or 3 nucleotides, or has at least 15, 16, 17, 18, 19, 20, 21 consecutive nucleotides identical to the sequences in Table 16.

In some embodiments, the antisense strand conjugated to 5'MVIP and/or 3'MVIP in Table 16 can be obtained by conjugating the sequence in Tables 7-1 and 7-2 with 5'MVIP and/or 3'MVIP.

In some embodiments, the 5' end and/or 3' end of the sense strand of the RNA inhibitor UsCsUCAGCUfUfCfAUGCAGGGUsUsA (SEQ ID NO: 412) is connected to 5'MVIP and/or 3'MVIP of different structures, and the sense strand and its carrier structure are selected from the following Table 17.

Table 17 sense strand conjugated to 5'MVIP and/or 3'MVIP

| sense strand code | sense strand sequence 5'→3' |
|---|---|
| S19601 | 5'MVIP09-UsCsUCAGCUfUfCfAUGCAGGGUsUsA |
| S20701 | UsCsUCAGCUfUfCfAUGCAGGGUsUsA - 3'MVIP17 |
| S20801 | UsCsUCAGCUfUfCfAUGCAGGGUsUsA -3'MVIP09 |
| S20901 | UsCsUCAGCUfUfCfAUGCAGGGUsUsA-3'MVIP01 |
| S21001 | 5'MVIP17- UsCsUCAGCUfUfCfAUGCAGGGUsUsA |
| S21101 | 5'MVIP01- UsCsUCAGCUfUfCfAUGCAGGGUsUsA |
| S21201 | 5'MVIP01-UsCsUCAGCUfUfCfAUGCAGGGUsUsA -3'MVIP01 |
| S21301 | 5'MVIP09-UsCsUCAGCUfUfCfAUGCAGGGUsUsA -3'MVIP09 |
| S21401 | 5'MVIP17-UsCsUCAGCUfUfCfAUGCAGGGUsUsA -3'MVIP17 |
| S21501 | 5'MVIP01-UsCsUCAGCUfUfCfAUGCAGGGUsUsA -3'MVIP17 |
| S21601 | 5'MVIP17-UsCsUCAGCUfUfCfAUGCAGGGUsUsA -3'MVIP01 |
| S21701 | 5'MVIP01-UsCsUCAGCUfUfCfAUGCAGGGUsUsA -3'MVIP09 |
| S21801 | 5'MVIP09-UsCsUCAGCUfUfCfAUGCAGGGUsUsA -3'MVIP01 |
| S21901 | 5'MVIP09-UsCsUCAGCUfUfCfAUGCAGGGUsUsA -3'MVIP17 |
| S22001 | 5'MVIP17-UsCsUCAGCUfUfCfFAUGCAGGGUSsUsA -3'MVIP09 |
| S22101 | 5'MVIP12-UsCsUCAGCUfUfCfAUGCAGGGUsUsA |
| S22201 | UsCsUCAGCUfUfCfAUGCAGGGUsUsA -3'MVIP19 |
| S22301 | 5'MVIP16-UsCsUCAGCUfUfCfAUGCAGGGUsUsA -3'MVIP16 |
| S22401 | UsCsUCAGCUfUfCfAUGCAGGGUsUsA -3'MVIP17 |
| S22501 | UsCsUCAGCUfUfCfAUGCAGGGUsUsA -3'MVIP18 |
| S22601 | 5' MVIP03-UsCsUCAGCUfUfCfAUGCAGGGUsUsA |
| S22701 | 5' MVIP08-UsCsUCAGCUfUfCfAUGCAGGGUsUsA |
| S19601 | 5'MVIP09-UsCsUCAGCUfUfCfAUGCAGGGUsUsA |
| S22801 | 5' MVIP16-UsCsUCAGCUfUfCfAUGCAGGGUsUsA |
| S22901 | 5'MVIP13-UsCsUCAGCUfUfCfAUGCAGGGUsUsA -3'MVIP06 |
| S23001 | 5'MVIP04-UsCsUCAGCUfUfCfAUGCAGGGUsUsA -3'MVIP06 |
| S23101 | 5'MVIP11-UsCsUCAGCUfUfCfAUGCAGGGUsUsA |
| S23201 | 5'MVIP11-UsCsUCAGCUfUfCfAUGCAGGGUsUsA -3'MVIP14 |
| S23301 | 5'MVIP15-UsCsUCAGCUfUfCfAUGCAGGGUsUsA |
| S23401 | 5'MVIP02-UsCsUCAGCUfUfCfAUGCAGGGUsUsA |
| S23501 | 5'MVIP05-UsCsUCAGCUfUfCfAUGCAGGGUsUsA |
| S23601 | 5'MVIP06-UsCsUCAGCUfUfCfAUGCAGGGUsUsA |
| S23701 | 5'MVIP07-UsCsUCAGCUfUfCfAUGCAGGGUsUsA |

(continued)

| sense strand code | sense strand sequence 5'→3' |
|---|---|
| S23801 | 5'MVIP10-UsCsUCAGCUfUfCfAUGCAGGGUsUsA |
| S23901 | 5'MVIP14-UsCsUCAGCUfUfCfAUGCAGGGUsUsA |
| S24001 | 5'MVIP18-UsCsUCAGCUfUfCfAUGCAGGGUsUsA |
| S24101 | UsCsUCAGCUfUfCfAUGCAGGGUsUsA -3' MVIP02 |
| S24201 | UsCsUCAGCUfUfCfAUGCAGGGUsUsA -3' MVIP03 |
| S24301 | UsCsUCAGCUfUfCfAUGCAGGGUsUsA -3' MVIP04 |
| S24401 | 5' MVIP04-UsCsUCAGCUfUfCfAUGCAGGGUsUsA -3' MVIP04 |
| S24501 | 5' MVIP03-UsCsUCAGCUfUfCfAUGCAGGGUsUsA -3' MVIP19 |
| S24601 | 5' MVIP18-UsCsUCAGCUfUfCfAUGCAGGGUsUsA -3' MVIP18 |
| S24701 | 5' MVIP08-UsCsUCAGCUfUfCfAUGCAGGGUsUsA -3' MVIP18 |
| S24801 | UsCsUCAGCUfUfCfAUGCAGGGUsUsA -3' MVIP05 |
| S24901 | UsCsUCAGCUfUfCfAUGCAGGGUsUsA -3' MVIP07 |
| S25001 | UsCsUCAGCUfUfCfAUGCAGGGUsUsA -3' MVIP10 |
| S25101 | UsCsUCAGCUfUfCfAUGCAGGGUsUsA -3' MVIP11 |
| S25201 | 5' MVIP11-UsCsUCAGCUfUfCfAUGCAGGGUsUsA -3' MVIP11 |
| S25301 | 5' MVIP15-UsCsUCAGCUfUfCfAUGCAGGGUsUsA -3' MVIP15 |
| S19601 | 5'MVIP09-UsCsUCAGCUfUfCfAUGCAGGGUsUsA |
| S25401 | UsCsUCAGCUfUfCfAUGCAGGGUsUsA -3' MVIP06 |
| S25501 | UsCsUCAGCUfUfCfAUGCAGGGUsUsA -3' MVIP08 |
| S25601 | UsCsUCAGCUfUfCfAUGCAGGGUsUsA -3' MVIP12 |
| S25701 | UsCsUCAGCUfUfCfAUGCAGGGUsUsA -3' MVIP13 |
| S25801 | UsCsUCAGCUfUfCfAUGCAGGGUsUsA -3' MVIP14 |
| S25901 | UsCsUCAGCUfUfCfAUGCAGGGUsUsA -3' MVIP15 |
| S26001 | UsCsUCAGCUfUfCfAUGCAGGGUsUsA -3' MVIP16 |
| S26101 | 5' MVIP19-UsCsUCAGCUfUfCfAUGCAGGGUsUsA |
| S26201 | 5' MVIP20-UsCsUCAGCUfUfCfAUGCAGGGUsUsA |
| S26301 | 5' MVIP21-UsCsUCAGCUfUfCfAUGCAGGGUsUsA |
| S26401 | 5' MVIP22-UsCsUCAGCUfUfCfAUGCAGGGUsUsA |

In some embodiments, the sense strand of the RNA inhibitor described in the present invention differs from each sequence in Table 17 by 1, 2, or 3 nucleotides, or has at least 15, 16, 17, 18, 19, 20, 21 consecutive nucleotides identical to the sequences in Table 17.

In some embodiments, the 5' end and/or 3' end of the antisense strand of the RNA inhibitor AsfCsAfCUfGAfGAAUACfUGfUCfGCsfUsC (SEQ ID NO: 970) is connected to 5'MVIP and/or 3'MVIP of different structures, and the antisense strand and its carrier structure are selected from the following Table 18.

Table 18 antisense strand conjugated to 5'MVIP and/or 3'MVIP

| antisense strand code | antisense strand sequence 5'→3' |
|---|---|
| Ky-12AS01 | AsfCsAfCUfGAfGAAUACfUGfUCfGCsfUsC-3'MVIP09 |
| Ky-12AS02 | 5'MVIP17-AsfCsAfCUfGAfGAAUACfUGfUCfGCsfUsC |
| Ky-12AS03 | AsfCsAfCUfGAfGAAUACfUGfUCfGCsfUsC-3'MVIP01 |

(continued)

| antisense strand code | antisense strand sequence 5'→3' |
|---|---|
| Ky-12AS04 | AsfCsAfCUfGAfGAAUACfUGfUCfGCsfUsC-3'MVIP17 |
| Ky-12AS05 | 5'MVIP01-AsfCsAfCUfGAfGAAUACfUGfUCfGCsfUsC-3'MVIP01 |
| Ky-12AS06 | 5'MVIP09-AsfCsAfCUfGAfGAAUACfUGfUCfGCsfUsC-3'MVIP09 |
| Ky-12AS07 | 5'MVIP17-AsfCsAfCUfGAfGAAUACfUGfUCfGCsfUsC-3'MVIP17 |
| Ky-12AS08 | 5'MVIP01-AsfCsAfCUfGAfGAAUACfUGfUCfGCsfUsC-3'MVIP17 |
| Ky-12AS09 | 5'MVIP17-AsfCsAfCUfGAfGAAUACfUGfUCfGCsfUsC-3'MVIP01 |
| Ky-12AS10 | 5'MVIP01-AsfCsAfCUfGAfGAAUACfUGfUCfGCsfUsC-3'MVIP09 |
| Ky-12AS11 | 5'MVIP09-AsfCsAfCUfGAfGAAUACfUGfUCfGCsfUsC-3'MVIP01 |
| Ky-12AS12 | 5'MVIP09-AsfCsAfCUfGAfGAAUACfUGfUCfGCsfUsC-3'MVIP17 |
| Ky-12AS13 | 5'MVIP17-AsfCsAfCUfGAfGAAUACfUGfUCfGCsfUsC-3'MVIP09 |
| Ky-12AS14 | 5'MVIP12-AsfCsAfCUfGAfGAAUACfUGfUCfGCsfUsC |
| Ky-12AS15 | AsfCsAfCUfGAfGAAUACfUGfUCfGCsfUsC-3'MVIP19 |
| Ky-12AS16 | 5'MVIP16-AsfCsAfCUfGAfGAAUACfUGfUCfGCsfUsC-3'MVIP16 |
| Ky-12AS17 | AsfCsAfCUfGAfGAAUACfUGfUCfGCsfUsC-3'MVIP17 |
| Ky-12AS18 | AsfCsAfCUfGAfGAAUACfUGfUCfGCsfUsC-3'MVIP18 |
| Ky-12AS19 | 5'MVIP03-AsfCsAfCUfGAfGAAUACfUGfUCfGCsfUsC |
| Ky-12AS20 | 5'MVIP08-AsfCsAfCUfGAfGAAUACfUGfUCfGCsfUsC |
| Ky-12AS21 | 5'MVIP16-AsfCsAfCUfGAfGAAUACfUGfUCfGCsfUsC |
| Ky-12AS22 | 5'MVIP13-AsfCsAfCUfGAfGAAUACfUGfUCfGCsfUsC-3'MVIP06 |
| Ky-12AS23 | 5'MVIP04-AsfCsAfCUfGAfGAAUACfUGfUCfGCsfUsC-3'MVIP06 |
| Ky-12AS24 | 5'MVIP11-AsfCsAfCUfGAfGAAUACfUGfUCfGCsfUsC |
| Ky-12AS25 | 5'MVIP11-AsfCsAfCUfGAfGAAUACfUGfUCfGCsfUsC-3'MVIP14 |
| Ky-12AS26 | 5'MVIP15-AsfCsAfCUfGAfGAAUACfUGfUCfGCsfUsC |
| Ky-12AS27 | 5'MVIP02-AsfCsAfCUfGAfGAAUACfUGfUCfGCsfUsC |
| Ky-12AS28 | 5'MVIP05-AsfCsAfCUfGAfGAAUACfUGfUCfGCsfUsC |
| Ky-12AS29 | 5'MVIP06-AsfCsAfCUfGAfGAAUACfUGfUCfGCsfUsC |
| Ky-12AS30 | 5'MVIP07-AsfCsAfCUfGAfGAAUACfUGfUCfGCsfUsC |
| Ky-12AS31 | 5'MVIP10-AsfCsAfCUfGAfGAAUACfUGfUCfGCsfUsC |
| Ky-12AS32 | 5'MVIP14-AsfCsAfCUfGAfGAAUACfUGfUCfGCsfUsC |
| Ky-12AS33 | 5'MVIP18-AsfCsAfCUfGAfGAAUACfUGfUCfGCsfUsC |
| Ky-12AS34 | AsfCsAfCUfGAfGAAUACfUGfUCfGCsfUsC-3'MVIP02 |
| Ky-12AS35 | AsfCsAfCUfGAfGAAUACfUGfUCfGCsfUsC-3'MVIP03 |
| Ky-12AS36 | AsfCsAfCUfGAfGAAUACfUGfUCfGCsfUsC-3'MVIP04 |
| Ky-12AS37 | 5'MVIP04-AsfCsAfCUfGAfGAAUACfUGfUCfGCsfUsC-3'MVIP04 |
| Ky-12AS38 | 5'MVIP03-AsfCsAfCUfGAfGAAUACfUGfUCfGCsfUsC-3'MVIP19 |
| Ky-12AS39 | 5'MVIP18-AsfCsAfCUfGAfGAAUACfUGfUCfGCsfUsC-3'MVIP18 |
| Ky-12AS40 | 5'MVIP08-AsfCsAfCUfGAfGAAUACfUGfUCfGCsfUsC-3'MVIP18 |
| Ky-12AS41 | AsfCsAfCUfGAfGAAUACfUGfUCfGCsfUsC-3'MVIP05 |
| Ky-12AS42 | AsfCsAfCUfGAfGAAUACfUGfUCfGCsfUsC-3'MVIP07 |

(continued)

| antisense strand code | antisense strand sequence 5'→3' |
|---|---|
| Ky-12AS43 | AsfCsAfCUfGAfGAAUACfUGfUCfGCsfUsC-3'MVIP10 |
| Ky-12AS44 | AsfCsAfCUfGAfGAAUACfUGfUCfGCsfUsC-3'MVIP11 |
| Ky-12AS45 | 5'MVIP11-AsfCsAfCUfGAfGAAUACfUGfUCfGCsfUsC-3'MVIP11 |
| Ky-12AS46 | 5'MVIP15-AsfCsAfCUfGAfGAAUACfUGfUCfGCsfUsC-3'MVIP15 |
| Ky-12AS47 | AsfCsAfCUfGAfGAAUACfUGfUCfGCsfUsC-3'MVIP06 |
| Ky-12AS48 | AsfCsAfCUfGAfGAAUACfUGfUCfGCsfUsC-3'MVIP08 |
| Ky-12AS49 | AsfCsAfCUfGAfGAAUACfUGfUCfGCsfUsC-3'MVIP12 |
| Ky-12AS50 | AsfCsAfCUfGAfGAAUACfUGfUCfGCsfUsC-3'MVIP13 |
| Ky-12AS51 | AsfCsAfCUfGAfGAAUACfUGfUCfGCsfUsC-3'MVIP14 |
| Ky-12AS52 | AsfCsAfCUfGAfGAAUACfUGfUCfGCsfUsC-3'MVIP15 |
| Ky-12AS53 | AsfCsAfCUfGAfGAAUACfUGfUCfGCsfUsC-3'MVIP16 |
| Ky-12AS54 | AsfCsAfCUfGAfGAAUACfUGfUCfGCsfUsC-3'MVIP24 |
| Ky-12AS55 | AsfCsAfCUfGAfGAAUACfUGfUCfGCsfUsC-3'MVIP27 |
| Ky-12AS56 | 5'MVIP19-AsfCsAfCUfGAfGAAUACfUGfUCfGCsfUsC |
| Ky-12AS57 | 5'MVIP20-AsfCsAfCUfGAfGAAUACfUGfUCfGCsfUsC |
| Ky-12AS58 | 5'MVIP21-AsfCsAfCUfGAfGAAUACfUGfUCfGCsfUsC |
| Ky-12AS59 | 5'MVIP22-AsfCsAfCUfGAfGAAUACfUGfUCfGCsfUsC |
| Ky-12AS60 | 5'MVIP01-AsfCsAfCUfGAfGAAUACfUGfUCfGCsfUsC |
| Ky-12AS61 | 5'MVIP09-AsfCsAfCUfGAfGAAUACfUGfUCfGCsfUsC |

In some embodiments, the antisense strand of the RNA inhibitor described in the present invention differs from each sequence in Table 18 by 1, 2, or 3 nucleotides, or has at least 15, 16, 17, 18, 19, 20, 21 consecutive nucleotides identical to the sequences in Table 18.

It should be noted that, the combinations in Table 18 are not exhaustive. The sequences of the present invention can be connected to MVIPs of any structure, and the connection sites and number of connections are not limited. The "connection site is not limited" means that the site can be at 5' end, 3' end, or not at the end. The "number of connections is not limited" means that the number can be one or more and there is no limit.

In some embodiments, the 5' end and/or 3' end of the sense strand of the RNA inhibitor GsAsGCGACAfGfUfAUUCUCAGUsGsU (SEQ ID NO: 1033) is connected to 5'MVIP and/or 3'MVIP of different structures, and the sense strand and its carrier structure are selected from the following Table 19:

Table 19 sense strand conjugated to 5'MVIP and/or 3'MVIP

| sense strand code | sense strandsequence 5'→3' |
|---|---|
| Ky-12S01 | 5'MVIP09-GsAsGCGACAfGfUfAUUCUCAGUsGsU |
| Ky-12S02 | GsAsGCGACAfGfUfAUUCUCAGUsGsU-3'MVIP17 |
| Ky-12S03 | GsAsGCGACAfGfUfAUUCUCAGUsGsU-3'MVIP09 |
| Ky-12S04 | GsAsGCGACAfGfUfAUUCUCAGUsGsU-3'MVIP01 |
| Ky-12S05 | 5'MVIP17-GsAsGCGACAfGfUfAUUCUCAGUsGsU |
| Ky-12S06 | 5'MVIP01-GsAsGCGACAfGfUfAUUCUCAGUsGsU |
| Ky-12S07 | 5'MVIP01-GsAsGCGACAfGfUfAUUCUCAGUsGsU-3'MVIP01 |
| Ky-12S08 | 5'MVIP09-GsAsGCGACAfGfUfAUUCUCAGUsGsU-3'MVIP09 |
| Ky-12S09 | 5'MVIP17-GsAsGCGACAfGfUfAUUCUCAGUsGsU-3'MVIP17 |
| Ky-12S10 | 5'MVIP01-GsAsGCGACAfGfUfAUUCUCAGUsGsU-3'MVIP17 |

(continued)

| sense strand code | sense strandsequence 5'→3' |
|---|---|
| Ky-12S11 | 5'MVIP17-GsAsGCGACACUCAGUsGsU-3'MVIP01 |
| Ky-12S12 | 5'MVIP01-GsAsGCGACAfGfUfAUUCUCAGUsGsU-3'MVIP09 |
| Ky-12S13 | 5'MVIP09-GsAsGCGACAfGfUfAUUCUCAGUsGsU-3'MVIP01 |
| Ky-12S14 | 5'MVIP09-GsAsGCGACAfGfUfAUUCUCAGUsGsU-3'MVIP17 |
| Ky-12S15 | 5'MVIP17-GsAsGCGACAfGfUfAUUCUCAGUsGsU-3'MVIP09 |
| Ky-12S16 | 5'MVIP12-GsAsGCGACAfGfUfAUUCUCAGUsGsU |
| Ky-12S17 | GsAsGCGACAfGfUfAUUCUCAGUsGsU-3'MVIP19 |
| Ky-12S18 | 5'MVIP16-GsAsGCGACAfGfUfAUUCUCAGUsGsU-3'MVIP16 |
| Ky-12S19 | GsAsGCGACAfGfUfAUUCUCAGUsGsU-3'MVIP17 |
| Ky-12S20 | GsAsGCGACAfGfUfAUUCUCAGUsGsU-3'MVIP18 |
| Ky-12S21 | 5'MVIP03-GsAsGCGACAfGfUfAUUCUCAGUsGsU |
| Ky-12S22 | 5'MVIP08-GsAsGCGACAfGfUfAUUCUCAGUsGsU |
| Ky-12S23 | 5'MVIP16-GsAsGCGACAfGfUfAUUCUCAGUsGsU |
| Ky-12S24 | 5'MVIP13-GsAsGCGACAfGfUfAUUCUCAGUsGsU-3'MVIP06 |
| Ky-12S25 | 5'MVIP04-GsAsGCGACAfGfUfAUUCUCAGUsGsU-3'MVIP06 |
| Ky-12S26 | 5'MVIP11-GsAsGCGACAfGfUfAUUCUCAGUsGsU |
| Ky-12S27 | 5'MVIP11-GsAsGCGACAfGfUfAUUCUCAGUsGsU-3'MVIP14 |
| Ky-12S28 | 5'MVIP15-GsAsGCGACAfGfUfAUUCUCAGUsGsU |
| Ky-12S29 | 5'MVIP02-GsAsGCGACAfGfUfAUUCUCAGUsGsU |
| Ky-12S30 | 5'MVIP05-GsAsGCGACAfGfUfAUUCUCAGUsGsU |
| Ky-12S31 | 5'MVIP06-GsAsGCGACAfGfUfAUUCUCAGUsGsU |
| Ky-12S32 | 5'MVIP07-GsAsGCGACAfGfUfAUUCUCAGUsGsU |
| Ky-12S33 | 5'MVIP10-GsAsGCGACAfGfUfAUUCUCAGUsGsU |
| Ky-12S34 | 5'MVIP14-GsAsGCGACAfGfUfAUUCUCAGUsGsU |
| Ky-12S35 | 5'MVIP18-GsAsGCGACAfGfUfAUUCUCAGUsGsU |
| Ky-12S36 | GsAsGCGACAfGfUfAUUCUCAGUsGsU-3'MVIP02 |
| Ky-12S37 | GsAsGCGACAfGfUfAUUCUCAGUsGsU-3'MVIP03 |
| Ky-12S38 | GsAsGCGACAfGfUfAUUCUCAGUsGsU-3'MVIP04 |
| Ky-12S39 | 5'MVIP04-GsAsGCGACAfGfUfAUUCUCAGUsGsU-3'MVIP04 |
| Ky-12S40 | 5'MVIP03-GsAsGCGACAfGfUfAUUCUCAGUsGsU-3'MVIP19 |
| Ky-12S41 | 5'MVIP18-GsAsGCGACAfGfUfAUUCUCAGUsGsU-3'MVIP18 |
| Ky-12S42 | 5'MVIP08-GsAsGCGACAfGfUfAUUCUCAGUsGsU-3'MVIP18 |
| Ky-12S43 | GsAsGCGACAfGfUfAUUCUCAGUsGsU-3'MVIP05 |
| Ky-12S44 | GsAsGCGACAfGfUfAUUCUCAGUsGsU-3'MVIP07 |
| Ky-12S45 | GsAsGCGACAfGfUfAUUCUCAGUsGsU-3'MVIP10 |
| Ky-12S46 | GsAsGCGACAfGfUfAUUCUCAGUsGsU-3'MVIP11 |
| Ky-12S47 | 5'MVIP11-GsAsGCGACAfGfUfAUUCUCAGUsGsU-3'MVIP11 |
| Ky-12S48 | 5'MVIP15-GsAsGCGACAfGfUfAUUCUCAGUsGsU-3'MVIP15 |
| Ky-12S49 | GsAsGCGACAfGfUfAUUCUCAGUsGsU-3'MVIP06 |

(continued)

| sense strand code | sense strandsequence 5'→3' |
|---|---|
| Ky-12S50 | GsAsGCGACAfGfUfAUUCUCAGUsGsU-3'MVIP08 |
| Ky-12S51 | GsAsGCGACAfGfUfAUUCUCAGUsGsU-3'MVIP12 |
| Ky-12S52 | GsAsGCGACAfGfUfAUUCUCAGUsGsU-3'MVIP13 |
| Ky-12S53 | GsAsGCGACAfGfUfAUUCUCAGUsGsU-3'MVIP14 |
| Ky-12S54 | GsAsGCGACAfGfUfAUUCUCAGUsGsU-3'MVIP15 |
| Ky-12S55 | GsAsGCGACAfGfUfAUUCUCAGUsGsU-3'MVIP16 |
| Ky-12S56 | 5'MVIP 19-GsAsGCGACAfGfUfAUUCUCAGU sGsU |
| Ky-12S57 | 5'MVIP20-GsAsGCGACAfGfUfAUUCUCAGUsGsU |
| Ky-12S58 | 5'MVIP21-GsAsGCGACAfGfUfAUUCUCAGUsGsU |
| Ky-12S59 | 5'MVIP22-GsAsGCGACAfGfUfAUUCUCAGUsGsU |

In some embodiments, the sense strand of the RNA inhibitor described in the present invention differs from each sequence in Table 19 by 1, 2, or 3 nucleotides, or has at least 15, 16, 17, 18, 19, 20, 21 consecutive nucleotides identical to the sequences in Table 19.

It should be noted that, the combinations in Table 19 are not exhaustive. The sequences of the present invention can be connected to MVIPs of any structure, and the connection sites and number of connections are not limited. The "connection site is not limited" means that the site can be at 5' end, 3' end, or not at the end. The "number of connections is not limited" means that the number can be one or more and there is no limit.

In some embodiments, the antisense strand of the RNA inhibitor can be obtained by conjugating the antisense strand sequence exemplified above with any 5'MVIP and/or 3'MVIP.

In some embodiments, the sense strand of the RNA inhibitor can be obtained by conjugating the sense strand sequence exemplified above with any 5'MVIP and/or 3'MVIP.

In some embodiments, the sequences within the protection scope of the present invention can be connected to any number of 5' MVIP or 3' MVIP in any structure at any position to form RNA inhibitors. These RNA inhibitors are all within the protection scope of the present invention and are inspired by the present invention.

Chinese patent CN113171371B provides a detailed examination of the effects of different X, L, B, D, $R_1$, and $R_2$ in the structures of 5'MVIP and/or 3'MVIP on the activity of RNA inhibitors. The full text of this patent is incorporated herein by reference. When one of X, L, B, D, $R_1$, and $R_2$ is different, the other parts of the corresponding 5'MVIP and/or 3'MVIP are the same as those of 5'MVIP 09/3'MVIP 09.

When X is galactose, galactosamine, N-acetylgalactosamine and their derivatives respectively, the RNA inhibitor provided by the present invention preferably uses N-acetylgalactosamine and its derivatives as liver targeting specific ligands.

| X code | X structure |
|---|---|
| X1 | 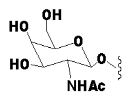 |
| X2 | 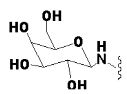 |
| X3 | 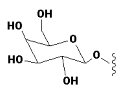 |
| X4 | 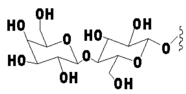 |

(continued)

| X code | X structure |
|---|---|
| X5 | 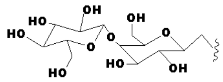 |
| X6 | 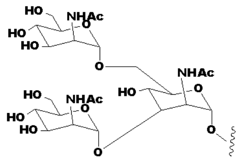 |

The length of L has a great impact on the effect of RNA inhibitors. The L chain should not be too short or too long. When L contains -NH-, C=O, O, S, amido, phosphoryl, thiophosphoryl, aliphatic carbocyclyl such as cyclohexane, or a combination of these groups, or has different structures in the same 5'MVIP, 3'MVIP structures or between 5'MVIP and 3'MVIP, the activities of the resulting RNA inhibitors do not differ significantly in the range of carbon chain length of C7-C18.

| L code | L structure |
|---|---|
| L1 | 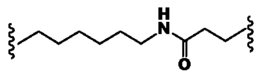 |
| L2 | 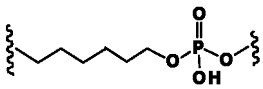 |
| L3 | 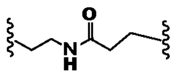 |
| L4 | 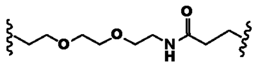 |
| L5 | 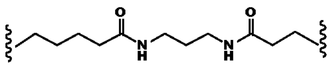 |
| L6 | 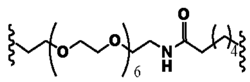 |
| L7 | 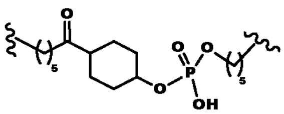 |
| L8 | 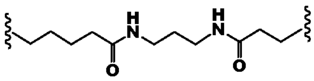 |
| L9 | 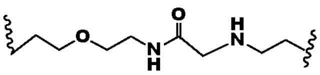 |
| L10 | 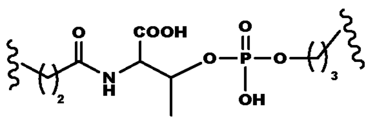 |

(continued)

| L code | L structure |
|---|---|
| L11 | L in 5' MVIP for S:<br>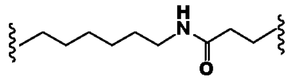<br>L in 3'MVIP for AS:<br>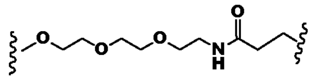 |
| L12 | L in 5' MVIP for S:<br>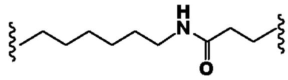<br>L in 3'MVIP for AS:<br>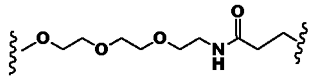 |
| L13 | L in 5' MVIP for S:<br>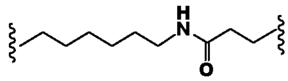 |
| | L in 3'MVIP for AS:<br>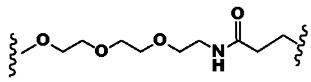 |
| L14 | 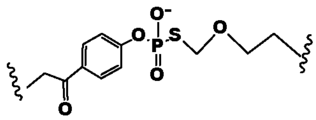 |

Except for the change in the structure of the linker B, when X, L, D and $R_1/R_2$ are consistent with those in the combination 5'MVIP09/3'MVIP09, $A_1$ and $A_2$ in the general formula of linker B are each independently C, O, S, -NH-, carbonyl, amido, phosphoryl or thiophosphoryl, and r is any integer of 0-4, and when the linker B is the same or different between 5'MVIP and 3'MVIP, the inhibitory activity of resulting RNA inhibitor does not differ significantly.

| B code | B structure |
|---|---|
| B1 | 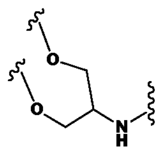 |
| B2 | 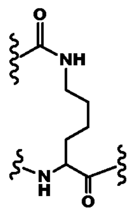 |

(continued)

| B code | B structure |
|---|---|
| B3 | 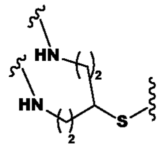 |
| B4 | 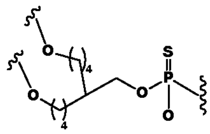 |
| B5 | 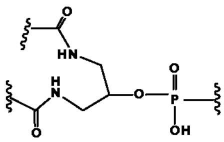 |
| B6 | 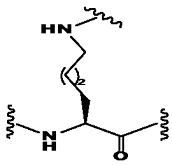 |
| B7 | 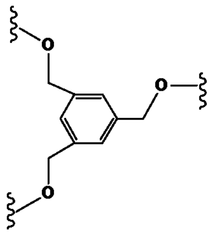 |
| B8 | B in 5'MVIP for S: 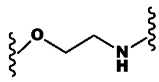 |
| | B in 3'MVIP for AS: 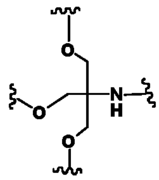 |
| B9 | B in 5'MVIP for S: 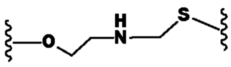 |
| | B in 3'MVIP for AS: 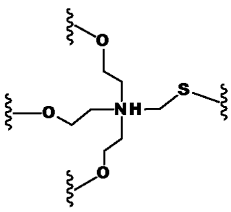 |

(continued)

| B code | B structure |
|---|---|
| B10 | B in 5'MVIP for S: 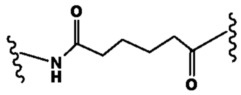 |
| | B in 3'MVIP for AS: 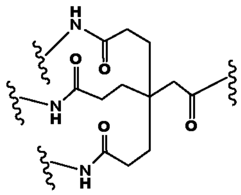 |
| B11 | B in 5'MVIP for S: 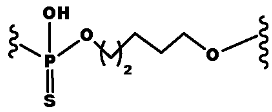 |
| | B in 3'MVIP for AS: |
| | 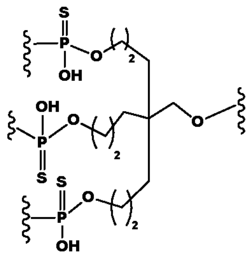 |
| B12 | B in 5'MVIP for S: 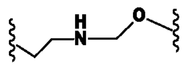 |
| | B in 3'MVIP for AS: 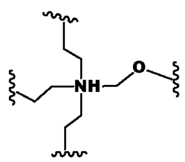 |
| B13 | B in 5'MVIP for S: 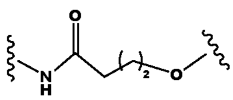 |
| | B in 3'MVIP for AS: 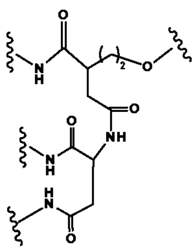 |

(continued)

| B code | B structure |
|---|---|
| B14 | B in 5'MVIP for S: 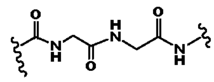 |
| | B in 3'MVIP for AS: 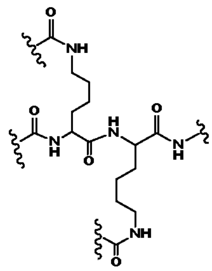 |
| B15 | B in 5'MVIP for S: |
| | 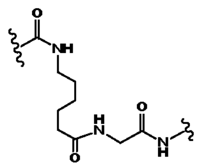 |
| | B in 3'MVIP for AS: 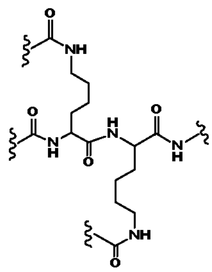 |
| B16 | B in 5'MVIP for S: 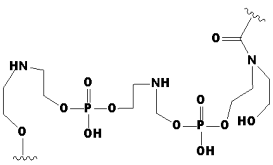 |
| | B in 3'MVIP for AS: 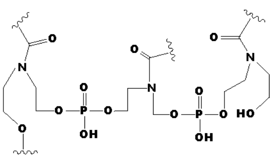 |

(continued)

| B code | B structure |
|---|---|
| B17 | B in 5'MVIP for S: 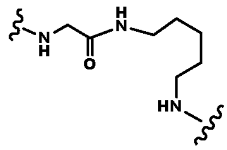 |
| | B in 3'MVIP for AS: 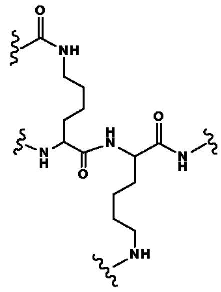 |
| B18 | B in 5'MVIP for S: 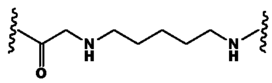 |
| | B in 3'MVIP for AS: 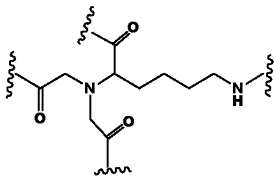 |
| B19 | B in 5'MVIP for S: 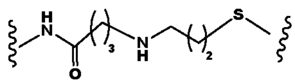 |
| | B in 3'MVIP for AS: 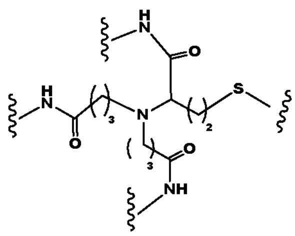 |
| B20 | 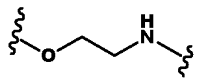 |
| B21 | 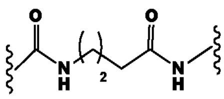 |
| B22 | 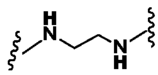 |

(continued)

| B code | B structure |
|---|---|
| B23 | 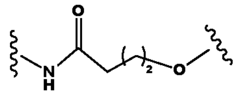 |
| B24 | 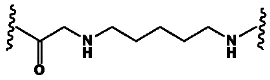 |
| B25 | 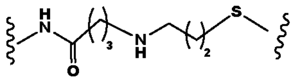 |
| B26 | 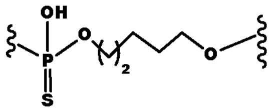 |
| B27 | B in 5'MVIP for S: 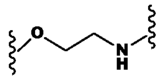 |
| | B in 3'MVIP for AS: 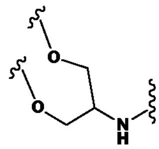 |
| B28 | B in 5'MVIP for S: 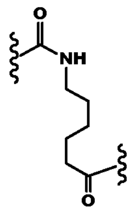 |
| | B in 3'MVIP for AS: 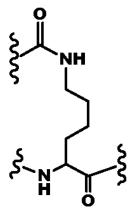 |
| B29 | B in 5'MVIP for S: 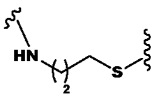 |
| | B in 3'MVIP for AS: 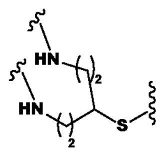 |

(continued)

| B code | B structure |
|---|---|
| B30 | 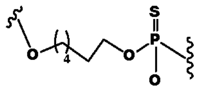 B in 5'MVIP for S: |
| | 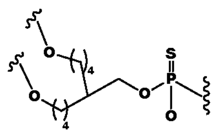 B in 3'MVIP for AS: |
| B31 | 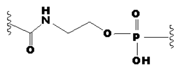 B in 5'MVIP for S: |
| | 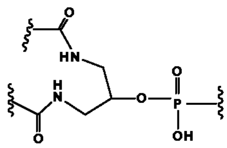 B in 3'MVIP for AS: |
| B32 | B in 5'MVIP for S: 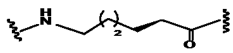 |
| | B in 3'MVIP for AS: 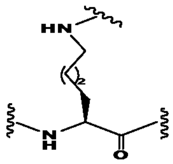 |
| B33 | B in 5'MVIP for S: 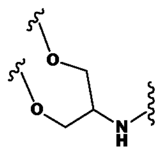 |
| | B in 3'MVIP for AS: 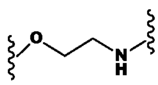 |

(continued)

| B code | B structure |
|---|---|
| B34 | B in 5'MVIP for S: 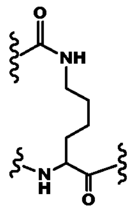 |
| | B in 3'MVIP for AS: 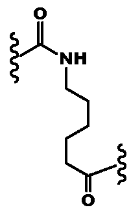 |
| B35 | B in 5'MVIP for S: 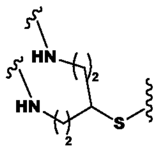 |
| | B in 3'MVIP for AS: 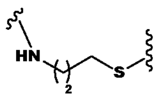 |
| B36 | 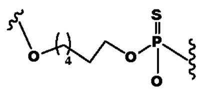 B in 5'MVIP for S: |
| | 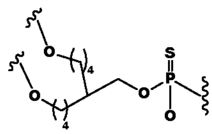 B in 3'MVIP for AS: |
| B37 | 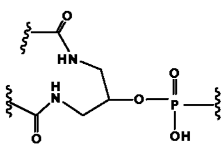 B in 5'MVIP for S: |
| | 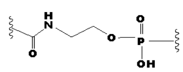 B in 3'MVIP for AS: |

(continued)

| B code | B structure |
|---|---|
| B38 | B in 5'MVIP for S:<br>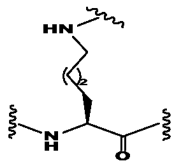 |
| | B in 3'MVIP for AS:<br>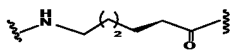 |

When the MVIP structure and RNA inhibitor are the same, different linking chain D will have an impact on the activity of the RNA inhibitor. Among them, the effects of D1, D2, and D4 are similar and better than D3.

| D code | D structure |
|---|---|
| D1 | D in 5'MVIP for S:<br>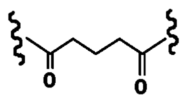 |
| | D in 3'MVIP for AS:<br>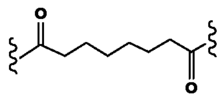 |
| D2 | D in 5'MVIP for S:<br>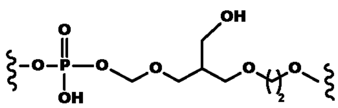 |
| | D in 3'MVIP for AS:<br>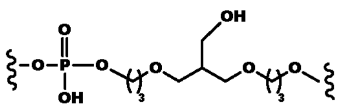 |
| D3 | D in 5'MVIP for S:<br>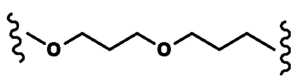 |
| | D in 3'MVIP for AS:<br>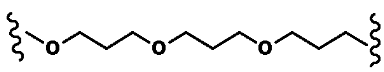 |
| D4 | D in 5'MVIP for S:<br>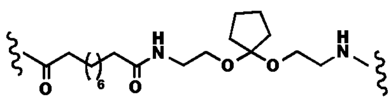 |
| | D in 3'MVIP for AS:<br>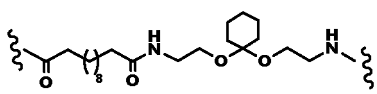 |

(continued)

| D code | D structure |
|---|---|
| D5 | D in 5'MVIP for S:<br>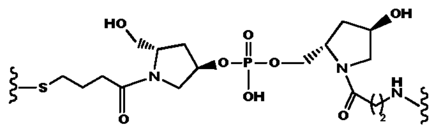 |
| | D in 3'MVIP for AS:<br>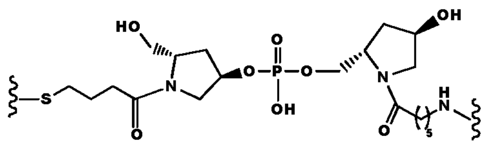 |

Different transition point $R_1$ will affect the activity of RNA inhibitor, among which the RNA inhibitor obtained by using R1-1 as the transition point has the best activity.

| $R_1$ code | $R_1$ structure |
|---|---|
| R1-1 | $-NH(CH_2)_6O-$ |
| R1-2 | $-O(CH_2)_6O-$ |
| R1-3 | $-S(CH_2)_6O-$ |
| R1-4 | $-NH(CH_2)_8$ O- |
| R1-5 | $-NH(CH_2)_5CH(CH_2CH_3)O-$ |
| R1-6 | $-S(CH_2)_4CH(CH_3)O-$ |

Different transition point $R_2$ will affect the activity of RNA inhibitor, among which the RNA inhibitor obtained by using R2-1 as the transition point has the best activity.

| $R_2$ code | $R_2$ structure |
|---|---|
| R2-1 | 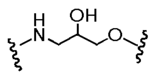 |
| R2-2 | 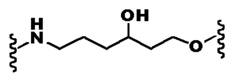 |
| R2-3 | 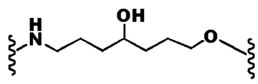 |
| R2-4 | 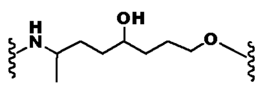 |
| R2-5 | 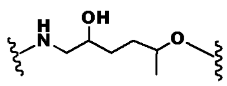 |
| R2-6 | 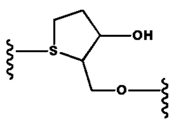 |

(continued)

| $R_2$ code | $R_2$ structure |
|---|---|
| R2-7 | 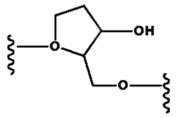 |
| R2-8 | 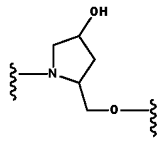 |
| R2-9 | 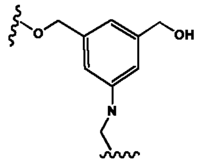 |
| R2-10 | 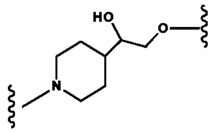 |
| R2-11 | 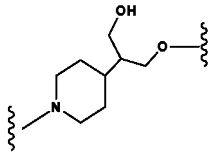 |
| R2-12 | 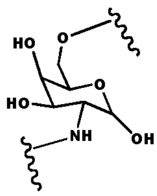 |

In some embodiments, the n+m of the RNA inhibitor described in the present invention is 2, 3, 4, 5 and 6, respectively. The positions for 5'MVIP and/or 3'MVIP conjugating include 5' end and/or 3' end of the antisense strand, 5' end and/or 3' end of the sense strand, 5' end of the antisense strand and 3' end of the sense strand, and 5' end of the sense strand and 3' end of the antisense strand. The antisense strand and the sense strand are base-paired to anneal, where n + m=2,3,4,5 and 6, as shown in Table 20.

Table 20 Positions of 5'MVIP and 3'MVIP conjugated to sense strand and/or antisense strand

| Carrier structure | | Carrier structure | | n+ m | Position of carrier structure conjugated to sense strand and/or antisense strand (S:AS) |
|---|---|---|---|---|---|
| 5'MVIP09 | n=2 | 3'MVIP09 | m=2 | 4 | n:m |
| 5'MVIP01 | n=1 | 3' MVIP01 | m=1 | 2 | n:m |
| 5'MVIP21 | n=1 | 3' MVIP01 | m=1 | 2 | n:m |
| 5'MVIP01 | n=1 | 3' MVIP07 | m=1 | 2 | n:m |
| 5'MVIP01/3'MVIP0 1 | n=1/m=1 | / | / | 2 | nm:/ |
| 3'MVIP01 | m=1 | 5'MVIP01 | n=1 | 2 | m:n |
| 3' MVIP07 | m=1 | 5'MVIP21 | n=1 | 2 | m:n |
| 5'MVIP01 | n=1 | 3'MVIP09 | m=2 | 3 | n:m |

(continued)

| Carrier structure | | Carrier structure | | n+ m | Position of carrier structure conjugated to sense strand and/or antisense strand (S:AS) |
|---|---|---|---|---|---|
| 5' MVIP08 | n=1 | 3'MVIP15 | m=2 | 3 | n:m |
| 5'MVIP09 | n=2 | 3'MVIP01 | m=1 | 3 | n:m |
| 5' MVIP19 | n=2 | 3' MVIP07 | m=1 | 3 | n:m |
| 5'MVIP01/3'MVIP0 9 | n=1/m=2 | / | / | 3 | nm:/ |
| 5'MVIP09/3'MVIP0 1 | n=2/m=1 | / | / | 3 | nm:/ |
| 3'MVIP09 | m=2 | 5'MVIP01 | n=1 | 3 | m:n |
| 3'MVIP11 | m=2 | 5'MVIP06 | n=1 | 3 | m:n |
| 3'MVIP01 | m=1 | 5' MVIP20 | n=2 | 3 | m:n |
| 3' MVIP07 | m=1 | 5' MVIP19 | n=2 | 3 | m:n |
| 5'MVIP01 | n=1 | 3' MVIP17 | m=3 | 4 | n:m |
| 5' MVIP21 | n=1 | 3' MVIP17 | m=3 | 4 | n:m |
| 5'MVIP17 | n=3 | 3' MVIP01 | m=1 | 4 | n:m |
| 5' MVIP22 | n=3 | 3' MVIP07 | m=1 | 4 | n:m |
| 5' MVIP20 | n=2 | 3' MVIP10 | m=2 | 4 | n:m |
| 5' MVIP19 | n=2 | 3'MVIP15 | m=2 | 4 | n:m |
| 5' MVIP10 | n=2 | 3'MVIP12 | m=2 | 4 | n:m |
| 5'MVIP09/3'MVIP0 9 | n=2/m=2 | / | / | 4 | nm:/ |
| 3'MVIP09 | m=2 | 5' MVIP09 | n=2 | 4 | m:n |
| 3'MVIP10 | m=2 | 5' MVIP20 | n=2 | 4 | m:n |
| 3'MVIP01 | m=1 | 5' MVIP17 | n=3 | 4 | m:n |
| 3'MVIP02 | m=1 | 5' MVIP18 | n=3 | 4 | m:n |
| 3'MVIP17 | m=3 | 5' MVIP01 | n=1 | 4 | m:n |
| 3'MVIP18 | m=3 | 5' MVIP21 | n=1 | 4 | m:n |
| 5'MVIP09 | n=2 | 3' MVIP17 | m=3 | 5 | n:m |
| 5'MVIP20 | n=2 | 3' MVIP19 | m=3 | 5 | n:m |
| 5'MVIP17 | n=3 | 3'MVIP09 | m=2 | 5 | n:m |
| 5'MVIP22 | n=3 | 3'MVIP11 | m=2 | 5 | n:m |
| 5'MVIP09/3'MVIP1 7 | n=2/m=3 | / | / | 5 | nm:/ |
| 5'MVIP17/3'MVIP0 9 | n=3/m=2 | / | / | 5 | nm:/ |
| 3'MVIP17 | m=3 | 5' MVIP20 | n=2 | 5 | m:n |
| 3'MVIP19 | m=3 | 5' MVIP16 | n=2 | 5 | m:n |
| 3'MVIP09 | m=2 | 5' MVIP17 | n=3 | 5 | m:n |
| 3' MVIP15 | m=2 | 5' MVIP17 | n=3 | 5 | m:n |
| 5'MVIP17 | n=3 | 3' MVIP17 | m=3 | 6 | n:m |
| 5' MVIP22 | n=3 | 3' MVIP19 | m=3 | 6 | n:m |
| 5'MVIP17/3'MVIP1 7 | n=3/m=3 | / | | 6 | nm:/ |
| 3'MVIP17 | m=3 | 5' MVIP17 | n=3 | 6 | m:n |
| 3'MVIP18 | m=3 | 5' MVIP22 | n=3 | 6 | m:n |

In some embodiments, n and m are each independently any integer of 0-4, each independently preferably an integer of 1-3, and n+m= an integer of 2-6, preferably n+m=2, 3 or 4, more preferably 4.

In some embodiments, the sense strand and antisense strand conjugated to 5'MVIP and/or 3'MVIP are selected from the following Table 21.

Table 21 Sense strand and antisense strand conjugated to 5'MVIP and/or 3'MVIP

| sense strand/antisense strand code | sense strand/antisense strand sequence 5'→3' |
|---|---|
| Ky-12AS62 | UsfGsAfGGfUCfUCAGGCfAGfCCfACsfGsG-3'MVIP09 |
| Ky-12AS63 | UsfGsAfGGfUCfUCAGGCfAGfCCfACsfGsG-3'MVIP01 |
| Ky-12AS64 | UsfGsAfGGfUCfUCAGGCfAGfCCfACsfGsG-3'MVIP17 |
| Ky-12AS65 | UsfGsGfAUfAGfGCAGGUfGGfACfUUsfGsG-3'MVIP09 |
| Ky-12AS66 | UsfGsGfAUfAGfGCAGGUfGGfACfUUsfGsG-3'MVIP01 |
| Ky-12AS67 | UsfGsGfAUfAGfGCAGGUfGGfACfUUsfGsG-3'MVIP17 |
| Ky-12AS68 | CsfAsGfGAfUGfGAUAGGfCAfGGfUGsfGsA-3'MVIP09 |
| Ky-12AS69 | CsfAsGfGAfUGfGAUAGGfCAfGGfUGsfGsA-3'MVIP01 |
| Ky-12AS70 | CsfAsGfGAfUGfGAUAGGfCAfGGfUGsfGsA-3'MVIP17 |
| Ky-12AS71 | GsfCsAfCUfGAfGAAUACfUGfUCfCCsfUsU-3'MVIP09 |
| Ky-12AS72 | GsfCsAfCUfGAfGAAUACfUGfUCfCCsfUsU-3'MVIP01 |
| Ky-12AS73 | GsfCsAfCUfGAfGAAUACfUGfUCfCCsfUsU-3'MVIP17 |
| Ky-12AS74 | GsfAsGfCAfCUfGAGAAUfACfUGfUCsfCsC-3'MVIP09 |
| Ky-12AS75 | GsfAsGfCAfCUfGAGAAUfACfUGfUCsfCsC-3'MVIP01 |
| Ky-12AS76 | GsfAsGfCAfCUfGAGAAUfACfUGfUCsfCsC-3'MVIP17 |
| Ky-12AS77 | UsfGsAfGGfUCfUCfAGGCfAGfCCfACsGsG-3'MVIP09 |
| Ky-12AS78 | UsfGsAfGGfUCfUCfAGGCfAGfCCfACsGsG-3'MVIP01 |
| Ky-12AS79 | UsfGsAfGGfUCfUCfAGGCfAGfCCfACsGsG-3'MVIP17 |
| Ky-12AS80 | UsfGsGfAUfAGfGCfAGGUfGGfACfUUsGsG-3'MVIP09 |
| Ky-12AS81 | UsfGsGfAUfAGfGCfAGGUfGGfACfUUsGsG-3'MVIP01 |
| Ky-12AS82 | UsfGsGfAUfAGfGCfAGGUfGGfACfUUsGsG-3'MVIP17 |
| Ky-12AS83 | CsfAsGfGAfUGfGAfUAGGfCAfGGfUGsGsA-3'MVIP09 |
| Ky-12AS84 | CsfAsGfGAfUGfGAfUAGGfCAfGGfUGsGsA-3'MVIP01 |
| Ky-12AS85 | CsfAsGfGAfUGfGAfUAGGfCAfGGfUGsGsA-3'MVIP17 |
| Ky-12AS86 | GsfCsAfCUfGAfGAfAUACfUGfUCfCCsUsU-3'MVIP09 |
| Ky-12AS87 | GsfCsAfCUfGAfGAfAUACfUGfUCfCCsUsU-3'MVIP01 |
| Ky-12AS88 | GsfCsAfCUfGAfGAfAUACfUGfUCfCCsUsU-3'MVIP17 |
| Ky-12AS89 | GsfAsGfCAfCUfGAfGAAUfACfUGfUCsCsC-3'MVIP09 |
| Ky-12AS90 | GsfAsGfCAfCUfGAfGAAUfACfUGfUCsCsC-3'MVIP01 |
| Ky-12AS91 | GsfAsGfCAfCUfGAfGAAUfACfUGfUCsCsC-3'MVIP17 |
| Ky-12AS01 | AsfCsAfCUfGAfGAAUACfUGfUCfGCsfUsC-3'MVIP09 |
| Ky-12AS03 | AsfCsAfCUfGAfGAAUACfUGfUCfGCsfUsC-3'MVIP01 |
| Ky-12AS04 | AsfCsAfCUfGAfGAAUACfUGfUCfGCsfUsC-3'MVIP17 |
| Ky-12AS92 | AsfCsAfCUfGAfGAfAUACfUGfUCfGCsUsC-3'MVIP09 |
| Ky-12AS93 | AsfCsAfCUfGAfGAfAUACfUGfUCfGCsUsC-3'MVIP01 |
| Ky-12AS94 | AsfCsAfCUfGAfGAfAUACfUGfUCfGCsUsC-3'MVIP17 |

(continued)

| sense strand/antisense strand code | sense strand/antisense strand sequence 5'→3' |
|---|---|
| Ky-12AS95 | AsfCsAfCUfGAfGAAUACfUGfUCfGCfUCsUsU-3'MVIP09 |
| Ky-12AS96 | AsfCsAfCUfGAfGAAUACfUGfUCfGCfUCsUsU-3'MVIP01 |
| Ky-12AS97 | AsfCsAfCUfGAfGAAUACfUGfUCfGCfUCsUsU-3'MVIP17 |
| Ky-12AS98 | UsfGsAfGGfUCfUCAGGCfAGfCCfACfGG-3'MVIP09 |
| Ky-12AS99 | UsfGsGfAUfAGfGCAGGUfGGfACfUUfGG-3'MVIP09 |
| Ky-12AS100 | CsfAsGfGAfUGfGAUAGGfCAfGGfUGfGA-3'MVIP09 |
| Ky-12AS101 | GsfCsAfCUfGAfGAAUACfUGfUCfCCfUU-3'MVIP09 |
| Ky-12AS102 | GsfAsGfCAfCUfGAGAAUfACfUGfUCfCC-3'MVIP09 |
| Ky-12AS103 | UsfGsAfGGfUCfUCfAGGCfAGfCCfACGG-3'MVIP09 |
| Ky-12AS104 | UsfGsGfAUfAGfGCfAGGUfGGfACfUUGG-3'MVIP09 |
| Ky-12AS105 | CsfAsGfGAfUGfGAfUAGGfCAfGGfUGGA-3'MVIP09 |
| Ky-12AS106 | GsfCsAfCUfGAfGAfAUACfUGfUCfCCUU-3'MVIP09 |
| Ky-12AS107 | GsfAsGfCAfCUfGAfGAAUfACfUGfUCCC-3'MVIP09 |
| Ky-12AS108 | AsfCsAfCUfGAfGAAUACfUGfUCfGCfUC-3'MVIP09 |
| Ky-12AS109 | AsfCsAfCUfGAfGAAUACfUGfUCfGCfUC-3'MVIP17 |
| Ky-12AS110 | AsfCsAfCUfGAfGAfAUACfUGfUCfGCUC-3'MVIP09 |
| Ky-12AS111 | AsfCsAfCUfGAfGAAUACfUGfUCfGCfUCUU-3'MVIP09 |
| Ky-12AS112 | UsfGsAfGGfUCfUCAGGCfAGfCCfACfGsG-3'MVIP09 |
| Ky-12AS113 | UsfGsGfAUfAGfGCAGGUfGGfACfUUfGsG-3'MVIP09 |
| Ky-12AS114 | CsfAsGfGAfUGfGAUAGGfCAfGGfUGfGsA-3'MVIP09 |
| Ky-12AS115 | GsfCsAfCUfGAfGAAUACfUGfUCfCCfUsU-3'MVIP09 |
| Ky-12AS116 | GsfAsGfCAfCUfGAGAAUfACfUGfUCfCsC-3'MVIP09 |
| Ky-12AS117 | UsfGsAfGGfUCfUCfAGGCfAGfCCfACGsG-3'MVIP09 |
| Ky-12AS118 | UsfGsGfAUfAGfGCfAGGUfGGfACfUUGsG-3'MVIP09 |
| Ky-12AS119 | CsfAsGfGAfUGfGAfUAGGfCAfGGfUGGsA-3'MVIP09 |
| Ky-12AS120 | GsfCsAfCUfGAfGAfAUACfUGfUCfCCUsU-3'MVIP09 |
| Ky-12AS121 | GsfAsGfCAfCUfGAfGAAUfACfUGfUCCsC-3'MVIP09 |
| Ky-12AS122 | AsfCsAfCUfGAfGAAUACfUGfUCfGCfUsC-3'MVIP09 |
| Ky-12AS123 | AsfCsAfCUfGAfGAAUACfUGfUCfGCfUsC-3'MVIP17 |
| Ky-12AS124 | AsfCsAfCUfGAfGAfAUACfUGfUCfGCUsC-3'MVIP09 |
| Ky-12AS125 | AsfCsAfCUfGAfGAAUACfUGfUCfGCfUCUsU-3'MVIP09 |

(continued)

| sense strand/antisense strand code | sense strand/antisense strand sequence 5'→3' |
|---|---|
| Ky-12AS128 | GsfCsAfCUfGAfGAAUACfUGfUCfCCfUUsUsU-3'MVIP09 |
| Ky-12AS129 | GsfCsAfCUfGAfGAAUACfUGfUCfCCfUUsUsU-3'MVIP17 |
| Ky-12S60 | 5'MVIP09-GsUsGGCUGCfCfUfGAGACCUCAsAsU |
| Ky-12S61 | 5'MVIP01-GsUsGGCUGCfCfUfGAGACCUCAsAsU |
| Ky-12S62 | 5'MVIP17-GsUsGGCUGCfCfUfGAGACCUCAsAsU |
| Ky-12S63 | GsUsGGCUGCfCfUfGAGACCUCAsAsU-3'MVIP17 |
| Ky-12S64 | 5'MVIP01-GsUsGGCUGCfCfUfGAGACCUCAsAsU-3'MVIP09 |
| Ky-12S65 | 5'MVIP09-GsUsGGCUGCfCfUfGAGACCUCAsAsU-3'MVIP01 |
| Ky-12S66 | 5'MVIP09-AsAsGUCCACfCfUfGCCUAUCCAsUsC |
| Ky-12S67 | 5'MVIP01-AsAsGUCCACfCfUfGCCUAUCCAsUsC |
| Ky-12S68 | 5'MVIP17-AsAsGUCCACfCfUfGCCUAUCCAsUsC |
| Ky-12S69 | AsAsGUCCACfCfUfGCCUAUCCAsUsC-3'MVIP17 |
| Ky-12S70 | 5'MVIP01-AsAsGUCCACfCfUfGCCUAUCCAsUsC-3'MVIP09 |
| Ky-12S71 | 5'MVIP09-AsAsGUCCACfCfUfGCCUAUCCAsUsC-3'MVIP01 |
| Ky-12S72 | 5'MVIP09-CsAsCCUGCCfUfAfUCCAUCCUGsAsA |
| Ky-12S73 | 5'MVIP01-CsAsCCUGCCfUfAfUCCAUCCUGsAsA |
| Ky-12S74 | 5'MVIP17-CsAsCCUGCCfUfAfUCCAUCCUGsAsA |
| Ky-12S75 | CsAsCCUGCCfUfAfUCCAUCCUGsAsA-3'MVIP17 |
| Ky-12S76 | 5'MVIP01-CsAsCCUGCCfUfAfUCCAUCCUGsAsA-3'MVIP09 |
| Ky-12S77 | 5'MVIP09-CsAsCCUGCCfUfAfUCCAUCCUGsAsA-3'MVIP01 |
| Ky-12S78 | 5'MVIP09-GsGsGACAGUfAfUfUCUCAGUGCsUsU |
| Ky-12S79 | 5'MVIP01-GsGsGACAGUfAfUfUCUCAGUGCsUsU |
| Ky-12S80 | 5'MVIP17-GsGsGACAGUfAfUfUCUCAGUGCsUsU |
| Ky-12S81 | GsGsGACAGUfAfUfUCUCAGUGCsUsU-3'MVIP17 |

(continued)

| sense strand/antisense strand code | sense strand/antisense strand sequence 5'→3' |
|---|---|
| Ky-12S82 | 5'MVIP01-GsGsGACAGUfAfUfUCUCAGUGCsUsU-3'MVIP09 |
| Ky-12S83 | 5'MVIP09-GsGsGACAGUfAfUfUCUCAGUGCsUsU-3'MVIP01 |
| Ky-12S84 | 5'MVIP09-GsAsCAGUAUfUfCfUCAGUGCUCsUsU |
| Ky-12S85 | 5'MVIP01-GsAsCAGUAUfUfCfUCAGUGCUCsUsU |
| Ky-12S86 | 5'MVIP17-GsAsCAGUAUfUfCfUCAGUGCUCsUsU |
| Ky-12S87 | GsAsCAGUAUfUfCfUCAGUGCUCsUsU-3'MVIP17 |
| Ky-12S88 | 5'MVIP01-GsAsCAGUAUfUfCfUCAGUGCUCsUsU-3'MVIP09 |
| Ky-12S89 | 5'MVIP09-GsAsCAGUAUfUfCfUCAGUGCUCsUsU-3'MVIP01 |
| Ky-12S90 | 5'MVIP01-GsAsCAGUAUfUfCfUCAGUGCUCsUsU-3'MVIP01 |
| Ky-12S91 | 5'MVIP09-GsUsGGCUfGCfCfUfGAGACCUCAsAsU |
| Ky-12S92 | 5'MVIP01-GsUsGGCUfGCfCfUfGAGACCUCAsAsU |
| Ky-12S93 | 5'MVIP17-GsUsGGCUfGCfCfUfGAGACCUCAsAsU |
| Ky-12S94 | GsUsGGCUfGCfCfUfGAGACCUCAsAsU-3'MVIP17 |
| Ky-12S95 | 5'MVIP01-GsUsGGCUfGCfCfUfGAGACCUCAsAsU-3'MVIP09 |
| Ky-12S96 | 5'MVIP09-GsUsGGCUfGCfCfUfGAGACCUCAsAsU-3'MVIP01 |
| Ky-12S97 | 5'MVIP09-AsAsGUCCfACfCfUfGCCUAUCCAsUsC |
| Ky-12S98 | 5'MVIP01-AsAsGUCCfACfCfUfGCCUAUCCAsUsC |
| Ky-12S99 | 5'MVIP17-AsAsGUCCfACfCfUfGCCUAUCCAsUsC |
| Ky-12S100 | AsAsGUCCfACfCfUfGCCUAUCCAsUsC-3'MVIP17 |
| Ky-12S101 | 5'MVIP01-AsAsGUCCfACfCfUfGCCUAUCCAsUsC-3'MVIP09 |
| Ky-12S102 | 5'MVIP09-AsAsGUCCfACfCfUfGCCUAUCCAsUsC-3'MVIP01 |
| Ky-12S103 | 5'MVIP01-AsAsGUCCfACfCfUfGCCUAUCCAsUsC-3'MVIP01 |

(continued)

| sense strand/antisense strand code | sense strand/antisense strand sequence 5'→3' |
|---|---|
| Ky-12S104 | 5'MVIP09-CsAsCCUGfCCfUfAfUCCAUCCUGsAsA |
| Ky-12S105 | 5'MVIP01-CsAsCCUGfCCfUfAfUCCAUCCUGsAsA |
| Ky-12S106 | 5'MVIP17-CsAsCCUGfCCfUfAfUCCAUCCUGsAsA |
| Ky-12S107 | CsAsCCUGfCCfUfAfUCCAUCCUGsAsA-3'MVIP17 |
| Ky-12S108 | 5'MVIP01-CsAsCCUGfCCfUfAfUCCAUCCUGsAsA-3'MVIP09 |
| Ky-12S109 | 5'MVIP09-CsAsCCUGfCCfUfAfUCCAUCCUGsAsA-3'MVIP01 |
| Ky-12S110 | 5'MVIP09-GsGsGACAfGUfAfUfUCUCAGUGCsUsU |
| Ky-12S111 | 5'MVIP01-GsGsGACAfGUfAfUfUCUCAGUGCsUsU |
| Ky-12S112 | 5'MVIP17-GsGsGACAfGUfAfUfUCUCAGUGCsUsU |
| Ky-12S113 | GsGsGACAfGUfAfUfUCUCAGUGCsUsU-3'MVIP17 |
| Ky-12S114 | 5'MVIP01-GsGsGACAfGUfAfUfUCUCAGUGCsUsU-3'MVIP09 |
| Ky-12S115 | 5'MVIP09-GsGsGACAfGUfAfUfUCUCAGUGCsUsU-3'MVIP01 |
| Ky-12S116 | 5'MVIP09-GsAsCAGUfAUfUfCfUCAGUGCUCsUsU |
| Ky-12S117 | 5'MVIP01-GsAsCAGUfAUfUfCfUCAGUGCUCsUsU |
| Ky-12S118 | 5'MVIP17-GsAsCAGUfAUfUfCfUCAGUGCUCsUsU |
| Ky-12S119 | GsAsCAGUfAUfUfCfUCAGUGCUCsUsU-3'MVIP17 |
| Ky-12S120 | 5'MVIP01-GsAsCAGUfAUfUfCfUCAGUGCUCsUsU-3'MVIP09 |
| Ky-12S121 | 5'MVIP09-GsAsCAGUfAUfUfCfUCAGUGCUCsUsU-3'MVIP01 |
| Ky-12S01 | 5'MVIP09-GsAsGCGACAfGfUfAUUCUCAGUsGsU |
| Ky-12S06 | 5'MVIP01-GsAsGCGACAfGfUfAUUCUCAGUsGsU |
| Ky-12S05 | 5'MVIP17-GsAsGCGACAfGfUfAUUCUCAGUsGsU |
| Ky-12S01 | GsAsGCGACAfGfUfAUUCUCAGUsGsU-3'MVIP17 |
| Ky-12S12 | 5'MVIP01-GsAsGCGACAfGfUfAUUCUCAGUsGsU-3'MVIP09 |
| Ky-12S13 | 5'MVIP09-GsAsGCGACAfGfUfAUUCUCAGUsGsU-3'MVIP01 |

(continued)

| sense strand/antisense strand code | sense strand/antisense strand sequence 5'→3' |
|---|---|
| Ky-12S07 | 5'MVIP01-GsAsGCGACAfGfUfAUUCUCAGUsGsU-3'MVIP01 |
| Ky-12S122 | 5'MVIP09-GsAsGCGAfCAfGfUfAUUCUCAGUsGsU |
| Ky-12S123 | 5'MVIP01-GsAsGCGAfCAfGfUfAUUCUCAGUsGsU |
| Ky-12S124 | 5'MVIP17-GsAsGCGAfCAfGfUfAUUCUCAGUsGsU |
| Ky-12S125 | GsAsGCGAfCAfGfUfAUUCUCAGUsGsU-3'MVIP17 |
| Ky-12S126 | 5'MVIP01-GsAsGCGAfCAfGfUfAUUCUCAGUsGsU-3'MVIP09 |
| Ky-12S127 | 5'MVIP09-GsAsGCGAfCAfGfUfAUUCUCAGUsGsU-3'MVIP01 |
| Ky-12S128 | 5'MVIP01-GsAsGCGAfCAfGfUfAUUCUCAGUsGsU-3'MVIP01 |
| Ky-12S129 | 5'MVIP09-GsAsGCGACAfGfUfAUUCUCAsGsU |
| Ky-12S130 | 5'MVIP01-GsAsGCGACAfGfUfAUUCUCAsGsU |
| Ky-12S131 | 5'MVIP17-GsAsGCGACAfGfUfAUUCUCAsGsU |
| Ky-12S132 | GsAsGCGACAfGfUfAUUCUCAsGsU-3'MVIP17 |
| Ky-12S133 | 5'MVIP01-GsAsGCGACAfGfUfAUUCUCAsGsU-3'MVIP09 |
| Ky-12S134 | 5'MVIP09-GsAsGCGACAfGfUfAUUCUCAsGsU-3'MVIP01 |
| Ky-12S135 | 5'MVIP01-GsAsGCGACAfGfUfAUUCUCAsGsU-3'MVIP01 |
| Ky-12S136 | 5'MVIP09-GsUGGCUGCfCfUfGAGACCUCAsAsU |
| Ky-12S137 | 5'MVIP01-GsUGGCUGCfCfUfGAGACCUCAsAsU |
| Ky-12S138 | 5'MVIP17-GsUGGCUGCfCfUfGAGACCUCAsAsU |
| Ky-12S139 | GsUsGGCUGCfCfUfGAGACCUCAAsU-3'MVIP17 |
| Ky-12S140 | 5'MVIP01-GsUGGCUGCfCfUfGAGACCUCAsAsU-3'MVIP09 |
| Ky-12S141 | 5'MVIP09-GsUGGCUGCfCfUfGAGACCUCAsAsU-3'MVIP01 |
| Ky-12S142 | 5'MVIP09-AsAGUCCACfCfUfGCCUAUCCAsUsC |
| Ky-12S143 | 5'MVIP01-AsAGUCCACfCfUfGCCUAUCCAsUsC |
| Ky-12S144 | 5'MVIP17-AsAGUCCACfCfUfGCCUAUCCAsUsC |

(continued)

| sense strand/antisense strand code | sense strand/antisense strand sequence 5'→3' |
|---|---|
| Ky-12S145 | AsAsGUCCACfCfUfGCCUAUCCAUsC-3'MVIP17 |
| Ky-12S146 | 5'MVIP01-AsAGUCCACfCfUfGCCUAUCCAsUsC-3'MVIP09 |
| Ky-12S147 | 5'MVIP09-AsAGUCCACfCfUfGCCUAUCCAsUsC-3'MVIP01 |
| Ky-12S148 | 5'MVIP09-CsACCUGCCfUfAfUCCAUCCUGsAsA |
| Ky-12S149 | 5'MVIP01-CsACCUGCCfUfAfUCCAUCCUGsAsA |
| Ky-12S150 | 5'MVIP17-CsACCUGCCfUfAfUCCAUCCUGsAsA |
| Ky-12S151 | CsAsCCUGCCfUfAfUCCAUCCUGAsA-3'MVIP17 |
| Ky-12S152 | 5'MVIP01-CsACCUGCCfUfAfUCCAUCCUGsAsA-3'MVIP09 |
| Ky-12S153 | 5'MVIP09-CsACCUGCCfUfAfUCCAUCCUGsAsA-3'MVIP01 |
| Ky-12S154 | 5'MVIP09-GsGGACAGUfAfUfUCUCAGUGCsUsU |
| Ky-12S155 | 5'MVIP01-GsGGACAGUfAfUfUCUCAGUGCsUsU |
| Ky-12S156 | 5'MVIP17-GsGGACAGUfAfUfUCUCAGUGCsUsU |
| Ky-12S157 | GsGsGACAGUfAfUfUCUCAGUGCUsU-3'MVIP17 |
| Ky-12S158 | 5'MVIP01-GsGGACAGUfAfUfUCUCAGUGCsUsU-3'MVIP09 |
| Ky-12S159 | 5'MVIP09-GsGGACAGUfAfUfUCUCAGUGCsUsU-3'MVIP01 |
| Ky-12S160 | 5'MVIP09-GsACAGUAUfUfCfUCAGUGCUCsUsU |
| Ky-12S161 | 5'MVIP01-GsACAGUAUfUfCfUCAGUGCUCsUsU |
| Ky-12S162 | 5'MVIP17-GsACAGUAUfUfCfUCAGUGCUCsUsU |
| Ky-12S163 | GsAsCAGUAUfUfCfUCAGUGCUCUsU-3'MVIP17 |
| Ky-12S164 | 5'MVIP01-GsACAGUAUfUfCfUCAGUGCUCsUsU-3'MVIP09 |
| Ky-12S165 | 5'MVIP09-GsACAGUAUfUfCfUCAGUGCUCsUsU-3'MVIP01 |
| Ky-12S166 | 5'MVIP09-GsUGGCUfGCfCfUfGAGACCUCAsAsU |
| Ky-12S167 | 5'MVIP09-AsAGUCCfACfCfUfGCCUAUCCAsUsC |
| Ky-12S168 | 5'MVIP01-AsAGUCCfACfCfUfGCCUAUCCAsUsC |
| Ky-12S169 | 5'MVIP17-AsAGUCCfACfCfUfGCCUAUCCAsUsC |
| Ky-12S170 | AsAsGUCCfACfCfUfGCCUAUCCAUsC-3'MVIP17 |

(continued)

| sense strand/antisense strand code | sense strand/antisense strand sequence 5'→3' |
|---|---|
| Ky-12S171 | 5'MVIP01-AsAGUCCfACfCfUfGCCUAUCCAsUsC-3'MVIP09 |
| Ky-12S172 | 5'MVIP09-AsAGUCCfACfCfUfGCCUAUCCAsUsC-3'MVIP01 |
| Ky-12S173 | 5'MVIP01-AsAGUCCfACfCfUfGCCUAUCCAUsCsU-3'MVIP01 |
| Ky-12S174 | 5'MVIP09-AsAGUCCfACfCfUfGCCUAUCCAUsCsU |
| Ky-12S175 | 5'MVIP01-AsAGUCCfACfCfUfGCCUAUCCAUsCsU |
| Ky-12S176 | 5'MVIP17-AsAGUCCfACfCfUfGCCUAUCCAUsCsU |
| Ky-12S177 | AsAsGUCCfACfCfUfGCCUAUCCAUCsU-3'MVIP17 |
| Ky-12S178 | 5'MVIP01-AsAGUCCfACfCfUfGCCUAUCCAUsCsU-3'MVIP09 |
| Ky-12S179 | 5'MVIP09-AsAGUCCfACfCfUfGCCUAUCCAUsCsU-3'MVIP01 |
| Ky-12S180 | 5'MVIP09-CsACCUGfCCfUfAfUCCAUCCUGsAsA |
| Ky-12S181 | 5'MVIP01-CsACCUGfCCfUfAfUCCAUCCUGsAsA |
| Ky-12S182 | 5'MVIP17-CsACCUGfCCfUfAfUCCAUCCUGsAsA |
| Ky-12S183 | CsAsCCUGfCCfUfAfUCCAUCCUGAsA-3'MVIP17 |
| Ky-12S184 | 5'MVIP01-CsACCUGfCCfUfAfUCCAUCCUGAsA-3'MVIP09 |
| Ky-12S185 | 5'MVIP09-CsACCUGfCCfUfAfUCCAUCCUGAsA-3'MVIP01 |
| Ky-12S186 | 5'MVIP09-GsGGACAfGUfAfUfUCUCAGUGCsUsU |
| Ky-12S187 | 5'MVIP01-GsGGACAfGUfAfUfUCUCAGUGCsUsU |
| Ky-12S188 | 5'MVIP17-GsGGACAfGUfAfUfUCUCAGUGCsUsU |
| Ky-12S189 | GGGACAfGUfAfUfUCUCAGUGCsUsU-3'MVIP17 |
| Ky-12S190 | 5'MVIP01-GsGGACAfGUfAfUfUCUCAGUGCsUsU-3'MVIP09 |
| Ky-12S191 | 5'MVIP09-GsGGACAfGUfAfUfUCUCAGUGCsUsU-3'MVIP01 |
| Ky-12S192 | 5'MVIP09-GsACAGUfAUfUfCfUCAGUGCUCsUsU |
| Ky-12S193 | 5'MVIP01-GsACAGUfAUfUfCfUCAGUGCUCsUsU |
| Ky-12S194 | 5'MVIP17-GsACAGUfAUfUfCfUCAGUGCUCsUsU |

(continued)

| sense strand/antisense strand code | sense strand/antisense strand sequence 5'→3' |
|---|---|
| Ky-12S195 | GsAsCAGUfAUfUfCfUCAGUGCUCsUsU-3'MVIP17 |
| Ky-12S196 | 5'MVIP01-GsACAGUfAUfUfCfUCAGUGCUCsUsU-3'MVIP09 |
| Ky-12S197 | 5'MVIP09-GsACAGUfAUfUfCfUCAGUGCUCsUsU-3'MVIP01 |
| Ky-12S198 | 5'MVIP09-GsAGCGACAfGfUfAUUCUCAGUsGsU |
| Ky-12S199 | 5'MVIP01-GsAGCGACAfGfUfAUUCUCAGUsGsU |
| Ky-12S200 | 5'MVIP17-GsAGCGACAfGfUfAUUCUCAGUsGsU |
| Ky-12S201 | GsAsGCGACAfGfUfAUUCUCAGUGsU-3'MVIP17 |
| Ky-12S202 | 5'MVIP01-GsAGCGACAfGfUfAUUCUCAGUsGsU-3'MVIP09 |
| Ky-12S203 | 5'MVIP09-GsAGCGACAfGfUfAUUCUCAGUsGsU-3'MVIP01 |
| Ky-12S204 | 5'MVIP01-GsAGCGACAfGfUfAUUCUCAGUsGsU-3'MVIP01 |
| Ky-12S205 | 5'MVIP09-GsAGCGAfCAfGfUfAUUCUCAGUsGsU |
| Ky-12S206 | 5'MVIP01-GsAGCGAfCAfGfUfAUUCUCAGUsGsU |
| Ky-12S207 | 5'MVIP17-GsAGCGAfCAfGfUfAUUCUCAGUsGsU |
| Ky-12S208 | GsAsGCGAfCAfGfUfAUUCUCAGUGsU-3'MVIP17 |
| Ky-12S209 | 5'MVIP01-GsAGCGAfCAfGfUfAUUCUCAGUsGsU-3'MVIP09 |
| Ky-12S210 | 5'MVIP09-GsAGCGAfCAfGfUfAUUCUCAGUsGsU-3'MVIP01 |
| Ky-12S211 | 5'MVIP09-GsAGCGACAfGfUfAUUCUCAsGsU |
| Ky-12S212 | 5'MVIP01-GsAGCGACAfGfUfAUUCUCAsGsU |
| Ky-12S213 | 5'MVIP17-GsAGCGACAfGfUfAUUCUCAsGsU |
| Ky-12S214 | GsAsGCGACAfGfUfAUUCUCAGsU-3'MVIP17 |
| Ky-12S215 | 5'MVIP01-GsAGCGACAfGfUfAUUCUCAsGsU-3'MVIP09 |
| Ky-12S216 | 5'MVIP09-GsAGCGACAfGfUfAUUCUCAsGsU-3'MVIP01 |
| Ky-12S217 | 5'MVIP09-GsUsGGCUGCfCfUfGAGACCUCAsAsA |
| Ky-12S218 | 5'MVIP01-GsUsGGCUGCfCfUfGAGACCUCAsAsA |

(continued)

| sense strand/antisense strand code | sense strand/antisense strand sequence 5'→3' |
|---|---|
| Ky-12S219 | 5'MVIP17-GsUsGGCUGCfCfUfGAGACCUCAsAsA |
| Ky-12S220 | GsUsGGCUGCfCfUfGAGACCUCAsAsA-3'MVIP17 |
| Ky-12S221 | 5'MVIP01-GsUsGGCUGCfCfUfGAGACCUCAsAsA-3'MVIP09 |
| Ky-12S222 | 5'MVIP09-GsUsGGCUGCfCfUfGAGACCUCAsAsA-3'MVIP01 |
| Ky-12S223 | 5'MVIP09-AsAsGUCCACfCfUfGCCUAUCCAsUsA |
| Ky-12S224 | 5'MVIP01-AsAsGUCCACfCfUfGCCUAUCCAsUsA |
| Ky-12S225 | 5'MVIP17-AsAsGUCCACfCfUfGCCUAUCCAsUsA |
| Ky-12S226 | AsAsGUCCACfCfUfGCCUAUCCAsUsA-3'MVIP17 |
| Ky-12S227 | 5'MVIP01-AsAsGUCCACfCfUfGCCUAUCCAsUsA-3'MVIP09 |
| Ky-12S228 | 5'MVIP09-AsAsGUCCACfCfUfGCCUAUCCAsUsA-3'MVIP01 |
| Ky-12S229 | 5'MVIP09-CsGsCCUGCCfUfAfUCCAUCCUGsAsA |
| Ky-12S230 | 5'MVIP01-CsGsCCUGCCfUfAfUCCAUCCUGsAsA |
| Ky-12S231 | 5'MVIP17-CsGsCCUGCCfUfAfUCCAUCCUGsAsA |
| Ky-12S232 | CsGsCCUGCCfUfAfUCCAUCCUGsAsA-3'MVIP17 |
| Ky-12S233 | 5'MVIP01-CsGsCCUGCCfUfAfUCCAUCCUGsAsA-3'MVIP09 |
| Ky-12S234 | 5'MVIP09-CsGsCCUGCCfUfAfUCCAUCCUGsAsA-3'MVIP01 |
| Ky-12S235 | 5'MVIP09-GsGsGACAGUfAfUfUCUCAGUGCsUsA |
| Ky-12S236 | 5'MVIP01-GsGsGACAGUfAfUfUCUCAGUGCsUsA |
| Ky-12S237 | 5'MVIP17-GsGsGACAGUfAfUfUCUCAGUGCsUsA |
| Ky-12S238 | GsGsGACAGUfAfUfUCUCAGUGCsUsA-3'MVIP17 |
| Ky-12S239 | 5'MVIP01-GsGsGACAGUfAfUfUCUCAGUGCsUsA-3'MVIP09 |
| Ky-12S240 | 5'MVIP09-GsGsGACAGUfAfUfUCUCAGUGCsUsA-3'MVIP01 |
| Ky-12S241 | 5'MVIP09-GsAsCAGUAUfUfCfUCAGUGCUCsUsA |
| Ky-12S242 | 5'MVIP01-GsAsCAGUAUfUfCfUCAGUGCUCsUsA |
| Ky-12S243 | 5'MVIP17-GsAsCAGUAUfUfCfUCAGUGCUCsUsA |
| Ky-12S244 | GsAsCAGUAUfUfCfUCAGUGCUCsUsA-3'MVIP17 |

(continued)

| sense strand/antisense strand code | sense strand/antisense strand sequence 5'→3' |
|---|---|
| Ky-12S245 | 5'MVIP01-GsAsCAGUAUfUfCfUCAGUGCUCsUsA-3'MVIP09 |
| Ky-12S246 | 5'MVIP09-GsAsCAGUAUfUfCfUCAGUGCUCsUsA-3'MVIP01 |
| Ky-12S247 | 5'MVIP09-GsUsGGCUfGCfCfUfGAGACCUCAsAsA |
| Ky-12S248 | 5'MVIP01-GsUsGGCUfGCfCfUfGAGACCUCAsAsA |
| Ky-12S249 | 5'MVIP17-GsUsGGCUfGCfCfUfGAGACCUCAsAsA |
| Ky-12S250 | GsUsGGCUfGCfCfUfGAGACCUCAsAsA-3'MVIP17 |
| Ky-12S251 | 5'MVIP01-GsUsGGCUfGCfCfUfGAGACCUCAsAsA-3'MVIP09 |
| Ky-12S252 | 5'MVIP09-GsUsGGCUfGCfCfUfGAGACCUCAsAsA-3'MVIP01 |
| Ky-12S253 | 5'MVIP09-AsAsGUCCfACfCfUfGCCUAUCCAsUsA |
| Ky-12S254 | 5'MVIP01-AsAsGUCCfACfCfUfGCCUAUCCAsUsA |
| Ky-12S255 | 5'MVIP17-AsAsGUCCfACfCfUfGCCUAUCCAsUsA |
| Ky-12S256 | AsAsGUCCfACfCfUfGCCUAUCCAsUsA-3'MVIP17 |
| Ky-12S257 | 5'MVIP01-AsAsGUCCfACfCfUfGCCUAUCCAsUsA-3'MVIP09 |
| Ky-12S258 | 5'MVIP09-AsAsGUCCfACfCfUfGCCUAUCCAsUsA-3'MVIP01 |
| Ky-12S259 | 5'MVIP09-CsGsCCUGfCCfUfAfUCCAUCCUGsAsA |
| Ky-12S260 | 5'MVIP01-CsGsCCUGfCCfUfAfUCCAUCCUGsAsA |
| Ky-12S261 | 5'MVIP17-CsGsCCUGfCCfUfAfUCCAUCCUGsAsA |
| Ky-12S262 | CsGsCCUGfCCfUfAfUCCAUCCUGsAsA-3'MVIP17 |
| Ky-12S263 | 5'MVIP01-CsGsCCUGfCCfUfAfUCCAUCCUGsAsA-3'MVIP09 |
| Ky-12S264 | 5'MVIP09-CsGsCCUGfCCfUfAfUCCAUCCUGsAsA-3'MVIP01 |
| Ky-12S265 | 5'MVIP09-GsGsGACAfGUfAfUfUCUCAGUGCsUsA |
| Ky-12S266 | 5'MVIP01-GsGsGACAfGUfAfUfUCUCAGUGCsUsA |
| Ky-12S267 | 5'MVIP17-GsGsGACAfGUfAfUfUCUCAGUGCsUsA |
| Ky-12S268 | GsGsGACAfGUfAfUfUCUCAGUGCsUsA-3'MVIP17 |

(continued)

| sense strand/antisense strand code | sense strand/antisense strand sequence 5'→3' |
|---|---|
| Ky-12S269 | 5'MVIP01-GsGsGACAfGUfAfUfUCUCAGUGCsUsA-3'MVIP09 |
| Ky-12S270 | 5'MVIP09-GsGsGACAfGUfAfUfUCUCAGUGCsUsA-3'MVIP01 |
| Ky-12S271 | 5'MVIP09-GsAsCAGUfAUfUfCfUCAGUGCUCsUsA |
| Ky-12S272 | 5'MVIP01-GsAsCAGUfAUfUfCfUCAGUGCUCsUsA |
| Ky-12S273 | 5'MVIP17-GsAsCAGUfAUfUfCfUCAGUGCUCsUsA |
| Ky-12S274 | GsAsCAGUfAUfUfCfUCAGUGCUCsUsA-3'MVIP17 |
| Ky-12S275 | 5'MVIP01-GsAsCAGUfAUfUfCfUCAGUGCUCsUsA-3'MVIP09 |
| Ky-12S276 | 5'MVIP09-GsAsCAGUfAUfUfCfUCAGUGCUCsUsA-3'MVIP01 |
| Ky-12S277 | 5'MVIP09-AsAsGCGACAfGfUfAUUCUCAGUsGsA |
| Ky-12S278 | 5'MVIP01-AsAsGCGACAfGfUfAUUCUCAGUsGsA |
| Ky-12S279 | 5'MVIP17-AsAsGCGACAfGfUfAUUCUCAGUsGsA |
| Ky-12S280 | AsAsGCGACAfGfUfAUUCUCAGUsGsA-3'MVIP17 |
| Ky-12S281 | 5'MVIP01-AsAsGCGACAfGfUfAUUCUCAGUsGsA-3'MVIP09 |
| Ky-12S282 | 5'MVIP09-AsAsGCGACAfGfUfAUUCUCAGUsGsA-3'MVIP01 |
| Ky-12S283 | 5'MVIP09-AsAsGCGAfCAfGfUfAUUCUCAGUsGsA |
| Ky-12S284 | 5'MVIP01-AsAsGCGAfCAfGfUfAUUCUCAGUsGsA |
| Ky-12S285 | 5'MVIP17-AsAsGCGAfCAfGfUfAUUCUCAGUsGsA |
| Ky-12S286 | AsAsGCGAfCAfGfUfAUUCUCAGUsGsA-3'MVIP17 |
| Ky-12S287 | 5'MVIP01-AsAsGCGAfCAfGfUfAUUCUCAGUsGsA-3'MVIP09 |
| Ky-12S288 | 5'MVIP09-AsAsGCGAfCAfGfUfAUUCUCAGUsGsA-3'MVIP01 |
| Ky-12S289 | 5'MVIP09-AsAsGCGACAfGfUfAUUCUCAsGsA |
| Ky-12S290 | 5'MVIP01-AsAsGCGACAfGfUfAUUCUCAsGsA |
| Ky-12S291 | 5'MVIP17-AsAsGCGACAfGfUfAUUCUCAsGsA |
| Ky-12S292 | AsAsGCGACAfGfUfAUUCUCAsGsA-3'MVIP17 |

(continued)

| sense strand/antisense strand code | sense strand/antisense strand sequence 5'→3' |
|---|---|
| Ky-12S293 | 5'MVIP01-AsAsGCGACAfGfUfAUUCUCAsGsA-3'MVIP09 |
| Ky-12S294 | 5'MVIP09-AsAsGCGACAfGfUfAUUCUCAsGsA-3'MVIP01 |
| Ky-12S295 | 5'MVIP09-GsUGGCUGCfCfUfGAGACCUCAsAsA |
| Ky-12S296 | 5'MVIP01-GsUGGCUGCfCfUfGAGACCUCAsAsA |
| Ky-12S297 | 5'MVIP17-GsUGGCUGCfCfUfGAGACCUCAsAsA |
| Ky-12S298 | GsUsGGCUGCfCfUfGAGACCUCAAsA-3'MVIP17 |
| Ky-12S299 | 5'MVIP01-GsUGGCUGCfCfUfGAGACCUCAsAsA-3'MVIP09 |
| Ky-12S300 | 5'MVIP09-GsUGGCUGCfCfUfGAGACCUCAsAsA-3'MVIP01 |
| Ky-12S301 | 5'MVIP09-AsAGUCCACfCfUfGCCUAUCCAsUsA |
| Ky-12S302 | 5'MVIP01-AsAGUCCACfCfUfGCCUAUCCAsUsA |
| Ky-12S303 | 5'MVIP17-AsAGUCCACfCfUfGCCUAUCCAsUsA |
| Ky-12S304 | AsAsGUCCACfCfUfGCCUAUCCAUsA-3'MVIP17 |
| Ky-12S305 | 5'MVIP01-AsAGUCCACfCfUfGCCUAUCCAsUsA-3'MVIP09 |
| Ky-12S306 | 5'MVIP09-AsAGUCCACfCfUfGCCUAUCCAsUsA-3'MVIP01 |
| Ky-12S307 | 5'MVIP09-CsGCCUGCCfUfAfUCCAUCCUGsAsA |
| Ky-12S308 | 5'MVIP01-CsGCCUGCCfUfAfUCCAUCCUGsAsA |
| Ky-12S309 | 5'MVIP17-CGCCUGCCfUfAfUCCAUCCUGsAsA |
| Ky-12S310 | CsGsCCUGCCfUfAfUCCAUCCUGAsA-3'MVIP17 |
| Ky-12S311 | 5'MVIP01-CsGCCUGCCfUfAfUCCAUCCUGsAsA-3'MVIP09 |
| Ky-12S312 | 5'MVIP09-CsGCCUGCCfUfAfUCCAUCCUGsAsA-3'MVIP01 |
| Ky-12S313 | 5'MVIP09-GsGGACAGUfAfUfUCUCAGUGCsUsA |
| Ky-12S314 | 5'MVIP01-GsGGACAGUfAfUfUCUCAGUGCsUsA |
| Ky-12S315 | 5'MVIP17-GsGGACAGUfAfUfUCUCAGUGCsUsA |
| Ky-12S316 | GsGsGACAGUfAfUfUCUCAGUGCUsA-3'MVIP17 |

(continued)

| sense strand/antisense strand code | sense strand/antisense strand sequence 5'→3' |
|---|---|
| Ky-12S317 | 5'MVIP01-GsGGACAGUfAfUfUCUCAGUGCsUsA-3'MVIP09 |
| Ky-12S318 | 5'MVIP09-GsGGACAGUfAfUfUCUCAGUGCsUsA-3'MVIP01 |
| Ky-12S319 | 5'MVIP01-GsGGACAGUfAfUfUCUCAGUGCsUsA-3'MVIP01 |
| Ky-12S320 | 5'MVIP09-GsACAGUAUfUfCfUCAGUGCUCsUsA |
| Ky-12S321 | 5'MVIP01-GsACAGUAUfUfCfUCAGUGCUCsUsA |
| Ky-12S322 | 5'MVIP17-GsACAGUAUfUfCfUCAGUGCUCsUsA |
| Ky-12S323 | GsAsCAGUAUfUfCfUCAGUGCUCUsA-3'MVIP17 |
| Ky-12S324 | 5'MVIP01-GsACAGUAUfUfCfUCAGUGCUCsUsA-3'MVIP09 |
| Ky-12S325 | 5'MVIP09-GsACAGUAUfUfCfUCAGUGCUCsUsA-3'MVIP01 |
| Ky-12S326 | 5'MVIP09-GsUGGCUfGCfCfUfGAGACCUCAsAsA |
| Ky-12S327 | 5'MVIP01-GsUGGCUfGCfCfUfGAGACCUCAsAsA |
| Ky-12S328 | 5'MVIP17-GsUGGCUfGCfCfUfGAGACCUCAsAsA |
| Ky-12S329 | GsUsGGCUfGCfCfUfGAGACCUCAAsA-3'MVIP17 |
| Ky-12S330 | 5'MVIP01-GsUGGCUfGCfCfUfGAGACCUCAsAsA-3'MVIP09 |
| Ky-12S331 | 5'MVIP09-GsUGGCUfGCfCfUfGAGACCUCAsAsA-3'MVIP01 |
| Ky-12S332 | 5'MVIP09-AsAGUCCfACfCfUfGCCUAUCCAsUsA |
| Ky-12S333 | 5'MVIP01-AsAGUCCfACfCfUfGCCUAUCCAsUsA |
| Ky-12S334 | 5'MVIP17-AsAGUCCfACfCfUfGCCUAUCCAsUsA |
| Ky-12S335 | AsAsGUCCfACfCfUfGCCUAUCCAUsA-3'MVIP17 |
| Ky-12S336 | 5'MVIP01-AsAGUCCfACfCfUfGCCUAUCCAsUsA-3'MVIP09 |
| Ky-12S337 | 5'MVIP09-AsAGUCCfACfCfUfGCCUAUCCAsUsA-3'MVIP01 |
| Ky-12S338 | 5'MVIP09-CsGCCUGfCCfUfAfUCCAUCCUGsAsA |
| Ky-12S339 | 5'MVIP01-CsGCCUGfCCfUfAfUCCAUCCUGsAsA |
| Ky-12S340 | 5'MVIP17-CsGCCUGfCCfUfAfUCCAUCCUGsAsA |

(continued)

| sense strand/antisense strand code | sense strand/antisense strand sequence 5'→3' |
|---|---|
| Ky-12S341 | CsGsCCUGfCCfUfAfUCCAUCCUGAsA-3'MVIP17 |
| Ky-12S342 | 5'MVIP01-CsGCCUGfCCfUfAfUCCAUCCUGsAsA-3'MVIP09 |
| Ky-12S343 | 5'MVIP09-CsGCCUGfCCfUfAfUCCAUCCUGsAsA-3'MVIP01 |
| Ky-12S344 | 5'MVIP09-GsGGACAfGUfAfUfUCUCAGUGCsUsA |
| Ky-12S345 | 5'MVIP01-GsGGACAfGUfAfUfUCUCAGUGCsUsA |
| Ky-12S346 | 5'MVIP17-GsGGACAfGUfAfUfUCUCAGUGCsUsA |
| Ky-12S347 | GsGsGACAfGUfAfUfUCUCAGUGCUsA-3'MVIP17 |
| Ky-12S348 | 5'MVIP01-GsGGACAfGUfAfUfUCUCAGUGCsUsA-3'MVIP09 |
| Ky-12S349 | 5'MVIP09-GsGGACAfGUfAfUfUCUCAGUGCsUsA-3'MVIP01 |
| Ky-12S350 | 5'MVIP09-GsACAGUfAUfUfCfUCAGUGCUCsUsA |
| Ky-12S351 | 5'MVIP01-GsACAGUfAUfUfCfUCAGUGCUCsUsA |
| Ky-12S352 | 5'MVIP17-GsACAGUfAUfUfCfUCAGUGCUCsUsA |
| Ky-12S353 | GsAsCAGUfAUfUfCfUCAGUGCUCUsA-3'MVIP17 |
| Ky-12S354 | 5'MVIP01-GsACAGUfAUfUfCfUCAGUGCUCsUsA-3'MVIP09 |
| Ky-12S355 | 5'MVIP09-GsACAGUfAUfUfCfUCAGUGCUCsUsA-3'MVIP01 |
| Ky-12S356 | 5'MVIP09-AsAGCGACAfGfUfAUUCUCAGUsGsA |
| Ky-12S357 | 5'MVIP01-AsAGCGACAfGfUfAUUCUCAGUsGsA |
| Ky-12S358 | 5'MVIP17-AsAGCGACAfGfUfAUUCUCAGUsGsA |
| Ky-12S359 | AsAsGCGACAfGfUfAUUCUCAGUGsA-3'MVIP17 |
| Ky-12S360 | 5'MVIP01-AsAGCGACAfGfUfAUUCUCAGUsGsA-3'MVIP09 |
| Ky-12S361 | 5'MVIP09-AsAGCGACAfGfUfAUUCUCAGUsGsA-3'MVIP01 |
| Ky-12S362 | 5'MVIP09-AsAGCGAfCAfGfUfAUUCUCAGUsGsA |
| Ky-12S363 | 5'MVIP01-AsAGCGAfCAfGfUfAUUCUCAGUsGsA |
| Ky-12S364 | 5'MVIP17-AsAGCGAfCAfGfUfAUUCUCAGUsGsA |
| Ky-12S365 | AsAsGCGAfCAfGfUfAUUCUCAGUGsA-3'MVIP17 |

(continued)

| sense strand/antisense strand code | sense strand/antisense strand sequence 5'→3' |
|---|---|
| Ky-12S366 | 5'MVIP01-AsAGCGAfCAfGfUfAUUCUCAGUsGsA-3'MVIP09 |
| Ky-12S367 | 5'MVIP09-AsAGCGAfCAfGfUfAUUCUCAGUsGsA-3'MVIP01 |
| Ky-12S368 | 5'MVIP09-AsAGCGACAfGfUfAUUCUCAsGsA |
| Ky-12S369 | 5'MVIP01-AsAGCGACAfGfUfAUUCUCAsGsA |
| Ky-12S370 | 5'MVIP17-AsAGCGACAfGfUfAUUCUCAsGsA |
| Ky-12S371 | AsAsGCGACAfGfUfAUUCUCAGsA-3'MVIP17 |
| Ky-12S372 | 5'MVIP01-AsAGCGACAfGfUfAUUCUCAsGsA-3'MVIP09 |
| Ky-12S373 | 5'MVIP09-AsAGCGACAfGfUfAUUCUCAsGsA-3'MVIP01 |
| Ky-12S374 | 5'MVIP09-GsUsGGCUGCfCfUfGAGACCUsCsA |
| Ky-12S375 | 5'MVIP01-GsUsGGCUGCfCfUfGAGACCUsCsA |
| Ky-12S376 | 5'MVIP17-GsUsGGCUGCfCfUfGAGACCUsCsA |
| Ky-12S377 | GsUsGGCUGCfCfUfGAGACCUsCsA-3'MVIP17 |
| Ky-12S378 | 5'MVIP01-GsUsGGCUGCfCfUfGAGACCUsCsA-3'MVIP09 |
| Ky-12S379 | 5'MVIP09-GsUsGGCUGCfCfUfGAGACCUsCsA-3'MVIP01 |
| Ky-12S380 | 5'MVIP09-GsAsGUCCACfCfUfGCCUAUCsCsA |
| Ky-12S381 | 5'MVIP01-GsAsGUCCACfCfUfGCCUAUCsCsA |
| Ky-12S382 | 5'MVIP17-GsAsGUCCACfCfUfGCCUAUCsCsA |
| Ky-12S383 | GsAsGUCCACfCfUfGCCUAUCsCsA-3'MVIP17 |
| Ky-12S384 | 5'MVIP01-GsAsGUCCACfCfUfGCCUAUCsCsA-3'MVIP09 |
| Ky-12S385 | 5'MVIP09-GsAsGUCCACfCfUfGCCUAUCsCsA-3'MVIP01 |
| Ky-12S386 | 5'MVIP09-CsAsCCUGCCfUfAfUCCAUCCsUsA |
| Ky-12S387 | 5'MVIP01-CsAsCCUGCCfUfAfUCCAUCCsUsA |
| Ky-12S388 | 5'MVIP17-CsAsCCUGCCfUfAfUCCAUCCsUsA |
| Ky-12S389 | CsAsCCUGCCfUfAfUCCAUCCsUsA-3'MVIP17 |

(continued)

| sense strand/antisense strand code | sense strand/antisense strand sequence 5'→3' |
|---|---|
| Ky-12S390 | 5'MVIP01-CsAsCCUGCCfUfAfUCCAUCCsUsA-3'MVIP09 |
| Ky-12S391 | 5'MVIP09-CsAsCCUGCCfUfAfUCCAUCCsUsA-3'MVIP01 |
| Ky-12S392 | 5'MVIP01-CsAsCCUGCCfUfAfUCCAUCCsUsA-3'MVIP01 |
| Ky-12S393 | 5'MVIP09-GsGsGACAGUfAfUfUCUCAGUsGsA |
| Ky-12S394 | 5'MVIP01-GsGsGACAGUfAfUfUCUCAGUsGsA |
| Ky-12S395 | 5'MVIP17-GsGsGACAGUfAfUfUCUCAGUsGsA |
| Ky-12S396 | GsGsGACAGUfAfUfUCUCAGUsGsA-3'MVIP17 |
| Ky-12S397 | 5'MVIP01-GsGsGACAGUfAfUfUCUCAGUsGsA-3'MVIP09 |
| Ky-12S398 | 5'MVIP09-GsGsGACAGUfAfUfUCUCAGUsGsA-3'MVIP01 |
| Ky-12S399 | 5'MVIP09-GsAsCAGUAUfUfCfUCAGUGCsUsA |
| Ky-12S400 | 5'MVIP01-GsAsCAGUAUTUfCfUCAGUGCsUsA |
| Ky-12S401 | 5'MVIP17-GsAsCAGUAUfUfCfUCAGUGCsUsA |
| Ky-12S402 | GsAsCAGUAUfUfCfUCAGUGCsUsA-3'MVIP17 |
| Ky-12S403 | 5'MVIP01-GsAsCAGUAUfUfCfUCAGUGCsUsA-3'MVIP09 |
| Ky-12S404 | 5'MVIP09-GsAsCAGUAUfUfCfUCAGUGCsUsA-3'MVIP01 |
| Ky-12S405 | 5'MVIP09-GsTsGGCUfGCfCfUfGAGACCUsCsA |
| Ky-12S406 | 5'MVIP01-GsTsGGCUfGCfCfUfGAGACCUsCsA |
| Ky-12S407 | 5'MVIP17-GsTsGGCUfGCfCfUfGAGACCUsCsA |
| Ky-12S408 | GsTsGGCUfGCfCfUfGAGACCUsCsA-3'MVIP17 |
| Ky-12S409 | 5'MVIP01-GsTsGGCUfGCfCfUfGAGACCUsCsA-3'MVIP09 |
| Ky-12S410 | 5'MVIP09-GsTsGGCUfGCfCfUfGAGACCUsCsA-3'MVIP01 |
| Ky-12S411 | 5'MVIP09-AsAsGUCCfACfCfUfGCCUAUCsCsA |
| Ky-12S412 | 5'MVIP01-AsAsGUCCfACfCfUfGCCUAUCsCsA |
| Ky-12S413 | 5'MVIP17-AsAsGUCCfACfCfUfGCCUAUCsCsA |

(continued)

| sense strand/antisense strand code | sense strand/antisense strand sequence 5'→3' |
|---|---|
| Ky-12S414 | AsAsGUCCfACfCfUfGCCUAUCsCsA-3'MVIP17 |
| Ky-12S415 | 5'MVIP01-AsAsGUCCfACfCfUfGCCUAUCsCsA-3'MVIP09 |
| Ky-12S416 | 5'MVIP09-AsAsGUCCfACfCfUfGCCUAUCsCsA-3'MVIP01 |
| Ky-12S417 | 5'MVIP09-CsAsCCUGfCCfUfAfUCCAUCCsUsA |
| Ky-12S418 | 5'MVIP01-CsAsCCUGfCCfUfAfUCCAUCCsUsA |
| Ky-12S419 | 5'MVIP17-CsAsCCUGfCCfUfAfUCCAUCCsUsA |
| Ky-12S420 | CsAsCCUGfCCfUfAfUCCAUCCsUsA-3'MVIP17 |
| Ky-12S421 | 5'MVIP01-CsAsCCUGfCCfUfAfUCCAUCCsUsA-3'MVIP09 |
| Ky-12S422 | 5'MVIP09-CsAsCCUGfCCfUfAfUCCAUCCsUsA-3'MVIP01 |
| Ky-12S423 | 5'MVIP09-GsGsGACAfGUfAfUfUCUCAGUsGsA |
| Ky-12S424 | 5'MVIP01-GsGsGACAfGUfAfUfUCUCAGUsGsA |
| Ky-12S425 | 5'MVIP17-GsGsGACAfGUfAfUfUCUCAGUsGsA |
| Ky-12S426 | GsGsGACAfGUfAfUfUCUCAGUsGsA-3'MVIP17 |
| Ky-12S427 | 5'MVIP01-GsGsGACAfGUfAfUfUCUCAGUsGsA-3'MVIP09 |
| Ky-12S428 | 5'MVIP09-GsGsGACAfGUfAfUfUCUCAGUsGsA-3'MVIP01 |
| Ky-12S429 | 5'MVIP09-GsAsCAGUfAUfUfCfUCAGUGCsUsA |
| Ky-12S430 | 5'MVIP01-GsAsCAGUfAUfUfCfUCAGUGCsUsA |
| Ky-12S431 | 5'MVIP17-GsAsCAGUfAUfUfCfUCAGUGCsUsA |
| Ky-12S432 | GsAsCAGUfAUfUfCfUCAGUGCsUsA-3'MVIP17 |
| Ky-12S433 | 5'MVIP01-GsAsCAGUfAUfUfCfUCAGUGCsUsA-3'MVIP09 |
| Ky-12S434 | 5'MVIP09-GsAsCAGUfAUfUfCfUCAGUGCsUsA-3'MVIP01 |
| Ky-12S435 | 5'MVIP09-GsAsGCGACAfGfUfAUUCTCAsGsU |
| Ky-12S436 | 5'MVIP01-GsAsGCGACAfGfUfAUUCTCAsGsU |
| Ky-12S437 | 5'MVIP17-GsAsGCGACAfGfUfAUUCTCAsGsU |
| Ky-12S438 | GsAsGCGACAfGfUfAUUCTCAsGsU-3'MVIP09 |
| Ky-12S439 | GsAsGCGACAfGfUfAUUCTCAsGsU-3'MVIP17 |

(continued)

| sense strand/antisense strand code | sense strand/antisense strand sequence 5'→3' |
|---|---|
| Ky-12S440 | 5'MVIP01-GsAsGCGACAfGfUfAUUCTCAsGsU-3'MVIP09 |
| Ky-12S441 | 5'MVIP09-GsAsGCGACAfGfUfAUUCTCAsGsU-3'MVIP01 |
| Ky-12S442 | 5'MVIP09-GsCsGCGAfCAfGfUfAUUCTCAsGsU |
| Ky-12S443 | 5'MVIP01-GsCsGCGAfCAfGfUfAUUCTCAsGsU |
| Ky-12S444 | 5'MVIP17-GsCsGCGAfCAfGfUfAUUCTCAsGsU |
| Ky-12S445 | GsCsGCGAfCAfGfUfAUUCTCAsGsU-3'MVIP09 |
| Ky-12S446 | GsCsGCGAfCAfGfUfAUUCTCAsGsU-3'MVIP17 |
| Ky-12S447 | 5'MVIP01-GsCsGCGAfCAfGfUfAUUCTCAsGsU-3'MVIP09 |
| Ky-12S448 | 5'MVIP09-GsCsGCGAfCAfGfUfAUUCTCAsGsU-3'MVIP01 |
| Ky-12S449 | 5'MVIP09-GsCsGCGACAfGfUfAUUCTCAsGsA |
| Ky-12S450 | 5'MVIP01-GsCsGCGACAfGfUfAUUCTCAsGsA |
| Ky-12S451 | 5'MVIP17-GsCsGCGACAfGfUfAUUCTCAsGsA |
| Ky-12S452 | GsCsGCGACAfGfUfAUUCTCAsGsA-3'MVIP09 |
| Ky-12S453 | GsCsGCGACAfGfUfAUUCTCAsGsA-3'MVIP17 |
| Ky-12S454 | 5'MVIP01-GsCsGCGACAfGfUfAUUCTCAsGsA-3'MVIP09 |
| Ky-12S455 | 5'MVIP09-GsCsGCGACAfGfUfAUUCTCAsGsA-3'MVIP01 |
| Ky-12S456 | 5'MVIP09-AsAsGCGACAfGfUfAUUCUCAGUsGsU |
| Ky-12S457 | 5'MVIP01-AsAsGCGACAfGfUfAUUCUCAGUsGsU |
| Ky-12S458 | 5'MVIP01-AsAsGCGACAfGfUfAUUCUCAGUsGsU-3'MVIP09 |

In some embodiments, the RNA inhibitor is selected from Tables 22-1 and 22-2, wherein the sense strand and/or antisense strand differ from each sequence in Tables 22-1 and 22-2 by 1, 2 or 3 nucleotides, or have sequences of at least 15 consecutive nucleotides identical to those in Tables 22-1 and 22-2.

Table 22-1 RNA inhibitor

| RNA inhibitor | sense strand 5’→3’ | antisense strand 5’→3’ |
|---|---|---|
| Ky-12-DS23001 | 5’MVIP09-GsUsGGCUGCfCfUfGAGACCUCAsAsU | UsfGsAfGGfUCfUCAGGCfAGfCCfACsfGsG-3’MVIP09 |
| Ky-12-DS25001 | 5’MVIP09-AsAsGUCCACfCfUfGCCUAUCCAsUsC | UsfGsGfAUfAGfGCAGGUfGGfACfUUsfGsG-3’MVIP09 |
| Ky-12-DS25401 | 5’MVIP09-CsAsCCUGCCfUfAfUCCAUCCUGsAsA | CsfAsGfGAfUGfGAUAGGfCAfGGfUGsfGsA-3’MVIP09 |
| Ky-12-DS29701 | 5’MVIP09-GsGsGACAGUfAfUfUCUCAGUGCsUsU | GsfCsAfCUfGAfGAAUACfUGfUCfCCsfUsU-3’MVIP09 |
| Ky-12-DS29801 | 5’MVIP09-GsAsCAGUAUfUfCfUCAGUGCUCsUsU | GsfAsGfCAfCUfGAGAAUfACfUGfUCsfCsC-3’MVIP09 |

(continued)

| RNA inhibitor | sense strand 5'→3' | antisense strand 5'→3' |
|---|---|---|
| Ky-12-DS31701 | 5'MVIP09-GsAsGCGACAfGfUfAUUCUCAGUsGsU | AsfCsAfCUfGAfGAAUACfUGfUCfGCsfUsC-3'MVIP09 |
| Ky-12-DS31702 | 5'MVIP09-GsAsGCGAfCAfGfUfAUUCUCAGUsGsU | AsfCsAfCUfGAfGAfAUACfUGfUCfGCsUsC-3'MVIP09 |
| Ky-12-DS31703 | 5'MVIP09-GsAsGCGACAfGfUfAUUCUCAGUsGsU | AsfCsAfCUfGAfGAAUACfUGfUCfGCfUCsUsU-3'MVIP09 |
| Ky-12-DS31704 | 5'MVIP09-GsAsGCGACAfGfUfAUUCUCAsGsU | AsfCsAfCUfGAfGAAUACfUGfUCfGCsfUsC-3'MVIP09 |
| Ky-12-DS31705 | 5'MVIP01-GsAsGCGACAfGfUfAUUCUCAGUsGsU | AsfCsAfCUfGAfGAAUACfUGfUCfGCsfUsC-3'MVIP17 |
| Ky-12-DS31706 | 5'MVIP01-GsAsGCGACAfGfUfAUUCUCAGUsGsU-3'MVIP01 | AsfCsAfCUfGAfGAAUACfUGfUCfGCsfUsC |

(continued)

| RNA inhibitor | sense strand 5'→3' | antisense strand 5'→3' |
|---|---|---|
| Ky-12-DS31707 | 5'MVIP01-GsAsGCGACAfGfUfAUUCUCAGUsGsU-3'MVIP09 | AsfCsAfCUfGAfGAAUACfUGfUCfGCsfUsC |
| Ky-12-DS31708 | 5'MVIP09-GsAsGCGACAfGfUfAUUCUCAGUsGsU-3'MVIP01 | AsfCsAfCUfGAfGAAUACfUGfUCfGCsfUsC |
| Ky-12-DS31709 | 5'MVIP01-GsAsGCGACAfGfUfAUUCUCAsGsU | AsfCsAfCUfGAfGAAUACfUGfUCfGCsfUsC-3'MVIP01 |
| Ky-12-DS31710 | GsAsGCGACAfGfUfAUUCUCAsGsU-3'MVIP09 | AsfCsAfCUfGAfGAAUACfUGfUCfGCsfUsC-3'MVIP09 |
| Ky-12-DS31711 | 5'MVIP17-GsAsGCGACAfGfUfAUUCUCAsGsU | AsfCsAfCUfGAfGAAUACfUGfUCfGCsfUsC |
| Ky-12-DS31712 | GsAsGCGACAfGfUfAUUCUCAsGsU-3'MVIP17 | AsfCsAfCUfGAfGAAUACfUGfUCfGCsfUsC |
| Ky-12-DS31713 | 5'MVIP17-GsAsGCGACAfGfUfAUUCUCAsGsU | AsfCsAfCUfGAfGAAUACfUGfUCfGCsfUsC-3'MVIP01 |

(continued)

| RNA inhibitor | sense strand 5'→3' | antisense strand 5'→3' |
|---|---|---|
| Ky-12-DS33001 | 5'MVIP09-AsAsGCGACAfGfUfAUUCUCAGUsGsU | AsfCsAfCUfGAfGAAUACfUGfUCfGCsfUsU-3'MVIP09 |
| Ky-12-DS33002 | 5'MVIP09-AsAGCGACAfGfUfAUUCUCAGUsGsU | AsfCsAfCUfGAfGAAUACfUGfUCfGCfUsU-3'MVIP09 |
| Ky-12-DS33003 | 5'MVIP09-AsAGCGACAfGfUfAUUCUCAsGsU | AsfCsAfCUfGAfGAAUACfUGfUCfGCfUsU-3'MVIP09 |
| Ky-12-DS33004 | 5'MVIP01-AsAGCGACAfGfUfAUUCUCAGUsGsU | AsfCsAfCUfGAfGAAUACfUGfUCfGCfUsU-3'MVIP17 |
| Ky-12-DS33005 | 5'MVIP09-AsAGCGACAfGfUfAUUCUCAGUsGsU | AsfCsAfCUfGAfGAAUACfUGfUCfGCfUsU-3'MVIP09 |
| Ky-12-DS23002 | 5'MVIP09-GsUGGCUGCfCfUfGAGACCUCAsAsU | UsfGsAfGGfUCfUCAGGCfAGfCCfACfGsG-3'MVIP09 |

(continued)

| RNA inhibitor | sense strand 5'→3' | antisense strand 5'→3' |
|---|---|---|
| Ky-12-DS25002 | 5'MVIP09-AsAGUCCACfCfUfGCCUAUCCAsUsC | UsfGsGfAUfAGfGCAGGUfGGfACfUUfGsG-3'MVIP09 |
| Ky-12-DS25402 | 5'MVIP09-CsACCUGCCfUfAfUCCAUCCUGsAsA | CsfAsGfGAfUGfGAUAGGfCAfGGfUGfGsA-3'MVIP09 |
| Ky-12-DS29702 | 5'MVIP09-GsGGACAGUfAfUfUCUCAGUGCsUsU | GsfCsAfCUfGAfGAAUACfUGfUCfCCfUsU-3'MVIP09 |
| Ky-12-DS29802 | 5'MVIP09-GsACAGUAUfUfCfUCAGUGCUCsUsU | GsfAsGfCAfCUfGAGAAUfACfUGfUCfCsC-3'MVIP09 |
| Ky-12-DS31714 | 5'MVIP09-GsAGCGACAfGfUfAUUCUCAGUsGsU | AsfCsAfCUfGAfGAAUACfUGfUCfGCfUsC-3'MVIP09 |
| Ky-12-DS31715 | 5'MVIP09-GsAGCGAfCAfGfUfAUUCUCAGUsGsU | AsfCsAfCUfGAfGAfAUACfUGfUCfGCUsC-3'MVIP09 |

(continued)

| RNA inhibitor | sense strand 5'→3' | antisense strand 5'→3' |
|---|---|---|
| Ky-12-DS31716 | 5'MVIP09-GsAGCGACAfGfUfAUUCUCAGUsGsU | AsfCsAfCUfGAfGAAUACfUGfUCfGCfUCUsU-3'MVIP09 |
| Ky-12-DS31717 | 5'MVIP09-GsAGCGACAfGfUfAUUCUCAsGsU | AsfCsAfCUfGAfGAAUACfUGfUCfGCfUsC-3'MVIP09 |
| Ky-12-DS33006 | 5'MVIP09-AsAGCGACAfGfUfAUUCUCAGUsGsU | AsfCsAfCUfGAfGAAUACfUGfUCfGCfUCUsU-3'MVIP09 |

Table 22-2 RNA inhibitor

| single strand code | carrier structure | | single strand code | carrier structure | | Double Strand code | n + m | Position of carrier structure conjugate d to siRNA (S:AS) |
|---|---|---|---|---|---|---|---|---|
| S196 | 5'MVIP09 | n=2 | AS 196 | 3'MVIP 09 | m=2 | Kylo-12-DS1081 | 4 | n:m |
| S211 | 5'MVIP01 | n=1 | AS208 | 3' MVIP0 1 | m=1 | Kylo-12-DS131 | 2 | n:m |
| S263 | 5'MVIP21 | n=1 | AS208 | 3' MVIP0 1 | m=1 | Kylo-12-DS132 | 2 | n:m |
| S211 | 5'MVIP01 | n=1 | AS247 | 3' MVIP0 7 | m=1 | Kylo-12-DS133 | 2 | n:m |
| S212 | 5'MVIP01/3' MVIP01 | n=1/ m=1 | AS108 | / | / | Kylo-12-DS134 | 2 | nm:/ |
| S209 | 3'MVIP01 | m=1 | AS265 | 5'MVIP 01 | n=1 | Kylo-12-DS135 | 2 | m:n |
| S249 | 3' MVIP07 | m=1 | AS263 | 5'MVIP 21 | n=1 | Kylo-12-DS136 | 2 | m:n |
| S211 | 5'MVIP01 | n=1 | AS196 | 3'MVIP 09 | m=2 | Kylo-12-DS137 | 3 | n:m |
| S227 | 5' MVIP08 | n=1 | AS257 | 3'MVIP 15 | m=2 | Kylo-12-DS138 | 3 | n:m |
| S196 | 5'MVIP09 | n=2 | AS208 | 3'MVIP 01 | m=1 | Kylo-12-DS139 | 3 | n:m |
| S261 | 5' MVIP19 | n=2 | AS247 | 3' MVIP0 7 | m=1 | Kylo-12-DS140 | 3 | n:m |
| S217 | 5'MVIP01/3' MVIP09 | n=1/ m=2 | AS108 | / | / | Kylo-12-DS141 | 3 | nm:/ |
| S218 | 5'MVIP09/3' MVIP01 | n=2/ m=1 | AS108 | / | / | Kylo-12-DS142 | 3 | nm:/ |
| S208 | 3'MVIP09 | m=2 | AS265 | 5'MVIP 01 | n=1 | Kylo-12-DS143 | 3 | m:n |
| S251 | 3'MVIP 11 | m=2 | AS234 | 5'MVIP 06 | n=1 | Kylo-12-DS144 | 3 | m:n |
| S209 | 3'MVIP01 | m=1 | AS262 | 5' MVIP2 0 | n=2 | Kylo-12-DS145 | 3 | m:n |
| S249 | 3' MVIP07 | m=1 | AS261 | 5' MVIP1 9 | n=2 | Kylo-12-DS146 | 3 | m:n |
| S231 | 5'MVIP01 | n=1 | AS209 | 3' MVIP1 7 | m=3 | Kylo-12-DS147 | 4 | n:m |
| S263 | 5' MVIP21 | n=1 | AS209 | 3' MVIP1 7 | m=3 | Kylo-12-DS148 | 4 | n:m |
| S210 | 5'MVIP17 | n=3 | AS208 | 3' MVIP0 1 | m=1 | Kylo-12-DS149 | 4 | n:m |
| S264 | 5' MVIP22 | n=3 | AS247 | 3' MVIP0 7 | m=1 | Kylo-12-DS150 | 4 | n:m |
| S262 | 5' MVIP20 | n=2 | AS248 | 3' MVIP1 0 | m=2 | Kylo-12-DS151 | 4 | n:m |

(continued)

| single strand code | carrier structure | | single strand code | carrier structure | | Double Strand code | n + m | Position of carrier structure conjugate d to siRNA (S:AS) |
|---|---|---|---|---|---|---|---|---|
| S261 | 5' MVIP19 | n=2 | AS257 | 3'MVIP 15 | m=2 | Kylo-12-DS152 | 4 | n:m |
| S238 | 5' MVIP10 | n=2 | AS254 | 3'MVIP 12 | m=2 | Kylo-12-DS153 | 4 | n:m |
| S213 | 5'MVIP09/3' MVIP09 | n=2/ m=2 | AS108 | / | / | Kylo-12-DS154 | 4 | nm:/ |
| S208 | 3'MVIP09 | m=2 | AS266 | 5' MVIP0 9 | n=2 | Kylo-12-DS155 | 4 | m:n |
| S250 | 3'MVIP10 | m=2 | AS262 | 5' MVIP2 0 | n=2 | Kylo-12-DS156 | 4 | m:n |
| S209 | 3'MVIP01 | m=1 | AS207 | 5' MVIP1 7 | n=3 | Kylo-12-DS157 | 4 | m:n |
| S241 | 3'MVIP02 | m=1 | AS238 | 5' MVIP1 8 | n=3 | Kylo-12-DS158 | 4 | m:n |
| S207 | 3'MVIP17 | m=3 | AS265 | 5' MVIP0 1 | n=1 | Kylo-12-DS159 | 4 | m:n |
| S225 | 3'MVIP18 | m=3 | AS263 | 5' MVIP2 1 | n=1 | Kylo-12-DS160 | 4 | m:n |
| S196 | 5'MVIP09 | n=2 | AS209 | 3' MVIP1 7 | m=3 | Kylo-12-DS161 | 5 | n:m |
| S262 | 5'MVIP20 | n=2 | AS220 | 3' MVIP1 9 | m=3 | Kylo-12-DS162 | 5 | n:m |
| S210 | 5'MVIP17 | n=3 | AS 196 | 3'MVIP 09 | m=2 | Kylo-12-DS163 | 5 | n:m |
| S264 | 5'MVIP22 | n=3 | AS249 | 3'MVIP 11 | m=2 | Kylo-12-DS164 | 5 | n:m |
| S219 | 5'MVIP09/3' MVIP17 | n=2/ m=3 | AS108 | / | / | Kylo-12-DS165 | 5 | nm:/ |
| S220 | 5'MVIP17/3' MVIP09 | n=3/ m=2 | AS108 | / | / | Kylo-12-DS166 | 5 | nm:/ |
| S207 | 3'MVIP17 | m=3 | AS262 | 5' MVIP2 0 | n=2 | Kylo-12-DS167 | 5 | m:n |
| S222 | 3'MVIP19 | m=3 | AS226 | 5' MVIP1 6 | n=2 | Kylo-12-DS168 | 5 | m:n |
| S208 | 3'MVIP09 | m=2 | AS207 | 5' MVIP1 7 | n=3 | Kylo-12-DS169 | 5 | m:n |
| S259 | 3' MVIP15 | m=2 | AS207 | 5' MVIP1 7 | n=3 | Kylo-12-DS170 | 5 | m:n |
| S210 | 5'MVIP17 | n=3 | AS209 | 3' MVIP1 7 | m=3 | Kylo-12-DS171 | 6 | n:m |
| S264 | 5' MVIP22 | n=3 | AS220 | 3' MVIP1 9 | m=3 | Kylo-12-DS172 | 6 | n:m |
| S214 | 5'MVIP17/3' MVIP17 | n=3/ m=3 | AS108 | / | / | Kylo-12-DS173 | 6 | nm:/ |

(continued)

| single strand code | carrier structure | | single strand code | carrier structure | | Double Strand code | n + m | Position of carrier structure conjugate d to siRNA (S:AS) |
|---|---|---|---|---|---|---|---|---|
| S207 | 3'MVIP17 | m=3 | AS207 | 5' MVIP1 7 | n=3 | Kylo-12-DS174 | 6 | m:n |
| S225 | 3'MVIP18 | m=3 | AS264 | 5' MVIP2 2 | n=3 | Kylo-12-DS175 | 6 | m:n |

In some embodiments, the RNA inhibitor or its pharmaceutically acceptable salt described in the present invention is preferably prepared or synthesized in the form of sodium salt, triethylamine salt or other pharmaceutically acceptable salt.

In some embodiments, the RNA inhibitor or its pharmaceutically acceptable salt is more preferably sodium salt or triethylamine salt.

The present invention also provides a pharmaceutical composition comprising the above-mentioned RNA inhibitor or pharmaceutically acceptable salt thereof.

In one embodiment, the present invention also provides a pharmaceutical composition comprising the above-mentioned RNA inhibitor or pharmaceutically acceptable salt thereof and pharmaceutically acceptable pharmaceutical adjuvants. The pharmaceutical composition containing the RNA inhibitor can be used to prevent and/or treat diseases associated with elevated levels of APOC3, such as hepatic diseases, inflammatory, cardiovascular and cerebrovascular, and metabolic diseases, wherein the cardiovascular and cerebrovascular diseases include hyperlipidemia, stroke, atherosclerosis, thrombosis, coronary heart disease, aortic valve stenosis, hypertriglyceridemia (HTG), severe hypertriglyceridemia (sHTG) or familial chylomicronemia syndrome (FCS). Such pharmaceutical compositions are formulated according to the mode of delivery. An embodiment is to formulate as a composition for systemic administration by parenteral delivery, such as, subcutaneous (SC), intramuscular (IM), or intravenous (IV) delivery. The pharmaceutical composition of the present invention can be administered at a dose sufficient to inhibit APOC3 gene expression.

The pharmaceutically acceptable "adjuvants" or "excipients" are pharmaceutically acceptable solvents, suspensions or any other pharmaceutically inert vehicles used to deliver one or more nucleic acids to animals. The excipients may be liquid or solid and are selected taking account of the intended mode of administration to provide the required volume, consistency, etc. when combined with nucleic acids and other components in a given pharmaceutical composition. The RNA inhibitor can be delivered in a manner that targets specific tissues (e.g., hepatocytes).

In some embodiments, the pharmaceutical composition further comprises a delivery vehicle (such as nanoparticles, dendrimers, polymers, liposomes, or cation delivery systems).

In some embodiments, the delivery vehicle comprises liposomes.

In some embodiments, the delivery vehicle comprises nanolipids that is capable of forming a liposome-nucleic acid nanoparticle with nucleic acid molecule.

In some embodiments, the delivery vehicle comprises amphoteric lipid compounds M10C1.

The pharmaceutical composition provided in the present invention includes (but is not limited to) solutions, emulsions, and preparations containing liposomes. These compositions can be produced from a variety of components, including (but not limited to) pre-formed liquids, self-emulsifying solids, and self-emulsifying semisolids. Preparations include those targeting the liver. The pharmaceutical preparations of the present invention, which can conveniently exist in a unit dosage form, can be prepared according to the conventional technology known to the pharmaceutical industry. Such technologies include steps of combining active ingredients with pharmaceutical adjuvants or excipients.

**Purpose**

In yet another aspect, the present invention further provides a method for reducing APOC3 mRNA or protein expression in cell or tissue, which includes contacting the cell or tissue with an effective amount of the aforementioned RNA inhibitor or pharmaceutically acceptable salt thereof that inhibits APOC3 gene expression, and/or the aforementioned pharmaceutical composition.

The cell suitable for treatment using the method of the present invention can be any cell expressing APOC3 gene, such as liver cell, brain cell, gallbladder cell, heart cell or kidney cell, but preferably liver cell. The cell suitable for the method of the present invention can be mammalian cell. When in contact with cell expressing APOC3 gene, RNA inhibitor inhibits the expression of APOC3 gene (for example, human, primate, non-primate or rat APOC3 gene) by at least about 50%. For example, it can be determined by PCR or methods based on branched DNA (bDNA), or methods based on protein, such as immunofluorescence, Western blotting or flow cytometry.

In some embodiments, the tissue is liver tissue.

The term "inhibit" used herein may be used interchangeably with "reduce", "decrease", "silence", "downregulate", "repress" and other similar terms, and includes any level of inhibition. The expression of APOC3 gene can be evaluated according to the level or level change of any variable related to APOC3 gene expression, for example, APOC3 mRNAlevel or APOC3 protein level. Such level can be analyzed in single cell or population of cells (including, for example, a sample from a subject). Inhibition can be evaluated by the decrease in absolute or relative levels of one or more variables associated with APOC3 expression compared with control levels. The control level can be any type of control level adopted in the art, for example, the baseline level before dosing or the level measured from similar subjects, cells or samples that have never been treated or received control treatment (e.g., only buffer control or inactive agent control).

The inhibition of APOC3 gene expression can be represented by a decrease in the amount of mRNA expressed by a first cell or cells population that inhibit APOC3 gene expression (such cells may be, for example, present in a sample from a subject) in which the APOC3 gene is transcribed and treated (for example, by contacting one or more cells with the RNA inhibitor of the present invention, or by administering the RNA inhibitor of the present invention to a subject in which the cell is present) compared to a second cell or cells population (control cells not treated with RNA inhibitor or RNA inhibitor targeting the gene of interest) that is essentially the same as that first cell or cells population but not so treated. In a preferred embodiment, the inhibition is evaluated in a cell line that highly expresses APOC3 by the method provided in Example 2 using an appropriate concentration of siRNA, and the mRNA level in the interfered cells is expressed as a percentage of the mRNA levels in the uninterfered control cells.

In other embodiments, the inhibition of APOC3 gene expression can be evaluated by the reduction of parameters functionally related to APOC3 gene expression, such as APOC3 protein levels in the blood or serum of subjects. APOC3 gene silencing can be determined in any APOC3 expressing cell (endogenous or exogenous from the expression constructs) and by any assay known in the art.

The inhibition of APOC3 protein expression can be manifested by the reduction of APOC3 protein levels expressed by cell or cells population or subject samples (e.g., protein levels in blood samples derived from subjects). As mentioned above, for the evaluation of mRNA inhibition, the inhibition of protein expression levels of treated cell or cells population can be similarly shown as a percentage of protein levels of control cell or cells population, or changes in protein levels in subject samples (e.g., blood or serum derived from it).

The cell, cells population or subject samples in control group that can be used to evaluate APOC3 gene inhibition include cell, cells population or subject samples that have not been exposed to the RNA inhibitor of the present invention. For example, control cell, cells population, or subject samples may be derived from a single subject (e.g., human or animal subjects) or an appropriately matched group controls before treatment with RNA inhibitor.

APOC3 mRNA levels expressed by cell or cells population can be determined using any method known in the art for evaluating mRNA expression. For example, qRT-PCR evaluates the reduction of gene expression. The reduction in protein production can be evaluated by any method known in the art, for example, ELISA. In some embodiments, the liver biopsy sample is used as tissue material to monitor the reduction of APOC3 gene or protein expression. In other embodiments, blood samples are used as subject samples for monitoring the reduction of APOC3 protein expression.

In yet another aspect, the present invention further provides use of the aforementioned RNA inhibitor or pharmaceutically acceptable salt thereof for inhibiting APOC3 gene expression, or the aforementioned pharmaceutical composition in the preparation of drugs for preventing and/or treating diseases or conditions or reducing the risk of diseases or conditions.

In some embodiments, the disease or condition includes APOC3 related disease or condition.

In some embodiments, the disease or condition is selected from: atherosclerosis, vascular disease, myocardial infarction, angina, stroke, kidney disease, renal failure, obesity, glucose intolerance, type 2 diabetes (non-insulin-dependent diabetes mellitus), and metabolic syndrome.

In yet another aspect, the present invention provides a method for preventing and/or treating diseases or conditions, which includes administering to a subject in need an effective amount of the aforementioned RNA inhibitor or pharmaceutically acceptable salt thereof that inhibits APOC3 gene expression, and/or the aforementioned pharmaceutical composition.

The in vivo method of the present invention may comprise administering to a subject a composition comprising an RNA inhibitor, wherein the RNA inhibitor comprises a nucleotide sequence complementary to at least a portion of the RNA transcript of the APOC3 gene of a mammal receiving the administration of the RNA inhibitor. The composition may be administered in any manner known in the art, including (but not limited to): oral, intraperitoneal or parenteral routes, including intracranial (e.g., intraventricular, intraparenchymal, and intrathecal), intravenous, intramuscular, subcutaneous, transdermal, airway (aerosol), nasal, rectal, and local (including buccal and sublingual) administration. In some embodiments, the composition is administered by intravenous infusion or injection. In some embodiments, the composition is administered by subcutaneous injection. In some embodiments, the composition is administered by intramuscular injection.

The RNA inhibitor of the present invention can also be administered as a "free RNA inhibitor". The free RNA

inhibitors are administered in the absence of a pharmaceutical composition. Naked RNA inhibitors can be in a suitable buffer. The buffer may contain acetate, citrate, prolamin, carbonate, or phosphate, or any combination thereof. In one embodiment, the buffer is phosphate buffered saline (PBS). The pH and osmotic pressure of the buffer containing RNA inhibitor can be adjusted to be suitable for administration to subjects.

Alternatively, the RNA inhibitor of the present invention may be administered as a pharmaceutical composition, such as a liposome preparation.

The pharmaceutical composition of the present invention can be administered at a dosage sufficient to inhibit APOC3 gene expression. In some examples, the appropriate dosage of the RNA inhibitor of the present invention is in the range of about 0.001 to about 1000.0 mg per kilogram of recipient body weight per day. In some examples, the appropriate dosage of the RNA inhibitor of the present invention is in the range of about 1 to 50 mg per kilogram body weight per day. In some examples, the appropriate dosage of the RNA inhibitor of the present invention is in the range of about 0.1 mg/kg to about 10.0.0 mg/kg, for example, in the range of about 0.3mg/kg to about 3.0mg/kg.

In one embodiment, the method includes administering the pharmaceutical composition of the present invention such that the target APOC3 gene expression is reduced for, such as approximately 1, 2, 3, 4, 5, 6, 1-6, 1-3, or 3-6 months per dose. In some embodiments, the composition is administered every 3-6 months.

In some embodiments, after the initial treatment regimen, treatment is administered at less frequency. The repeated dosage regimen may include regular administration of therapeutic amounts of the RNA inhibitor, such as once a month to once a year. In some embodiments, the RNA inhibitor is administered approximately once a month to approximately once every three months, or approximately once every three months to approximately once every six months.

After the initial treatment regimen, treatment can be administered at a lower frequency. The duration of treatment can be determined according to the severity of the disease.

In other embodiments, a single dose of the pharmaceutical composition may be long-acting, such that the dose is administered at intervals of no more than 1, 2, 3, or 4 months. In some embodiments of the present invention, a single dose of the pharmaceutical composition is administered approximately once a month. In other embodiments of the present invention, a single dose of the pharmaceutical composition is administered quarterly (i.e. about every 3 months). In other embodiments of the present invention, a single dose of the pharmaceutical composition is administered twice a year (i.e., about once every 6 months).

The person skilled in the art should understand that certain factors can affect the dosage and duration of administration required to effectively treat a subject, including (but not limited to): the mutation existing in the subject, previous treatment, the general health/age and presence of other diseases of the subject. In addition, treating a subject with a prophylactically and/or therapeutically effective amount of the composition as needed may include a single treatment or a series of treatments.

In some embodiments, the method further includes determining APOC3 levels in samples from the subject.

For example, the method further includes determining the APOC3 level in blood sample, serum sample or urine sample from the subject.

In some embodiments, the method further includes administering to the subject an additional therapeutic agent for the treatment of hyperlipidemia.

For example, the additional therapeutic agent can be selected from statins, such as atorvastatin, rosuvastatin, etc.; cholesterol absorption inhibitors such as ezetimibe; PCSK9 inhibitors.

In yet another aspect, the present invention further provides a cell, which contains the aforementioned RNA inhibitor or pharmaceutically acceptable salt thereof for inhibiting APOC3 gene expression.

In yet another aspect, the present invention further provides a kit, which contains the aforementioned RNA inhibitor or pharmaceutically acceptable salt thereof for inhibiting APOC3 gene expression, or the aforementioned pharmaceutical composition.

Without intending to be bound by any theory, the following examples are only for the purpose of explaining the RNA inhibitor, preparation method and use of the present invention, and are not used to limit the scope of the invention.

## Examples

Explanation:

The name of DMSO is dimethyl sulfoxide;
The name of DMF is N, N-dimethylformamide;
The name of HOBt is 1-hydroxybenzotriazole;
The name of HBTU is O-benzotriazole-tetramethylurea hexafluorophosphate;

The name of DIPEA (DIEA) is N, N-diisopropylethylamine;
The name of DCM is dichloromethane;
The name of DMAP is 4-dimethylaminopyridine;
The name of DMT-CL is 4,4'- dimethoxytriphenylchloromethane;
The name of MEOH is methanol;
The name of TBTU is O-benzotriazole-N, N, N', N'- tetramethylurea tetrafluoroboric acid;

The name of 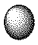 is solid support, such as macroporous aminomethyl resin (Resin).

**Example 1 Synthesis of RNA inhibitor**

The sense strand and antisense strand of the unconjugated carrier structure were synthesized using a solid phase synthesizer according to the standard solid-phase phosphoramidite method. The sense strand and the corresponding antisense strand were annealed to obtain RNA inhibitor.

The basic steps of the solid-phase phosphoramidite method include:

1) Deprotection: removing hydroxyl protecting group (DMTr) of starting monomer Solid Support;
2) Conjugating: adding a first phosphoramidite monomer, the conjugating occurs through the 3' to 5'direction;
3) Oxidation: oxidizing the resulting nucleoside phosphite to a more stable nucleoside phosphate (that is, trivalent phosphorus is oxidized to pentavalent phosphorus);
4) Blocking: adding a cap to the 5'-OH of the failed nucleotide sequence in the previous step to prevent from further participating in subsequent reaction; repeating the above steps until the last phosphoramidite monomer is introduced; then cleaving the ester bond between Solid Support and the starting monomer with methylamine aqueous solution and ammonia water, and removing the protecting groups on each base and phosphate on the resulting nucleotide sequence; after separation and purification by HPLC, filter sterilizing and lyophilizing to obtain the corresponding sense strand or antisense strand.

Description of the synthesis process of RNA inhibitors:

Redissolved the sense strand and antisense strand freeze-dried powders separately, mixed them in an equal molar ratio, added into an appropriate amount of water for injection, and added into an appropriate amount of TRIS buffer solution. Shaked gently for about 1 to 2 min to mix the solution evenly. Heated a water bath to 92°C~95 °C and placed the above reaction solution in the water bath to heat for 3~ 5min, shaked gently to heat the solution evenly. Let it to cool naturally to room temperature to obtain colorless or yellowish transparent liquid. Took samples for testing to measure concentrations.

**Example 2-1 Experiment 1 of RNA inhibitor to inhibit APOC3 gene expression in vitro**

The RNA inhibitors in this Example (DS52~DS102) were prepared by the method described in Example 1. The aqueous solution of RNA inhibitor agent and organic solution of DOTMA were mixed to form water-insoluble precipitate, which was separated, dried, dissolved in chloroform, and further mixed with a solution of other lipid chloroform, including M10C1 and PEG600-cholesterol. The mixture was evaporated and dried overnight by vacuum centrifugation to obtain nanolipid-encapsulated RNA inhibitor, in which the weight ratios of DOTMA, M10C1 and PEG600-cholesterol to RNA inhibitor were 1~1.6, 1.5~2.5 and 2.5~3.5.

DMEM containing 10% fetal bovine serum was used to prepare the nanolipid-encapsulated RNA inhibitor sample solution with corresponding concentration. HepG 2 cells were inoculated at a density of $10^5$ cells. After incubation for 24h in DMEM medium supplemented with 10% fetal bovine serum, 37°C, 5%$CO_2$, a sample of 10nM was added for interference. After incubation for 72h, collected the cell samples, added 1 ml Ezol lysate to the collected cell samples and mixed well by a vortex shaker. Added 0.2 ml trichloromethane, shook vigorously for 10 s, and left at room temperature for 1 min. Centrifuged at 12000 x g for 15 min at 4 °C. Transferred the upper clear water phase to another new RNase free centrifuge tube, and added an equal volume of 100% ethanol. Pipetted all of the sample and added it to a mini-spin centrifuge column with a 2 ml collection tube. Centrifuged at 8000 x g, room temperature for 15 seconds, and discarded the flow-through liquid. Transferred the remaining sample to a centrifuge column and repeated the previous step. Added 700 μl WB to the centrifuge column, gently closed the cover, centrifuged at 8000 × g, room temperature for 15 seconds, discarded the flow-through liquid. Repeated the previous step, and used 500 μ L WB to wash the centrifuge column twice. APOC3 mRNA levels were determined by qRT-PCR. Compared with the supernatant of HepG 2 cells without intervention, the relative expression levels of APOC3 mRNAin the intervention group were calibrated.

Data analysis ΔΔCt method (Ct difference comparative method):

The housekeeping gene GAPDH is expressed in all cells, and its product is necessary for maintaining cell survival. The expression level or genome copy number in the cell is constant and is less affected by the environment. Therefore, GAPDH is used as the internal reference gene. After qRT-PCR, the CT value of the internal reference will be recorded at the same time, which is called Ct (GAPDH), and the CT value of the sample is called Ct (sample).

$$\Delta\,\text{Ct (sample)} = \text{Ct (sample)-Ct (GAPDH)}$$

$$\Delta\,\text{Ct (control)} = \text{Ct (control)-Ct (GAPDH)}$$

$$\Delta\,\Delta\,\text{Ct}=\Delta\text{Ct (sample)}-\Delta\text{Ct (control)}$$

$$\text{Relative expression level of gene} = 2\,\hat{}\,-\triangle\triangle\text{Ct}$$

The experimental results are shown in Table 23 below.

Table 23 APOC3 mRNA levels in HepG2 cell after RNA inhibitor intervention

| RNA inhibitor | CT value | | APOC3-GAPDH | Relative expression level | |
|---|---|---|---|---|---|
| | GAPDH | APOC3 | | 2 ^ -ΔΔCt | Mean value |
| DS52 | 25.06 | 36.73 | 11.67 | 0.040949794 | 0.059042056 |
| | 25.01 | 36.81 | 11.8 | 0.03742121 | |
| | 25.04 | 35.44 | 10.4 | 0.098755164 | |
| DS53 | 21.47 | 32.2 | 10.73 | 0.07856334 | 0.06000476 |
| | 21.4 | 32.61 | 11.21 | 0.05632815 | |
| | 21.3 | 32.83 | 11.53 | 0.04512279 | |
| DS54 | 23.13 | 33.31 | 10.18 | 0.11502346 | 0.0984873 |
| | 23.25 | 33.67 | 10.42 | 0.09739557 | |
| | 22.73 | 33.38 | 10.65 | 0.08304286 | |
| DS55 | 20.53 | 33.19 | 12.66 | 0.02061731 | 0.01934869 |
| | 20.82 | 33.26 | 12.44 | 0.02401367 | |
| | 20.85 | 34.13 | 13.28 | 0.01341508 | |
| DS56 | 19.97 | 31.99 | 12.02 | 0.03212856 | 0.03902712 |
| | 19.9 | 31.43 | 11.53 | 0.04512279 | |
| | 19.84 | 31.55 | 11.71 | 0.03983002 | |
| DS57 | 24.35 | 34.31 | 9.96 | 0.13397168 | 0.20733798 |
| | 24.44 | 33.55 | 9.11 | 0.24148408 | |
| | 24.31 | 33.39 | 9.08 | 0.24655818 | |
| DS58 | 20.28 | 31.4 | 11.12 | 0.05995401 | 0.06233004 |
| | 20.33 | 31.22 | 10.89 | 0.07031616 | |
| | 20.22 | 31.42 | 11.2 | 0.05671995 | |
| DS59 | 22.01 | 30.22 | 8.21 | 0.45062523 | 0.54905108 |
| | 22.11 | 30.08 | 7.97 | 0.53218509 | |
| | 22.56 | 30.21 | 7.65 | 0.66434291 | |

(continued)

| RNA inhibitor | CT value | | APOC3-GAPDH | Relative expression level | |
|---|---|---|---|---|---|
| | GAPDH | APOC3 | | 2 ^ -ΔΔCt | Mean value |
| DS60 | 22.22 | 32.2 | 9.98 | 0.13212726 | 0.12281597 |
| | 22.07 | 32.32 | 10.25 | 0.10942972 | |
| | 21.97 | 32.01 | 10.04 | 0.12674493 | |
| DS61 | 23.66 | 29.66 | 6 | 2.08493152 | 2.10135682 |
| | 23.66 | 29.72 | 6.06 | 2 | |
| | 23.6 | 29.51 | 5.91 | 2.21913894 | |
| DS62 | 21.19 | 32.95 | 11.76 | 0.03847326 | 0.03523392 |
| | 21.26 | 33.24 | 11.98 | 0.03303181 | |
| | 21.22 | 33.15 | 11.93 | 0.03419668 | |
| DS63 | 20.83 | 31.03 | 10.2 | 0.11343989 | 0.11783809 |
| | 21.03 | 31.42 | 10.39 | 0.09944206 | |
| | 21.32 | 31.21 | 9.89 | 0.14063231 | |
| DS64 | 22.04 | 31.39 | 9.35 | 0.20447551 | 0.21274677 |
| | 22.09 | 31.24 | 9.15 | 0.23488069 | |
| | 21.96 | 31.35 | 9.39 | 0.19888412 | |
| DS65 | 26.65 | 34.7 | 8.05 | 0.50347778 | 0.4294787 |
| | 26.65 | 34.74 | 8.09 | 0.48971015 | |
| | 26.63 | 35.45 | 8.82 | 0.29524817 | |
| DS66 | 20.5 | 31.66 | 11.16 | 0.0241807 | 0.02752831 |
| | 20.56 | 31.53 | 10.97 | 0.02758447 | |
| | 20.52 | 31.33 | 10.81 | 0.03081977 | |
| DS67 | 20.19 | 31.06 | 10.87 | 0.0295643 | 0.02970701 |
| | 20.24 | 30.98 | 10.74 | 0.03235203 | |
| | 20.24 | 31.23 | 10.99 | 0.02720471 | |
| DS68 | 19.35 | 31.59 | 12.24 | 0.01143817 | 0.01242943 |
| | 19.36 | 31.36 | 12 | 0.01350839 | |
| | 19.29 | 31.37 | 12.08 | 0.01277972 | |
| DS69 | 22.1 | 33.31 | 11.21 | 0.02335702 | 0.02969232 |
| | 22.15 | 32.69 | 10.54 | 0.03716272 | |
| | 22.25 | 33.17 | 10.92 | 0.02855723 | |
| DS70 | 19.32 | 32.88 | 13.56 | 0.00458139 | 0.00418037 |
| | 19.33 | 32.77 | 13.44 | 0.00497875 | |
| | 19.3 | 33.48 | 14.18 | 0.00298098 | |
| DS71 | 21.66 | 32.75 | 11.09 | 0.02538289 | 0.02743428 |
| | 21.59 | 32.5 | 10.91 | 0.02875586 | |
| | 21.64 | 32.58 | 10.94 | 0.02816408 | |

(continued)

| RNA inhibitor | CT value | | APOC3-GAPDH | Relative expression level | |
|---|---|---|---|---|---|
| | GAPDH | APOC3 | | 2 ^ -ΔΔCt | Mean value |
| DS72 | 20.83 | 32.4 | 11.57 | 0.01819896 | 0.0167851 |
| | 20.88 | 32.57 | 11.69 | 0.01674646 | |
| | 20.95 | 32.76 | 11.81 | 0.01540989 | |
| DS73 | 20.09 | 31 | 10.91 | 0.02875586 | 0.0321474 |
| | 20.38 | 31.06 | 10.68 | 0.03372588 | |
| | 20.56 | 31.23 | 10.67 | 0.03396046 | |
| DS74 | 22.53 | 31.38 | 8.85 | 0.11990801 | 0.14334036 |
| | 22.51 | 31.04 | 8.53 | 0.14968484 | |
| | 22.47 | 30.9 | 8.43 | 0.16042824 | |
| DS75 | 21.4 | 31.59 | 10.19 | 0.04736614 | 0.04188562 |
| | 21.47 | 32.06 | 10.59 | 0.03589682 | |
| | 21.38 | 31.73 | 10.35 | 0.04239389 | |
| DS76 | 24.82 | 30.54 | 5.72 | 1.04971668 | 1.0337533 |
| | 24.76 | 30.75 | 5.99 | 0.87055056 | |
| | 24.82 | 30.37 | 5.55 | 1.18099266 | |
| DS77 | 24.95 | 34.52 | 9.57 | 0.07279585 | 0.09858007 |
| | 25.11 | 34.52 | 9.41 | 0.08133387 | |
| | 25.09 | 33.7 | 8.61 | 0.14161049 | |
| DS78 | 27.42 | 36.86 | 9.44 | 0.07966004 | 0.08846432 |
| | 27.47 | 36.4 | 8.93 | 0.11343989 | |
| | 27.42 | 37 | 9.58 | 0.07229301 | |
| DS79 | 23.29 | 34.53 | 11.24 | 0.02287634 | 0.04030962 |
| | 23.19 | 33.71 | 10.52 | 0.03768149 | |
| | 23.14 | 32.98 | 9.84 | 0.06037102 | |
| DS80 | 26.25 | 36.44 | 10.19 | 0.13869618 | 0.17461278 |
| | 26.18 | 35.8 | 9.62 | 0.20589775 | |
| | 26.07 | 35.89 | 9.82 | 0.17924441 | |
| DS81 | 23.34 | 33.68 | 10.34 | 0.125 | 0.1427191 |
| | 23.46 | 33.73 | 10.27 | 0.13121459 | |
| | 23.35 | 33.23 | 9.88 | 0.17194273 | |
| DS82 | 26.77 | 38.02 | 11.25 | 0.06652314 | 0.05729203 |
| | 26.77 | 38.12 | 11.35 | 0.06206828 | |
| | 26.65 | 38.52 | 11.87 | 0.04328467 | |
| DS83 | 25.69 | 37.66 | 11.97 | 0.04038603 | 0.04889128 |
| | 25.73 | 37.36 | 11.63 | 0.05111888 | |
| | 25.81 | 37.33 | 11.52 | 0.05516894 | |

(continued)

| RNA inhibitor | CT value | | APOC3-GAPDH | Relative expression level | |
|---|---|---|---|---|---|
| | GAPDH | APOC3 | | 2 ^ -ΔΔCt | Mean value |
| DS84 | 25.01 | 33.7 | 8.69 | 0.39229205 | 0.36010911 |
| | 24.87 | 33.79 | 8.92 | 0.33448189 | |
| | 24.81 | 33.65 | 8.84 | 0.35355339 | |
| DS85 | 24.79 | 34.31 | 9.52 | 0.22067429 | 0.20805055 |
| | 24.79 | 34.4 | 9.61 | 0.20732989 | |
| | 24.71 | 34.4 | 9.69 | 0.19614602 | |
| DS86 | 26.21 | 36.31 | 10.1 | 0.14762408 | 0.1700243 |
| | 26.25 | 35.93 | 9.68 | 0.19751033 | |
| | 26.21 | 36.15 | 9.94 | 0.16493849 | |
| DS87 | 23.5 | 34.78 | 11.28 | 0.06515411 | 0.06397548 |
| | 23.54 | 34.83 | 11.29 | 0.06470406 | |
| | 23.78 | 35.13 | 11.35 | 0.06206828 | |
| DS88 | 27.83 | 33.68 | 5.85 | 2.80888975 | 2.69058589 |
| | 27.33 | 33.41 | 6.08 | 2.39495741 | |
| | 27.72 | 33.54 | 5.82 | 2.8679105 | |
| DS89 | 28.04 | 37.2 | 9.16 | 0.28322097 | 0.42813277 |
| | 28.01 | 36.42 | 8.41 | 0.476319 | |
| | 28.04 | 36.31 | 8.27 | 0.52485834 | |
| DS90 | 24.08 | 33.39 | 9.31 | 0.25525303 | 0.23639568 |
| | 24.09 | 33.53 | 9.44 | 0.23325825 | |
| | 24.13 | 33.65 | 9.52 | 0.22067429 | |
| DS91 | 26.34 | 36.75 | 10.41 | 0.11907975 | 0.16009295 |
| | 26.49 | 36.25 | 9.76 | 0.18685616 | |
| | 26.51 | 36.37 | 9.86 | 0.17434296 | |
| DS92 | 23.55 | 34.18 | 10.63 | 0.10223776 | 0.09572549 |
| | 23.45 | 34.52 | 11.07 | 0.07536299 | |
| | 23.35 | 33.88 | 10.53 | 0.10942972 | |
| DS93 | 28.25 | 38.07 | 9.82 | 0.17924441 | 0.18518687 |
| | 28.36 | 39.4 | 11.04 | 0.07694653 | |
| | 28.26 | 37.34 | 9.08 | 0.29936968 | |
| DS94 | 24.52 | 35.54 | 11.02 | 0.0476956 | 0.06091932 |
| | 24.57 | 35.16 | 10.59 | 0.06425711 | |
| | 24.41 | 34.86 | 10.45 | 0.07080524 | |
| DS95 | 21.05 | 33.27 | 12.22 | 0.02076072 | 0.01817949 |
| | 21.01 | 33.45 | 12.44 | 0.01782443 | |
| | 21.04 | 33.64 | 12.6 | 0.01595331 | |

(continued)

| RNA inhibitor | CT value | | APOC3-GAPDH | Relative expression level | |
|---|---|---|---|---|---|
| | GAPDH | APOC3 | | 2 ^ -ΔΔCt | Mean value |
| DS96 | 26.81 | 38.08 | 11.27 | 0.04010706 | 0.06210346 |
| | 26.9 | 37.36 | 10.46 | 0.07031616 | |
| | 26.82 | 37.17 | 10.35 | 0.07588718 | |
| DS97 | 25.26 | 35.26 | 10 | 0.09672281 | 0.10383615 |
| | 25.33 | 35.51 | 10.18 | 0.08537752 | |
| | 25.6 | 35.18 | 9.58 | 0.12940812 | |
| DS98 | 27.65 | 37.03 | 9.38 | 0.14865089 | 0.16856626 |
| | 27.66 | 36.89 | 9.23 | 0.16493849 | |
| | 27.57 | 36.58 | 9.01 | 0.1921094 | |
| DS99 | 30.82 | 38.29 | 7.47 | 0.55864357 | 1.5028866 |
| | 30.65 | 37.37 | 6.72 | 0.93952275 | |
| | 30.81 | 35.85 | 5.04 | 3.01049349 | |
| DS100 | 24.46 | 34.53 | 10.07 | 0.09214183 | 0.09505216 |
| | 24.43 | 34.58 | 10.15 | 0.08717148 | |
| | 24.58 | 34.45 | 9.87 | 0.10584316 | |
| DS101 | 24.97 | 34.69 | 9.72 | 0.11744034 | 0.10968828 |
| | 25.01 | 35 | 9.99 | 0.09739557 | |
| | 25.15 | 34.91 | 9.76 | 0.11422893 | |
| DS102 | 26.75 | 37.24 | 10.49 | 0.06886907 | 0.08281502 |
| | 26.52 | 36.08 | 9.56 | 0.13121459 | |
| | 26.42 | 37.42 | 11 | 0.04836141 | |

The test results showed that the RNA inhibitors in Table 6 showed varying degrees of inhibitory effects on APOC3 mRNA expression levels in HepG2 cells at different concentrations, among which DS52-58, DS60, DS62-64, DS66-75, DS77-87, DS90-98 and DS100-102 significantly inhibited APOC3 mRNA expression levels in HepG2 cells.

**Example 2-2** Experiment 2 of RNA inhibitor to inhibit APOC3 gene expression in vitro

The sequence of the RNA inhibitor in this example is selected from Table 5 and prepared by the method described in Example 1. The RNA inhibitor HepG2 cells were seeded in a 96-well plate to spread. At the same time, the siRNA compound was transfected into the cells with a transfection reagent and the siRNA was tested at concentrations of 1 nM and 0.1nM. The cells were cultured overnight in a 37°C, 5% $CO_2$ incubator, and 3 replicates were measured in parallel. A control group was also set up.

qPCR detection of target gene mRNA expression level:

After 48 hours of transfection, RNA was extracted, and the target cDNA was detected by qPCR. GAPDH cDNA was also detected as an internal control for parallel detection. 8 μL of the prepared qPCR reaction solution and 2 μL of sample cDNA were added to 384 wells. The qPCR reaction program was: heating at 95 °C for 10 min, then going into cycle mode, heating at 95 °C for 15 sec, then 60°C for 1 min, for a total of 40 cycles. Compared with the supernatant of HepG 2 cells without intervention, the relative expression level of APOC3 mRNA in the sample intervention group was calibrated. Tested three times and took the average of the relative expression levels of APOC3. The test results obtained at concentrations of 1 nM and 0.1 nM are shown in Tables 24 and 25 below.

Table 24 Effect of RNA inhibitors on APOC3 gene inhibition in vitro (1nM)

| RNA inhibitor | relative expression level (2 ^ -ΔΔCt mean) | SD |
|---|---|---|
| Ky-12-DS69 | 0.44 | 0.01 |
| Ky-12-DS70 | 0.58 | 0.01 |
| Ky-12-DS71 | 0.75 | 0.01 |
| Ky-12-DS72 | 0.67 | 0.01 |
| Ky-12-DS73 | 0.78 | 0.04 |
| Ky-12-DS74 | 0.50 | 0.04 |
| Ky-12-DS75 | 0.64 | 0.02 |
| Ky-12-DS76 | 0.76 | 0.02 |
| Ky-12-DS77 | 0.88 | 0.01 |
| Ky-12-DS78 | 0.79 | 0.02 |
| Ky-12-DS79 | 0.67 | 0.01 |
| Ky-12-DS80 | 0.45 | 0.07 |
| Ky-12-DS81 | 0.39 | 0.04 |
| Ky-12-DS82 | 0.59 | 0.01 |
| Ky-12-DS83 | 0.68 | 0.03 |
| Ky-12-DS84 | 0.70 | 0.01 |
| Ky-12-DS85 | 0.90 | 0.09 |
| Ky-12-DS86 | 0.51 | 0.07 |
| Ky-12-DS87 | 0.53 | 0.05 |
| Ky-12-DS88 | 0.77 | 0.09 |
| Ky-12-DS89 | 0.51 | 0.05 |
| Ky-12-DS90 | 0.83 | 0.03 |
| Ky-12-DS91 | 0.84 | 0.08 |
| Ky-12-DS92 | 0.86 | 0.07 |
| Ky-12-DS93 | 0.87 | 0.06 |
| Ky-12-DS94 | 0.89 | 0.06 |
| Ky-12-DS95 | 0.75 | 0.08 |
| Ky-12-DS96 | 0.65 | 0.03 |
| Ky-12-DS97 | 0.84 | 0.05 |
| Ky-12-DS98 | 0.88 | 0.08 |
| Ky-12-DS99 | 0.78 | 0.10 |
| Ky-12-DS100 | 0.77 | 0.02 |
| Ky-12-DS101 | 0.75 | 0.02 |
| Ky-12-DS102 | 0.82 | 0.09 |
| Ky-12-DS103 | 0.90 | 0.09 |
| Ky-12-DS104 | 0.59 | 0.04 |
| Ky-12-DS105 | 0.56 | 0.04 |
| Ky-12-DS106 | 0.64 | 0.09 |
| Ky-12-DS107 | 0.58 | 0.10 |

(continued)

| RNA inhibitor | relative expression level (2 ^ -ΔΔCt mean) | SD |
|---|---|---|
| Ky-12-DS108 | 0.49 | 0.02 |
| Ky-12-DS109 | 0.94 | 0.07 |
| Ky-12-DS110 | 0.59 | 0.09 |
| Ky-12-DS111 | 0.37 | 0.05 |
| Ky-12-DS112 | 0.89 | 0.01 |
| Ky-12-DS113 | 0.69 | 0.01 |
| Ky-12-DS114 | 0.67 | 0.10 |
| Ky-12-DS115 | 0.65 | 0.02 |
| Ky-12-DS116 | 0.90 | 0.06 |
| Ky-12-DS117 | 0.94 | 0.04 |
| Ky-12-DS118 | 0.55 | 0.05 |
| Ky-12-DS119 | 0.59 | 0.04 |
| Ky-12-DS120 | 0.62 | 0.02 |
| Ky-12-DS121 | 0.96 | 0.01 |
| Ky-12-DS122 | 0.94 | 0.07 |
| Ky-12-DS123 | 0.77 | 0.06 |
| Ky-12-DS124 | 0.71 | 0.06 |
| Ky-12-DS125 | 0.80 | 0.03 |
| Ky-12-DS126 | 0.52 | 0.06 |
| Ky-12-DS127 | 0.94 | 0.03 |
| Ky-12-DS128 | 0.67 | 0.04 |
| Ky-12-DS129 | 0.52 | 0.08 |
| Ky-12-DS130 | 0.74 | 0.06 |
| Ky-12-DS131 | 0.92 | 0.02 |
| Ky-12-DS132 | 0.51 | 0.02 |
| Ky-12-DS133 | 0.23 | 0.09 |
| Ky-12-DS134 | 0.50 | 0.01 |
| Ky-12-DS135 | 0.78 | 0.09 |
| Ky-12-DS136 | 0.73 | 0.10 |
| Ky-12-DS137 | 0.62 | 0.09 |
| Ky-12-DS138 | 0.53 | 0.01 |
| Ky-12-DS139 | 0.85 | 0.04 |
| Ky-12-DS140 | 0.80 | 0.09 |
| Ky-12-DS141 | 0.34 | 0.02 |
| Ky-12-DS142 | 0.80 | 0.03 |
| Ky-12-DS143 | 0.66 | 0.03 |
| Ky-12-DS144 | 0.98 | 0.09 |
| Ky-12-DS145 | 1.00 | 0.04 |
| Ky-12-DS146 | 0.86 | 0.08 |

(continued)

| RNA inhibitor | relative expression level (2 ^ -ΔΔCt mean) | SD |
|---|---|---|
| Ky-12-DS147 | 0.95 | 0.07 |
| Ky-12-DS148 | 0.70 | 0.07 |
| Ky-12-DS149 | 0.53 | 0.02 |
| Ky-12-DS150 | 0.70 | 0.06 |
| Ky-12-DS151 | 0.97 | 0.03 |
| Ky-12-DS152 | 0.85 | 0.09 |
| Ky-12-DS153 | 0.65 | 0.05 |
| Ky-12-DS154 | 0.94 | 0.01 |
| Ky-12-DS155 | 0.97 | 0.08 |
| Ky-12-DS156 | 0.47 | 0.03 |
| Ky-12-DS157 | 0.56 | 0.02 |
| Ky-12-DS158 | 0.61 | 0.02 |
| Ky-12-DS159 | 0.56 | 0.02 |
| Ky-12-DS160 | 0.83 | 0.10 |
| Ky-12-DS161 | 0.93 | 0.06 |
| Ky-12-DS162 | 0.78 | 0.04 |
| Ky-12-DS163 | 0.30 | 0.01 |
| Ky-12-DS164 | 0.23 | 0.05 |
| Ky-12-DS165 | 0.55 | 0.11 |
| Ky-12-DS166 | 0.48 | 0.02 |
| Ky-12-DS167 | 0.54 | 0.07 |
| Ky-12-DS168 | 0.79 | 0.07 |
| Ky-12-DS169 | 0.78 | 0.03 |
| Ky-12-DS170 | 0.41 | 0.08 |
| Ky-12-DS171 | 0.46 | 0.05 |
| Ky-12-DS172 | 0.76 | 0.02 |
| Ky-12-DS173 | 0.60 | 0.08 |
| Ky-12-DS174 | 0.49 | 0.03 |
| Ky-12-DS175 | 0.97 | 0.06 |
| Ky-12-DS176 | 0.29 | 0.05 |
| Ky-12-DS177 | 0.83 | 0.07 |
| Ky-12-DS178 | 0.60 | 0.02 |
| Ky-12-DS179 | 0.40 | 0.02 |
| Ky-12-DS180 | 0.34 | 0.06 |
| Ky-12-DS181 | 0.54 | 0.06 |
| Ky-12-DS182 | 0.62 | 0.07 |
| Ky-12-DS183 | 0.60 | 0.02 |
| Ky-12-DS184 | 0.45 | 0.04 |
| Ky-12-DS185 | 0.69 | 0.02 |

(continued)

| RNA inhibitor | relative expression level (2 ^ -ΔΔCt mean) | SD |
|---|---|---|
| Ky-12-DS186 | 0.46 | 0.03 |
| Ky-12-DS187 | 0.51 | 0.01 |
| Ky-12-DS188 | 0.51 | 0.04 |
| Ky-12-DS189 | 0.42 | 0.09 |
| Ky-12-DS190 | 0.57 | 0.12 |
| Ky-12-DS191 | 0.41 | 0.08 |
| Ky-12-DS192 | 0.61 | 0.06 |
| Ky-12-DS193 | 0.53 | 0.02 |
| Ky-12-DS194 | 0.67 | 0.09 |
| Ky-12-DS195 | 0.46 | 0.04 |
| Ky-12-DS196 | 0.47 | 0.05 |
| Ky-12-DS197 | 0.41 | 0.05 |
| Ky-12-DS198 | 0.52 | 0.03 |
| Ky-12-DS199 | 0.45 | 0.07 |
| Ky-12-DS200 | 0.95 | 0.08 |
| Ky-12-DS201 | 0.56 | 0.02 |
| Ky-12-DS202 | 0.50 | 0.08 |
| Ky-12-DS203 | 0.61 | 0.07 |
| Ky-12-DS204 | 0.94 | 0.07 |
| Ky-12-DS205 | 1.00 | 0.08 |
| Ky-12-DS206 | 0.96 | 0.03 |
| Ky-12-DS207 | 0.96 | 0.10 |
| Ky-12-DS208 | 0.51 | 0.03 |
| Ky-12-DS209 | 0.72 | 0.09 |
| Ky-12-DS210 | 0.84 | 0.02 |
| Ky-12-DS211 | 0.76 | 0.02 |
| Ky-12-DS212 | 0.97 | 0.02 |
| Ky-12-DS213 | 0.89 | 0.01 |
| Ky-12-DS214 | 0.52 | 0.09 |
| Ky-12-DS215 | 0.60 | 0.10 |
| Ky-12-DS216 | 0.50 | 0.03 |
| Ky-12-DS217 | 0.94 | 0.05 |
| Ky-12-DS218 | 0.57 | 0.05 |
| Ky-12-DS219 | 0.54 | 0.04 |
| Ky-12-DS220 | 0.68 | 0.05 |
| Ky-12-DS221 | 0.68 | 0.01 |
| Ky-12-DS222 | 0.70 | 0.09 |
| Ky-12-DS223 | 0.58 | 0.03 |
| Ky-12-DS224 | 0.68 | 0.09 |

(continued)

| RNA inhibitor | relative expression level (2 ^ -ΔΔCt mean) | SD |
|---|---|---|
| Ky-12-DS225 | 0.80 | 0.09 |
| Ky-12-DS226 | 0.83 | 0.06 |
| Ky-12-DS227 | 0.57 | 0.11 |
| Ky-12-DS228 | 0.75 | 0.11 |
| Ky-12-DS229 | 1.00 | 0.07 |
| Ky-12-DS230 | 0.16 | 0.04 |
| Ky-12-DS231 | 0.46 | 0.06 |
| Ky-12-DS232 | 0.70 | 0.07 |
| Ky-12-DS233 | 0.94 | 0.06 |
| Ky-12-DS234 | 0.63 | 0.02 |
| Ky-12-DS235 | 0.94 | 0.07 |
| Ky-12-DS236 | 0.66 | 0.03 |
| Ky-12-DS237 | 0.86 | 0.08 |
| Ky-12-DS238 | 0.52 | 0.01 |
| Ky-12-DS239 | 0.66 | 0.02 |
| Ky-12-DS240 | 0.80 | 0.01 |
| Ky-12-DS241 | 0.23 | 0.01 |
| Ky-12-DS242 | 0.47 | 0.02 |
| Ky-12-DS243 | 0.95 | 0.05 |
| Ky-12-DS244 | 0.98 | 0.04 |
| Ky-12-DS245 | 0.95 | 0.11 |
| Ky-12-DS246 | 0.32 | 0.03 |
| Ky-12-DS247 | 0.29 | 0.07 |
| Ky-12-DS248 | 0.36 | 0.08 |
| Ky-12-DS249 | 0.59 | 0.05 |
| Ky-12-DS250 | 0.14 | 0.11 |
| Ky-12-DS251 | 0.35 | 0.03 |
| Ky-12-DS252 | 0.77 | 0.05 |
| Ky-12-DS253 | 0.39 | 0.06 |
| Ky-12-DS254 | 0.12 | 0.02 |
| Ky-12-DS255 | 0.49 | 0.11 |
| Ky-12-DS256 | 0.20 | 0.07 |
| Ky-12-DS257 | 0.67 | 0.02 |
| Ky-12-DS258 | 0.53 | 0.04 |
| Ky-12-DS259 | 0.42 | 0.03 |
| Ky-12-DS260 | 0.51 | 0.10 |
| Ky-12-DS261 | 0.43 | 0.02 |
| Ky-12-DS262 | 0.52 | 0.07 |
| Ky-12-DS263 | 0.53 | 0.09 |

(continued)

| RNA inhibitor | relative expression level (2 ^ -ΔΔCt mean) | SD |
|---|---|---|
| Ky-12-DS264 | 0.94 | 0.09 |
| Ky-12-DS265 | 0.85 | 0.07 |
| Ky-12-DS266 | 0.73 | 0.03 |
| Ky-12-DS267 | 0.47 | 0.06 |
| Ky-12-DS268 | 0.63 | 0.03 |
| Ky-12-DS269 | 0.34 | 0.05 |
| Ky-12-DS270 | 0.52 | 0.10 |
| Ky-12-DS271 | 0.69 | 0.06 |
| Ky-12-DS272 | 0.63 | 0.07 |
| Ky-12-DS273 | 0.91 | 0.03 |
| Ky-12-DS274 | 0.89 | 0.03 |
| Ky-12-DS275 | 0.79 | 0.05 |
| Ky-12-DS276 | 0.66 | 0.10 |
| Ky-12-DS277 | 0.88 | 0.09 |
| Ky-12-DS278 | 0.64 | 0.04 |
| Ky-12-DS279 | 0.78 | 0.04 |
| Ky-12-DS280 | 0.67 | 0.08 |
| Ky-12-DS281 | 0.47 | 0.07 |
| Ky-12-DS282 | 0.79 | 0.03 |
| Ky-12-DS283 | 0.57 | 0.03 |
| Ky-12-DS284 | 0.76 | 0.09 |
| Ky-12-DS285 | 0.45 | 0.04 |
| Ky-12-DS286 | 0.78 | 0.02 |
| Ky-12-DS287 | 0.76 | 0.04 |
| Ky-12-DS288 | 0.48 | 0.06 |
| Ky-12-DS289 | 0.78 | 0.03 |
| Ky-12-DS290 | 0.58 | 0.05 |
| Ky-12-DS291 | 0.63 | 0.02 |
| Ky-12-DS292 | 0.98 | 0.04 |
| Ky-12-DS293 | 0.82 | 0.09 |
| Ky-12-DS294 | 0.90 | 0.05 |
| Ky-12-DS295 | 0.82 | 0.05 |
| Ky-12-DS296 | 0.46 | 0.11 |
| Ky-12-DS297 | 0.19 | 0.05 |
| Ky-12-DS298 | 0.14 | 0.07 |
| Ky-12-DS299 | 0.58 | 0.05 |
| Ky-12-DS300 | 0.50 | 0.08 |
| Ky-12-DS301 | 0.98 | 0.05 |
| Ky-12-DS302 | 0.25 | 0.05 |

(continued)

| RNA inhibitor | relative expression level (2 ^ -ΔΔCt mean) | SD |
|---|---|---|
| Ky-12-DS303 | 0.94 | 0.08 |
| Ky-12-DS304 | 0.59 | 0.08 |
| Ky-12-DS305 | 0.58 | 0.01 |
| Ky-12-DS306 | 0.62 | 0.05 |
| Ky-12-DS307 | 0.95 | 0.02 |
| Ky-12-DS308 | 0.89 | 0.02 |
| Ky-12-DS309 | 0.79 | 0.04 |
| Ky-12-DS310 | 0.48 | 0.08 |
| Ky-12-DS311 | 0.90 | 0.02 |
| Ky-12-DS312 | 0.55 | 0.10 |
| Ky-12-DS313 | 0.36 | 0.05 |
| Ky-12-DS314 | 0.40 | 0.04 |
| Ky-12-DS315 | 0.37 | 0.04 |
| Ky-12-DS316 | 0.45 | 0.02 |
| Ky-12-DS317 | 0.10 | 0.05 |
| Ky-12-DS318 | 0.44 | 0.01 |
| Ky-12-DS319 | 0.53 | 0.10 |
| Ky-12-DS320 | 0.56 | 0.04 |
| Ky-12-DS321 | 0.53 | 0.02 |
| Ky-12-DS322 | 0.70 | 0.02 |
| Ky-12-DS323 | 0.66 | 0.03 |
| Ky-12-DS324 | 0.66 | 0.06 |
| Ky-12-DS325 | 0.73 | 0.02 |
| Ky-12-DS326 | 0.78 | 0.09 |
| Ky-12-DS327 | 0.54 | 0.05 |
| Ky-12-DS328 | 0.50 | 0.01 |
| Ky-12-DS329 | 0.41 | 0.06 |
| Ky-12-DS330 | 0.12 | 0.02 |

Table 25 Effect of RNA inhibitors on APOC3 gene inhibition in vitro (0.1nM)

| RNA inhibitor | relative expression level (2 ^ -ΔΔCt mean) | SD |
|---|---|---|
| Ky-12-DS74 | 0.91 | 0.05 |
| Ky-12-DS80 | 0.76 | 0.01 |
| Ky-12-DS81 | 0.70 | 0.05 |
| Ky-12-DS86 | 0.93 | 0.07 |
| Ky-12-DS89 | 0.89 | 0.06 |
| Ky-12-DS107 | 0.85 | 0.01 |
| Ky-12-DS108 | 0.81 | 0.03 |
| Ky-12-DS111 | 0.81 | 0.02 |

(continued)

| RNA inhibitor | relative expression level (2 ^ -ΔΔCt mean) | SD |
|---|---|---|
| Ky-12-DS118 | 0.80 | 0.07 |
| Ky-12-DS119 | 0.97 | 0.02 |
| Ky-12-DS126 | 0.79 | 0.08 |
| Ky-12-DS132 | 0.96 | 0.07 |
| Ky-12-DS133 | 0.70 | 0.01 |
| Ky-12-DS134 | 0.90 | 0.05 |
| Ky-12-DS141 | 0.65 | 0.09 |
| Ky-12-DS156 | 0.97 | 0.06 |
| Ky-12-DS157 | 0.96 | 0.07 |
| Ky-12-DS163 | 0.75 | 0.11 |
| Ky-12-DS164 | 0.97 | 0.01 |
| Ky-12-DS166 | 0.95 | 0.01 |
| Ky-12-DS167 | 0.87 | 0.10 |
| Ky-12-DS170 | 0.80 | 0.06 |
| Ky-12-DS171 | 0.79 | 0.10 |
| Ky-12-DS174 | 0.91 | 0.09 |
| Ky-12-DS176 | 0.96 | 0.09 |
| Ky-12-DS179 | 0.89 | 0.03 |
| Ky-12-DS180 | 0.89 | 0.04 |
| Ky-12-DS184 | 0.76 | 0.08 |
| Ky-12-DS185 | 0.95 | 0.07 |
| Ky-12-DS186 | 0.95 | 0.06 |
| Ky-12-DS187 | 0.53 | 0.08 |
| Ky-12-DS188 | 0.61 | 0.06 |
| Ky-12-DS189 | 0.73 | 0.05 |
| Ky-12-DS190 | 0.96 | 0.05 |
| Ky-12-DS192 | 0.83 | 0.04 |
| Ky-12-DS195 | 1.16 | 0.08 |
| Ky-12-DS196 | 0.74 | 0.03 |
| Ky-12-DS197 | 0.51 | 0.04 |
| Ky-12-DS199 | 0.96 | 0.09 |
| Ky-12-DS201 | 0.73 | 0.07 |
| Ky-12-DS202 | 0.68 | 0.02 |
| Ky-12-DS216 | 0.60 | 0.07 |
| Ky-12-DS223 | 0.97 | 0.03 |
| Ky-12-DS230 | 0.33 | 0.05 |
| Ky-12-DS231 | 0.90 | 0.06 |
| Ky-12-DS241 | 0.44 | 0.02 |
| Ky-12-DS242 | 0.53 | 0.08 |

(continued)

| RNA inhibitor | relative expression level (2 ^ -ΔΔCt mean) | SD |
|---|---|---|
| Ky-12-DS246 | 0.52 | 0.03 |
| Ky-12-DS247 | 0.32 | 0.10 |
| Ky-12-DS248 | 0.55 | 0.04 |
| Ky-12-DS250 | 0.31 | 0.06 |
| Ky-12-DS251 | 0.66 | 0.05 |
| Ky-12-DS253 | 0.36 | 0.01 |
| Ky-12-DS254 | 0.25 | 0.02 |
| Ky-12-DS255 | 0.55 | 0.09 |
| Ky-12-DS256 | 0.53 | 0.04 |
| Ky-12-DS259 | 0.56 | 0.05 |
| Ky-12-DS261 | 0.87 | 0.08 |
| Ky-12-DS267 | 0.64 | 0.09 |
| Ky-12-DS269 | 0.78 | 0.04 |
| Ky-12-DS284 | 0.76 | 0.02 |
| Ky-12-DS285 | 0.88 | 0.02 |
| Ky-12-DS286 | 1.23 | 0.07 |
| Ky-12-DS287 | 0.88 | 0.08 |
| Ky-12-DS288 | 0.92 | 0.03 |
| Ky-12-DS289 | 0.89 | 0.07 |
| Ky-12-DS290 | 0.69 | 0.03 |
| Ky-12-DS291 | 0.72 | 0.01 |
| Ky-12-DS292 | 0.86 | 0.07 |
| Ky-12-DS293 | 0.83 | 0.07 |
| Ky-12-DS294 | 0.59 | 0.03 |
| Ky-12-DS295 | 0.91 | 0.10 |
| Ky-12-DS296 | 0.76 | 0.04 |
| Ky-12-DS297 | 0.29 | 0.07 |
| Ky-12-DS298 | 0.28 | 0.05 |
| Ky-12-DS299 | 0.69 | 0.09 |
| Ky-12-DS300 | 0.96 | 0.02 |
| Ky-12-DS301 | 0.86 | 0.09 |
| Ky-12-DS302 | 0.95 | 0.03 |
| Ky-12-DS303 | 0.87 | 0.01 |
| Ky-12-DS304 | 0.85 | 0.09 |
| Ky-12-DS305 | 0.97 | 0.00 |
| Ky-12-DS306 | 0.60 | 0.03 |
| Ky-12-DS307 | 0.83 | 0.05 |
| Ky-12-DS308 | 0.57 | 0.02 |
| Ky-12-DS309 | 0.80 | 0.05 |

(continued)

| RNA inhibitor | relative expression level (2 ^ -ΔΔCt mean) | SD |
|---|---|---|
| Ky-12-DS310 | 0.76 | 0.07 |
| Ky-12-DS313 | 0.49 | 0.05 |
| Ky-12-DS314 | 0.50 | 0.05 |
| Ky-12-DS315 | 0.50 | 0.04 |
| Ky-12-DS316 | 0.55 | 0.04 |
| Ky-12-DS317 | 0.26 | 0.04 |
| Ky-12-DS318 | 0.56 | 0.03 |
| Ky-12-DS319 | 0.59 | 0.09 |
| Ky-12-DS320 | 0.62 | 0.06 |
| Ky-12-DS321 | 0.62 | 0.05 |
| Ky-12-DS322 | 0.62 | 0.06 |
| Ky-12-DS323 | 0.66 | 0.03 |
| Ky-12-DS324 | 0.69 | 0.17 |
| Ky-12-DS325 | 0.74 | 0.06 |
| Ky-12-DS326 | 0.78 | 0.02 |
| Ky-12-DS327 | 0.79 | 0.03 |
| Ky-12-DS328 | 0.83 | 0.07 |
| Ky-12-DS329 | 0.84 | 0.03 |
| Ky-12-DS330 | 0.31 | 0.02 |

RNA inhibitors Ky-12-DS230, Ky-12-DS250, Ky-12-DS254, Ky-12-DS297, Ky-12-DS298, Ky-12-DS317, and Ky-12-DS330 were perfectly selected for $EC_{50}$ value study. The experimental results are shown in Table 26.

Table 26 $EC_{50}$ value

| RNA inhibitor | HepG 2 cells | Huh-7 cells |
|---|---|---|
| | $EC_{50}$ (nM) | $EC_{50}$ (nM) |
| Ky-12-DS230 | 0.052 | 0.005 |
| Ky-12-DS250 | 0.020 | 0.003 |
| Ky-12-DS254 | 0.034 | 0.020 |
| Ky-12-DS297 | 0.020 | 0.002 |
| Ky-12-DS298 | 0.027 | 0.003 |
| Ky-12-DS317 | 0.022 | 0.001 |
| Ky-12-DS330 | 0.093 | 0.005 |

**Example 3 Experiment 2 of RNA inhibitor to inhibit APOC3 gene expression in vitro**

The sequences of this example were selected from Table 8. The 2' positions of the glycosyls in sequences of the sense strand and antisense strand were methoxylated or fluorinated. The modified sequences of the sense strand and antisense strand were shown in Table 6-1 and Table 7-1, respectively. Selected the corresponding sequences in Table 6-1 and Table 7-1 to anneal and synthesize RNA inhibitors.

Took the RNA inhibitor prepared by the method described in Example 1. The RNA inhibitor was transfected into HepG 2 cells to study the intervention effect of the RNA inhibitor on cells at a concentration of 1.0 nM. The APOC3 mRNA level was measure by qRT-PCR. Compare with the supernatant of HepG 2 cells without intervention, the relative

expression level of APOC3 mRNA in the sample intervention group was calibrated. The obtained test results were shown in Table 27.

Table 27 APOC3 mRNA levels in HepG2 cells after RNA inhibitor intervention

| RNA inhibitor | GAPDH | APOC3 | APOC3-GAPDH | 2 ^ -ΔΔCt | Mean value |
|---|---|---|---|---|---|
| DS103 | 21.22 | 31.59 | 10.37 | 2.143547 | 1.818354 |
| | 21.18 | 31.57 | 10.39 | 2.114036 | |
| | 21.04 | 32.25 | 11.21 | 1.197479 | |
| DS104 | 19.18 | 31.87 | 12.69 | 0.429283 | 0.43886 |
| | 19.32 | 31.61 | 12.29 | 0.566442 | |
| | 19.06 | 32.17 | 13.11 | 0.320856 | |
| DS105 | 18.9 | 31.66 | 12.76 | 0.408951 | 0.366182 |
| | 18.79 | 32.01 | 13.22 | 0.297302 | |
| | 18.97 | 31.79 | 12.82 | 0.392292 | |
| DS106 | 19.41 | 31.54 | 12.13 | 0.632878 | 0.590938 |
| | 19.56 | 32.16 | 12.6 | 0.456916 | |
| | 19.52 | 31.54 | 12.02 | 0.68302 | |
| DS107 | 18.45 | 30.72 | 12.27 | 0.574349 | 0.442755 |
| | 18.38 | 31.08 | 12.7 | 0.426317 | |
| | 18.21 | 31.29 | 13.08 | 0.327598 | |
| DS108 | 18.52 | 31.21 | 12.69 | 0.429283 | 0.426716 |
| | 18.78 | 31.23 | 12.45 | 0.50698 | |
| | 19.04 | 32.05 | 13.01 | 0.343885 | |
| DS109 | 18.75 | 31.11 | 12.36 | 0.539614 | 0.416712 |
| | 18.26 | 31.15 | 12.89 | 0.373712 | |
| | 18.41 | 31.45 | 13.04 | 0.336808 | |
| DS110 | 17.62 | 30.25 | 12.63 | 0.447513 | 0.549706 |
| | 17.25 | 30.04 | 12.79 | 0.400535 | |
| | 18.26 | 30.05 | 11.79 | 0.80107 | |
| DS111 | 17.27 | 29.9 | 12.63 | 0.447513 | 0.407824 |
| | 17.18 | 29.8 | 12.62 | 0.450625 | |
| | 17.05 | 30.14 | 13.09 | 0.325335 | |
| DS112 | 18.87 | 31.26 | 12.39 | 0.528509 | 0.768558 |
| | 19.26 | 30.79 | 11.53 | 0.959264 | |
| | 19.32 | 31.08 | 11.76 | 0.817902 | |
| | 18.17 | 29.64 | 11.47 | 1 | |
| | 18.23 | 29.97 | 11.74 | 0.82932 | |
| DS113 | 18.66 | 31.06 | 12.4 | 0.479632 | 0.468569 |
| | 18.51 | 30.72 | 12.21 | 0.547147 | |
| | 18.49 | 31.23 | 12.74 | 0.378929 | |

(continued)

| RNA inhibitor | GAPDH | APOC3 | APOC3-GAPDH | 2 ^ -ΔΔCt | Mean value |
|---|---|---|---|---|---|
| DS114 | 18.51 | 31.38 | 12.87 | 0.346277 | 0.302763 |
| | 18.67 | 31.45 | 12.78 | 0.368567 | |
| | 18.23 | 31.94 | 13.71 | 0.193446 | |
| DS115 | 18.66 | 30.83 | 12.17 | 0.562529 | 0.576077 |
| | 18.61 | 30.92 | 12.31 | 0.510506 | |
| | 18.58 | 30.53 | 11.95 | 0.655197 | |
| DS116 | 18.65 | 29.42 | 10.77 | 1.484524 | 1.295607 |
| | 18.43 | 29.68 | 11.25 | 1.06437 | |
| | 18.4 | 29.32 | 10.92 | 1.337928 | |
| DS117 | 18.53 | 29.42 | 10.89 | 1.36604 | 1.028067 |
| | 18.37 | 29.98 | 11.61 | 0.82932 | |
| | 18.39 | 29.9 | 11.51 | 0.888843 | |
| DS118 | 17.97 | 30.68 | 12.71 | 0.386891 | 0.4461 |
| | 18.05 | 30.67 | 12.62 | 0.411796 | |
| | 18.56 | 30.79 | 12.23 | 0.539614 | |
| DS119 | 19.55 | 31.09 | 11.54 | 0.870551 | 0.587075 |
| | 18.72 | 30.97 | 12.25 | 0.532185 | |
| | 18.8 | 31.62 | 12.82 | 0.358489 | |
| DS120 | 18.44 | 30.79 | 12.35 | 0.496546 | 0.592164 |
| | 18.31 | 30.8 | 12.49 | 0.450625 | |
| | 18.75 | 30.36 | 11.61 | 0.82932 | |
| DS122 | 18.38 | 31.36 | 12.98 | 0.320856 | 0.229129 |
| | 18.15 | 31.5 | 13.35 | 0.248273 | |
| | 17.78 | 32.2 | 14.42 | 0.118257 | |
| DS123 | 20.19 | 31.08 | 10.89 | 1.36604 | 1.335014 |
| | 20.3 | 31.08 | 10.78 | 1.474269 | |
| | 20.44 | 31.56 | 11.12 | 1.164734 | |
| DS124 | 18.65 | 30.81 | 12.16 | 0.429283 | 0.436352 |
| | 18.71 | 30.55 | 11.84 | 0.535887 | |
| | 18.58 | 31.06 | 12.48 | 0.343885 | |
| DS125 | 19.39 | 30.01 | 10.62 | 1.248331 | 1.396799 |
| | 19.57 | 29.78 | 10.21 | 1.658639 | |
| | 19.32 | 29.9 | 10.58 | 1.283426 | |
| DS126 | 20.26 | 31.57 | 11.31 | 0.773782 | 0.712237 |
| | 20.21 | 31.5 | 11.29 | 0.784584 | |
| | 20.33 | 32.06 | 11.73 | 0.578344 | |
| DS127 | 18.05 | 31.16 | 13.11 | 0.222211 | 0.262199 |
| | 18.32 | 30.74 | 12.42 | 0.358489 | |
| | 18.02 | 31.24 | 13.22 | 0.205898 | |

(continued)

| RNA inhibitor | GAPDH | APOC3 | APOC3-GAPDH | 2 ^ -ΔΔCt | Mean value |
|---|---|---|---|---|---|
| DS128 | 19.98 | 31.35 | 11.37 | 0.742262 | 0.708325 |
| | 19.77 | 31.16 | 11.39 | 0.732043 | |
| | 19.74 | 31.3 | 11.56 | 0.650671 | |
| DS129 | 19.37 | 31.2 | 11.83 | 0.539614 | 0.600899 |
| | 19.58 | 31.11 | 11.53 | 0.664343 | |
| | 19.84 | 31.52 | 11.68 | 0.598739 | |
| DS130 | 19.92 | 31.12 | 11.2 | 0.835088 | 0.582446 |
| | 19.18 | 31.17 | 11.99 | 0.482968 | |
| | 19.35 | 31.51 | 12.16 | 0.429283 | |
| DS131 | 18.78 | 31.86 | 13.08 | 0.22688 | 0.346228 |
| | 18.72 | 31.25 | 12.53 | 0.332171 | |
| | 19.15 | 31.15 | 12 | 0.479632 | |
| DS132 | 18.93 | 32.21 | 13.28 | 0.19751 | 0.188774 |
| | 18.65 | 32.26 | 13.61 | 0.157127 | |
| | 18.65 | 31.83 | 13.18 | 0.211686 | |

The RNA inhibitors DS104-105, DS107-109, DS111, DS113-114, DS118, DS122, DS124, DS127, DS131 and DS132 were selected for further activity studies.

**Example 4 Experiment 3 of RNA inhibitor to inhibit APOC3 gene expression in vitro**

The sequences of this example were selected from Table 8. The 2' positions of the glycosyls in sequences of the sense strand and antisense strand were methoxylated or fluorinated. The modified sequences of the sense strand and antisense strand were shown in Table 6-1 and Table 7-1, respectively. The corresponding sequences in Table 6-1 and Table 7-1 were annealed to synthesize RNA inhibitors. Investigation of the effect of RNA inhibitors on APOC3 in cells at a low concentration of 0.1 nM. Took the RNA inhibitor prepared by the method described in Example 1. The RNA inhibitor was transfected into HepG 2 cells and the APOC3 mRNA level was measure by qRT-PCR. Compare with the supernatant of HepG 2 cells without intervention, the relative expression level of APOC3 mRNA in the sample intervention group was calibrated. The obtained test results were shown in Table 28.

Table 28

| RNA inhibitor | GAPDH | APOC3 | APOC3-GAPDH | 2 ^ -ΔΔCt | Mean value |
|---|---|---|---|---|---|
| DS104 | 15.88 | 30.31 | 14.43 | 0.939522749 | 1.043193741 |
| | 16.27 | 30.21 | 13.94 | 1.319507911 | |
| | 15.77 | 30.31 | 14.54 | 0.870550563 | |
| DS105 | 15.64 | 30.4 | 14.76 | 0.747424624 | 0.675779913 |
| | 15.49 | 30.53 | 15.04 | 0.615572207 | |
| | 15.47 | 30.4 | 14.93 | 0.664342907 | |
| DS107 | 15.2 | 30.42 | 15.22 | 0.543367431 | 0.607211557 |
| | 15.66 | 30.54 | 14.88 | 0.687770909 | |
| | 15.52 | 30.62 | 15.1 | 0.590496331 | |

(continued)

| RNA inhibitor | GAPDH | APOC3 | APOC3-GAPDH | 2 ^ -ΔΔCt | Mean value |
|---|---|---|---|---|---|
| DS108 | 15.3 | 31.36 | 16.06 | 0.303548721 | 0.269799651 |
| | 15.84 | 32.03 | 16.19 | 0.277392368 | |
| | 16.01 | 32.48 | 16.47 | 0.228457863 | |
| DS109 | 15.81 | 30.86 | 15.05 | 0.611320139 | 0.617098626 |
| | 15.55 | 30.61 | 15.06 | 0.607097442 | |
| | 15.35 | 30.35 | 15 | 0.632878297 | |
| DS111 | 14.62 | 29.75 | 15.13 | 0.578344092 | 0.656424156 |
| | 14.66 | 29.58 | 14.92 | 0.668963777 | |
| | 14.92 | 29.73 | 14.81 | 0.721964598 | |
| DS113 | 15.3 | 30.77 | 15.47 | 0.456915725 | 0.521149998 |
| | 15.35 | 30.49 | 15.14 | 0.574349177 | |
| | 15.34 | 30.59 | 15.25 | 0.532185091 | |
| DS114 | 14.99 | 31.04 | 16.05 | 0.305660069 | 0.304330291 |
| | 15.23 | 31.25 | 16.02 | 0.312082637 | |
| | 15.29 | 31.39 | 16.1 | 0.295248165 | |
| DS118 | 16.17 | 31.04 | 14.87 | 0.692554734 | 0.823958851 |
| | 15.84 | 31.07 | 15.23 | 0.539614118 | |
| | 15.85 | 29.88 | 14.03 | 1.2397077 | |
| DS122 | 15.23 | 29.8 | 14.57 | 0.852634892 | 0.770469348 |
| | 15.35 | 30.6 | 15.25 | 0.532185091 | |
| | 15.98 | 30.43 | 14.45 | 0.926588062 | |
| DS124 | 14.84 | 31.13 | 16.29 | 0.258816231 | 0.280412995 |
| | 14.95 | 31.05 | 16.1 | 0.295248165 | |
| | 15.17 | 31.31 | 16.14 | 0.287174589 | |
| DS127 | 15.22 | 30.14 | 14.92 | 0.668963777 | 0.71356103 |
| | 15.81 | 30.51 | 14.7 | 0.77916458 | |
| | 15.72 | 30.59 | 14.87 | 0.692554734 | |
| DS131 | 15.5 | 31.36 | 15.86 | 0.348685917 | 0.35881844 |
| | 15.37 | 30.94 | 15.57 | 0.426317446 | |
| | 15.34 | 31.41 | 16.07 | 0.301451957 | |
| DS132 | 15.5 | 32.05 | 16.55 | 0.216134308 | 0.242261641 |
| | 15.48 | 31.92 | 16.44 | 0.233258248 | |
| | 15.44 | 31.63 | 16.19 | 0.277392368 | |

Results: DS107, DS108, DS113, DS114, DS124, DS131 and DS132 were further selected for the next $EC_{50}$ test.

**Example 5 Experiment of RNA inhibitor to inhibit APOC3 gene expression in vitro**

This example detects the $EC_{50}$ values of DS107, DS108, DS113, DS114, DS124, DS131 and DS132.
The RNA inhibitor was transfected into HepG 2 cells to study the intervention effect of the RNA inhibitor on cells at a concentration of 10 nM, 5 nM, 2 nM, 1 nM, 0.5 nM, 0.1 nM, 0.05 nM and 0.01 nM. The APOC3 mRNA level was measure by qRT-PCR. $EC_{50}$ was calculated and the test results were shown in Table 29.

Table 29 $EC_{50}$ value of RNA inhibitor

| RNA inhibitor | Absolute $EC_{50}$ (nM) |
|---|---|
| DS107 | 0.035 |
| DS108 | <0.01 |
| DS113 | <0.01 |
| DS114 | 0.029 |
| DS124 | 0.042 |
| DS131 | 0.018 |
| DS132 | 0.019 |

DS107, DS108, DS113, DS114, DS124, DS131 and DS132 were selected for the next step of conjugating with 5'MVIP/3'MVIP.

**Example 6 Synthesis of 5'MVIP and 3'MVIP compounds**

When the 3' end of the sense strand or antisense strand of the RNA inhibitor of the present invention is conjugated with a carrier structure 3'MVIP, the Solid Support of 3'MVIP is used as the starting monomer for solid-phase synthesis. When the 5' end of the sense strand or antisense strand of the RNA inhibitor of the present invention is conjugated with a carrier structure 5'MVIP, the 5'MVIP phosphoramidite monomer is used as the last monomer for solid-phase synthesis.

When the 3' end of the sense strand or antisense strand of the RNA inhibitor of the present invention is conjugated with 3'MVIP, the solid support of 3'MVIP is used as the starting monomer for solid-phase synthesis. The general formula of solid support of 3'MVIP is as follows:

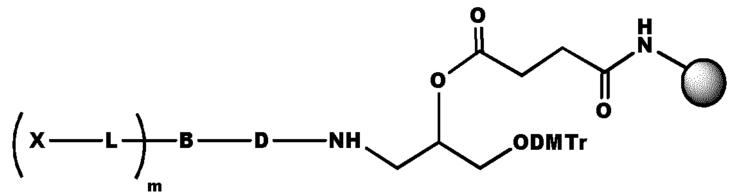

When m is 1-4, the linker B part in the general formula is branched for 1 to 4 times respectively to obtain the corresponding Solid Support of 3'MVIP.

For example, when m is 1, the obtained Solid Support serves as the starting monomer for solid-phase synthesis of the sense strand of the RNA inhibitors Kylo-12-DS134~ Kylo-12-DS136, Kylo-12-DS142, Kylo-12-DS145, Kylo-12-DS146, Kylo-12-DS157 and Kylo-12-DS158 and the antisense strand of Kylo-12-DS131~ Kylo-12-DS133, Kylo-12-DS139, Kylo-12-DS140, Kylo-12-DS149, Kylo-12-DS150 and other RNAinhibitors described in the present invention;

when m is 2, the obtained Solid Support serves as the starting monomer for solid-phase synthesis of the sense strand of the RNA inhibitors Kylo-12-DS141, Kylo-12-DS143, Kylo-12-DS144, Kylo-12-DS154~Kylo-12-DS156, Kylo-12-DS166, Kylo-12-DS169 and Kylo-12-DS170 and the antisense strand of Kylo-12-DS1081, Kylo-12-DS137, Kylo-12-DS138, Kylo-12-DS151~ Kylo-12-DS153, Kylo-12-DS163, Kylo-12-DS164 and other RNA inhibitors described in the present invention;

when m is 3, the obtained Solid Support serves as the starting monomer for solid-phase synthesis of the sense strand of the RNA inhibitors Kylo-12-DS159, Kylo-12-DS160, Kylo-12-DS165, Kylo-12-DS167, Kylo-12-DS168, Kylo-12-DS174 and Kylo-12-DS175 and the antisense strand of Kylo-12-DS147, Kylo-12-DS148, Kylo-12-DS161, Kylo-12-DS162, Kylo-12-DS171, Kylo-12-DS172 and other RNA inhibitors described in the present invention.

When the 5' end of the sense strand or antisense strand of the RNAinhibitor of the present invention has 5'MVIP, the 5'MVIP phosphoramidite monomer is the last phosphoramidite monomer for solid-phase synthesis of the sense strand or antisense strand. The general formula of 5'MVIP phosphoramidite monomer is as follows:

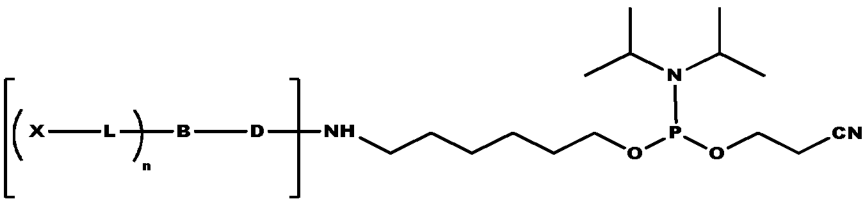

When n is 1-4, the linker B part in the general formula is branched for 1 to 4 times respectively to obtain the corresponding 5'MVIP phosphoramidite monomer.

For example, when n is 1, the resulting 5'MVIP phosphoramidite monomer is used as the last monomer for solid-phase synthesis of the sense strand of RNA inhibitors Kylo-12-DS131~ Kylo-12-DS134, Kylo-12-DS137, Kylo-12-DS138, Kylo-12-DS141, Kylo-12-DS147, Kylo-12-DS148 and other RNA inhibitors described in the present invention and the antisense strand of Kylo-12-DS135, Kylo-12-DS136, Kylo-12-DS143, Kylo-12-DS144, Kylo-12-DS159, Kylo-12-DS160 and other RNA inhibitors described in the present invention.

When n is 2, the resulting 5'MVIP phosphoramidite monomer is used as the last monomer for solid-phase synthesis of the sense strand of Kylo-12-DS1081, Kylo-12-DS139, Kylo-12-DS140, Kylo-12-DS142, Kylo-12-DS151~Kylo-12-DS154, Kylo-12-DS161, Kylo-12-DS162, Kylo-12-DS165 and other RNA inhibitors described in the present invention and the antisense strand of Kylo-12-DS145, Kylo-12-DS146, Kylo-12-DS155, Kylo-12-DS156, Kylo-12-DS167, Kylo-12-DS168 and other RNA inhibitors described in the present invention.

When n is 3, the resulting 5'MVIP phosphoramidite monomer with three liver targeting specific ligands X is used as the last monomer for solid-phase synthesis of the sense strand of Kylo-12-DS149, Kylo-12-DS150, Kylo-12-DS163, Kylo-12-DS164, Kylo-12-DS166, Kylo-12-DS171~Kylo-12-DS173 and other RNA inhibitors described in the present invention and the antisense strand of Kylo-12-DS157, Kylo-12-DS158, Kylo-12-DS169, Kylo-12-DS170, Kylo-12-DS174, Kylo-12-DS175 and other RNA inhibitors described in the present invention.

The above is only an exemplary list of some RNA inhibitors. This rule is also applicable to the RNAinhibitors described in the present invention but not listed, that is, when the 3' end of the sense strand or antisense strand of the RNA inhibitor of the present invention is conjugated with the carrier structure 3'MVIP, the solid support of 3'MVIP is used as the starting monomer for solid phase synthesis. When the 5' end of the sense strand or antisense strand of the RNAinhibitor of the present invention is conjugated with the carrier structure 5'MVIP, the 5'MVIP phosphoramidite monomer is used as the last monomer of solid phase synthesis.

Before the phosphoramidite solid-phase synthesis of the sense and antisense strands of these RNA inhibitors described in the present invention, the corresponding 3'MVIP Solid Support and 5'MVIP phosphoramidite monomers need to be chemically synthesized first.

This example only provides an exemplary chemical synthesis process of several 3'MVIP Solid Supports and 5'MVIP phosphoramidite monomers of the RNA inhibitor of the present invention. Those skilled in the art can easily synthesize other 3'MVIP Solid Support and 5'MVIP phosphoramidite monomers not listed herein. The synthesis process is described as follows:

### 4.1 Synthesis of Solid Support of 3'MVIP

#### 4.1.1 Synthesis of Solid Support of 3'MVIP09

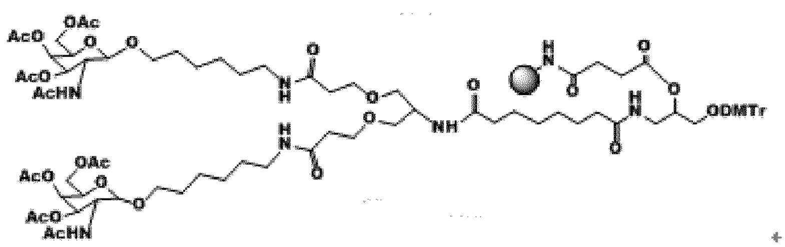

Solid Support of 3'MVIP09

Description of synthesis process:

4.1.1.1 Synthesis of ERC-01-c1

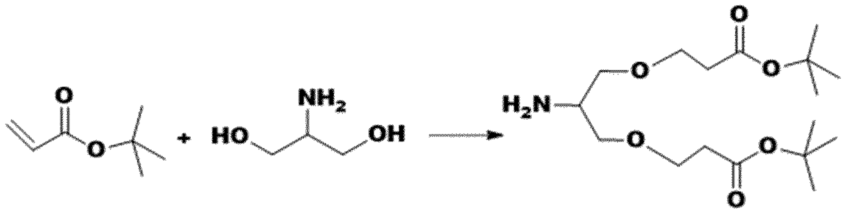

tert-butyl acrylate 2-amino-1,3-propanediol ERC-01-c1

Weighed 2-amino-1,3-propanediol (5.0g, 54.9mmol) and added 50ml of DMSO, 5ml of sodium hydroxide solution (1g/ml), cooled it down to 0°C, added tert-butyl acrylate (20ml, 137.8mol) dropwise for 2 hours, let it react at room temperature for 48h, added petroleum ether (100ml), washed twice with saturated salt water, and dried the organic layer. Passed it through a chromatography column (eluent: ethyl acetate: petroleum ether = 25%-75%), added 0.05% triethylamine to the column, and obtained 6.2g of colorless oil.

4.1.1.2 Synthesis of ERC-01-c2

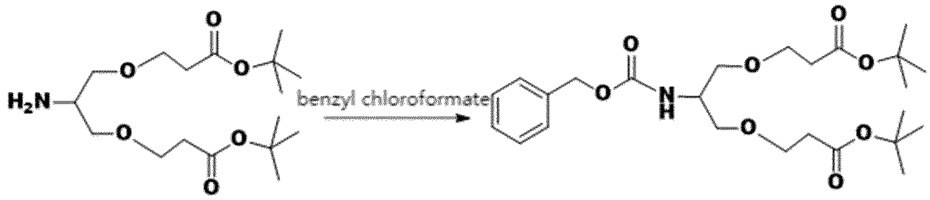


**ERC-01-c1** **ERC-01-c2**

Weighed ERC-01-c1 (6.2g, 17.9mmol), added 50ml of dichloromethane, 23ml of sodium carbonate solution (25%), added benzyl chloroformate (8.2ml, 57.4mmol) dropwise at room temperature for 2 hours, let it react overnight at room temperature, washed with saturated salt water for three times, dried with anhydrous sodium sulfate, evaporated the solvent, passed it through a chromatography column (ethyl acetate: petroleum ether =5%-30%), and obtained 4.0g of oil.

4.1.1.3 Synthesis of ERC-01-c3

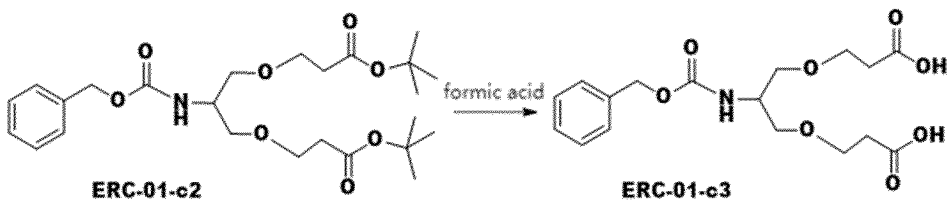


**ERC-01-c2** **ERC-01-c3**

Weighed ERC-01-c2 (4.0g, 8.3mmol), added 12ml of formic acid, let it react overnight at room temperature, evaporated the solvent under reduced pressure to obtain 2.8g of product.

4.1.1.4 Synthesis of ERCd-01-c1

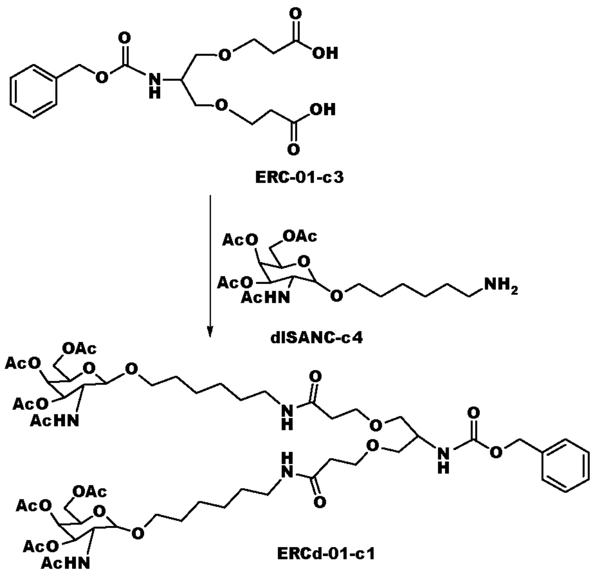

ERC-01-c3

dISANC-c4

ERCd-01-c1

Added compounds ERC-01-c3 (1.11g, 3.0mmol) and dISANC-c4 (3.6g, 8.04mmol) to DMF (60 ml), then added HOBt (2.24g) and HBTU (3.36g), and then slowly added DIEA (4.16ml). Stirred the reaction solution for 3 hours at room temperature. Then added water and extracted the aqueous layer with dichloromethane (2x10ml). Combined the organic layers, and then washed with saturated sodium bicarbonate (80 ml), water (2x60 ml), and saturated salt water (60ml) successively. Dried with anhydrous sodium sulfate, evaporated to dryness under reduced pressure, and purified by silica gel column chromatography (eluent: 3-15% MeOH in DCM). Obtained light yellow solid 3.24g.

4.1.1.5 Synthesis of ERCd-01-c2

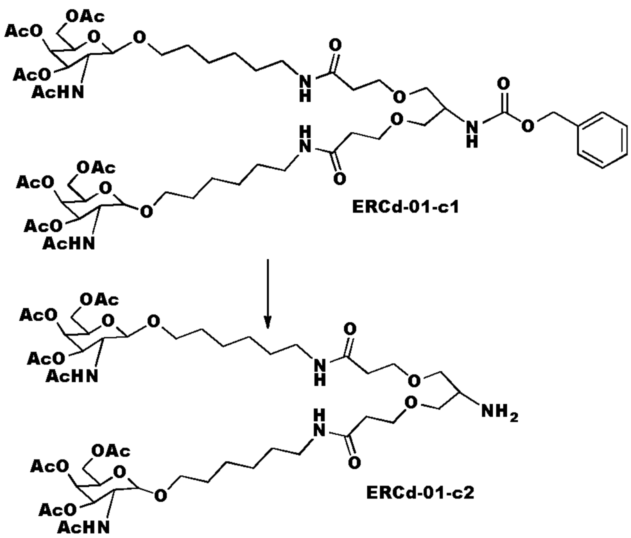

ERCd-01-c1

ERCd-01-c2

Dissolved ERCd-01-c1 (3.24g, 2.6mmol) in methanol (60ml), and added 10% palladium carbon (0.3g) and acetic acid (2.0ml). Then introduced hydrogen at atmospheric pressure to react overnight. The reaction solution was filtered with diatomite, and the filtrate was evaporated to dryness under reduced pressure to obtain 2.9g of oil ERCd-01-c2.

4.1.1.6 Synthesis of 3'MVIP09-c1

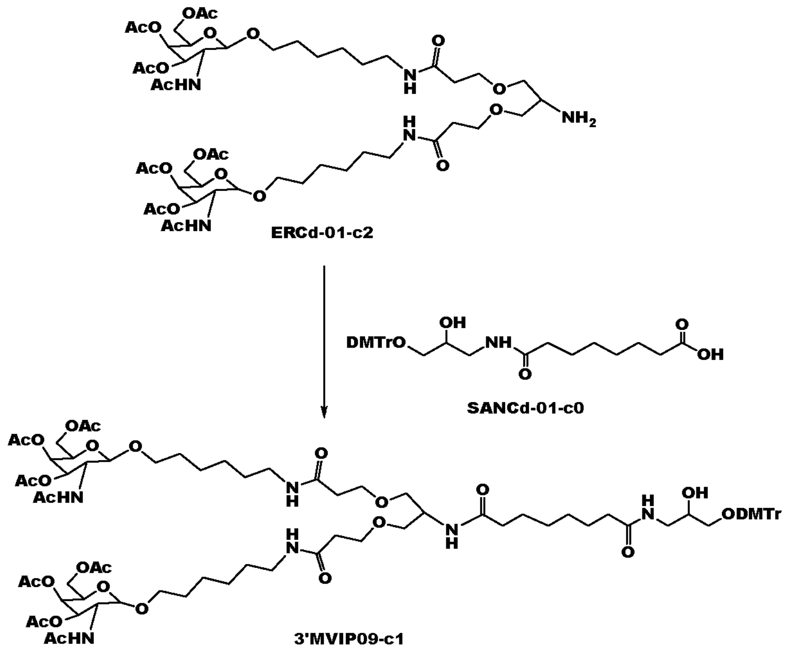

ERCd-01-c2

SANCd-01-c0

3'MVIP09-c1

Successively added SANCd-01-c0 (0.824g, 1.5mmol) and ERCd-01-c2 (1.09g, 1.0mmol) into a vial, then added 10ml of DCM, stirred until dissolved, then successively added TBTU (0.963g) and DIPEA (0.517g), let is react overnight, added water, extracted with DCM, washed the organic phase with saturated salt water, dried, filtered and concentrated, and finally purified by a silica gel column to obtain 1.3g of product.

4.1.1.7 Synthesis of 3'MVIP09-c2

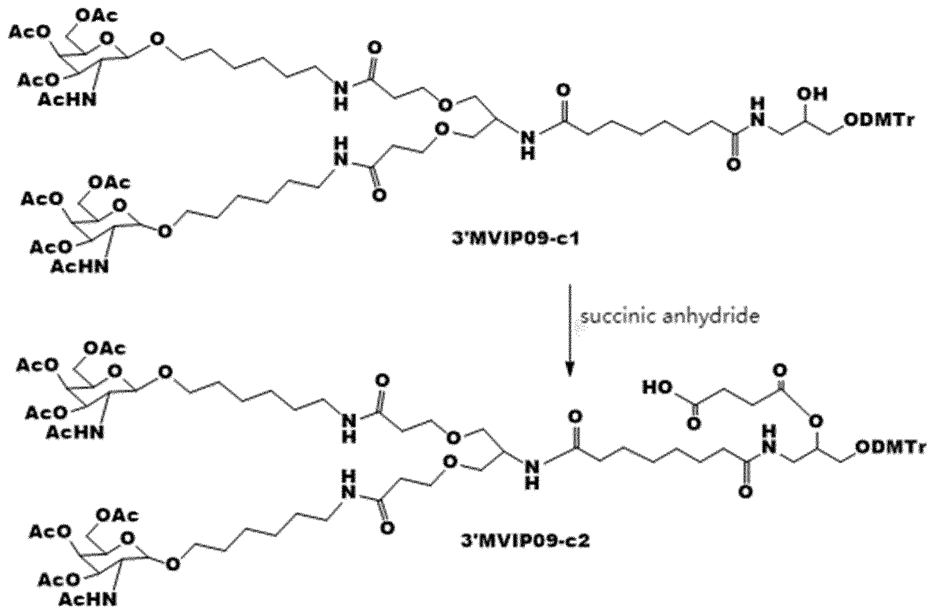


3'MVIP09-c1

3'MVIP09-c2

Added 3'MVIP09-c1 (1.62g, 1 μmol) and 10ml of DCM into a vial in turn, stirred at room temperature until dissolved, then added DMAP (0.366g) and succinic anhydride (0.2g, 3 μmol) in turn, stirred at room temperature to react. Concentrated DCM after the reaction is validated by TLC analysis, added water, extracted with DCM, washed the organic phase with saturated salt water, dried the organic phase by anhydrous sodium sulfate, filtered and concentrated, and finally purified by a silica gel column to obtain 1.55g product.

4.1.1.8 Synthesis of Solid Support of 3'MVIP09

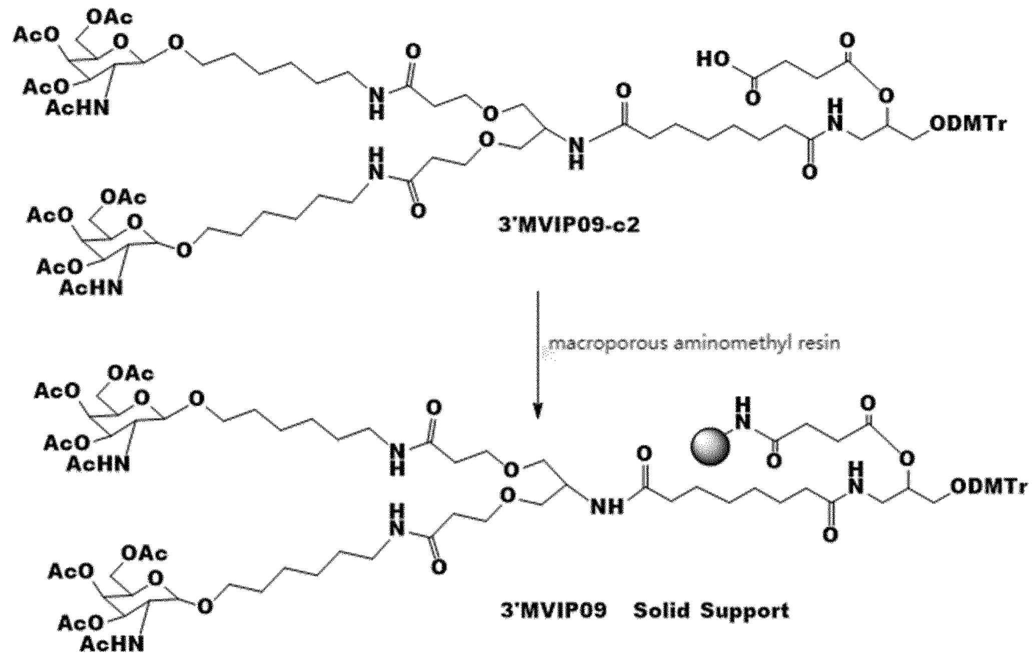


3'MVIP09-c2

3'MVIP09 Solid Support

Added 3'MVIP09-c2 (0.86g, 0.5 μmol) and 10ml DMF into a vial in turn, after dissolution, added HBTU (0.19g), DIPEA (0.194g) and macroporous aminomethyl resin (2.0g) in turn, placed on the shaker for 24h, filtered, washed the resin with 10% methanol/DCM, and then used 25% acetic acid/pyridine for end capping, with a degree of substitution of 150 μmol/g.

4.1.2 Synthesis of Solid Support of 3'MVIP17

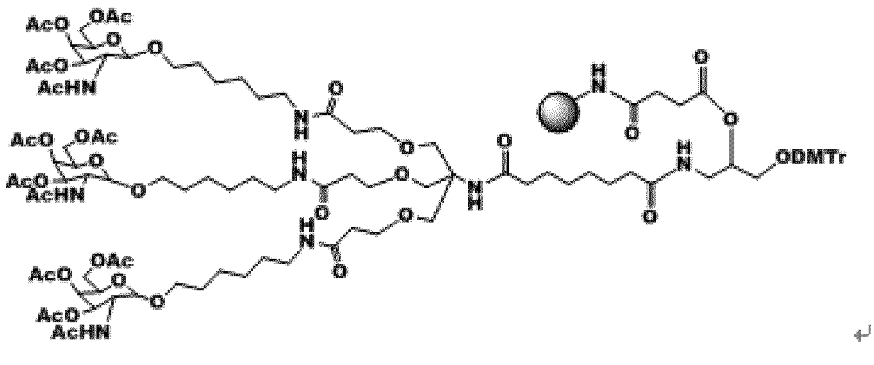

3'MVIP17 Solid Support

4.1.2.1 Synthesis of SANC-01-c1

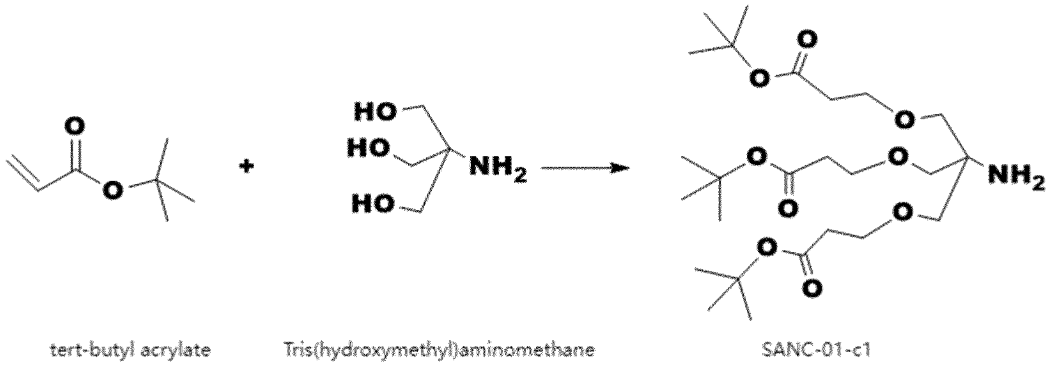


The synthesis steps are referred to 4.1.1.1 synthesis of ERC-01-c1.

4.1.2.2 Synthesis of SANC-01-c2

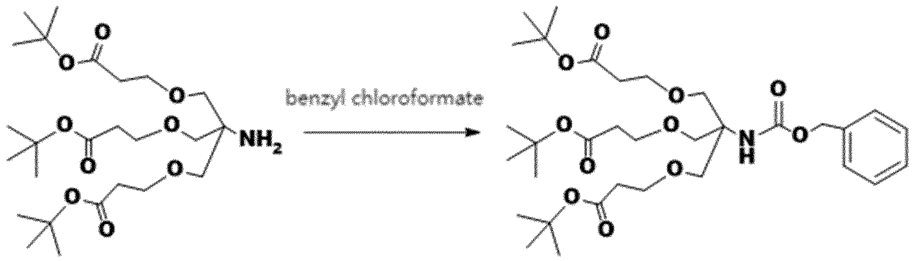


**SANC-01-c1** **SANC-01-c2**

The synthesis steps are referred to 4.1.1.2. synthesis of ERC-01-c2.

4.1.2.3 Synthesis of SANC-01-c3

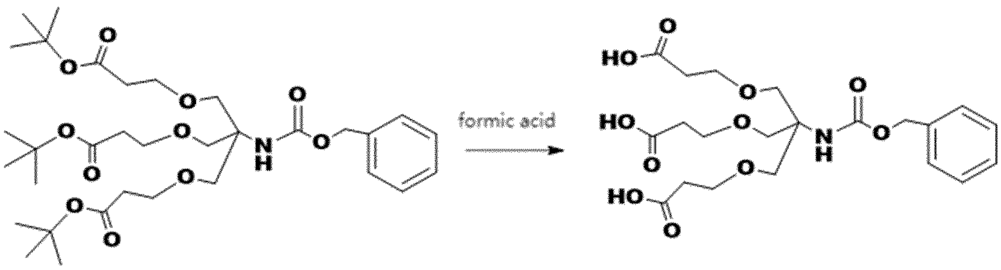


**SANC-01-c3**

**SANC-01-c2**

The synthesis steps are referred to 4.1.1.3. Synthesis of ERC-01-c3.

4.1.2.4 Synthesis of SANCd-01-c1

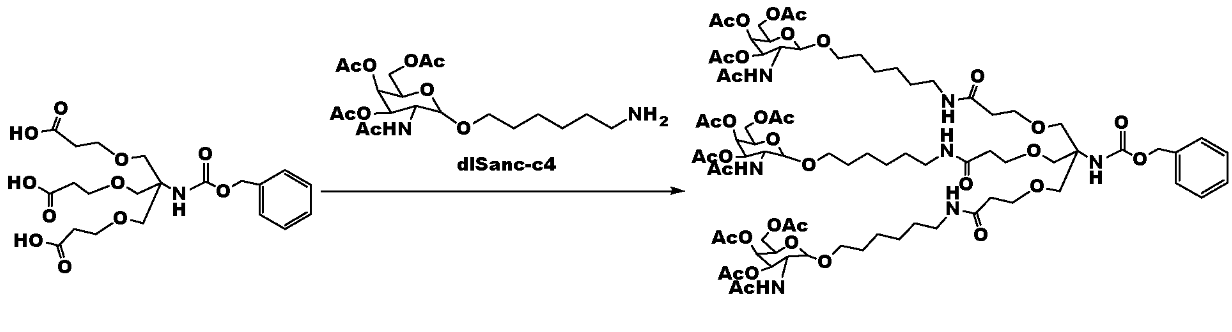


SANC-01-c3 SANCd-01-c1

The synthesis steps are referred to 4.1.1.4. Synthesis of ERCd-01-c1.

4.1.2.5 Synthesis of SANCd-01-c2

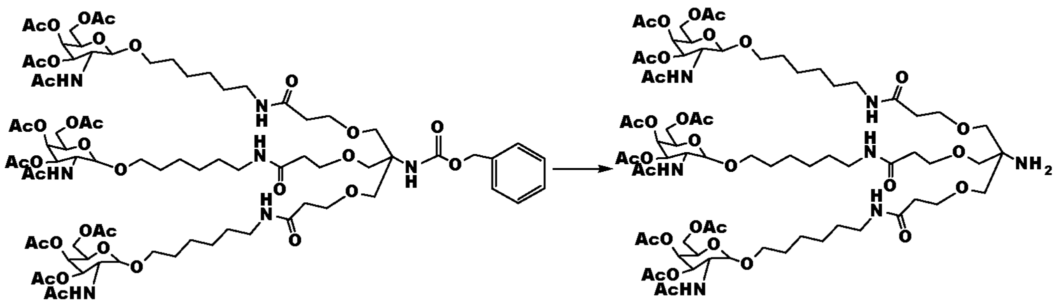

SANCd-01-c1 SANCd-01-c2

The synthesis steps are referred to 4.1.1.5. Synthesis of ERCd-01-c2.

4.1.2.6 Synthesis of 3'MVIP17-c1

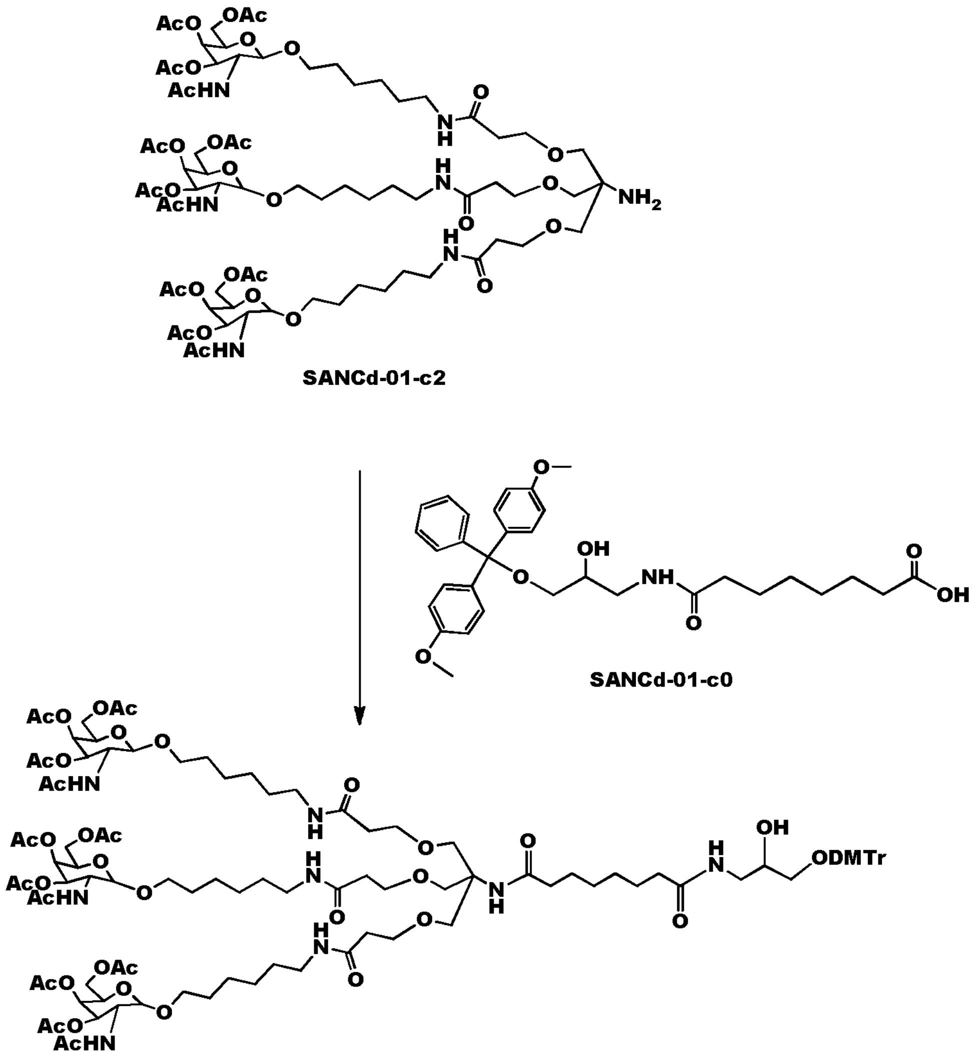

SANCd-01-c2

SANCd-01-c0

3'MVIP17-c1

The synthesis steps are referred to 4.1.1.6. Synthesis of 3'MVIP09-c1, 3'MVIP17-c1 was synthesized.

4.1.2.7 Synthesis of 3'MVIP17-c2

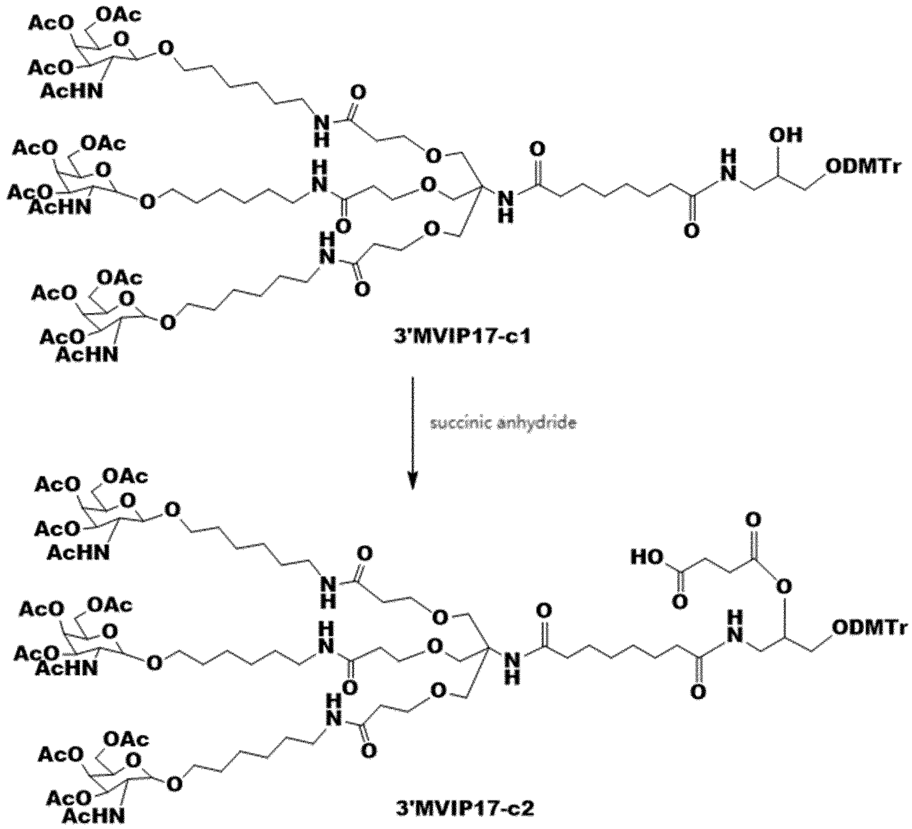


3'MVIP17-c1

3'MVIP17-c2

The synthesis steps are referred to 4.1.1.7. Synthesis of 3'MVIP09-c2.

4.1.2.8 Synthesis of Solid Support of 3'MVIP17

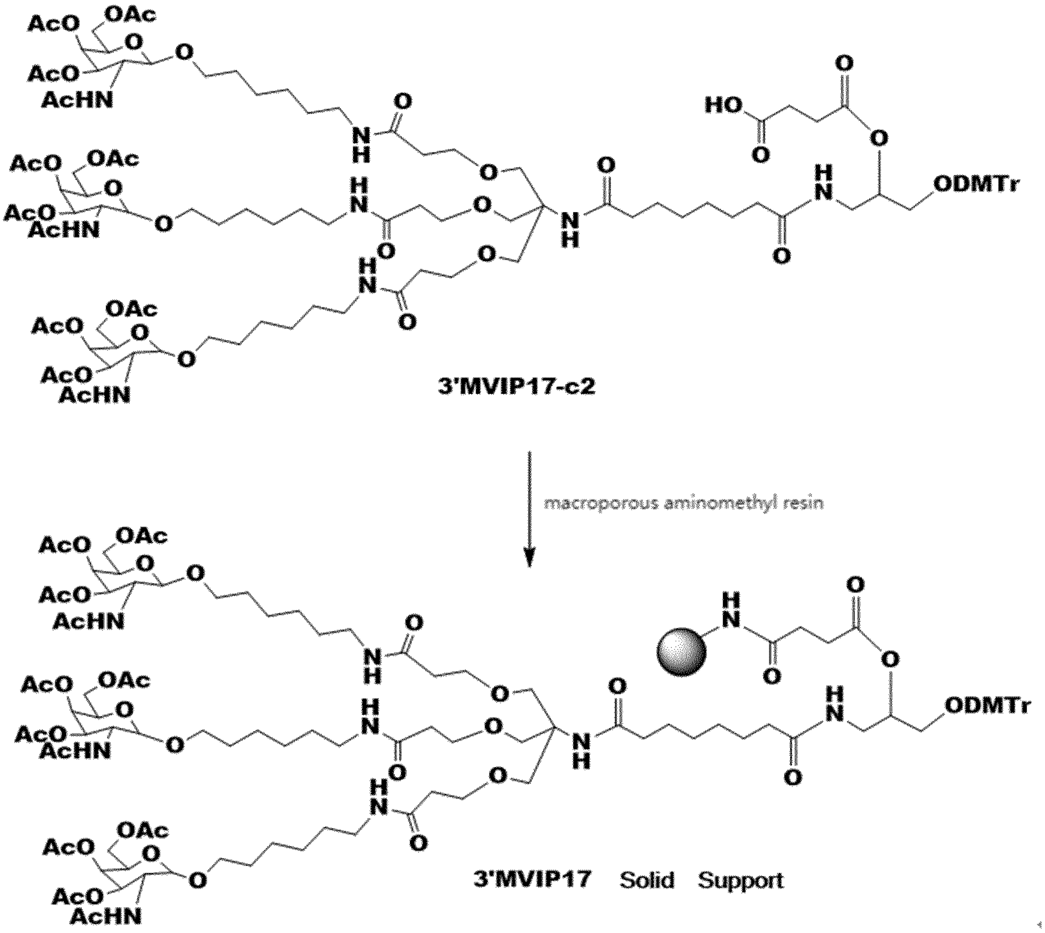


3'MVIP17-c2

3'MVIP17 Solid Support

The synthesis steps are referred to 4.1.1.8 Synthesis of Solid Support of 3'MVIP09.

4.1.3 Synthesis of Solid Support of 3'MVIP01:

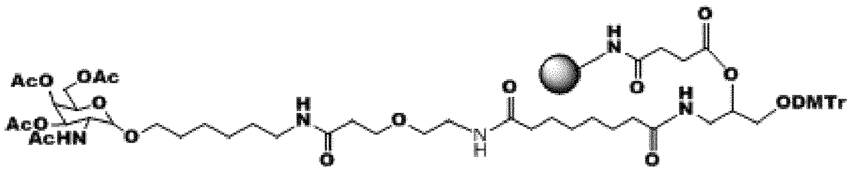

3'MVIP01 Solid Support

Description of synthesis process:

4.1.3.1 Synthesis of 3'MVIP01-c1

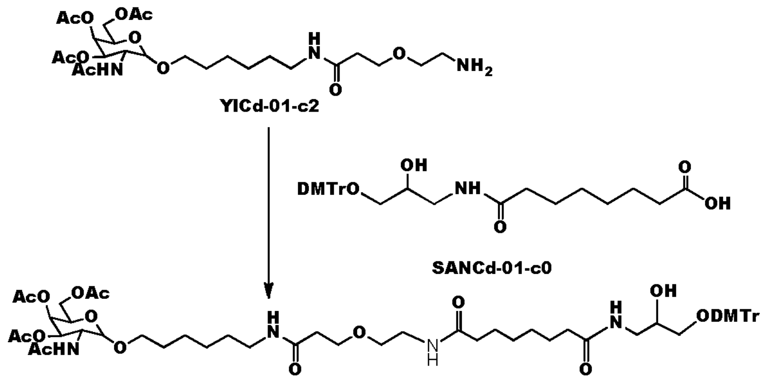


3'MVIP01-c1

The synthesis steps are referred to 4.1.1.6. synthesis of 3'MVIP09-c1.

4.1.3.2 Synthesis of 3'MVIP01-c2

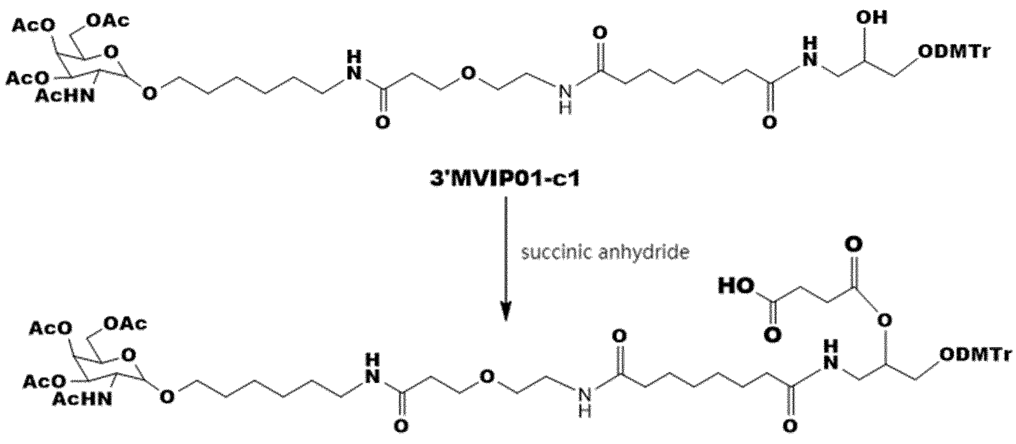


3'MVIP01-c2

The synthesis steps are referred to5.1.1.7. synthesis of 3'MVIP09-c2.

4.1.3.3 Synthesis of Solid Support of 3'MVIP01

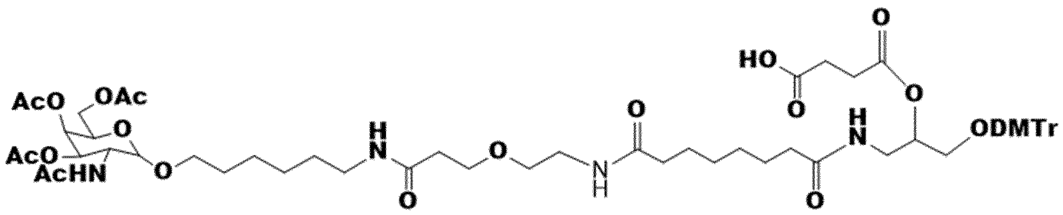

3'MVIP01-c2

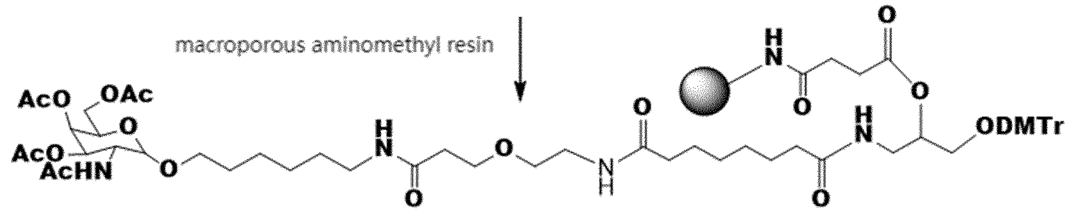


3'MVIP01 Solid Support

The synthesis steps are referred to4.1.1.8. synthesis of Solid Support of 3'MVIP09.

4.2. Synthesis of 5'MVIP phosphoramidite monomer

4.2.1 Synthesis of 5'MVIP09 phosphoramidite monomer:

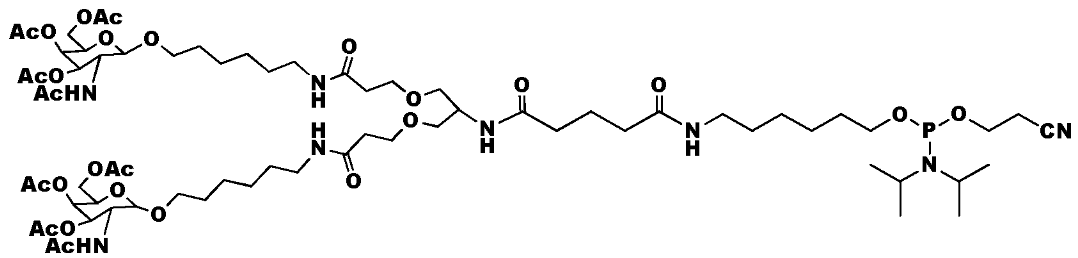

5'MVIP09 phosphoramidite monomer

4.2.1.1 Synthesis of 5'MVIP09-ERCd-PFP-c1

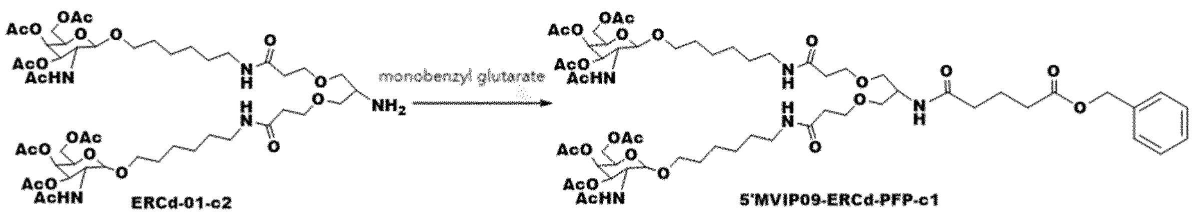


ERCd-01-c2 5'MVIP09-ERCd-PFP-c1

Weighed ERCd-01-c2 (2.18g, 2.0mmol) to dissolve in DMF (50ml), added monobenzyl glutarate (0.53g, 2.4mmol), DIPEA (0.78g) and TBTU (0.84g), stirred at room temperature overnight, added water to quench (50ml), extracted with DCM (30ml*3), washed with 10% citric acid (50ml*3), 50ml saturated sodium bicarbonate and 100ml pyridine, dried with anhydrous sodium sulfate, filtered, conducted rotary evaporation, and purified by a column to obtain the product 5'MVIP09-ERCd-PFP-c1 (2.15g).

4.2.1.2 Synthesis of 5'MVIP09-ERCd-PFP-c2

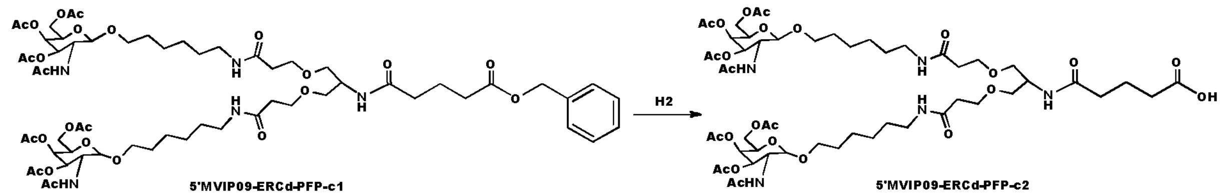


Weighed 5'MVIP09-ERCd-PFP-c1 (2.15g, 1.66mmol) and 10% palladium carbon (0.21g), added methanol (50ml), and hydrogenated overnight with stirring at room temperature, filtered palladium carbon with diatomite after the reaction, conducted rotary evaporation to obtain 5'MVIP09-ERCd-PFP-c2 crude product (1.9g).

4.2.1.3 Synthesis of 5'MVIP09-ERCd-PFP

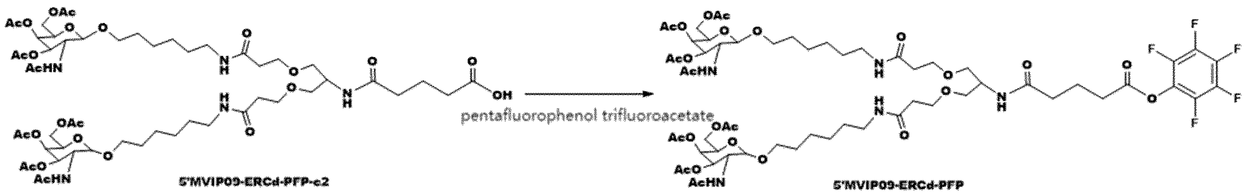


Weighed 5'MVIP09-ERCd-PFP-c2 crude product (1.9g, 1.58mmol) to dissolve in DCM (60ml), added DIPEA (1.33g), cooled, added pentafluorophenol trifluoroacetate (2.21g, 7.9mmol), let it react for 2h with stirring at room temperature, then conducted rotary evaporation, redissolved in DCM (60ml), washed with saturated sodium bicarbonate (30ml*3), 10% citric acid (30ml*1), saturated salt water (50ml*1), dried with anhydrous sodium sulfate, filtered, conducted rotary evaporation to obtain 5'MVIP09-ERCd-PFP crude product (2.35g). After being drained, it was directly used for the next reaction without purification.

4.2.1.4 Synthesis of 5'MVIP09 phosphoramidite monomer-c1

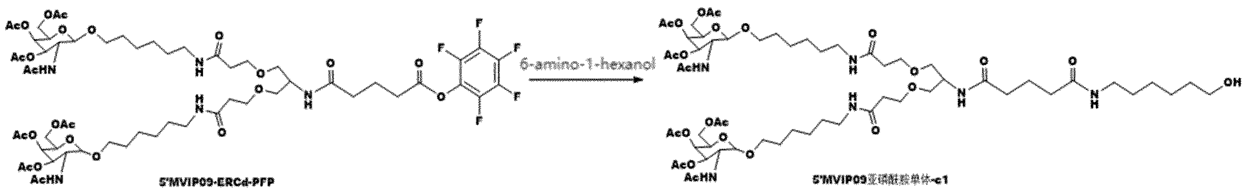


Dissolved 5'MVIP09-ERCd-PFP crude product (2.35g, 1.58mmol) in DCM (60ml), added DIPEA (0.82g, 6.32mmol), 6-amino-1-hexanol (0.37g, 3.16mmol), and let it react overnight with stirring at room temperature. Added 10% citric acid (30ml), extracted with DCM (30ml*3), washed with saturated salt water (50ml), dried with anhydrous sodium sulfate, filtered, conducted rotary evaporation, and purified by a column to obtain the product 5'MVIP09 monomer-c1 (1.73g). 4.2.1.5 5'MVIP09 phosphoramidite monomer

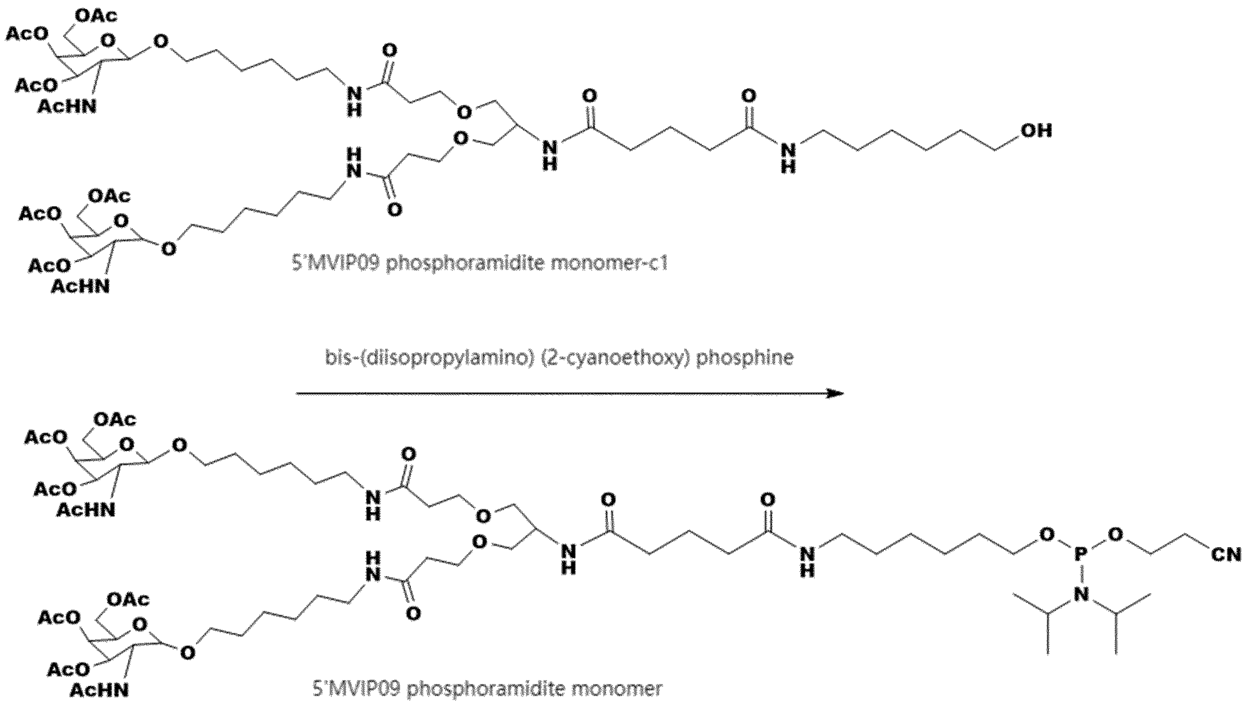


5'MVIP09 phosphoramidite monomer-c1

5'MVIP09 phosphoramidite monomer

Weighed 5'MVIP09 phosphoramidite monomer-c1 (1.3g, 1.0mmol) to dissolve in acetonitrile (30ml), added diisopropylamine triazole (0.22g), added bis-(diisopropylamino) (2-cyanoethoxy) phosphine (0.36g, 1.2mmol) dropwise in an ice bath, let it react for 4h at room temperature, after the reaction is validated by HPLC in-process control , concentrated and purified by a column to obtain the product 5'MVIP09 monomer (1.2g).

**4.2.2 Synthesis of 5'MVIP01 phosphoramidite monomer:**

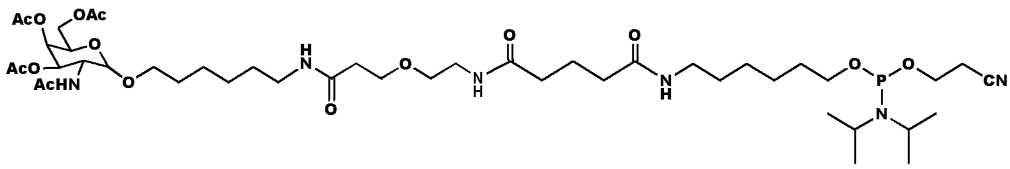

5'MVIP01 phosphoramidite monomer

Except for weighing YICd-01-c2 (1.12g, 2.0mmol) as the phosphoramidite monomer of 5'MVIP01, refer to 4.2.1.1.~ 4.2.1.5 for the remaining operations.

**Example 7 Synthesis of RNA inhibitor with different siRNA conjugated to 5'MVIP09/** 3'MVIP09

Description of synthesis of the antisense strand and its carrier structure: purged a reagent bottle with argon for at least 2 min. Added phosphoramidite monomer and acetonitrile into the reagent bottle in turn, tightened the bottle cap and shook until the solid is completely dissolved by visual inspection. Then added 3A molecular sieve and let it stand for more than 8 h for later use. Purged a reagent bottle with argon for at least 2 min. Added xanthane hydride and dried pyridine into the reagent bottle in turn, tightened the bottle cap, shook until the solid is completely dissolved by visual inspection, and stored temporarily for later use. Ensure to carry out the following operations at room temperature of 20~30°C : weighed 3'MVIP carrier, added it to a reagent bottle, then added acetonitrile, shook until mix evenly. Transferred the carrier to a synthesis column, and eluted the residual carrier in the reagent bottle with acetonitrile and transferred to the synthesis column. After elution, added acetonitrile to fill the synthetic column, and recorded the amount of acetonitrile used. Installed and fixed the synthetic column according to the instrument instructions.

Connected the monomer solution prepared above, CAP A, CAP B, oxidant, thioreagent, activator, decapping agent and acetonitrile to the pipeline corresponding to AKTA PILOT100, and ensured that the pipeline is inserted into the

bottom of the reagent bottle.

After the synthesis method is set, the instrument is ready for all work, clicked Run to start the synthesis. Observed and recorded each detritylation peak area online. During the synthesis process, added additional amount according to the actual amount of deprotection reagent used.

After the synthesis, purged the synthetic column with argon for at least 2 h, and unloaded the synthetic column according to the operating procedures. Transferred the solid phase support in the synthesis column to a reaction bottle, added methylamine aqueous solution and ammonia water, and put the reaction bottle into a shaker at 35 °C for 2-3 hours. Filtered the solution into a round bottom flask, washed the residual solid phase with 50% ethanol aqueous solution, filtered again and mixed with the previous filtrate, connected the round bottom flask with a rotary evaporator, set the water temperature at 50°C and evaporated until there is no distillate, added ethanol into the round bottom flask, mixed well and evaporated again until there is no distillate. Repeated the operation until white powder appeared at the bottom of the flask. Prepared the obtained white powder into a solution, purified by a reverse chromatography column, and sampled to detect OD260 and purity. Divided the purified antisense strand solution into vials and lyophilized for future use, and stored the product sealed in a -20°C refrigerator.

The synthesis process of sense strand and its carrier structure is the same as that of antisense strand and its carrier structure, in which the carrier loaded in the column is Universal carrier. Added DIPEA to the obtained intermediate to prepare a solution, added 5'MVIP phosphoramidite monomer, mixed well, and put the reaction bottle into a shaker at 35 °C for 2-3 hours.

Description of synthetic annealing process of RNA inhibitor:

Took a sense strand and its carrier structure, took an antisense strand and its carrier structure, mixed them in a reaction bottle in an equimolar ratio of 1:1. Turned off the power of the water bath after 5 minutes of 95°C water bath, let it naturally cool down to below 40 °C. Added 3M sodium acetate aqueous solution to the solution containing double strands, mixed well, then added an appropriate volume of absolute ethanol, mixed well, and put the reaction solution into a -20 °C refrigerator for 45min. Set the high-speed freezing centrifuge to pre-cooling at 4 °C, put in the solution containing double strands after the temperature is reached, and started the centrifuge. Took out the centrifuged solution containing double strands, removed the supernatant, added ultrapure water to completely dissolve the solid, and took samples to detect OD260 and purity, obtained the RNA inhibitors in Table 14. Divided the purified finished solution into vials and lyophilized for future use, and stored the product sealed in a -20 °C refrigerator.

**Example 8-1 Study on the use of PHH to evaluate the activity of RNA inhibitors containing 5'MVIP09/3'MVIP09 structure**

Primary human hepatocytes (PHH) were used to evaluate the in vitro activity of the RNA inhibitors in Table 15. Thawed frozen PHH and adjusted the cell density to $6\times10^5$ cells per ml. Took from the dilution plate the prepared RNA inhibitors and added it to a 96-well cell culture plate (10 μL/well), and added 90 μL/well of cells to the 96-well plate, with a final volume of 100 μL per well. The RNA inhibitors were diluted 10-fold starting from 200 nM, with a total of 3 concentration points and triplicate wells. After addition, the cells were placed in a 5% CO2, 37°C incubator for 48 hours. Extracted RNA from cells and then reverse transcribed it into cDNA. Detected target gene cDNA using qPCR. GAPDH was used as an internal reference gene and qPCR was performed in a 384-well plate. The qPCR reaction program was: 95°C for 10 minutes; then 95°C for 15 seconds and 60°C for 1 minute for 40 cycles. Detected the relative expression level of APOC3 mRNA. The test results were shown in Table 31.

Table 31 Relative expression level of APOC3 mRNA after RNA inhibitor intervention

| RNA inhibitor | APOC3 mRNA Relative expression level (2 ^ -ΔΔCt) | | | SD | | |
|---|---|---|---|---|---|---|
| | 200 nM | 20 nM | 2 nM | 200 nM | 20 nM | 2 nM |
| Kylo-12-DS1071 | 0.35 | 0.49 | 0.77 | 0.00 | 0.07 | 0.04 |
| Kylo-12-DS1081 | 0.46 | 0.58 | 0.84 | 0.21 | 0.09 | 0.07 |
| Kylo-12-DS1131 | 0.31 | 0.55 | 0.81 | 0.03 | 0.13 | 0.02 |
| Kylo-12-DS1141 | 0.39 | 0.47 | 0.74 | 0.04 | 0.15 | 0.05 |
| Kylo-12-DS1241 | 0.41 | 0.57 | 0.80 | 0.02 | 0.12 | 0.06 |
| Kylo-12-DS1311 | 0.37 | 0.56 | 0.75 | 0.03 | 0.11 | 0.04 |
| Kylo-12-DS1321 | 0.34 | 0.45 | 0.65 | 0.00 | 0.07 | 0.03 |

This example demonstrated that the 5'MVIP09/3'MVIP09 carrier combination can achieve self-delivery of siRNA.

**Example 8-2 Evaluation of the activity of RNA inhibitors with 5'MVIP/3'MVIP using PHH**

This example used primary human hepatocytes to evaluate the in vitro activity of the modified RNA inhibitors in Table 22-1. Thawed frozen PHH and adjusted the cell density to $6\times10^5$ cells per ml. Took from the dilution plate the prepared RNA inhibitors and added it to a 96-well cell culture plate (10 μL/well), and added 90 μL/well of cells to a 96-well plate, with a final volume of 100 μL per well. The RNA inhibitors were at 2 concentrations: 200 nM and 20nM, in triplicate wells. After addition, the cells were placed in a 5% CO2, 37°C incubator for 48 hours. Extracted RNA from cells and then reverse transcribed it into cDNA. Detected target gene cDNA using qPCR. GAPDH was used as an internal reference gene and qPCR was performed in a 384-well plate. The qPCR reaction program was: 95°C for 10 minutes; then 95°C for 15 seconds and 60°C for 1 minute for 40 cycles. Detected the relative expression level of APOC3 mRNA. The test results were shown in Table 32.

Table 32 Relative expression level of APOC3 mRNA

| RNA inhibitor | APOC3 mRNA relative expression level (2 ^ -ΔΔCt) | | SD | |
|---|---|---|---|---|
| | 200nM | 20nM | 200nM | 20nM |
| Ky-12-DS23001 | 0.2915 | 0.3771 | 0.0511 | 0.0302 |
| Ky-12-DS25001 | 0.2386 | 0.3765 | 0.0616 | 0.0131 |
| Ky-12-DS25401 | 0.3168 | 0.39 | 0.0971 | 0.1088 |
| Ky-12-DS29701 | 0.2174 | 0.3763 | 0.0257 | 0.0749 |
| Ky-12-DS29801 | 0.2075 | 0.3136 | 0.0517 | 0.0802 |
| Ky-12-DS31701 | 0.1899 | 0.211 | 0.0496 | 0.0702 |
| Ky-12-DS31702 | 0.2568 | 0.378 | 0.0237 | 0.0359 |
| Ky-12-DS31703 | 0.2378 | 0.3218 | 0.0329 | 0.0219 |
| Ky-12-DS31704 | 0.2977 | 0.4077 | 0.0642 | 0.0528 |
| Ky-12-DS31705 | 0.2193 | 0.397 | 0.0384 | 0.0512 |
| Ky-12-DS31706 | 0.242 | 0.3585 | 0.0725 | 0.0233 |
| Ky-12-DS31707 | 0.2364 | 0.3901 | 0.0459 | 0.0126 |
| Ky-12-DS31708 | 0.2936 | 0.3789 | 0.0669 | 0.0402 |
| Ky-12-DS31709 | 0.2872 | 0.3422 | 0.019 | 0.0712 |
| Ky-12-DS31711 | 0.23 | 0.3988 | 0.071 | 0.0321 |
| Ky-12-DS31712 | 0.2482 | 0.3679 | 0.0209 | 0.0715 |
| Ky-12-DS31713 | 0.2641 | 0.4012 | 0.0362 | 0.0556 |
| Ky-12-DS33001 | 0.2066 | 0.3198 | 0.0433 | 0.0521 |
| Ky-12-DS33006 | 0.2577 | 0.3834 | 0.0236 | 0.0424 |

The test results showed that RNA inhibitors, which were formed by the carrier structure 5'MVIP17 at 5' end of the modified sense strand, the carrier structure 3'MVIP17 at 3' end of the modified sense strand, the combination 5' MVIP01/3'MVIP01, 5'MVIP01/3'MVIP17, 5'MVIP17/3'MVIP01 or 5'MVIP09/3'MVIP09 of the modified sense strand and antisense strand conjugating carrier 5'MVIP/3'MVIP, or the combination 5'MVIP01/3'MVIP09, 5'MVIP09/3'MVIP01 or 5'MVIP01/3'MVIP01 of the sense strand 5'MVIP and the sense strand 3'MVIP, can be freely taken up into PHH cells and exhibit significant inhibitory effects on APOC3 mRNA in PHH cells.

**Example 9 Study on the effect of different structures of 5'MVIP and 3'MVIP conjugated to the same siRNA on the activity of RNA inhibitors**

Selected the antisense strand and the sense strand respectively, paired and annealed to synthesize RNA

inhibitors according to the method described in Example 7 (see Table 22-2). Examined the effect on the activity of RNA inhibitors when X, L, B, D, $R_1$ or $R_2$ are different in the structures of 5'MVIP and/or 3'MVIP. Selected APOC3 Tg mouse model of appropriate age for experimental evaluation. Administered 3mg/kg by subcutaneous injection on Day0. Collected blood on Day14 after administration, isolated serum and determined the levels of APOC3 in serum by ELISA method. The test results were shown in Table 33 and Figure 1.

Table 33 Average level of APOC3 in serum of APOC3 Tg mice (normalized)

| RNA inhibitor | average APOC3 level after RNA inhibitor intervention |
|---|---|
| Saline group | 1.000 |
| Kylo-12-DS131 | 0.331 |
| Kylo-12-DS141 | 0.312 |
| Kylo-12-DS142 | 0.291 |
| Kylo-12-DS147 | 0.138 |
| Kylo-12-DS148 | 0.136 |
| Kylo-12-DS149 | 0.101 |
| Kylo-12-DS150 | 0.127 |
| Kylo-12-DS1081 | 0.179 |
| Kylo-12-DS151 | 0.193 |
| Kylo-12-DS152 | 0.184 |
| Kylo-12-DS153 | 0.100 |
| Kylo-12-DS154 | 0.134 |
| Kylo-12-DS155 | 0.199 |
| Kylo-12-DS156 | 0.142 |
| Kylo-12-DS157 | 0.178 |
| Kylo-12-DS158 | 0.211 |
| Kylo-12-DS159 | 0.243 |
| Kylo-12-DS160 | 0.191 |
| Note: Normalization is to divide the TG level of an animal at a time point by the TG level of the animal on Day 0 to obtain a ratio A1, and to divide the average TG level of the control group at a time point by the average TG level of the control group on Day 0 to obtain a ratio A2. A1 divided by A2 is the normalized average TG level in the blood. The test results showed that the overall activity of the RNA inhibitors with n+m=4 in the 5'MVIP/3'MVIP combination is slightly higher than that of the combination with n+m=3. | |

**Example 10 Investigation of the effect of different 5' or 3' end nucleotides on RNA inhibitor activity**

Transfected RNA inhibitor into HepG2 cells, and measured APOC3 mRNA level by qRT-PCR. Compared with the supernatant of HepG 2 cells without intervention, the relative percentage of APOC3 mRNA in the intervention group was calibrated. The test results are shown in Table 34 and Figure 2.

Table 34 Inhibition rate of APOC3 mRNA after RNA inhibitor intervention

| SEQ ID NO. | sense strand code | sense strand sequence 5'→3' | SEQ ID NO. | antisense strand code | antisense strand sequence 5'→3' | Double strands code | Average Inhibition | |
|---|---|---|---|---|---|---|---|---|
| | | | | | | | 1nM | 0.1n M |
| 947 | S439 | aagggacaguauuc tcagugu | 683 | AS439 | acacugagaauacug ucccuu | DS321 | 0.951 | 0.819 |

(continued)

| SEQ ID NO. | sense strand code | sense strand sequence 5'→3' | SEQ ID NO. | antisense strand code | antisense strand sequence 5'→3' | Double strands code | Average Inhibition | |
|---|---|---|---|---|---|---|---|---|
| | | | | | | | 1nM | 0.1n M |
| 948 | S266 | gagggacaguauuc ucagugu | 684 | AS268 | acacugagaauacug ucccuc | DS331 | 0.941 | 0.782 |
| 949 | S267 | cagggacaguauuc ucagugu | 685 | AS269 | acacugagaauacug ucccug | DS341 | 0.941 | 0.664 |
| 950 | S268 | gggacaguauucuc agugcua | 686 | AS270 | gcacugagaauacu gucccuu | DS351 | 0.924 | 0.768 |
| 951 | S269 | gagcgacaguauuc tcagugu | 687 | AS271 | acacugagaauacug ucgcuc | DS361 | 0.961 | 0.54 |
| 952 | S270 | uagggacaguauuc tcagugu | 688 | AS272 | acacugagaauacug ucccua | DS371 | 0.922 | 0.643 |
| 953 | S271 | augggacaguauuc tcagugu | 689 | AS273 | acacugagaauacug ucccau | DS381 | 0.573 | 0.411 |
| 954 | S272 | agggacaguauucu cagugua | 690 | AS274 | ucacugagaauacu gucccuu | DS391 | 0.954 | 0.352 |
| 955 | S273 | aagggacaguauuc ucagu | 691 | AS275 | ucacugagaauacu gucccuu | DS401 | 0.906 | 0.513 |
| 956 | S274 | gcuuaaaagggaca guauuca | 692 | AS276 | ugaauacugucccu uuuaagc | DS411 | 0.951 | 0.819 |
| 957 | S275 | gcuuaaaagggaca guauucu | 693 | AS277 | aauacugucccuuu uaagcaa | DS601 | 0.903 | 0.956 |
| 958 | S276 | cuuaaaagggacag uauucuc | 694 | AS278 | gaauacugucccuu uuaagca | DS611 | 0.825 | 0.892 |
| 959 | S277 | uuaaaagggacagu auucuca | 695 | AS279 | agaauacugucccu uuuaagc | DS621 | 0.882 | 0.877 |

(continued)

| SEQ ID NO. | sense strand code | sense strand sequence 5'→3' | SEQ ID NO. | antisense strand code | antisense strand sequence 5'→3' | Double strands code | Average Inhibition | |
|---|---|---|---|---|---|---|---|---|
| | | | | | | | 1nM | 0.1n M |
| 960 | S278 | uaaaagggacagua uucucag | 696 | AS280 | gagaauacugucccc uuuuaag | DS631 | 0.452 | 0.383 |
| 961 | S279 | aaaagggacaguau ucucagc | 697 | AS281 | ugagaauacugucc cuuuuaa | DS641 | 0.419 | 0.973 |
| 962 | S280 | aaagggacaguauu cucagug | 698 | AS282 | cugagaauacuguc ccuuuua | DS651 | 0.913 | 0.897 |
| 963 | S281 | aagggacaguauuc ucagugc | 699 | AS283 | acugagaauacugu cccuuua | DS661 | 0.88 | 0.908 |
| 964 | S282 | agggacaguauucu cagugcu | 700 | AS284 | cacugagaauacug ucccuua | DS671 | 0.919 | 0.847 |
| 960 | S283 | gcuuaaaagggaca guauucu | 684 | AS285 | ugaauacugucccu uuuaagcaagc | DS681 | 0.884 | 0.961 |

The results showed that the 5' or 3' end of the sense and antisense strands of RNA inhibitors can differ by 1, 2 or even 3 nucleotides, and the activities of RNA inhibitors are not significantly affected.

**Example 11 Investigation of the effect of different modifications at 2' position of nucleotide glycosyls on RNA inhibitor activity**

Investigated the effect on the activity of RNA inhibitors of fluorine modification at 2' position of nucleotide glycosyls at different positions starting from the 5' end of the sense strand, and fluorine modification at 2' position of nucleotide glycosyls at different positions starting from the 5' end of the antisense strand.

Transfected RNA inhibitor into HepG2 cells, and measured APOC3 mRNA level by qRT-PCR. Compared with the supernatant of HepG 2 cells without intervention, the average expression level of APOC3 mRNA in the intervention group was calibrated, and the inhibition rate was calculated. The test results were shown in Table 35 and Figure 3.

Table 35 The inhibition rate of APOC3 mRNA after RNA inhibitor intervention

| SE Q ID NO. | sens e stran d code | sense strand sequence 5'→3' | SEQ ID NO. | antise nse strand code | antisense strand sequence 5'→3' | Dou ble stran ds code | Average Inhibition | |
|---|---|---|---|---|---|---|---|---|
| | | | | | | | 1nM | 0.1nM |
| 111 2 | S54 1 | AsAsGGfGAfCfA fGUAUUCTCAG UsGsU | 1142 | AS52 0 | AsCsACUGfAGA AUfACfUGUCCC sUsU | DS2 01 | 0.4396 8408 | 0 |
| 111 3 | S54 2 | GsAsGGfGAfCAf GUAUUCUCAG UsGsU | 1143 | AS52 1 | AsCsACUGfAGA AUfACfUGUCCC sUsC | DS2 11 | 0.5320 4033 | 0.2090 261 |
| 111 4 | S54 3 | CsAsGGfGAfCfA fGUAUUCUCAG UsGsU | 1144 | AS52 2 | AsCsACUGfAGA AUfACfUGUCCC sUsG | DS2 21 | 0.6190 0211 | 0 |
| 111 5 | S54 4 | GsGsGAfCAfGfU fAUUCUCAGUG CsUsA | 1145 | AS52 3 | GsCsACUGfAGA AUfACfUGUCCC sUsU | DS2 31 | 0.8282 0992 | 0.8646 5283 |
| 111 6 | S54 5 | GsAsGGfGAfCfA fGUAUUCUCAG UsGsU | 1146 | AS52 4 | AsCsACUGfAGA AUfACfUGUCCC sUsC | DS2 41 | 0.4761 101 | 0 |
| 111 7 | S54 6 | GsAsGCfGAfCfA fGUAUUCTCAG UsGsU | 1147 | AS52 5 | AsCsACUGfAGA AUfACfUGUCGC sUsC | DS2 51 | 0.4664 62 | 0 |

(continued)

| SE Q ID NO. | sens e stran d code | sense strand sequence 5'→3' | SEQ ID NO. | antise nse strand code | antisense strand sequence 5'→3' | Dou ble stran ds code | Average Inhibition | |
|---|---|---|---|---|---|---|---|---|
| | | | | | | | 1nM | 0.1nM |
| 111 8 | S54 7 | UsAsGGfGAfCfA fGUAUUCTCAG UsGsU | 1148 | AS52 6 | AsCsACUGfAGA AUfACfUGUCCC sUsA | DS2 61 | 0.9014 1965 | 0.8086 2764 |
| 111 9 | S54 8 | AsUsGGfGAfCfA fGUAUUCTCAG UsGsU | 1149 | AS52 7 | AsCsACUGfAGA AUfACfUGUCCC sAsU | DS2 71 | 0.4232 252 | 0.1985 1928 |
| 112 0 | S54 9 | AsGsGGfACfAfG fUAUUCUCAGU GsUsA | 1150 | AS52 8 | UsCsACUGfAGA AUfACfUGUCCC sUsU | DS2 81 | 0.8809 7941 | 0.6891 1395 |
| 112 1 | S55 0 | AsAsGGfGAfCfA fGUAUUCUCAG UsGsA | 1151 | AS52 9 | UsCsACUGfAGA AUfACfUGUCCC sUsU | DS2 91 | 0.7345 9528 | 0.7086 7407 |
| 112 2 | S55 1 | GsCsUUfAAfAfA fGGGACAGUAU UsCsA | 1152 | AS53 0 | UsGsAAUAfCUG UCfCCfUUUUAA sGsC | DS3 01 | 0 | 0.2302 3379 |
| 112 3 | S55 2 | GsCsUUAAAAfG fGfGACAGUAU UsCsA | 1153 | AS53 1 | UsfGsAfAUfACf UGfUCfCCfUUfU UfAAsGsC | DS3 11 | 0.9420 0427 | 0.8751 5849 |

(continued)

| SE Q ID NO. | sens e stran d code | sense strand sequence 5'→3' | SEQ ID NO. | antise nse strand code | antisense strand sequence 5'→3' | Dou ble stran ds code | Average Inhibition | |
|---|---|---|---|---|---|---|---|---|
| | | | | | | | 1nM | 0.1nM |
| 112 4 | S55 3 | GsCsUUfAAfAfA fGGGACAGUAU UsCsU | 1154 | AS53 2 | AsAsUACUfGUC CCfUUfUUAAGC sAsA | DS4 21 | 0.5039 7905 | 0.0169 2893 |
| 112 5 | S55 4 | GsCsfUUfAAfAf AfGGfGAfCAfG UAUUsCsU | 1155 | AS53 3 | AsfAsUfACfUGf UCCCUUfUUfAA GCsAsA | DS4 31 | 0.9205 1393 | 0.1046 0114 |
| 112 6 | S55 5 | CsUsUAfAAfAfG fGGACAGUAUU CsUsC | 1156 | AS53 4 | GsAsAUACfUGU CCfCUfUUUAAG sCsA | DS4 41 | 0.2717 1299 | 0 |
| 112 7 | S55 6 | CsUsfUAfAAfAf GfGGfACfAGfU AUUCsUsC | 1157 | AS53 5 | GsfAsAfUAfCUf GUCCCUfUUfUA AGsCsA | DS4 51 | 0.8492 3242 | 0.2706 6857 |
| 112 8 | S55 7 | UsUsAAfAAfGfG fGACAGUAUUC UsCsA | 1158 | AS53 6 | AsGsAAUAfCUG UCfCCfUUUUAA sGsC | DS4 61 | 0.8739 331 | 0.8306 664 |
| 112 9 | S55 8 | UsUsfAAfAAfGf GfGAfCAfGUfA UUCUsCsA | 1159 | AS53 7 | AsfGsAfAUfACf UGUCCCfUUfUU AAsGsC | DS4 71 | 0.9508 9087 | 0.7931 5352 |

(continued)

| SE Q ID NO. | sens e stran d code | sense strand sequence 5'→3' | SEQ ID NO. | antise nse strand code | antisense strand sequence 5'→3' | Dou ble stran ds code | Average Inhibition | |
|---|---|---|---|---|---|---|---|---|
| | | | | | | | 1nM | 0.1nM |
| 113 0 | S55 9 | UsAsAAfAGfGfG fACAGUAUUCU CsAsG | 1160 | AS53 8 | GsAsGAAUfACU GUfCCfCUUUUA sAsG | DS4 81 | 0.5044 4347 | 0.3883 753 |
| 113 1 | S56 0 | UsAsfAAfAGfGf GfACfAGfUAfU UCUCsAsG | 1161 | AS53 9 | GsfAsGfAAfUAf CUGUCCfCUfUU UAsAsG | DS4 91 | 0.7528 2363 | 0.3936 0064 |
| 113 2 | S56 1 | AsAsAAfGGfGfA fCAGUAUUCUC AsGsC | 1162 | AS54 0 | UsGsAGAAfUAC UGfUCfCCUUUU sAsA | DS5 01 | 0.5441 8952 | 0.7268 4271 |
| 113 3 | S56 2 | AsAsfAAfGGfGf AfCAfGUfAUfUC UCAsGsC | 1163 | AS54 1 | UsfGsAfGAfAUf ACUGUCfCCfUU UUsAsA | DS5 11 | 0.9354 0529 | 0.9899 81452 |
| 113 4 | S56 3 | AsAsAGfGGfAfC fAGUAUUCUCA GsUsG | 1164 | AS54 2 | CsfUsGfAGfAAf UACUGUfCCfCU UUsUsA | DS5 21 | 0.9135 8498 | 0.9844 0306 |
| 113 5 | S56 4 | AsAsAfGGfGAfC fAfGUfAUfUCfU CAGsUsG | 1165 | AS54 3 | CsfUsGfAGfAAf UACUGUfCCfCU UUsUsA | DS5 31 | 0.9339 3405 | 0.9015 5531 |

(continued)

| SE Q ID NO. | sens e stran d code | sense strand sequence 5'→3' | SEQ ID NO. | antise nse strand code | antisense strand sequence 5'→3' | Dou ble stran ds code | Average Inhibition | |
|---|---|---|---|---|---|---|---|---|
| | | | | | | | 1nM | 0.1nM |
| 113 6 | S56 5 | AsAsGGfGAfCfAfGUAUUCUCAGUsGsC | 1166 | AS54 4 | AsCsUGAGfAAUACfUGfUCCCUUsUsA | DS5 41 | 0.9308 5339 | 0.9814 1713 |
| 113 7 | S56 6 | AsAsfGGfGAfCfAfGUfAUfUCfUCAGUsGsC | 1167 | AS54 5 | AsfCsUfGAfGAfAUACUGfUCfCCUUsUsA | DS5 51 | 0.9723 2923 | 0.9250 3153 |
| 113 8 | S56 7 | AsGsGGfACfAfGfUAUUCUCAGUGsCsU | 1168 | AS54 6 | CsAsCUGAfGAAUAfCUfGUCCCUsUsA | DS5 61 | 0.5341 0452 | 0.7224 1722 |
| 113 9 | S56 8 | AsGsfGGfACfAfGfUAfUUfCUfCAGUGsCsU | 1169 | AS54 7 | CsfAsCfUGfAGfAAUACUfGUfCCCUsUsA | DS5 71 | 0.8624 7604 | 0.9514 6775 |
| 114 0 | S56 9 | GsCsUUfAAfAfAfGGGACAGUAUUsCsA | 1170 | AS54 8 | UsAsAUAfCUGUCfCCfUUUUAAGCsAsA | DS5 81 | 0.4202 7334 | 0.5183 2601 |
| 114 1 | S57 0 | GsCsUUAAAAfGfGfGACAGUAUUsCsA | 1171 | AS54 9 | UsAsfAUfACfUGfUCfCCfUUfUUfAAGCsAsA | DS5 91 | 0.9088 6421 | 0.8420 9032 |

According to the test results, the modification mode is sequence-specific. The ideal ones for the fluorine modification at 2'-position of the nucleotide glycosyls at positions 5, 7, 8, and 9 starting from 5' end of the sense chain, and the fluorine modification at 2'-position of the nucleotide glycosyls at positions 7, 14, and 16 starting from the 5' end of the antisense chain are DS231, DS261, DS281 and DS311.

DS541 and DS551 have the same sequence, and the 2' positions of the nucleotide glycosyls at positions 5, 7, 8, and 9 starting from the 5' end of the sense chain are fluorinated, and the 2' positions of the nucleotide glycosyls at positions 7, 14, and 16 starting from the 5' end of the antisense chain are fluorinated, or the 2' positions of the nucleotide glycosyls at positions 3, 5, 7, 8, 9, 11, 13, and 15 starting from the 5' end of the sense chain are fluorinated, and the 2' positions of the nucleotide glycosyls at positions 2, 4, 6, 8, 14, and 16 starting from the 5' end of the antisense chain are fluorinated, the RNA inhibitors maintain good activity.

DS571 has fluorine modification at 2' positions of nucleotide glycosyls at positions 3, 5, 7, 8, 9, 11, 13, and 15 starting from the 5' end of the sense strand, and fluorine modification at 2' positions of nucleotide glycosyls at positions 2, 4, 6, 8, 14, and 16 starting from the 5' end of the antisense strand, the effect is satisfactory.

DS591 has fluorine modification at 2' positions of nucleotide glycosyls at positions 9, 10 and 11 starting from the 5' end of the sense strand, and fluorine modification at 2' positions of nucleotide glycosyls at positions 2, 4, 6, 8, 14, and 16 starting from the 5' end of the antisense strand, the effect is satisfactory.

**Example 12 Evaluation of the effect of 5'MVIP09/3'MVIP09 combination on RNA inhibitor delivery using PHH screening**

Primary human hepatocytes (PHH) were used to evaluate the in vitro activity of the RNA inhibitors in Table 16. Thawed frozen PHH and adjusted the cell density to $6\times10^5$ cells per ml. Took from the dilution plate the prepared RNA inhibitors and added it to a 96-well cell culture plate (10 μL/well), and added 90 μL/well of cells to the 96-well plate, with a final volume of 100 μL per well. The RNA inhibitors were diluted 10-fold starting from 500 nM, with a total of 3 concentration points and triplicate wells. After addition, the cells were placed in a 5% $CO_2$, 37°C incubator for 48 hours. Extracted RNA from cells and then reverse transcribed it into cDNA. Detected target gene cDNA using qPCR. GAPDH was used as an internal reference gene and qPCR was performed in a 384-well plate. The qPCR reaction program was: 95°C for 10 minutes; then 95°C for 15 seconds and 60°C for 1 minute for 40 cycles. The relative expression level of APOC3 mRNA was detected and the inhibition rate was calculated. The results are shown in Table 36 and Figure 4.

Table 36 Inhibition rate of APOC3 mRNA after RNA inhibitor intervention

| sense strand code | sense strand sequence 5' strand sequence | antisen se strand code | antisense strand sequence 5'→3' | RNA inhibitor | Average Inhibition | | |
|---|---|---|---|---|---|---|---|
| | | | | | 500 nM | 100n M | 10nM |
| S313' | 5'MVIP09-GsGsGAfCAfGfUfAUUCUCAGUGCsUsA | AS316 , | GsCsACUGfAGAAUfACfUGUCCCsUsU-3'MVIP09 | Kylo-12-DS2311 | 0.81 21 | 0.595 4 | 0.432 1 |
| S314' | 5'MVIP09-UsAsGGfGAfCfAfGUAUUCTCAGUsGsU | AS317 , | AsCsACUGfAGAAUfACfUGUCCCsUsA-3'MVIP09 | Kylo-12-DS2611 | 0.79 26 | 0.612 35 | 0.402 1 |
| S315' | 5'MVIP09-AsAsGGfGAfCfAfGUAUUCUCAGUsGsA | AS318 , | UsCsACUGfAGAAUfACfUGUCCCsUsU-3'MVIP09 | Kylo-12-DS2911 | 0.80 33 | 0.679 1 | 0.501 7 |
| S316' | 5'MVIP09-AsAsGGfGAfCfAfGUAUUCUCAGUsGsC | AS319 , | AsCsUGAGfAAUACfUGfUCCCUUsUsA-3'MVIP09 | Kylo-12-DS5411 | 0.79 22 | 0.631 6 | 0.539 1 |

(continued)

| sense strand code | sense strand sequence 5' strand sequence | antisen se strand code | antisense strand sequence 5'→3' | RNA inhibitor | Average Inhibition | | |
|---|---|---|---|---|---|---|---|
| | | | | | 500 nM | 100n M | 10nM |
| S317' | 5'MVIP09-AsAsfGGfGAfCfAfGUfAUfUCfUCAGUsGsC | AS320 , | AsfCsUfGAfGAfAUACUGfUCfCCUUsUsA-3'MVIP09 | Kylo-12-DS5511 | 0.77 81 | 0.697 3 | 0.523 9 |
| S318' | 5'MVIP09-AsGsfGGfACfAfGfUAfUUfCUfCAGUGsCsU | AS321 , | CsfAsCfUGfAGfAAUACUfGUfCCCUsUsA-3'MVIP09 | Kylo-12-DS5711 | 0.81 26 | 0.618 5 | 0.475 6 |
| S319' | 5'MVIP09-GsCsUUAAAAfGfGfGACAGUAUUsCsA | AS322 , | AsfAUfACfUGfUCfCCfUUfUUfAAGCsAsA-3'MVIP09 | Kylo-12-DS5911 | 0.82 53 | 0.601 1 | 0.553 0 |
| S320' | 5'MVIP09-GsCsUUAAAAfG | AS323 , | UsfGsAfAUfACfUGfUCfCCfUUfUU | Kylo-12-DS3111 | 0.77 97 | 0.637 4 | 0.532 1 |
| | fGfGACAGUAUUsCsA | | fAAsGsC-3'MVIP09 | | | | |

The test results showed that the RNA inhibitor formed by the modified sense strand and antisense strand conjugating to carrier 5'MVIP09/3'MVIP09 can be freely taken up into PHH cells, inhibiting APOC3 mRNA in PHH cells and showing significant dose-effect effect.

**Example 13 Evaluation of the activity of RNA inhibitors using transgenic mouse model**

Examined the in vivo activity of the RNA inhibitors in Table 36. Selected APOC3 Tg mouse model of appropriate age for experimental evaluation. Administered 3mg/kg by subcutaneous injection on Day0. Collected blood on days 8, 15, 22, 29, 35 and 42 after administration, measured TG and APOC3 levels. After RNA inhibitor intervention, the normalized average APOC3 levels in serum were shown in Table 37 and Figure 5.

Table 37 Average APOC3 level in serum after normalization

| RNA inhibitor | d0 | d8 | d15 | d22 | d29 | d35 | d42 |
|---|---|---|---|---|---|---|---|
| Saline group | 1.000 | 1.000 | 1.000 | 1.000 | 1.000 | 1.000 | 1.000 |
| Kylo-12-DS2311 | 1.000 | 0.219 | 0.161 | 0.234 | 0.199 | 0.267 | 0.299 |
| Kylo-12-DS2611 | 1.000 | 0. 209 | 0.178 | 0.157 | 0.246 | 0.278 | 0.300 |
| Kylo-12-DS2911 | 1.000 | 0.159 | 0.169 | 0.210 | 0.269 | 0.250 | 0.289 |
| Kylo-12-DS5911 | 1.000 | 0.189 | 0.193 | 0.225 | 0.253 | 0.320 | 0.35 |
| Note: Normalization is to divide the APOC3 level of an animal at a time point by the APOC3 level of the animal on Day 0 to obtain a ratio A1, and to divide the average level of the control group at a time point by the average level of the control group on Day 0 to obtain a ratio A2. A1 divided by A2 is the normalized average APOC3 level in serum. | | | | | | | |

After RNA inhibitor intervention, the results of the normalized average TG levels in serum were shown in Table 38 and Figure 6:

Table 38 Normalized average TG level in serum

| RNA inhibitor | d0 | d8 | d15 | d22 | d29 | d35 | d42 |
|---|---|---|---|---|---|---|---|
| Saline group | 1.000 | 1.000 | 1.000 | 1.000 | 1.000 | 1.000 | 1.000 |
| Kylo-12-DS2311 | 1.000 | 0.356 | 0.307 | 0.389 | 0.394 | 0.450 | 0.560 |
| Kylo-12-DS2611 | 1.000 | 0.289 | 0.297 | 0.322 | 0.398 | 0.411 | 0.460 |
| Kylo-12-DS2911 | 1.000 | 0.199 | 0.213 | 0.278 | 0.321 | 0.345 | 0.384 |
| Kylo-12-DS5911 | 1.000 | 0.201 | 0.267 | 0.293 | 0.307 | 0.323 | 0.360 |
| Note: Normalization is to divide the TG level of an animal at a time point by the TG level of the animal on Day 0 to obtain a ratio A1, and to divide the average level of the control group at a time point by the average level of the control group on Day 0 to obtain a ratio A2. A1 divided by A2 is the normalized average TG level in serum. | | | | | | | |

**Example 14 Evaluation of the activity of RNA inhibitors using transgenic mouse model**

Examined the in vivo activity of the RNA inhibitors in Table 22-1. Took hAPOC3 Tg mouse model of appropriate age for experimental evaluation. Selected 55 6~8-week-old male hAPOC3 Tg mouse and divided into administration group (Ky-12-DS23001, Ky-12-DS25001, Ky-12-DS25401, Ky-12-DS29701, Ky-12-DS29801, Ky-12-DS31705, Ky-12-DS33001 and Ky-12-DS31701) and saline group. The mice were fasted after adaptive feeding for 2-3 days. Fasting blood collection was performed to separate serum to determine hAPOC3 protein, TG, TC and LDL-c levels. The mice were randomly divided into groups according to TG indexes, with 5 mice in each group. The day of administration was defined as day0. For Ky-12-DS31701, investigated 3 dosage groups. On Day0, administered 1 mg/kg, 3 mg/kg, and 6 mg/kg by subcutaneous injection. For the other groups, administered 3 mg/kg by subcutaneous injection. Collected blood on Day 7, 14, 21, 28, 35, 42, 49, 56, 63, 70 and 77, measured hAPOC3, TC, TG and LDL-c levels and normalized the results (the calculation method is shown in Example 13). The normalized interference effects of hAPOC3, TC, TG and LDL-c were shown in Figures 7, 8, 9 and 10.

The test results showed that these RNA inhibitors investigated in this example have varying degrees of effects on

hAPOC3 expression, TC, TG and LDL-c levels in the serum of hAPOC3 Tg mice, among which the hAPOC3 expression and TG levels were significantly and sustainedly affected. At the dose of 3 mg/kg: by day 35, the reduction rate of Ky-12-DS25401 on TG level could be as high as 89.89%. by day 77, the reduction rate of each RNA inhibitor on TG level remains above 50%. This example also examined the dose-effect relationship of the RNA inhibitor Ky-12-DS31701 on hAPOC3 expression, TC, TG and LDL-c levels. The test results showed that the effect of the RNAinhibitor on TG levels have a significant dose-effect relationship: by Day 14, 1mg/kg, 3mg/kg and 6mg/kg can reduce TG level by 84.21%, 88.98% and 95.06% respectively.

**Example 15 Evaluation of the activity of RNA inhibitors using cynomolgus monkey model**

Hyperlipidemia cynomolgus monkeys, male, 15, 3 in each group, administration group (Ky-12-DS31701, Ky-12-DS31712, Ky-12-DS31711 and Ky-12-DS33001) and saline group. The monkeys were randomly divided into groups according to TG level after 2 weeks of adaptive feeding. Administered subcutaneously at 4 mg/kg on the day of grouping. Collected blood from the saphenous vein or cephalic vein at the following time points: day 0 and days 7, 14, 21, 28, 35, 42, 49, 56 and 63 after administration. Measured APOC3, TC, TG, HDL-c and LDL-c levels and normalized the results. The normalized interference effects of APOC3, TG, HDL-c, LDL-c and TC were shown in Figures 11, 12, 13, 14 and 15. On day 28 and day 63, performed additional liver biopsies on the cynomolgus monkeys in Ky-12-DS31701 administration group respectively. Extracted liver tissue, measured APOC3 mRNA level in the liver of cynomolgus monkeys by RT-qPCR, and normalized the results.

The test results showed that the RNA inhibitors Ky-12-DS31701, Ky-12-DS31712, Ky-12-DS31711, and Ky-12-DS33001 all had significant inhibitory effect on APOC3 expression in serum of cynomolgus monkeys, among which Ky-12-DS31701 and Ky-12-DS33001 reduced TG level by 84.01% and 81.15% respectively, both TC and LDL-c levels were reduced to varying degrees, and HDL-c level was significantly increased. The above-mentioned compounds had significant and sustained inhibitory effects on APOC3 expression and TG level. Among them, the reduction level of TG by Ky-12-DS31701 could still reach 75.48% on day 63. On day 28 and day 63, performed liver biopsies on the cynomolgus monkeys in Ky-12-DS31701 administration group respectively. Extracted liver tissue, measured APOC3 mRNA level in the liver of cynomolgus monkeys by RT-qPCR, and normalized the results. The normalization calculation method was shown in Example 13. The test results showed that the inhibition rates of Ky-12-DS31701 on APOC3 mRNAlevel in cynomolgus monkeys were 91.65% and 87.23% respectively, and the highest individual inhibition rate was 95%.

The basic principles, main features and advantages of the present invention have been shown and described above. Those skilled in the art should understand that the above examples do not limit the present application in any way, and all technical solutions obtained by equivalent replacement or equivalent transformation shall fall within the scope of protection of the invention.

## Claims

1. An RNA inhibitor for inhibiting the expression of APOC3 gene or pharmaceutically acceptable salt thereof, **characterized in that** the RNA inhibitor is formed by base pairing of a sense strand and an antisense strand with chain length of 15-30 nucleotides each independently, wherein the chain length is preferably 19-23 nucleotides each independently, and at least 80% of the bases between the sense strand and the antisense strand are complementary.

2. The RNA inhibitor or pharmaceutically acceptable salt thereof according to claim 1, **characterized in that** the antisense strand is selected from a) the following sequences, b) sequences having at least 15 consecutive nucleotides identical to those in a), and c) sequences having no more than 3 nucleotides different from those in a) and b): 5'uugguggcgugcuucauguaatt 3' (SEQ ID NO. 212), 5'uaacccugcaugaagcugagatt 3'(SEQ ID NO. 216), 5'uuaacggugcuccaguagucutt 3' (SEQ ID NO. 224), 5'cagagaacuuguccuuaacggtt 3'(SEQ ID NO. 225), 5' uauugaggucucaggcagccatt 3' (SEQ ID NO. 241), 5'ugaaguuggucugaccucaggtt 3' (SEQ ID NO. 255), 5' gcacugagaauacugucccuuuu 3' (SEQ ID NO. 256), 5'gcacugagaauacugucccuu3'(SEQ ID NO. 459), 5' acacugagaauacugucccua 3' (SEQ ID NO.461), 5' ugaauacugucccuuuuaagc 3' (SEQ ID NO. 465) , 5' cugagaauacugucccuuuua 3' (SEQ ID NO.471), 5' acugagaauacugucccuuua 3' (SEQ ID NO. 472) , 5' uaauacugucccuuuuaagcaa 3' (SEQ ID NO.438), 5'ugaggucucaggcagccacgg3' (SEQ ID NO. 600), 5'uggauaggcagguggacuugg3' (SEQ ID NO. 620), 5'caggauggauaggcaggugga3' (SEQ ID NO. 624), 5'gagcacugagaauacuguccc3'(SEQ ID NO. 668), 5'acacugagaauacugucgcuc3'(SEQ ID NO. 687), 5'acacugagaauacugucgcuu3'(SEQ ID NO. 700), 5' ucacugagaauacugucccuu 3'(SEQ ID NO. 519);
wherein, g= guanylate, a= adenylate, u= uridylate, c= cytidylate, t= thymidylate.

3. The RNA inhibitor or pharmaceutically acceptable salt thereof according to claim 1, **characterized in that** the sense strand and antisense strand are selected from a) the following sequences, b) sequences having at least 15 consecutive nucleotides identical to those in a), and c) sequences having no more than 3 nucleotides different from those in a) and b), the sequences combination of the sense strand and the antisense strand is as follows:

| SEQ ID NO. | single strand code | sense strand 5'→3' | SEQ ID NO. | single strand code | antisense strand 5'→3' |
|---|---|---|---|---|---|
| 161 | S58 | uuacaugaagcacgccaccaatt | 212 | AS58 | uuggugGcgugcuucauguaatt |
| 165 | S62 | ucucagcuucaugcaggguuatt | 216 | AS62 | uaacccugcaugaagcugagatt |
| 173 | S70 | agacuacuggagcaccguuaatt | 224 | AS70 | uuaacggugcuccaguagucutt |
| 174 | S71 | ccguuaaggacaaguucucugtt | 225 | AS71 | cagagaacuuguccuuaacggtt |
| 190 | S87 | uggcugccugagaccucaauatt | 241 | AS87 | uauugaggucucaggcagccatt |
| 204 | S101 | ccugaggucagaccaacuucatt | 255 | AS10 1 | ugaaguuggucugaccucaggtt |
| 205 | S102 | aagggacaguauucucagugctt | 256 | AS10 2 | gcacugagaauacugucccuuuu |
| 440 | S268 | gggacaguauucucagugcua | 459 | AS27 0 | gcacugagaauacugucccuu |
| 442 | S270 | uagggacaguauuctcagugu | 461 | AS27 2 | acacugagaauacugucccua |
| 446 | S274 | gcuuaaaagggacaguauuca | 465 | AS27 6 | ugaauacugucccuuuuaagc |
| 452 | S280 | aaagggacaguauucucagug | 471 | AS28 2 | cugagaauacugucccuuuua |
| 453 | S281 | aagggacaguauucucagugc | 472 | AS28 3 | acugagaauacugucccuuua |
| 702 | S194 | gcuuaaaagggacaguauuca | 438 | AS19 4 | uaauacugucccuuuuaagcaa |
| 864 | S356 | guggcugccugagaccucaau | 600 | AS35 6 | ugaggucucaggcagccacgg |
| 884 | S376 | aaguccaccugccuauccauc | 620 | AS37 6 | uggauaggcagguggacuugg |
| 888 | S380 | caccugccuauccauccugaa | 624 | AS38 0 | caggauggauaggcaggugga |
| 931 | S423 | gggacaguauucucagugcuu | 667 | AS42 3 | gcacugagaauacugucccuu |
| 932 | S424 | gacaguauucucagugcucuu | 668 | AS42 4 | gagcacugagaauacuguccc |
| 951 | S443 | gagcgacaguauucucagugu | 687 | AS44 3 | acacugagaauacugucgcuc |
| 964 | S456 | aagcgacaguauucucagugu | 700 | AS45 6 | acacugagaauacugucgcuu |
| 781 | S273 | aagggacaguauucucaguga | 519 | AS27 5 | ucacugagaauacugucccuu |

wherein, g= guanylate, a= adenylate, u= uridylate, c= cytidylate, t= thymidylate.

4. The RNA inhibitor or pharmaceutically acceptable salt thereof according to claim 1, **characterized in that** the sense strand and/or antisense strand comprises at least one 2'-modified nucleotide, the 2'-modified nucleotide includes: 2'-O-methyl nucleotide, 2'-deoxy-2'-fluoro nucleotide, 2'-deoxy nucleotide, 2'-methoxyethyl nucleotide, 2'-amino nucleotide or 2'-alkyl nucleotide.

5. The RNA inhibitor or pharmaceutically acceptable salt thereof according to claim 4, **characterized in that** the sense strand and/or antisense strand comprises at least one 2'-O-methyl nucleotide or 2'-deoxy-2'-fluoro nucleotide.

6. The RNA inhibitor or pharmaceutically acceptable salt thereof according to claim 5, **characterized in that** the phosphate bonds among three adjacent nucleotides at at least one of the ends of the sense strand and/or antisense

strand could be thiolated.

7. The RNA inhibitor or pharmaceutically acceptable salt thereof according to claim 5, **characterized in that** the sense strand and the antisense strand are selected from: a) the following sequences, b) sequences having at least 15 consecutive nucleotides identical to those in a), and c) sequences having no more than 3 nucleotides different from those in a) and b):

| SE Q ID NO. | single strand code | sense strand 5'→3' | SE Q ID NO. | single strand code | antisense strand sequence 5'→3' |
|---|---|---|---|---|---|
| 411 | S16 7 | UsUsACAUGAfAfGfCACGCC ACCsAsA | 381 | AS16 7 | UsfUsGfGUfGGfCGUGCUfUCfA UGUsAsA |
| 412 | S16 8 | UsCsUCAGCUfUfCfAUGCAG GGUsUsA | 382 | AS16 8 | UsfAsAfCCfCUfGCAUGAfAGfC UGAsGsA |
| 417 | S17 3 | AsGsACUACUfGfGfAGCACC GUUsAsA | 387 | AS17 3 | UsfUsAfACfGGfUGCUCCfAGfU AGUsCsU |
| 418 | S17 4 | CsCsGUUAAGfGfAfCAAGU UCUCsUsG | 388 | AS17 4 | CsfAsGfAGfAAfCUUGUCfCUfU AACsGsG |
| 428 | S18 4 | UsGsGCUGCCfUfGfAGACCU CAAsUsA | 398 | AS18 4 | UsfAsUfUGfAGfGUCUCAfGGfC AGCsCsA |
| 435 | S19 1 | CsCsUGAGGUfCfAfGACCAA CUUsCsA | 405 | AS19 1 | UsfGsAfAGfUUfGGUCUGfACfC UCAsGsG |
| 436 | S19 2 | AsAsGGGACAfGfUfAUUCU CAGUsGsC | 406 | AS19 2 | GsfCsAfCUfGAfGAAUACfUGfU CCCUUsUsU |
| 478 | S28 7 | GsGsGAfCAfGfUfAUUCUCA GUGCsUsA | 507 | AS28 9 | GsCsACUGfAGAAUfACfUGUCC CsUsU |
| 481 | S29 0 | UsAsGGfGAfCfAfGUAUUCT CAGUsGsU | 510 | AS29 2 | AsCsACUGfAGAAUfACfUGUCC CsUsA |
| 486 | S29 5 | GsCsUUAAAAfGfGfGACAG UAUUsCsA | 515 | AS29 7 | UsfGsAfAUfACfUGfUCfCCfUUf UUfAAsGsC |

(continued)

| SE Q ID NO. | single strand code | sense strand 5'→3' | SE Q ID NO. | single strand code | antisense strand sequence 5'→3' |
|---|---|---|---|---|---|
| 497 | S30 6 | AsAsAGfGGfAfCfAGUAUUCUCAGsUsG | 526 | AS30 8 | CsfUsGfAGfAAfUACUGUfCCfCUUUsUsA |
| 499 | S30 8 | AsAsGGfGAfCfAfGUAUUCUCAGUsGsC | 527 | AS30 9 | AsCsUGAGfAAUACfUGfUCCCUUsUsA |
| 486 | S29 5 | GsCsUUAAAAfGfGfGACAGUAUUsCsA | 532 | AS31 4 | UsAsfAUfACfUGfUCfCCfUUfUUfAAGCsAsA |
| 102 8 | S45 7 | GsUsGGCUGCfCfUfGAGACCUCAsAsU | 965 | AS45 7 | UsfGsAfGGfUCfUCAGGCfAGfCCfACsfGsG |
| 102 9 | S45 8 | AsAsGUCCACfCfUfGCCUAUCCAsUsC | 966 | AS45 8 | UsfGsGfAUfAGfGCAGGUfGGfACfUUsfGsG |
| 103 0 | S45 9 | CsAsCCUGCCfUfAfUCCAUCCUGsAsA | 967 | AS45 9 | CsfAsGfGAfUGfGAUAGGfCAfGGfUGsfGsA |
| 103 1 | S46 0 | GsGsGACAGUfAfUfUCUCAGUGCsUsU | 968 | AS46 0 | GsfCsAfCUfGAfGAAUACfUGfUCfCCsfUsU |
| 103 2 | S46 1 | GsAsCAGUAUfUfCfUCAGUGCUCsUsU | 969 | AS46 1 | GsfAsGfCAfCUfGAGAAUfACfUGfUCsfCsC |
| 103 3 | S46 2 | GsAsGCGACAfGfUfAUUCUCAGUsGsU | 970 | AS46 2 | AsfCsAfCUfGAfGAAUACfUGfUCfGCsfUsC |
| 103 9 | S46 8 | GsAsGCGAfCAfGfUfAUUCUCAGUsGsU | 976 | AS46 8 | AsfCsAfCUfGAfGAfAUACfUGfUCfGCsUsC |

(continued)

| SE Q ID NO. | single strand code | sense strand 5'→3' | SE Q ID NO. | single strand code | antisense strand sequence 5'→3' |
|---|---|---|---|---|---|
| 111 0 | S53 9 | AsAsGCGACAfGfUfAUUCU CAGUsGsU | 102 4 | AS51 6 | AsfCsAfCUfGAfGAAUACfUGfU CfGCsfUsU |
| 112 1 | S55 0 | AsAsGGfGAfCfAfGUAUUCU CAGUsGsA | 115 1 | AS52 9 | UsCsACUGfAGAAUfACfUGUCC CsUsU |

wherein, G=2'-O-methylguanylate, A=2'-O-methyladenylate, U=2'-O-methyluridylate, C=2'-O-methylcytidylate; fG=2'-fluoroguanylate, fA=2'-fluoroadenylate, fU=2'-fluorouridylate, fC=2'-fluorocytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate, fGs=2'-fluoro-3'-thioguanylate, fAs=2'-fluoro-3'-thioadenylate, fUs=2'-fluoro-3'-thiouridylate, fCs=2'-fluoro-3'-thiocytidylate, T=thymidylate.

**8.** The RNA inhibitor or pharmaceutically acceptable salt thereof according to any of claims 1-7, **characterized in that** the RNA inhibitor further contains a carrier structure, and has the structure as shown in Formula Ia, Ib or Ic:

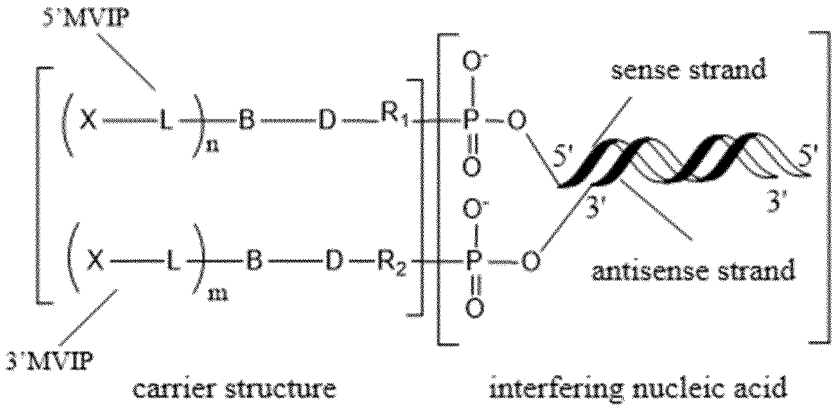


Ia

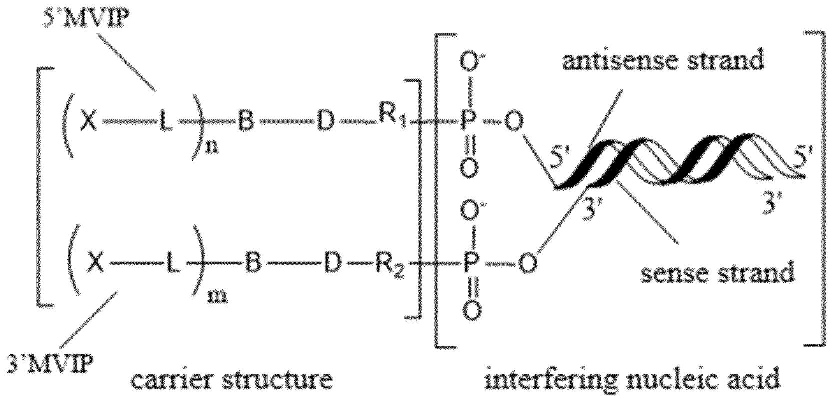


Ib

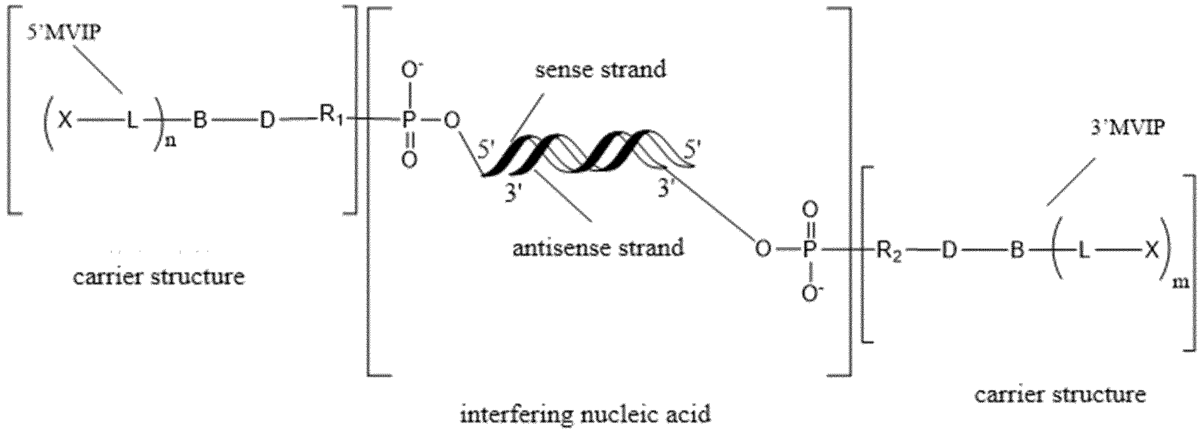


Ic

wherein,

the carrier structure includes a 5'MVIP and a 3 'MVIP;

the 5'MVIP is composed of a transition point $R_1$, a linking chain D, a linker B, a branched chain L and a liver targeting specific ligand X and is connected with 5' end of the sense strand or 5' end of the antisense strand through the transition point $R_1$, has the structure as shown in the formula I:

$$\mathbf{(X\text{-}L)_n\text{-}B\text{-}D\text{-}R_1} \qquad \text{I}$$

the 3'MVIP is composed of a transition point $R_2$, a linking chain D, a linker B, a branched chain L and a liver targeting specific ligand X and is connected with 3' end of the sense strand or 3' end of the antisense strand through the transition point $R_2$, has the structure as shown in the formula II:

$$\mathbf{(X\text{-}L)_m\text{-}B\text{-}D\text{-}R_2} \qquad \text{II}$$

wherein,

n and m are each independently any integer of 0-4, each independently preferably an integer of 1-3, and n+m = an integer of 2-6, preferably n+m=2, 3 or 4, more preferably 4,

the transition point $R_1$ is a heterocyclic or carbocyclic structure containing N, S or O as shown below,

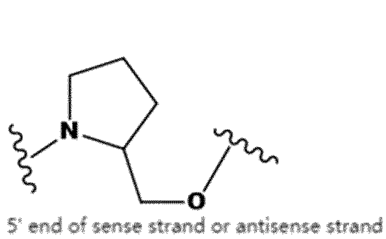


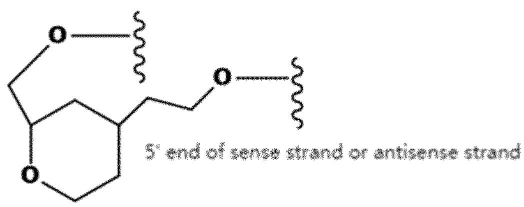


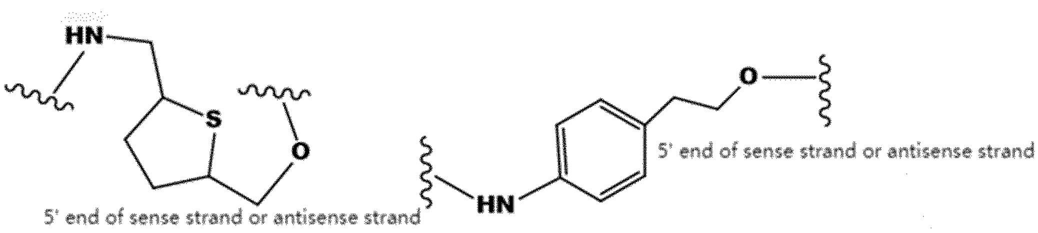


alternatively, $R_1$ is $-NH(CH_2)_xCH_2O-$, wherein x is any integer of 3-12, preferably any integer of 4-6;

the transition point $R_2$ is a heterocyclic or carbocyclic structure containing N, S or O as shown below,

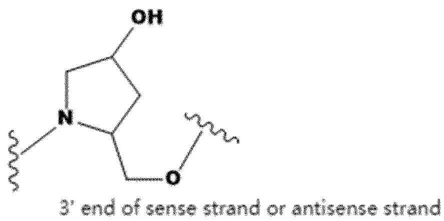


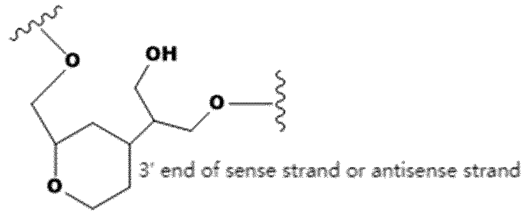


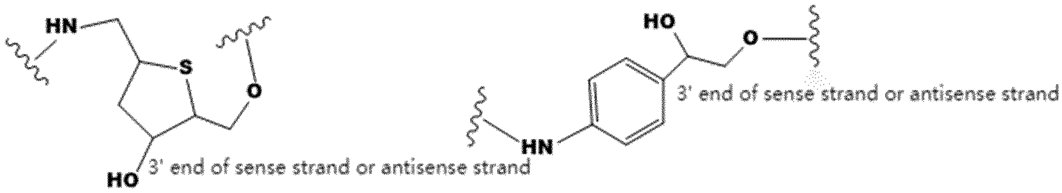


alternatively, the transition point $R_2$ is $-NH(CH_2)_{x1}CH(OH)(CH_2)_{x2}CH_2O-$, wherein x1 is any integer of 1-4, and x2 is any integer of 0-4;
the liver targeting specific ligand X may be the same or different within each of 5'MVIP and 3'MVIP or between 5'MVIP and 3'MVIP, and is selected from monosaccharides and their derivatives, preferably selected from N-acetylgalactosamine and their derivatives, and more preferably selected from the following structures::

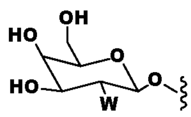 ; 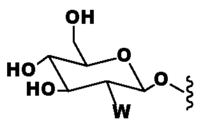 ; 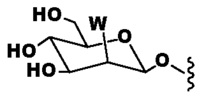 ;

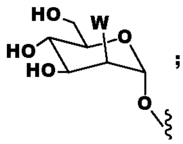 ; 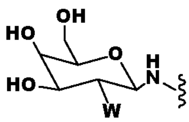 ; 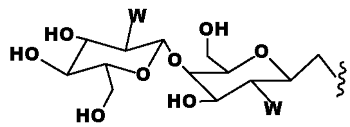 ;

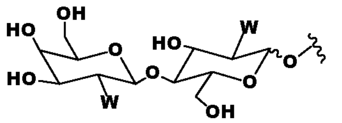 ; 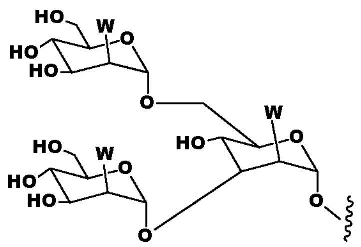 ;

wherein, W is selected from one or two of -OH, -NHCOOH and $-NHCO(CH_2)_qCH_3$, wherein q is an integer of 0-4.;
the branch chain L is the same or different within each of 5'MVIP and 3'MVIP or between 5'MVIP and 3'MVIP, which is selected from one or more of the following structures:

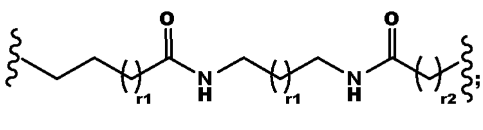 ; 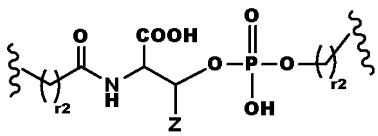 ;

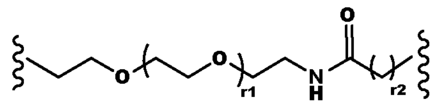 ; 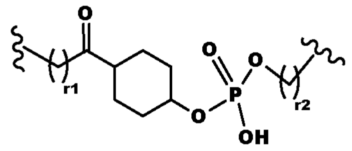 ;

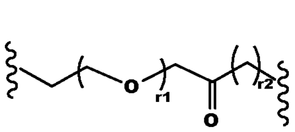 ; 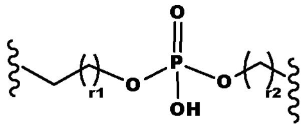 ; 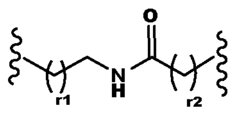 .

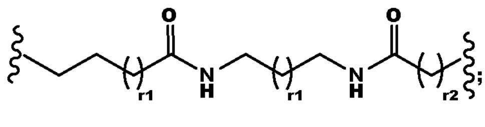 ; 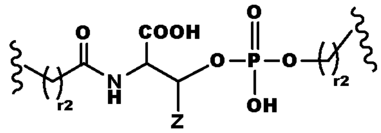 ;

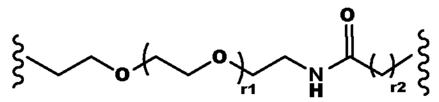 ; 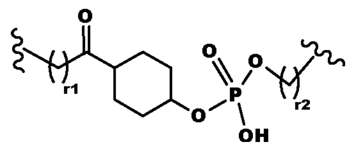 ;

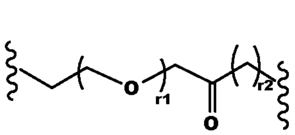 ; 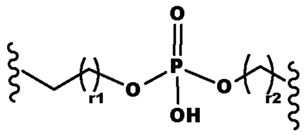 ; 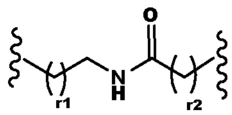 .

wherein, r1 is any integer of 1-12, r2 is any integer of 0-20, and Z is H, alkyl or amido, alkyl is, for example, $C_1$-$C_5$ alkyl;

the linker B is the same or different within each of 5'MVIP and 3'MVIP or between 5'MVIP and 3'MVIP, which is selected from the following structures:

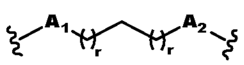 ; 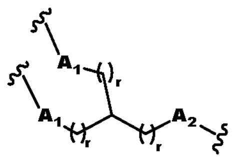 ; 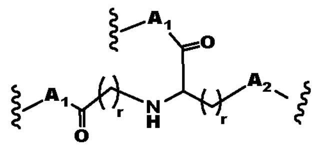 ;

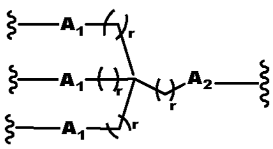 ; 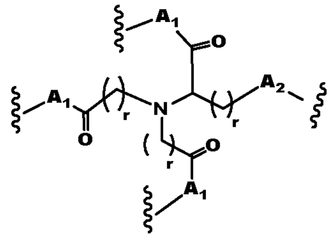 ; 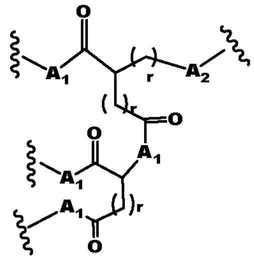 ;

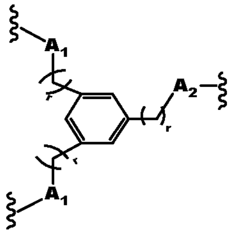 ; 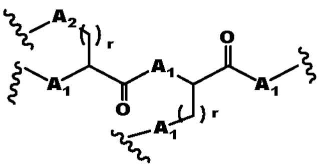 ;

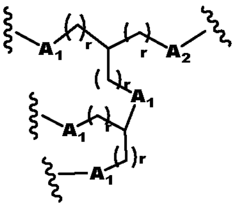 ; 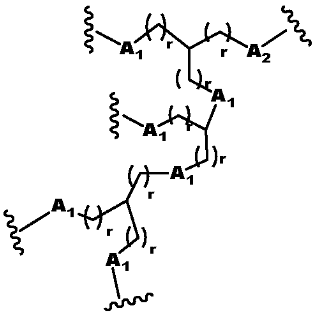 ; 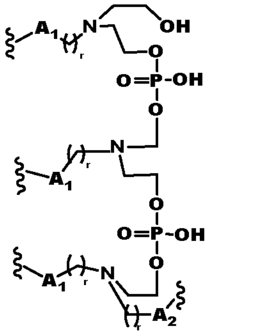 .

wherein, $A_1$ and $A_2$ are each independently C, O, S, -NH-, carbonyl, amido, phosphoryl or thiophosphoryl, and r is any integer of 0-4;
the linking chain D is the same or different within each of 5'MVIP and 3'MVIP or between 5'MVIP and 3'MVIP, which is selected from the following structures:

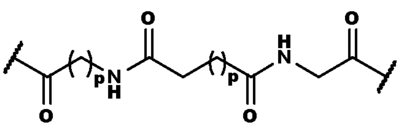 ; 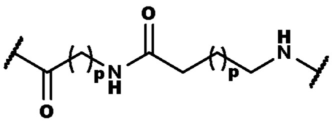 ; 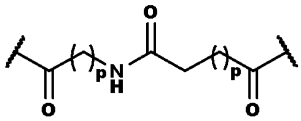 ;

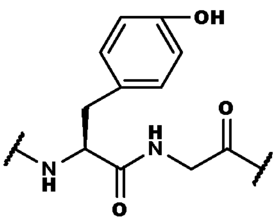 ; 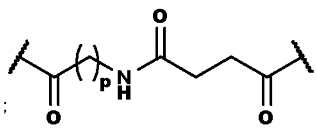 ; 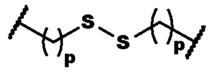 ; 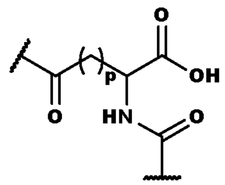 ;

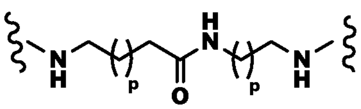 ; 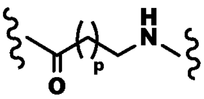 ; 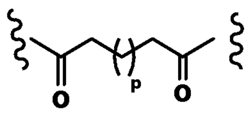 ;

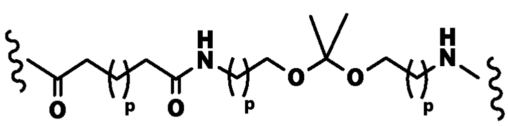 ; 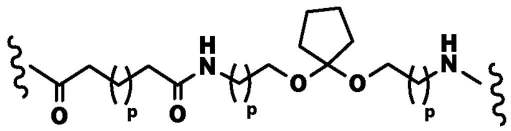 ;

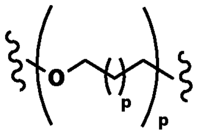 ; 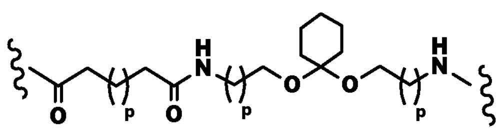 ; 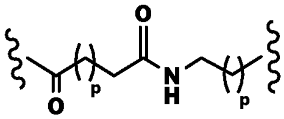 ;

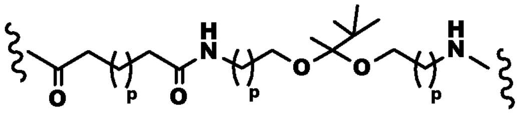 ; 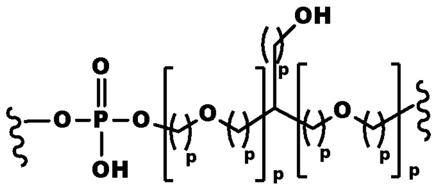 ;

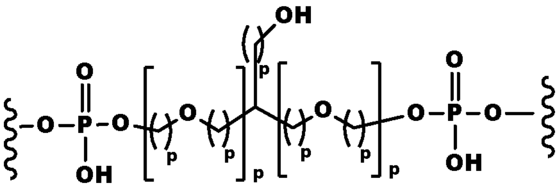 ; 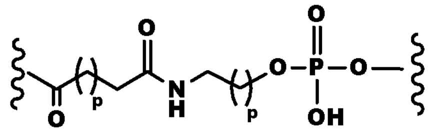 ;

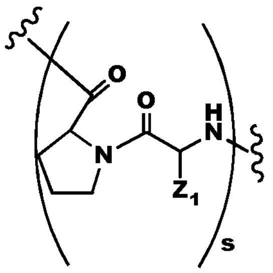 ; 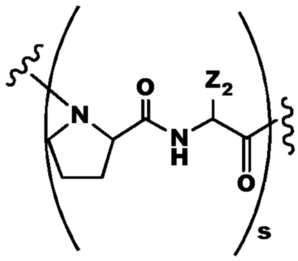 ; 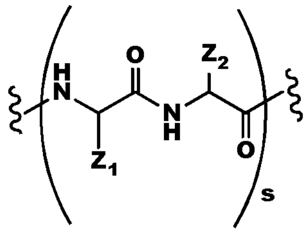 ;

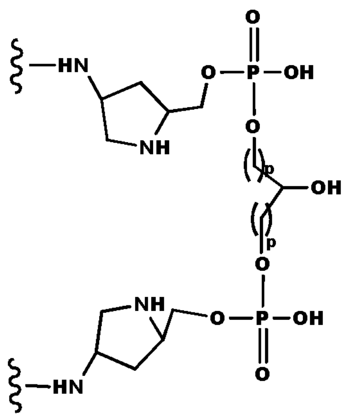 ; 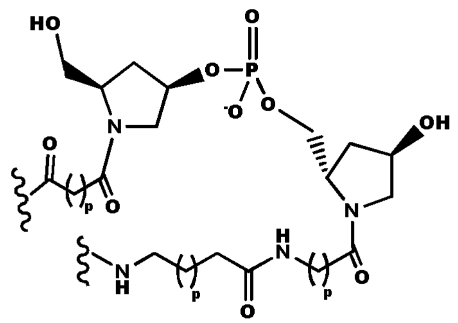 ;

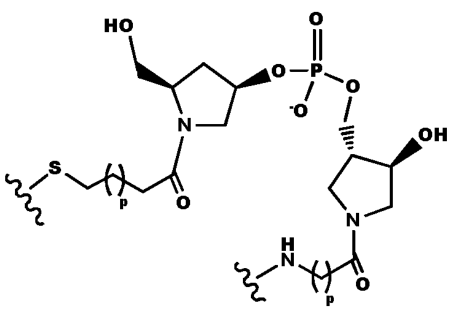 ; 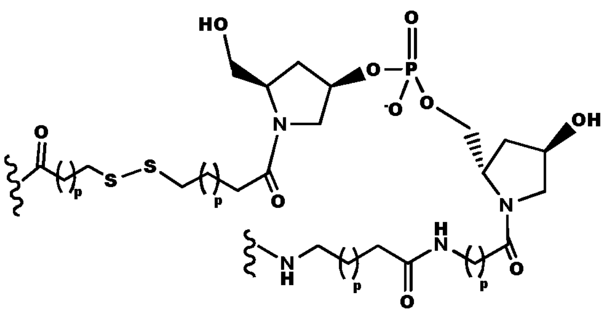 ;

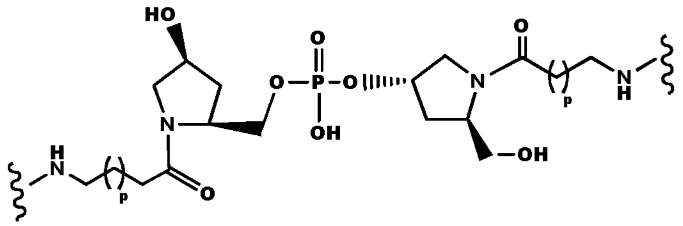

;

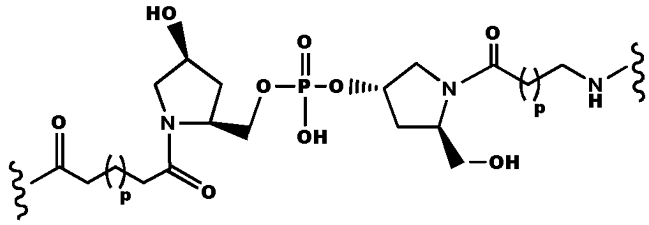

;

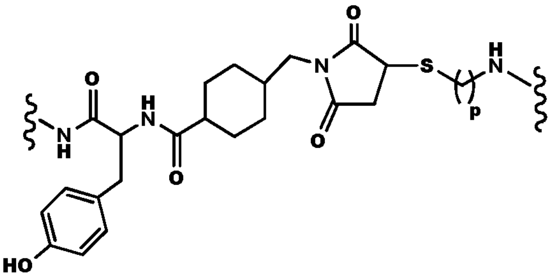

;

wherein, each p is independently any integer of 1-20; s is any integer of 2-13; $Z_1$ and $Z_2$ are the same or different substituent groups.

9. The RNA inhibitor or pharmaceutically acceptable salt thereof according to claim 8, **characterized in that** the 5'MVIP is 5'MVIP01, 5'MVIP09 or 5'MVIP17 as shown below, and the 3'MVIP is 3'MVIP01, 3'MVIP09 or 3'MVIP17 as shown below:

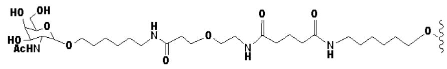

5'MVIP01

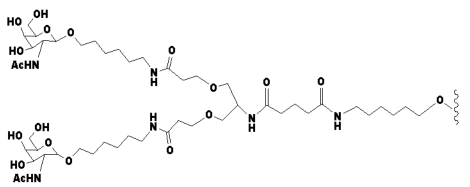

5'MVIP09

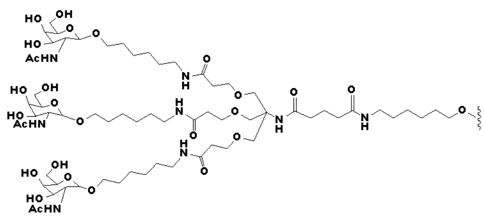

5'MVIP17

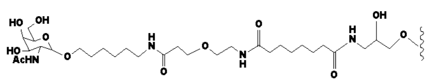

3'MVIP01

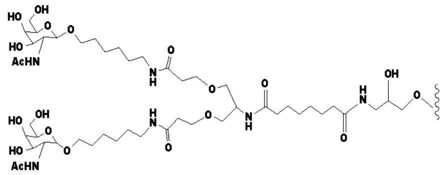

3'MVIP09

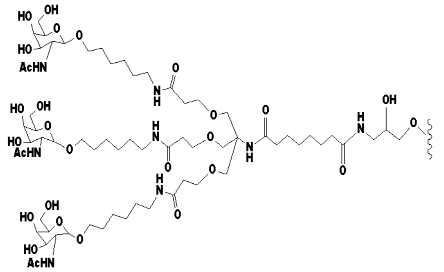

3'MVIP17

**10.** The RNA inhibitor or pharmaceutically acceptable salt thereof according to claim 9, **characterized in that** the carrier structure at 5' end of the sense strand is 5'MVIP17, the carrier structure at 3' end of the sense strand is 3'MVIP17, the combination of the sense strand 5'MVIP and the antisense strand 3'MVIP is 5'MVIP01/3'MVIP01, 5'MVIP01/3'MVIP17, 5'MVIP17/3'MVIP01 or 5'MVIP09/3'MVIP09, or the combination of the sense strand 5'MVIP and the antisense strand 3'MVIP is 5'MVIP01/3'MVIP09, 5'MVIP09/3'MVIP01 or 5'MVIP01/3'MVIP01.

**11.** The RNA inhibitor or pharmaceutically acceptable salt thereof according to claim 9, **characterized in that** the RNA inhibitor is selected from Ky-12-DS23001, Ky-12-DS25001, Ky-12-DS25401, Ky-12-DS29701, Ky-12-DS29801, Ky-12-DS31701, Ky-12-DS31702, Ky-12-DS31703, Ky-12-DS31704, Ky-12-DS31705, Ky-12-DS31706, Ky-12-DS31707, Ky-12-DS31708, Ky-12-DS31709, Ky-12-DS31711, Ky-12-DS31712, Ky-12-DS31713, Ky-12-DS33001, Ky-12-DS33006, Kylo-12-DS1071, Kylo-12-DS1081, Kylo-12-DS1131, Kylo-12-DS1141, Kylo-12-DS1241, Kylo-12-DS1311, Kylo-12-DS1321, Kylo-12-DS5911, Kylo-12-DS2911, Kylo-12-DS2611, Kylo-12-DS2311, Kylo-12-DS3111 and Kylo-12-DS5411.

**12.** Use of the RNA inhibitor or pharmaceutically acceptable salt thereof according to any of claims 1-11 in the preparation of a drug for treating and/or preventing a disease associated with an elevated APOC3 level, **characterized in that** the disease associated with an elevated APOC3 level includes hepatic diseases, which comprise inflammatory, cardiovascular and cerebrovascular and metabolic diseases, wherein the cardiovascular and cerebrovascular diseases include hyperlipidemia, stroke, atherosclerosis, thrombosis, coronary heart disease, aortic valve stenosis, hypertriglyceridemia (HTG), severe hypertriglyceridemia (sHTG) or familial chylomicronemia syndrome (FCS).

**13.** A pharmaceutical composition, **characterized in that** the composition comprises the APOC3 inhibitor or pharmaceutically acceptable salt thereof according to any of claims 1-11 and pharmaceutically acceptable excipients, wherein the pharmaceutical composition is an oral preparation, an intravenous injection, or a subcutaneous or intramuscular injection, preferably a subcutaneous injection.

Drawing

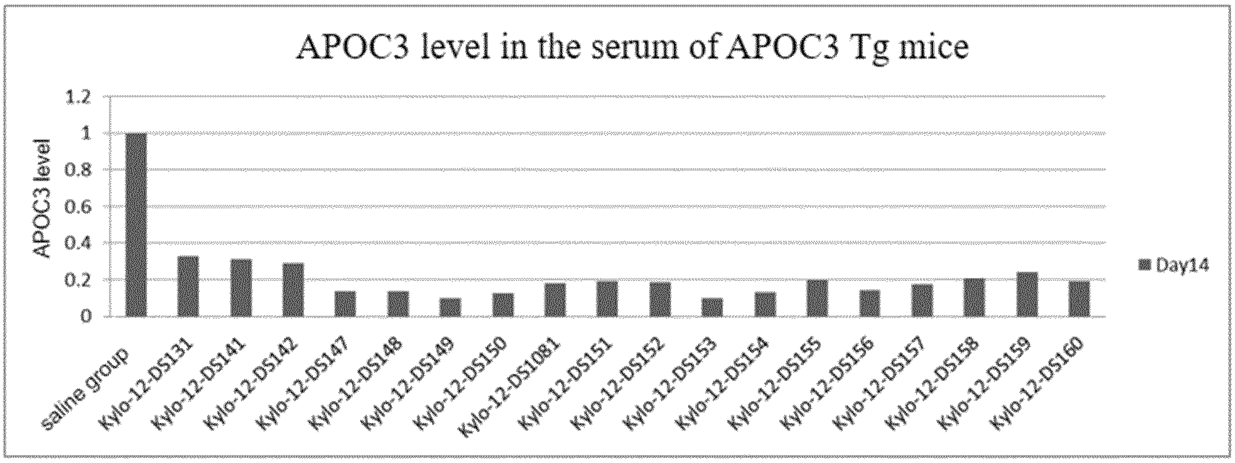


Figure 1

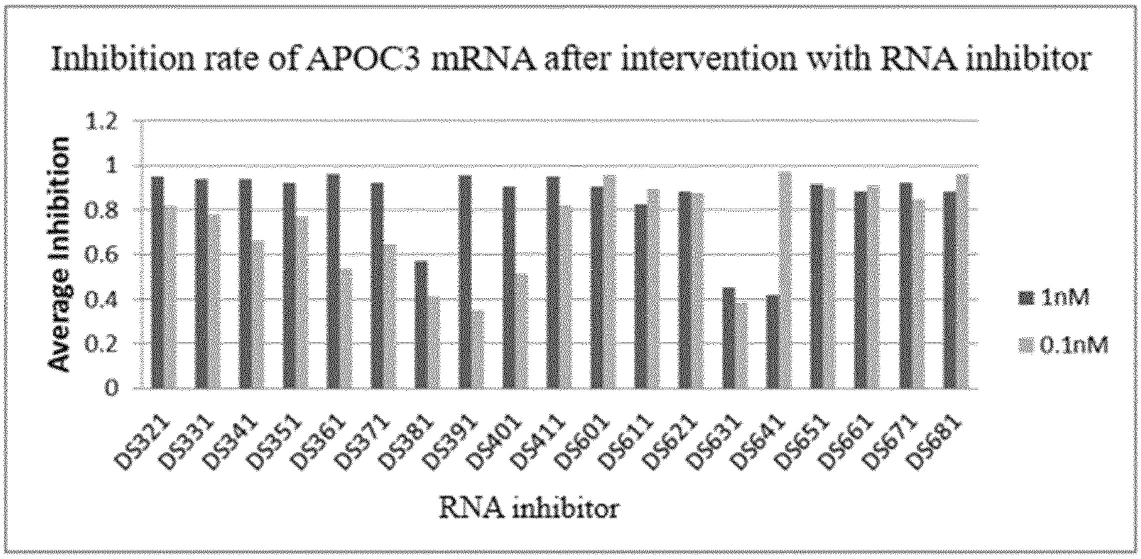


Figure 2

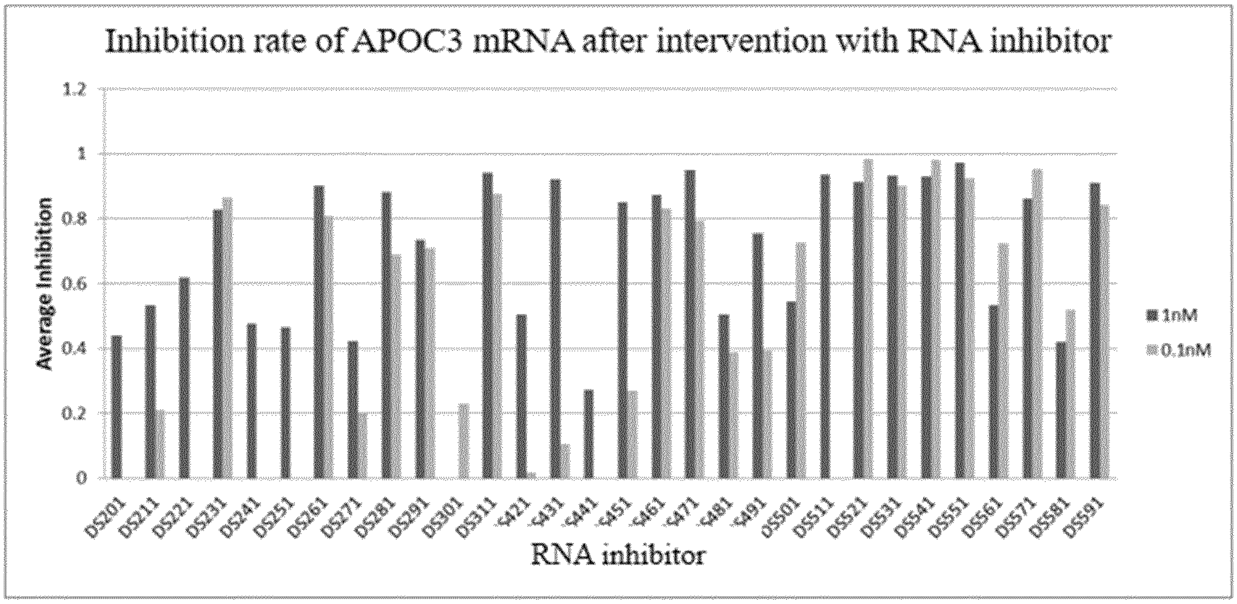


Figure 3

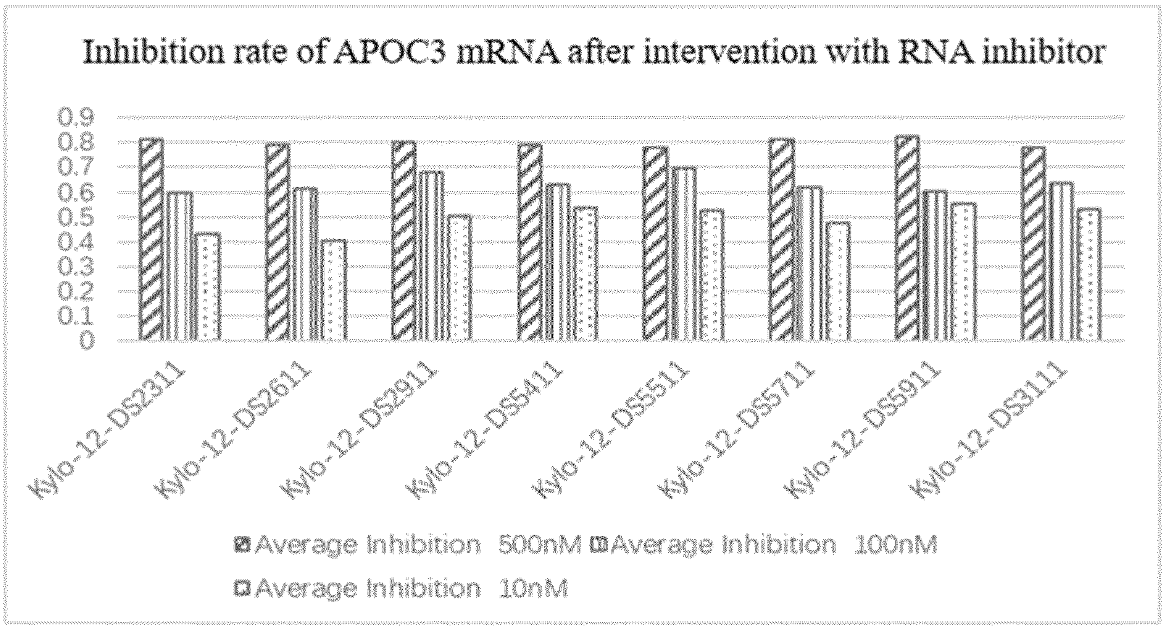


Figure 4

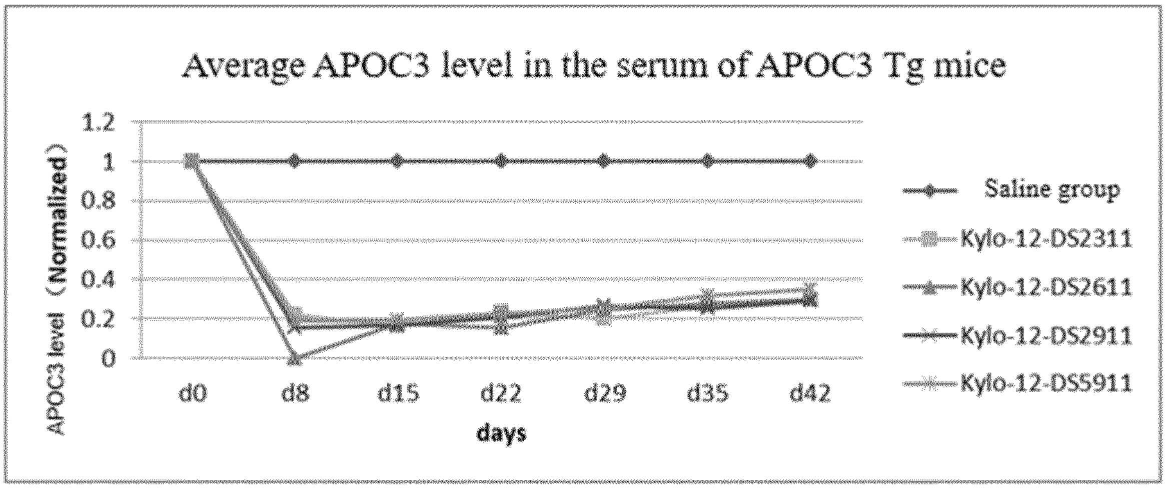


**Figure 5**

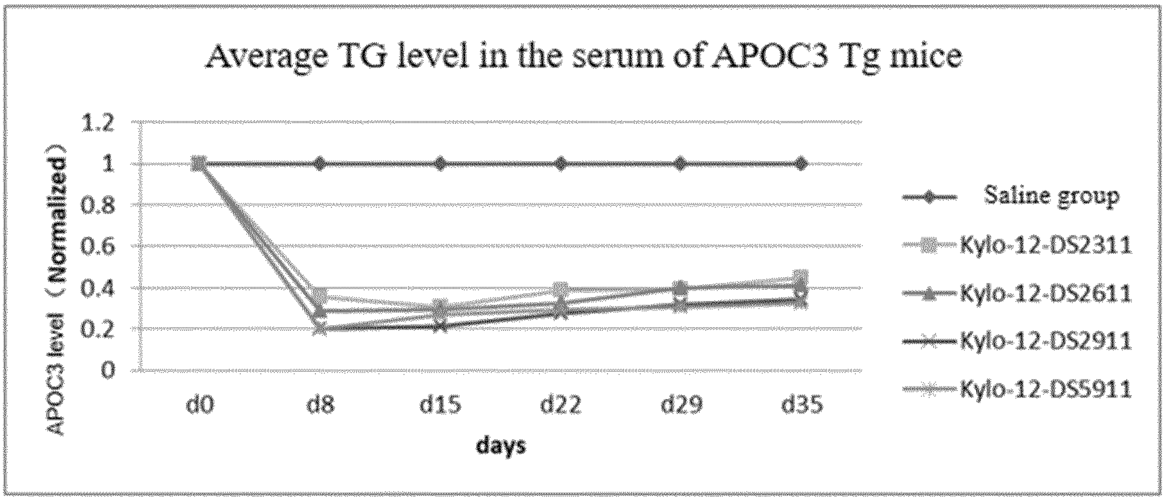


**Figure 6**

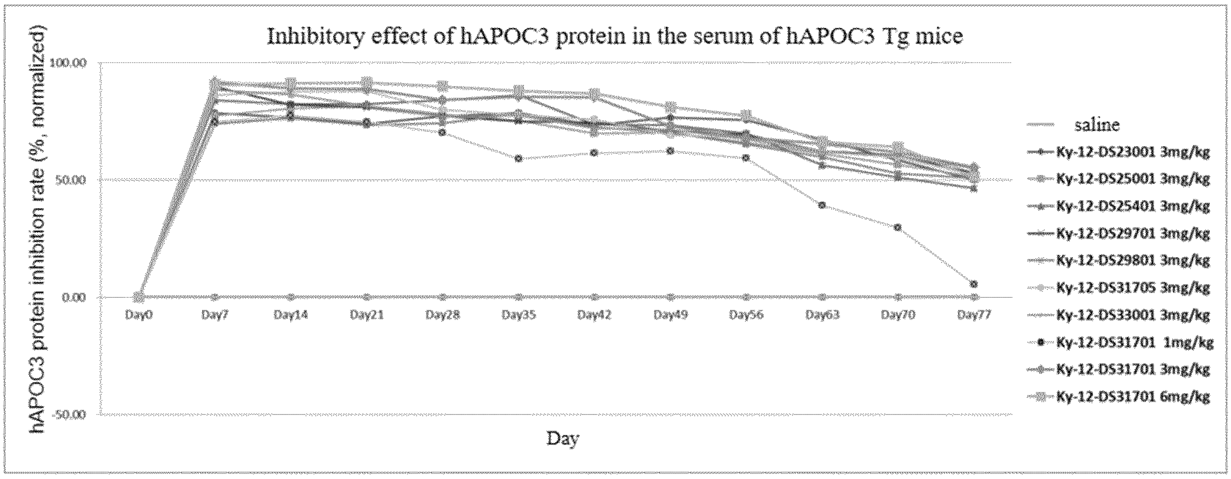


**Figure 7**

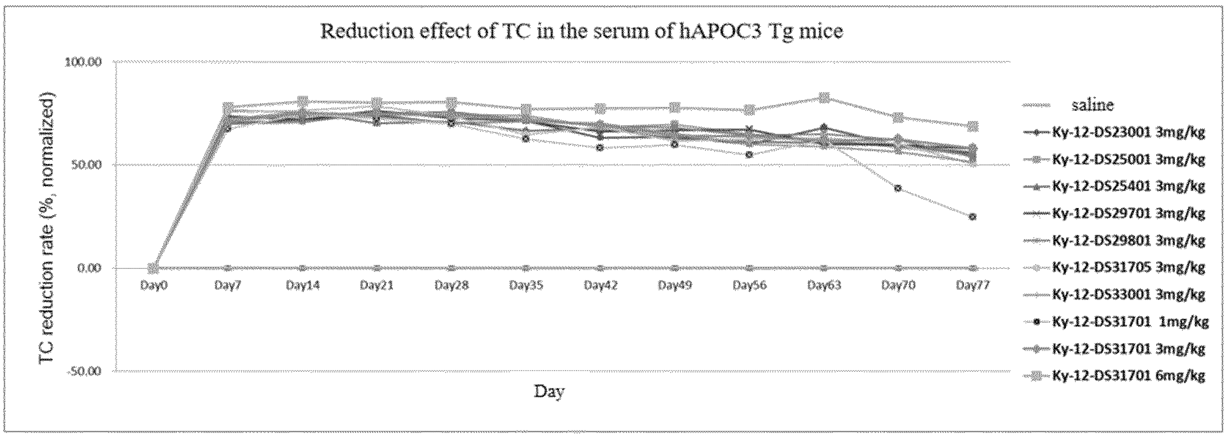


**Figure 8**

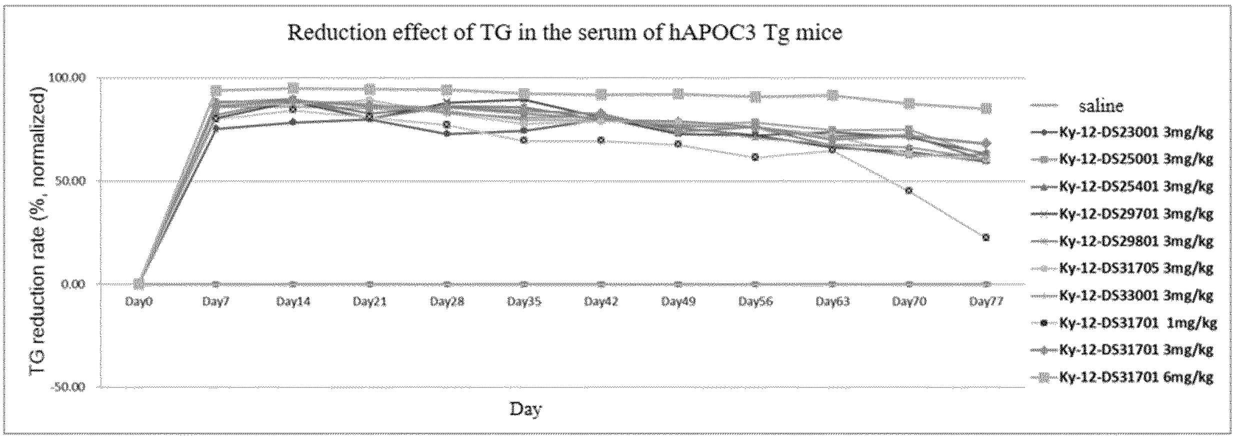


**Figure 9**

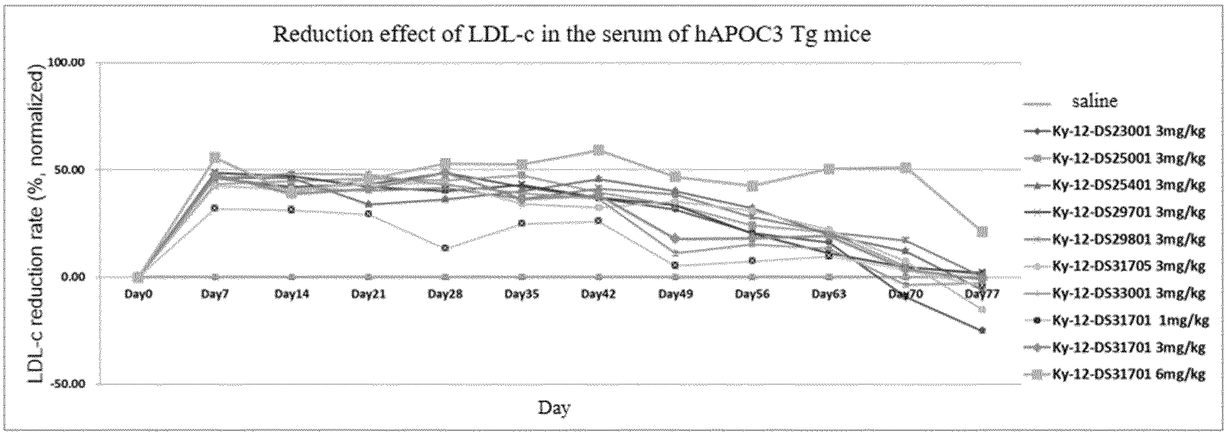


**Figure 10**

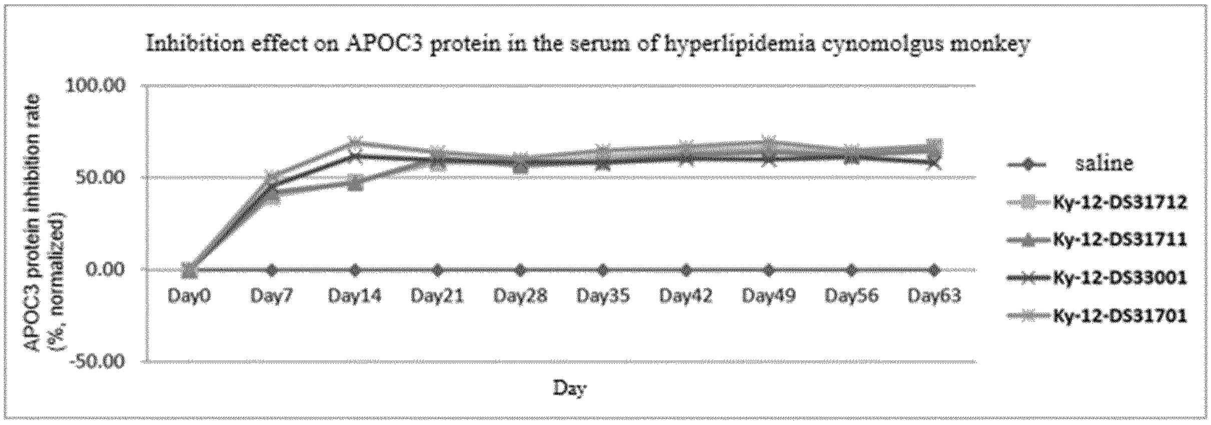


**Figure 11**

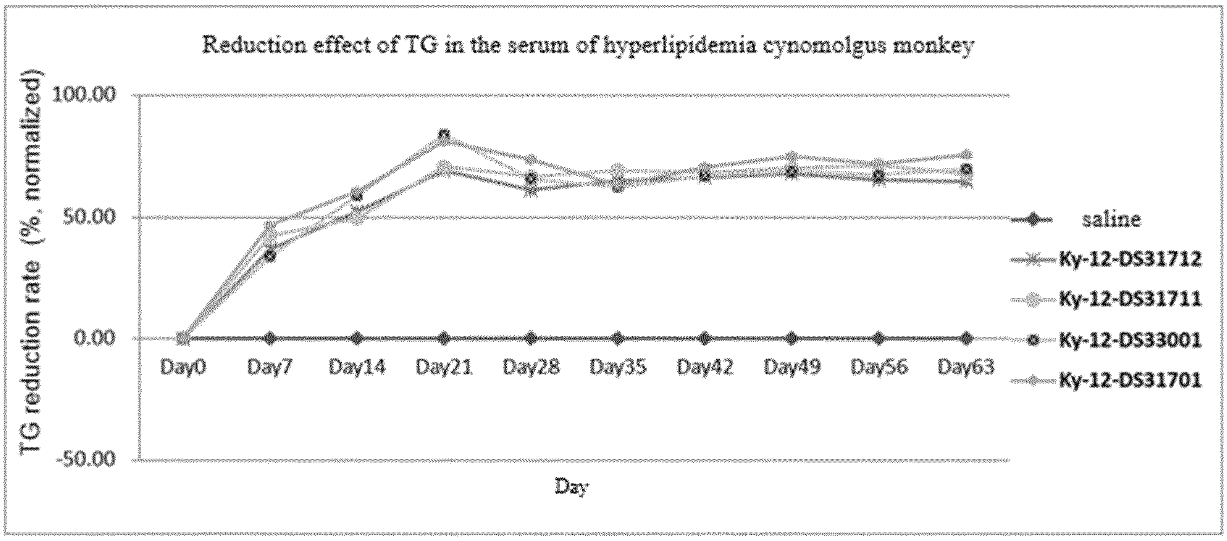


**Figure 12**

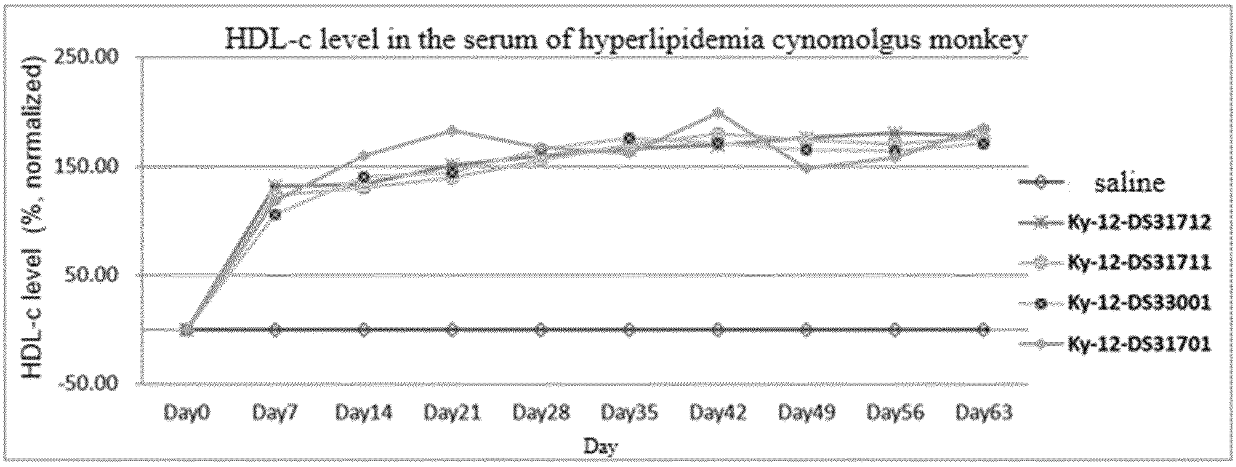


Figure 13

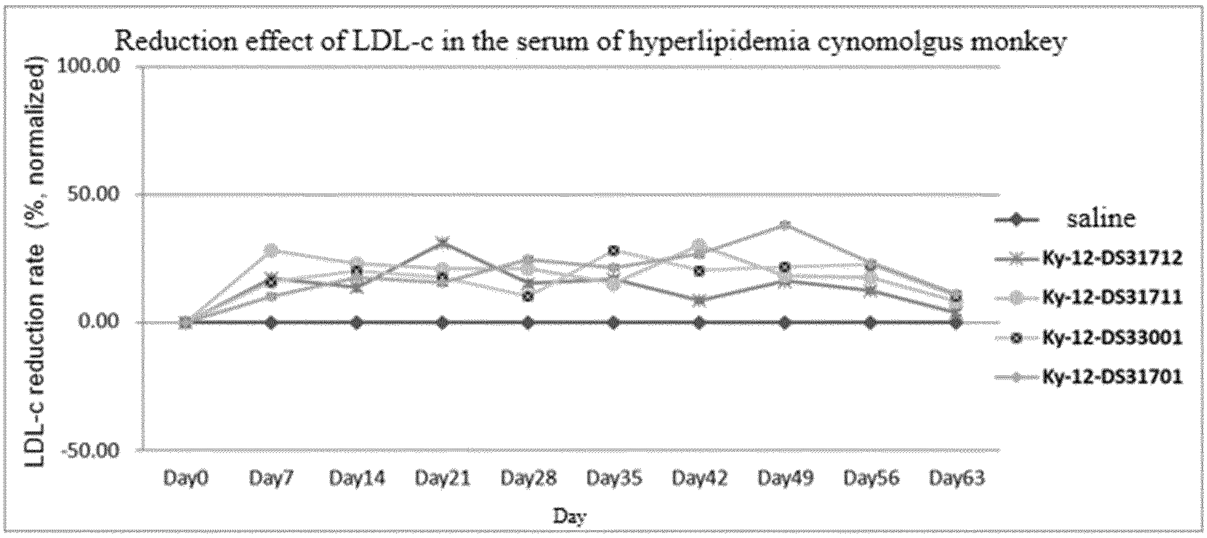


Figure 14

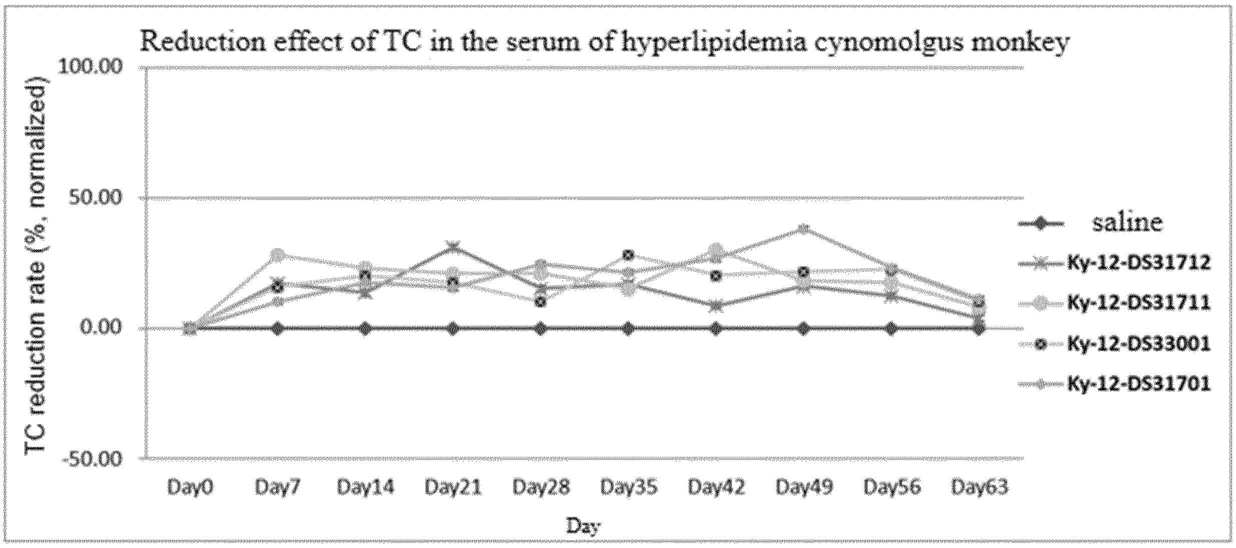
Reduction effect of TC in the serum of hyperlipidemia cynomolgus monkey
TC reduction rate (%, normalized)
100.00
50.00
0.00
-50.00
Day0
Day7
Day14
Day21
Day28
Day35
Day42
Day49
Day56
Day63
Day
saline
Ky-12-DS31712
Ky-12-DS31711
Ky-12-DS33001
Ky-12-DS31701

**Figure 15**

## INTERNATIONAL SEARCH REPORT

International application No.
**PCT/CN2023/111121**

**A. CLASSIFICATION OF SUBJECT MATTER**

C12N 15/113(2010.01)i; A61K 31/713(2006.01)i; A61P 1/16(2006.01)i; A61P 29/00(2006.01)i; A61P 3/06(2006.01)i; A61P 9/10(2006.01)i; A61P 7/02(2006.01)i; A61P 9/00(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

CNMED, CNABS, DWPI, WPABS, WPABSC, CJFD, AUABS, TWMED, ILABS, HKABS, MOABS, CNTXT, ENTXT, ENTXTC, WOTXT, USTXT, VCN, VEN, GBTXT, EPTXT, CATXT, CNKI, PubMed, EMBL, Web of Science, GoogleScholar: APOC3, APOCIII, apolipoprotein, 载脂蛋白, RNAi, siRNA, MVIP 中国专利生物序列检索系统, NCBI, EMBL, STN: 基于SEQ ID NO: 161和212的序列检索, China Patent Biological Sequence Search System, NCBI, EMBL, STN: search based on SEQ ID NOs: 161 and 212

**C. DOCUMENTS CONSIDERED TO BE RELEVANT**

| Category* | Citation of document, with indication, where appropriate, of the relevant passages | Relevant to claim No. |
|---|---|---|
| X | CN 111107853 A (ARROWHEAD PHARMACEUTICALS, INC.) 05 May 2020 (2020-05-05)<br>claims 1-6 and 51-53, and figure 1A | 12, 13 (all in part), 1, 4-6 |
| Y | CN 111107853 A (ARROWHEAD PHARMACEUTICALS, INC.) 05 May 2020 (2020-05-05)<br>claims 1-6 and 51-53, and figure 1A | 8-10 (in part) |
| Y | CN 113171371 A (KYLONOVA (XIAMEN) BIOPHARMA CO., LTD.) 27 July 2021 (2021-07-27)<br>description, paragraphs 30-41, 62-64, 70-76 and 89-92, tables 3-5, and claims 8-11 | 8-10 (in part) |
| A | US 2017260527 A1 (ALNYLAM PHARMACEUTICALS, INC.) 14 September 2017 (2017-09-14)<br>abstract | 2, 3, 7-13 (all in part), 1, 4-6 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 November 2023** | **25 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No. PCT/CN2023/111121

C. DOCUMENTS CONSIDERED TO BE RELEVANT

| Category* | Citation of document, with indication, where appropriate, of the relevant passages | Relevant to claim No. |
|---|---|---|
| A | CN 108743943 A (IONIS PHARMACEUTICALS, INC.) 06 November 2018 (2018-11-06) abstract, and sequence 3 | 2, 3, 7-13 (all in part), 1, 4-6 |
| A | CN 113293163 A (IONIS PHARMACEUTICALS, INC.) 24 August 2021 (2021-08-24) description, paragraphs 23-32, and sequences 1, 2 | 2, 3, 7-13 (all in part), 1, 4-6 |

Form PCT/ISA/210 (second sheet) (July 2022)

**INTERNATIONAL SEARCH REPORT**

International application No.
**PCT/CN2023/111121**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/111121**

**Box No. III Observations where unity of invention is lacking (Continuation of item 3 of first sheet)**

This International Searching Authority found multiple inventions in this international application, as follows:

Claim 1 sets forth an RNA inhibitor for inhibiting the expression of an APOC3 gene or a pharmaceutically acceptable salt thereof. The prior art document CN 111107853 A (publication date: 05 May 2020, see claims 1-3 and 40) discloses an RNAi reagent for inhibiting the expression of an APOC3 gene, the RNAi reagent comprising an antisense strand having a length of at least 17 nucleotides and a sense strand complementary to at least 85% of the antisense strand. Further disclosed is a composition comprising the RNAi reagent and a pharmaceutically acceptable excipient. Therefore, claim 1 lacks novelty and does not comply with PCT Article 33(2). Claims 2, 3 and 7-13, which refer to claim 1, each comprise 20 or more parallel technical solutions, do not have a special technical feature which makes a contribution over the prior art, cannot be associated with each other, and thus do not comply with PCT Rule 13.1 and 13.2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **the technical solutions of claims 2, 3 and 7-13 (all in part) that relate to a sense strand and an antisense strand being sequences respectively shown in SEQ ID NO: 161 and SEQ ID NO: 212, and claims 1 and 4-6**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2023/111121**

| Patent document cited in search report | | | Publication date (day/month/year) | Patent family member(s) | | | Publication date (day/month/year) |
|---|---|---|---|---|---|---|---|
| CN | 111107853 | A | 05 May 2020 | CL | 2020000593 | A1 | 25 September 2020 |
| | | | | PH | 12020500332 | A1 | 28 September 2020 |
| | | | | SG | 11201912178 | VA | 30 January 2020 |
| | | | | JP | 2020533334 | A | 19 November 2020 |
| | | | | UY | 37871 | A | 29 March 2019 |
| | | | | WO | 2019051402 | A1 | 14 March 2019 |
| | | | | JP | 2023073358 | A | 25 May 2023 |
| | | | | US | 2019078088 | A1 | 14 March 2019 |
| | | | | US | 10597657 | B2 | 24 March 2020 |
| | | | | US | 2020299690 | A1 | 24 September 2020 |
| | | | | US | 11214801 | B2 | 04 January 2022 |
| | | | | ECSP | 20017905 | A | 30 June 2020 |
| | | | | MX | 2020002648 | A | 22 July 2020 |
| | | | | JOP | 20200054 | A1 | 10 March 2020 |
| | | | | KR | 20200044013 | A | 28 April 2020 |
| | | | | CR | 20200108 | A | 28 June 2020 |
| | | | | CO | 2020002586 | A2 | 01 April 2020 |
| | | | | BR | 112020002413 | A2 | 28 July 2020 |
| | | | | AU | 2018329190 | A1 | 23 April 2020 |
| | | | | ZA | 202100806 | B | 29 March 2023 |
| | | | | TN | 2020000039 | A1 | 04 October 2021 |
| | | | | TW | 201920227 | A | 01 June 2019 |
| | | | | PE | 20201283 | A1 | 24 November 2020 |
| | | | | CA | 3074303 | A1 | 14 March 2019 |
| | | | | EP | 3681516 | A1 | 22 July 2020 |
| | | | | EP | 3681516 | A4 | 29 September 2021 |
| | | | | IL | 273172 | A | 30 April 2020 |
| | | | | US | 2022064646 | A1 | 03 March 2022 |
| | | | | US | 2023323350 | A1 | 12 October 2023 |
| CN | 113171371 | A | 27 July 2021 | None | | | |
| US | 2017260527 | A1 | 14 September 2017 | US | 11408001 | B1 | 09 August 2022 |
| | | | | US | 2023068339 | A1 | 02 March 2023 |
| | | | | US | 2020199591 | A1 | 25 June 2020 |
| | | | | US | 11142766 | B2 | 12 October 2021 |
| | | | | HK | 1244843 | A1 | 17 August 2018 |
| | | | | US | 2020080088 | A1 | 12 March 2020 |
| | | | | JP | 2020141685 | A | 10 September 2020 |
| | | | | WO | 2016081444 | A1 | 26 May 2016 |
| | | | | WO | 2016081444 | A9 | 21 July 2016 |
| | | | | AU | 2021212094 | A1 | 26 August 2021 |
| | | | | JP | 2017535552 | A | 30 November 2017 |
| | | | | AU | 2015350120 | A1 | 18 May 2017 |
| | | | | AU | 2015350120 | B2 | 27 May 2021 |
| | | | | EP | 3221451 | A1 | 27 September 2017 |
| | | | | US | 2021108203 | A1 | 15 April 2021 |
| | | | | US | 11034958 | B2 | 15 June 2021 |
| | | | | JP | 2023100887 | A | 19 July 2023 |
| | | | | CA | 2968114 | A1 | 26 May 2016 |
| | | | | US | 10407679 | B2 | 10 September 2019 |
| CN | 108743943 | A | 06 November 2018 | DK | 2956176 | T3 | 27 August 2018 |

Form PCT/ISA/210 (patent family annex) (July 2022)

**INTERNATIONAL SEARCH REPORT**
**Information on patent family members**

International application No.
**PCT/CN2023/111121**

| Patent document cited in search report | Publication date (day/month/year) | Patent family member(s) | Publication date (day/month/year) |
|---|---|---|---|
| | | US 2020017854 A1 | 16 January 2020 |
| | | EP 3400947 A1 | 14 November 2018 |
| | | EP 3400947 B1 | 23 August 2023 |
| | | IL 275246 A | 30 July 2020 |
| | | JP 2018135341 A | 30 August 2018 |
| | | JP 6681422 B2 | 15 April 2020 |
| | | CA 2901119 A1 | 21 August 2014 |
| | | CA 2901119 C | 18 October 2022 |
| | | ZA 201505628 B | 30 October 2019 |
| | | US 2017233732 A1 | 17 August 2017 |
| | | JP 2020111594 A | 27 July 2020 |
| | | BR 112015019515 A2 | 22 August 2017 |
| | | BR 112015019515 A8 | 03 October 2017 |
| | | JP 2016513110 A | 12 May 2016 |
| | | JP 6313789 B2 | 18 April 2018 |
| | | IL 240338 A0 | 24 September 2015 |
| | | IL 240338 B | 30 June 2020 |
| | | RU 2015139055 A | 20 March 2017 |
| | | RU 2661781 C2 | 19 July 2018 |
| | | AU 2020202300 A1 | 23 April 2020 |
| | | US 2015376614 A1 | 31 December 2015 |
| | | US 9593333 B2 | 14 March 2017 |
| | | NZ 631119 A | 31 March 2017 |
| | | RU 2018121529 A | 06 March 2019 |
| | | HK 1217441 A1 | 13 January 2017 |
| | | AU 2014216137 A1 | 20 August 2015 |
| | | AU 2014216137 B2 | 10 May 2018 |
| | | MX 2019011188 A | 24 October 2019 |
| | | AU 2018211325 A1 | 30 August 2018 |
| | | AU 2018211325 B2 | 02 January 2020 |
| | | US 2022213472 A1 | 07 July 2022 |
| | | ES 2680599 T3 | 10 September 2018 |
| | | CA 3170716 A1 | 21 August 2014 |
| | | EP 2956176 A2 | 23 December 2015 |
| | | EP 2956176 A4 | 26 October 2016 |
| | | EP 2956176 B1 | 27 June 2018 |
| | | WO 2014127268 A2 | 21 August 2014 |
| | | WO 2014127268 A3 | 16 October 2014 |
| | | KR 20200123263 A | 28 October 2020 |
| | | MX 2015010487 A | 30 October 2015 |
| | | MX 368155 B | 20 September 2019 |
| | | KR 20150118180 A | 21 October 2015 |
| | | KR 102169899 B1 | 26 October 2020 |
| CN 113293163 A | 24 August 2021 | ES 2778442 T3 | 10 August 2020 |
| | | RU 2019124314 A | 21 August 2019 |
| | | EA 201891479 A1 | 30 November 2018 |
| | | EA 036584 B1 | 26 November 2020 |
| | | IL 261901 A | 31 October 2018 |
| | | IL 261901 B | 31 May 2020 |
| | | RS 60796 B1 | 30 October 2020 |

Form PCT/ISA/210 (patent family annex) (July 2022)

**INTERNATIONAL SEARCH REPORT**
**Information on patent family members**

International application No.
**PCT/CN2023/111121**

| Patent document cited in search report | Publication date (day/month/year) | Patent family member(s) | | | Publication date (day/month/year) |
|---|---|---|---|---|---|
| | | MX | 2020004209 | A | 13 August 2020 |
| | | IL | 263843 | A | 31 January 2019 |
| | | IL | 263843 | B | 31 March 2020 |
| | | MX | 2019010441 | A | 17 October 2019 |
| | | US | 2016076032 | A1 | 17 March 2016 |
| | | US | 9714421 | B2 | 25 July 2017 |
| | | KR | 20190084138 | A | 15 July 2019 |
| | | KR | 102212275 | B1 | 05 February 2021 |
| | | WO | 2014179620 | A1 | 06 November 2014 |
| | | KR | 20230006933 | A | 11 January 2023 |
| | | JP | 2020058370 | A | 16 April 2020 |
| | | JP | 2016526874 | A | 08 September 2016 |
| | | JP | 6387084 | B2 | 05 September 2018 |
| | | IL | 242124 | B | 28 February 2019 |
| | | IL | 296543 | A | 01 November 2022 |
| | | EP | 2991656 | A2 | 09 March 2016 |
| | | EP | 2991656 | A4 | 22 February 2017 |
| | | EP | 2991656 | B1 | 18 December 2019 |
| | | HUE | 050394 | T2 | 30 November 2020 |
| | | AU | 2017200365 | A1 | 23 February 2017 |
| | | AU | 2017200365 | B2 | 08 November 2018 |
| | | AU | 2017200365 | C1 | 18 April 2019 |
| | | HRP | 20201378 | T1 | 27 November 2020 |
| | | EP | 3633039 | A1 | 08 April 2020 |
| | | JP | 2016523515 | A | 12 August 2016 |
| | | JP | 6456362 | B2 | 23 January 2019 |
| | | US | 2023151365 | A1 | 18 May 2023 |
| | | US | 2016017323 | A1 | 21 January 2016 |
| | | US | 2015176007 | A1 | 25 June 2015 |
| | | US | 9145558 | B2 | 29 September 2015 |
| | | JP | 2021020901 | A | 18 February 2021 |
| | | JP | 7177127 | B2 | 22 November 2022 |
| | | US | 2018273953 | A1 | 27 September 2018 |
| | | US | 10883104 | B2 | 05 January 2021 |
| | | AU | 2014259759 | A1 | 22 October 2015 |
| | | AU | 2014259759 | B2 | 18 June 2020 |
| | | IL | 274064 | A | 30 June 2020 |
| | | IL | 274064 | B | 30 June 2021 |
| | | PT | 2992098 | T | 05 July 2019 |
| | | IL | 242132 | B | 31 October 2018 |
| | | US | 2021395734 | A1 | 23 December 2021 |
| | | US | 2021130823 | A1 | 06 May 2021 |
| | | JP | 2023113843 | A | 16 August 2023 |
| | | CA | 2921162 | A1 | 06 November 2014 |
| | | HK | 1221404 | A1 | 02 June 2017 |
| | | AU | 2014259757 | A1 | 22 October 2015 |
| | | AU | 2014259757 | B2 | 02 March 2017 |
| | | AU | 2022202770 | A1 | 19 May 2022 |
| | | UA | 120287 | C2 | 11 November 2019 |
| | | IL | 264241 | A | 28 February 2019 |

Form PCT/ISA/210 (patent family annex) (July 2022)

**INTERNATIONAL SEARCH REPORT**
**Information on patent family members**

International application No.
**PCT/CN2023/111121**

| Patent document cited in search report | Publication date (day/month/year) | Patent family member(s) | | | Publication date (day/month/year) |
|---|---|---|---|---|---|
| | | IL | 264241 | B | 30 April 2020 |
| | | LT | 2992098 | T | 10 July 2019 |
| | | AU | 2020207820 | A1 | 06 August 2020 |
| | | US | 2018273952 | A1 | 27 September 2018 |
| | | US | 10927372 | B2 | 23 February 2021 |
| | | JP | 2020039355 | A | 19 March 2020 |
| | | ES | 2819213 | T3 | 15 April 2021 |
| | | MY | 178929 | A | 23 October 2020 |
| | | AU | 2014259755 | A1 | 22 October 2015 |
| | | AU | 2014259755 | B2 | 30 August 2018 |
| | | AU | 2019200820 | A1 | 28 February 2019 |
| | | AU | 2019200820 | B2 | 30 April 2020 |
| | | PT | 3524680 | T | 04 January 2021 |
| | | CR | 20190269 | A | 13 September 2019 |
| | | KR | 20210151260 | A | 13 December 2021 |
| | | KR | 102558571 | B1 | 21 July 2023 |
| | | US | 2016076030 | A1 | 17 March 2016 |
| | | US | 9932580 | B2 | 03 April 2018 |
| | | US | 2020224198 | A1 | 16 July 2020 |
| | | EP | 2992009 | A1 | 09 March 2016 |
| | | EP | 2992009 | A4 | 28 December 2016 |
| | | EP | 2992009 | B1 | 24 June 2020 |
| | | HK | 1221486 | A1 | 02 June 2017 |
| | | CL | 2015003217 | A1 | 08 July 2016 |
| | | AU | 2019203674 | A1 | 27 June 2019 |
| | | AU | 2019203674 | B2 | 25 March 2021 |
| | | SG | 11201508800 | WA | 27 November 2015 |
| | | MX | 2015015263 | A | 16 December 2016 |
| | | AU | 2018267625 | A1 | 13 December 2018 |
| | | AU | 2018267625 | B2 | 10 September 2020 |
| | | JP | 2020007361 | A | 16 January 2020 |
| | | NZ | 740338 | A | 29 April 2022 |
| | | IL | 264580 | A | 28 February 2019 |
| | | IL | 264580 | B | 30 April 2020 |
| | | HK | 1221475 | A1 | 02 June 2017 |
| | | MX | 2020002184 | A | 14 July 2020 |
| | | CY | 1123369 | T1 | 31 December 2021 |
| | | BR | 112015027319 | A2 | 26 September 2017 |
| | | BR | 112015027319 | A8 | 02 January 2018 |
| | | RU | 2018136140 | A | 17 December 2018 |
| | | RU | 2018136140 | A3 | 27 April 2022 |
| | | HUE | 043697 | T2 | 30 September 2019 |
| | | US | 2021024923 | A1 | 28 January 2021 |
| | | US | 2022275365 | A9 | 01 September 2022 |
| | | DOP | 2021000095 | A | 15 September 2021 |
| | | IL | 273205 | A | 30 April 2020 |
| | | BR | 112015027321 | A2 | 26 September 2017 |
| | | BR | 112015027321 | A8 | 02 January 2018 |
| | | PE | 20152002 | A1 | 21 January 2016 |
| | | IL | 273184 | A | 30 April 2020 |

Form PCT/ISA/210 (patent family annex) (July 2022)

**INTERNATIONAL SEARCH REPORT**
**Information on patent family members**

International application No.
**PCT/CN2023/111121**

| Patent document cited in search report | Publication date (day/month/year) | Patent family member(s) | | | Publication date (day/month/year) |
|---|---|---|---|---|---|
| | | IL | 273184 | B | 29 July 2021 |
| | | EP | 2991661 | A1 | 09 March 2016 |
| | | EP | 2991661 | A4 | 15 February 2017 |
| | | EP | 2991661 | B1 | 13 March 2019 |
| | | EP | 2992097 | A2 | 09 March 2016 |
| | | EP | 2992097 | A4 | 04 January 2017 |
| | | EP | 2992097 | B1 | 06 November 2019 |
| | | NZ | 631512 | A | 28 October 2016 |
| | | CL | 2016002262 | A1 | 09 June 2017 |
| | | RU | 2015151203 | A | 02 June 2017 |
| | | RU | 2650510 | C2 | 16 April 2018 |
| | | KR | 20210014758 | A | 09 February 2021 |
| | | WO | 2014179629 | A2 | 06 November 2014 |
| | | WO | 2014179629 | A3 | 22 January 2015 |
| | | WO | 2014179629 | A8 | 02 June 2016 |
| | | EP | 3828275 | A1 | 02 June 2021 |
| | | IL | 283660 | A | 29 July 2021 |
| | | SG | 11201508870 | VA | 27 November 2015 |
| | | KR | 20180051678 | A | 16 May 2018 |
| | | KR | 102138781 | B1 | 28 July 2020 |
| | | KR | 20160002976 | A | 08 January 2016 |
| | | KR | 102315836 | B1 | 22 October 2021 |
| | | ES | 2730015 | T3 | 07 November 2019 |
| | | PH | 12015502493 | A1 | 22 February 2016 |
| | | HK | 1221403 | A1 | 02 June 2017 |
| | | MX | 2015015234 | A | 03 October 2016 |
| | | AU | 2017203436 | A1 | 08 June 2017 |
| | | AU | 2017203436 | B2 | 18 October 2018 |
| | | ME | 03390 | B | 20 January 2020 |
| | | IL | 242125 | B | 28 February 2019 |
| | | RU | 2015151199 | A | 05 June 2017 |
| | | RU | 2015151199 | A3 | 27 March 2018 |
| | | RU | 2697152 | C2 | 12 August 2019 |
| | | PE | 20161430 | A1 | 06 January 2017 |
| | | DK | 3524680 | T3 | 14 December 2020 |
| | | KR | 20220108195 | A | 02 August 2022 |
| | | EP | 2992098 | A2 | 09 March 2016 |
| | | EP | 2992098 | A4 | 11 January 2017 |
| | | EP | 2992098 | B1 | 27 March 2019 |
| | | NZ | 753018 | A | 28 January 2022 |
| | | BR | 112015027369 | A2 | 26 September 2017 |
| | | BR | 112015027369 | A8 | 02 January 2018 |
| | | BR | 112015027369 | B1 | 08 June 2021 |
| | | SG | 10201801507 | RA | 28 March 2018 |
| | | JP | 2021107408 | A | 29 July 2021 |
| | | JP | 7339294 | B2 | 05 September 2023 |
| | | JP | 2016522817 | A | 04 August 2016 |
| | | JP | 6216444 | B2 | 18 October 2017 |
| | | JP | 2019056001 | A | 11 April 2019 |
| | | JP | 6639629 | B2 | 05 February 2020 |

Form PCT/ISA/210 (patent family annex) (July 2022)

**INTERNATIONAL SEARCH REPORT**
**Information on patent family members**

International application No.
**PCT/CN2023/111121**

| Patent document cited in search report | Publication date (day/month/year) | Patent family member(s) | | | Publication date (day/month/year) |
|---|---|---|---|---|---|
| | | JP | 2018027091 | A | 22 February 2018 |
| | | JP | 6592486 | B2 | 16 October 2019 |
| | | WO | 2014179627 | A2 | 06 November 2014 |
| | | WO | 2014179627 | A9 | 26 February 2015 |
| | | WO | 2014179627 | A3 | 16 April 2015 |
| | | CA | 2921514 | A1 | 06 November 2014 |
| | | DK | 2992098 | T3 | 17 June 2019 |
| | | NZ | 725538 | A | 26 February 2021 |
| | | DOP | 2016000287 | A | 15 February 2017 |
| | | PL | 2992098 | T3 | 30 September 2019 |
| | | JP | 2020074787 | A | 21 May 2020 |
| | | US | 2018044676 | A1 | 15 February 2018 |
| | | US | 10683499 | B2 | 16 June 2020 |
| | | JP | 2016522683 | A | 04 August 2016 |
| | | JP | 6995478 | B2 | 14 January 2022 |
| | | WO | 2014179626 | A2 | 06 November 2014 |
| | | WO | 2014179626 | A3 | 26 February 2015 |
| | | US | 2016090596 | A1 | 31 March 2016 |
| | | US | 9957504 | B2 | 01 May 2018 |
| | | RU | 2018112167 | A | 07 March 2019 |
| | | ZA | 201507218 | B | 27 September 2023 |
| | | NZ | 631552 | A | 24 February 2017 |
| | | AU | 2021204244 | A1 | 22 July 2021 |
| | | SG | 10201906382 | QA | 27 August 2019 |
| | | SI | 2992009 | T1 | 30 October 2020 |
| | | IL | 273312 | A | 30 April 2020 |
| | | JP | 2018183184 | A | 22 November 2018 |
| | | JP | 6652602 | B2 | 26 February 2020 |
| | | US | 2015126719 | A1 | 07 May 2015 |
| | | US | 9163239 | B2 | 20 October 2015 |
| | | JP | 2023012548 | A | 25 January 2023 |
| | | CA | 2921518 | A1 | 06 November 2014 |
| | | ZA | 201507216 | B | 30 August 2017 |
| | | NZ | 712737 | A | 27 August 2021 |
| | | AU | 2017200950 | A1 | 02 March 2017 |
| | | AU | 2017200950 | B2 | 17 January 2019 |
| | | NZ | 728517 | A | 24 December 2021 |
| | | PH | 12019501191 | A1 | 01 March 2021 |
| | | MY | 198359 | A | 28 August 2023 |
| | | EP | 3524680 | A1 | 14 August 2019 |
| | | EP | 3524680 | B1 | 11 November 2020 |
| | | MX | 2021008901 | A | 19 August 2021 |
| | | US | 2014343123 | A1 | 20 November 2014 |
| | | US | 9127276 | B2 | 08 September 2015 |
| | | US | 2018002693 | A1 | 04 January 2018 |
| | | US | 2016090595 | A1 | 31 March 2016 |
| | | US | 9932581 | B2 | 03 April 2018 |
| | | WO | 2014179625 | A1 | 06 November 2014 |
| | | ES | 2885174 | T3 | 13 December 2021 |
| | | IL | 272617 | A | 31 March 2020 |

Form PCT/ISA/210 (patent family annex) (July 2022)

**INTERNATIONAL SEARCH REPORT**
**Information on patent family members**

International application No.
**PCT/CN2023/111121**

| Patent document cited in search report | Publication date (day/month/year) | Patent family member(s) | | | Publication date (day/month/year) |
|---|---|---|---|---|---|
| | | EP | 3690049 | A1 | 05 August 2020 |
| | | BR | 112015027377 | A2 | 29 August 2017 |
| | | BR | 112015027377 | A8 | 03 October 2017 |
| | | BR | 112015027377 | B1 | 10 January 2023 |
| | | MX | 2021008899 | A | 19 August 2021 |
| | | AU | 2014259756 | A1 | 22 October 2015 |
| | | AU | 2014259756 | B2 | 23 February 2017 |
| | | UA | 121017 | C2 | 25 March 2020 |
| | | JP | 2020039354 | A | 19 March 2020 |
| | | JP | 6866459 | B2 | 28 April 2021 |
| | | US | 2015126720 | A1 | 07 May 2015 |
| | | US | 9181550 | B2 | 10 November 2015 |
| | | MX | 2015015264 | A | 12 August 2016 |
| | | KR | 20210037752 | A | 06 April 2021 |
| | | US | 2019367914 | A1 | 05 December 2019 |
| | | US | 10844379 | B2 | 24 November 2020 |
| | | JP | 2022017514 | A | 25 January 2022 |
| | | CA | 2921167 | A1 | 06 November 2014 |
| | | DK | 2992009 | T3 | 14 September 2020 |
| | | EA | 201592093 | A1 | 30 June 2016 |
| | | EA | 031393 | B1 | 28 December 2018 |
| | | AU | 2020233603 | A1 | 01 October 2020 |
| | | PL | 2992009 | T3 | 30 November 2020 |
| | | MX | 2015015220 | A | 12 January 2016 |
| | | JP | 2021074021 | A | 20 May 2021 |
| | | AU | 2020217347 | A1 | 27 August 2020 |
| | | HK | 1221485 | A1 | 02 June 2017 |
| | | IL | 284593 | A | 31 August 2021 |
| | | IL | 284593 | B | 01 October 2022 |
| | | IL | 284593 | B2 | 01 February 2023 |
| | | RU | 2015151204 | A | 02 June 2017 |
| | | RU | 2015151204 | A3 | 27 March 2018 |
| | | RU | 2686080 | C2 | 24 April 2019 |
| | | MX | 2015015239 | A | 03 October 2016 |
| | | AU | 2014259750 | A1 | 22 October 2015 |
| | | AU | 2014259750 | B2 | 28 February 2019 |
| | | IL | 242126 | B | 31 January 2019 |
| | | AU | 2019204784 | A1 | 25 July 2019 |
| | | AU | 2019204784 | B2 | 27 January 2022 |
| | | AU | 2019204784 | C1 | 03 November 2022 |
| | | KR | 20200090966 | A | 29 July 2020 |
| | | KR | 20160002977 | A | 08 January 2016 |
| | | KR | 20230113835 | A | 01 August 2023 |
| | | EP | 4155403 | A1 | 29 March 2023 |
| | | RU | 2015151202 | A | 06 June 2017 |
| | | RU | 2015151202 | A3 | 27 March 2018 |
| | | RU | 2670614 | C2 | 24 October 2018 |
| | | RU | 2670614 | C9 | 23 November 2018 |
| | | PT | 2992009 | T | 21 September 2020 |
| | | NZ | 631537 | A | 26 May 2017 |

Form PCT/ISA/210 (patent family annex) (July 2022)

**INTERNATIONAL SEARCH REPORT**
**Information on patent family members**

International application No.
**PCT/CN2023/111121**

| Patent document cited in search report | Publication date (day/month/year) | Patent family member(s) | | | Publication date (day/month/year) |
|---|---|---|---|---|---|
| | | PH | 12018501963 | A1 | 20 July 2020 |
| | | SI | 2992098 | T1 | 28 June 2019 |
| | | AU | 2019202598 | A1 | 02 May 2019 |
| | | DOP | 2015000268 | A | 30 November 2015 |
| | | BR | 112015027322 | A2 | 26 September 2017 |
| | | BR | 112015027322 | A8 | 02 January 2018 |
| | | US | 2015126718 | A1 | 07 May 2015 |
| | | US | 9181549 | B2 | 10 November 2015 |
| | | KR | 20160003723 | A | 11 January 2016 |
| | | KR | 102424855 | B1 | 26 July 2022 |
| | | CR | 20150612 | A | 03 March 2016 |
| | | RU | 2019110030 | A | 06 May 2019 |
| | | RU | 2015151200 | A3 | 14 January 2019 |
| | | RU | 2015151200 | A | 11 September 2019 |
| | | LT | 2992009 | T | 10 November 2020 |
| | | IL | 284000 | A | 29 July 2021 |
| | | CA | 2921509 | A1 | 06 November 2014 |
| | | RS | 58981 | B1 | 30 August 2019 |
| | | KR | 20160002975 | A | 08 January 2016 |
| | | KR | 101857707 | B1 | 14 May 2018 |
| | | US | 11299736 | B1 | 12 April 2022 |
| | | US | 2019055554 | A1 | 21 February 2019 |
| | | HRP | 20190987 | T1 | 20 September 2019 |
| | | MX | 2019010443 | A | 17 October 2019 |
| | | EP | 3546579 | A1 | 02 October 2019 |
| | | DK | 2991656 | T3 | 23 March 2020 |
| | | SG | 10201801813 | YA | 27 April 2018 |
| | | IL | 270464 | B | 29 July 2021 |
| | | US | 2021087566 | A1 | 25 March 2021 |
| | | KR | 20160002974 | A | 08 January 2016 |
| | | KR | 102235678 | B1 | 05 April 2021 |
| | | KR | 20210129257 | A | 27 October 2021 |
| | | KR | 102482890 | B1 | 30 December 2022 |
| | | JP | 2016526018 | A | 01 September 2016 |
| | | JP | 6769866 | B2 | 14 October 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210936491
- CN 202310260531
- CN 202310655563
- CN 103189057 B
- US 9233971 B2
- US 9080186 B2
- CN 102985548 B
- CN 113171371 B

### Non-patent literature cited in the description


- **ASHWELL G** ; **HARFORD J**. Carbohydrate specific Receptors of the Liver. *Ann Rev Biochem*, 1982, vol. 51, 531-554
- **KARLIN** ; **ALTSCHUL**. *PNAS*, 1990, vol. 87, 2264-2268
- **KARLIN** ; **ALTSCHUL**. *PNAS*, 1993, vol. 90, 5873-5877
- **ELBASHIR et al.** *EMBO*, 2001, vol. 20, 6877-6888
- **CHU** ; **RANA**. *RNA*, 2007, vol. 14, 1714-1719
- **KIM et al.** *Nat Biotech*, 2005, vol. 23, 222-226
- Current protocols in nucleic acid chemistry. JohnWiley & Sons, Inc